# EUROPEAN PATENT APPLICATION

(11) **EP 2 163 638 A1**
(43) Date of publication of application: **17.03.2010**
(21) Application number: 08162569.1
(22) Date of filing: 19.12.2005
(51) Int. Cl.: C12N 15/82, C12N 9/02, A01H 5/00

(54) **Nucleic acid molecules encoding fatty acid desaturase genes from plants and methods of use**

(30) Priority: 20.12.2004 US 637531 P
(62) Divisional of application: 05857138.1
(71) Applicant: BASF Plant Science GmbH, 67056 Ludwigshafen (DE)
(72) Inventor: Härtel, Heiko A., 98673 Eisfeld (DE); Gibson, Jermaine, Raleigh, NC 27612 (US); Ren, Peifeng, Cary, NC 27519 (US)
(74) Representative: Popp, Andreas

(57) **Abstract**

This invention relates generally to nucleic acid sequences encoding proteins that are related to the presence of seed storage compounds in plants. More specifically, the present invention relates to *FAD2-*like nucleic acid sequences from Oryza sativa encoding lipid metabolism regulator proteins and the use of these sequences in transgenic plants. In particular, the invention is directed to methods for manipulating lipid metabolism related compounds and for increasing oil level and altering the fatty acid composition in plants and seeds. The invention further relates to methods of using these novel plant polypeptides to stimulate plant growth and/or to increase yield and/or composition of seed storage compounds.

## Description

This application claims priority to U.S. provisional application 60/637531 filed on December 20, 2004, herein incorporated by reference in its entirety.

### FIELD OF THE INVENTION

Described herein are inventions in the field of genetic engineering of plants, including isolated nucleic acid molecules encoding *Fatty Acid Desaturase2*-like (FAD2-like) polypeptides to improve agronomic, horticultural and quality traits. This invention relates generally to nucleic acid sequences encoding proteins that are related to the presence of seed storage compounds in plants. More specifically, the present invention relates to *FAD2*-like nucleic acid sequences encoding lipid metabolism regulator proteins and the use of these sequences in transgenic plants. In particular, the invention is directed to methods for manipulating lipid metabolism related compounds and for increasing oil level and altering the fatty acid composition in plants and seeds. The invention further relates to methods of using these novel plant polypeptides to stimulate plant growth and/or to increase yield and/or composition of seed storage compounds.

### BACKGROUND OF THE INVENTION

The study and genetic manipulation of plants has a long history that began even before the famed studies of Gregor Mendel. In perfecting this science, scientists have accomplished modification of particular traits in plants ranging from potato tubers having increased starch content to oilseed plants such as canola and sunflower having increased or altered fatty acid content. With the increased consumption and use of plant oils, the modification of seed oil content and seed oil levels has become increasingly widespread (e.g. Töpfer et al. 1995, Science 268:681-686). Manipulation of biosynthetic pathways in transgenic plants provides a number of opportunities for molecular biologists and plant biochemists to affect plant metabolism giving rise to the production of specific higher-value products. The seed oil production or composition has been altered in numerous traditional oilseed plants such as soybean (U.S. Patent No. 5,955,650), canola (U.S. Patent No. 5,955,650), sunflower (U.S. Patent No. 6,084,164) and rapeseed (Töpfer et al. 1995, Science 268:681-686), and non-traditional oil seed plants such as tobacco (Cahoon et al. 1992, Proc. Natl. Acad. Sci. USA 89:11184-11188).

Plant seed oils comprise both neutral and polar lipids (see Table 1). The neutral lipids contain primarily triacylglycerol, which is the main storage lipid that accumulates in oil bodies in seeds. The polar lipids are mainly found in the various membranes of the seed cells, e.g. the endoplasmic reticulum, microsomal membranes and the cell membrane. The neutral and polar lipids contain several common fatty acids (see Table 2) and a range of less common fatty acids. The fatty acid composition of membrane lipids is highly regulated and only a select number of fatty acids are found in membrane lipids. On the other hand, a large number of unusual fatty acids can be incorporated into the neutral storage lipids in seeds of many plant species (Van de Loo F.J. et al. 1993, Unusual Fatty Acids in Lipid Metabolism in Plants pp. 91-126, editor TS Moore Jr. CRC Press; Millar et al. 2000, Trends Plant Sci. 5:95-101).

Lipids are synthesized from fatty acids and their synthesis may be divided into two parts: the prokaryotic pathway and the eukaryotic pathway (Browse et al. 1986, Biochemical J. 235:25-31; Ohlrogge & Browse 1995, Plant Cell 7:957-970). The prokaryotic pathway is located in plastids that are the primary site of fatty acid biosynthesis. Fatty acid synthesis begins with the conversion of acetyl-CoA to malonyl-CoA by acetyl-CoA carboxylase (ACCase). Malonyl-CoA is converted to malonyl-acyl carrier protein (ACP) by the malonyl-CoA:ACP transacylase. The enzyme beta-keto-acyl-ACP-synthase III (KAS III) catalyzes a condensation reaction, in which the acyl group from acetyl-CoA is transferred to malonyl-ACP to form 3-ketobutyryl-ACP. In a subsequent series of condensation, reduction and dehydration reactions the nascent fatty acid chain on the ACP cofactor is elongated by the step-by-step addition (condensation) of two carbon atoms donated by malonyl-ACP until a 16- or 18-carbon saturated fatty acid chain is formed. The plastidial delta-9 acyl-ACP desaturase introduces the first unsaturated double bond into the fatty acid. Thioesterases cleave the fatty acids from the ACP cofactor and free fatty acids are exported to the cytoplasm where they participate as fatty acyl-CoA esters in the eukaryotic pathway. In this pathway the fatty acids are esterified by glycerol-3-phosphate acyltransferase and lysophosphatidic acid acyl-transferase to the sn-1 and sn-2 positions of glycerol-3-phosphate, respectively, to yield phosphatidic acid (PA). The PA is the precursor for other polar and neutral lipids, the latter being formed in the Kennedy pathway (Voelker 1996, Genetic Engineering ed.: Setlow 18:111-113; Shanklin & Cahoon 1998, Annu. Rev. Plant Physiol. Plant Mol. Biol. 49:611-641; Frentzen 1998, Lipids 100:161-166; Millar et al. 2000, Trends Plant Sci. 5:95-101).

Storage lipids in seeds are synthesized from carbohydrate-derived precursors. Plants have a complete glycolytic pathway in the cytosol (Plaxton 1996, Annu. Rev. Plant Physiol. Plant Mol. Biol. 47:185-214) and it has been shown that a complete pathway also exists in the plastids of rape-seeds (Kang & Rawsthorne 1994, Plant J. 6:795-805). Sucrose is the primary source of carbon and energy, transported from the leaves into the developing seeds. During the storage phase of seeds, sucrose is converted in the cytosol to provide the metabolic precursors glucose-6-phosphate and pyruvate. These are transported into the plastids and converted into acetyl-CoA that serves as the primary precursor for the synthesis of fatty acids. Acetyl-CoA in the plastids is the central precursor for lipid biosynthesis. Acetyl-CoA can be formed in the plastids by different reactions and the exact contribution of each reaction is still being debated (Ohlrogge & Browse 1995, Plant Cell 7:957-970). It is however accepted that a large part of the acetyl-CoA is derived from glucose-6-phospate and pyruvate that are imported from the cytoplasm into the plastids. Sucrose is produced in the source organs (leaves, or anywhere that photosynthesis occurs) and is transported to the developing seeds that are also termed sink organs. In the developing seeds, sucrose is the precursor for all the storage compounds, i.e. starch, lipids and partly the seed storage proteins. Therefore, it is clear that carbohydrate metabolism, in which sucrose plays a central role is very important to the accumulation of seed storage compounds.

Storage compounds such as triacylglycerols (seed oil) serve as carbon and energy reserves, which are used during germination and growth of the young seedling. Seed (vegetable) oil is also an essential component of the human diet and a valuable commodity providing feed stocks for the chemical industry.

. Although the lipid and fatty acid content and/or composition of seed oil can be modified by the traditional methods of plant breeding, the advent of recombinant DNA technology has allowed for easier manipulation of the seed oil content of a plant, and in some cases, has allowed for the alteration of seed oils in ways that could not be accomplished by breeding alone (see, e.g., Töpfer et al., 1995, Science 268:681-686). For example, introduction of a □¹²-hydroxylase nucleic acid sequence into transgenic tobacco resulted in the introduction of a novel fatty acid, ricinoleic acid, into the tobacco seed oil (Van de Loo et al. 1995, Proc. Natl. Acad. Sci USA 92:6743-6747). Tobacco plants have also been engineered to produce low levels of petroselinic acid by the introduction and expression of an acyl-ACP desaturase from coriander (Cahoon et al. 1992, Proc. Natl. Acad. Sci USA 89:11184-11188).

The modification of seed oil content in plants has significant medical, nutritional and economic ramifications. With regard to the medical ramifications, the long chain fatty acids (C18 and longer) found in many seed oils have been linked to reductions in hypercholesterolemia and other clinical disorders related to coronary heart disease (Brenner 1976, Adv. Exp. Med. Biol. 83:85-101). Therefore, consumption of a plant having increased levels of these types of fatty acids may reduce the risk of heart disease. Enhanced levels of seed oil content also increase large-scale production of seed oils and thereby reduce the cost of these oils.

In order to increase or alter the levels of compounds such as seed oils in plants, nucleic acid sequences and proteins regulating lipid and fatty acid metabolism must be identified. As mentioned earlier, several desaturase nucleic acids such as the Δ⁶-desaturase nucleic acid, Δ¹²-desaturase nucleic acid and acyl-ACP desaturase nucleic acid have been cloned and demonstrated to encode enzymes required for fatty acid synthesis in various plant species (Miquel & Browse, in Seed Development and Germination, Galili et al. (eds.), Marcel Dekker, New York, pp. 169-193, 1994; Ohlrogge & Browse 1995, Plant Cell 7:957-970). Oleosin nucleic acid sequences from such different species as canola, soybean, carrot, pine and *Arabidopsis thaliana* have also been cloned and determined to encode proteins associated with the phospholipid monolayer membrane of oil bodies in those plants.

Although several compounds are known that generally affect plant and seed development, there is a clear need to specifically identify factors that are more specific for the developmental regulation of storage compound accumulation and to identify genes which have the capacity to confer altered or increased oil production to its host plant and to other plant species.

Another problem underlying the present invention was to provide a more efficient way of silencing fatty acid desatures. Another problem underlying the present invention was to specifically modify the fatty acid content of oil seeds. Another problem underlying the present invention was the increase of the oleic acid content of oil seeds. Another problem underlying the present invention was the decrease of the linolic acid content of oil seeds.

This invention discloses nucleic acid sequences from *Arabidopsis thaliana, Glycine max, Oryza sativa,* Zea *mays, Linum usitatissimum, Hordeum vulgare* or *Triticum aestivum.* These nucleic acid sequences can be used to alter or increase the levels of seed storage compounds such as proteins, sugars and oils, in plants, including transgenic plants, such as canola, linseed, soybean, sunflower, maize, oat, rye, barley, wheat, rice, pepper, tagetes, cotton, oil palm, coconut palm, flax, castor and peanut, which are oilseed plants containing high amounts of lipid compounds.

### BRIEF DESCRIPTION OF THE INVENTION

The present invention provides novel isolated nucleic acid and amino acid sequences associated with the metabolism of seed storage compounds in plants, in particular with sequences that are FAD2-like.
Another subject of the present invention is an isolated polypeptide comprising an amino acid sequence selected from the group consisting of
a. X¹X²X³X⁴X⁵X⁶X⁷LX⁸X⁹PX¹⁰YL, whereas X¹ is not M and X³ is not T and X⁶X⁷ are not FV,
b. GX¹¹X¹²X¹³X¹⁴X¹⁵X¹⁶X¹⁷X¹⁸HX¹⁹X²⁰PX²¹X²²X²³X²⁴X²⁵X²⁶X²⁷X²⁸ER, whereas X¹⁵ is not G and whereas X²⁰ is not F and whereas X²¹X²² are not NA,
c. HX²⁹X³⁰PX³¹X³²X³³X³⁴X³⁵X³⁶X³⁷X³⁸ER, whereas X³⁰ is not F and whereas X³¹X³² are not NA,
d. LX³⁹X⁴⁰X⁴¹X⁴²X⁴³X⁴⁴X⁴⁵GX⁴⁶X⁴⁷X⁴⁸X⁴⁹X⁵⁰X⁵¹X⁵²YX⁵³X⁵⁴P, whereas X⁴¹ is not Y and X⁴⁵ is not Q and X⁴⁸ is not S and X⁴⁹ is not M and X⁵⁰ is not I,
e. TX⁵⁵X⁵⁶X⁵⁷X⁵⁸HX⁵⁹X⁶⁰X⁶¹X⁶²X⁶³X⁶⁴X⁶⁵X⁶⁶X⁶⁷X⁶⁸X⁶⁹X⁷⁰X⁷¹T, whereas X⁶⁷ is not N,
f. PX⁷²X⁷³X⁷⁴X⁷⁵X⁷⁶X⁷⁷X⁷⁸X⁷⁹X⁸⁰X⁸¹X⁸²X⁸³X⁸⁴X⁸⁵X⁸⁶, whereas X⁸⁴X⁸⁵X⁸⁶ are not WYV,
and whereas X has the meaning of any amino acid if not defined elsewhise above.

A sequence alignment for determining the common peptide sequences a to f of claim 1 is preferably generated using using using Align X (Aug. 22, 2003) of Vector NTI Suite 9.0. The parameters used for the multiple alignment are preferably as follows: Gap opening penalty: 10; Gap extension penalty: 0.05; Gap separation penalty range: 8; % identity for alignment delay: 40.

In a preferred embodiment the present inventions claims an isolated polypeptide comprising an amino acid sequence selected from the group consisting of
a. AWYPYX⁸⁷YX⁸⁸NPX⁸⁹GRLVHIX⁹⁰VOLTLGWPLYLAX⁹¹NX⁹² SGRPYPRFACHFDPYGPIYNDRER,
b. FISDVGV,
c. ALX⁹³KLX⁹⁴SX⁹⁵FGFWWVVRVYGVP,
d. ILGEYYQFDX⁹⁶TPVAKAT,
e. and whereas X has the meaning of any amino acid.
A sequence alignment for determining the common peptide sequences a to d of claim 2 is preferably generated using using Align X (Aug. 22, 2003) of Vector NTI Suite 9.0. The parameters used for the multiple alignment are preferably as follows: Gap opening penalty: 10; Gap extension penalty: 0.05; Gap separation penalty range: 8; % identity for alignment delay: 40.

The isolated polypeptide of the present invention can include one, two, three, four, five or six of the amino acid sequences of claim 1. The isolated polypeptide of the present invention can include one, tow, three or four of the amino acid sequences of claim 2. X stands for any amino acid if not defined elsewhise in claim 1, especially G, A, V, L, I, F, Y, W, P, D, E, N, Q, S, T, C, M, K, R, H.

X¹ is not M. X¹ is in a preferred embodiment an amino acid selected from the group consisting of G, A, V, L, I, F, Y, W, P, D, E, N, Q, S, T, C, K, R and H, in a more preferred embodiment from the group consisting of F, T and H and in an even more preferred embodiment H.

X³ is not T. X³ is in a preferred embodiment an amino acid selected from the group consisting of G, A, V, L, I, F, Y, W, P, D, E, N, Q, S, C, M, K, R and H, in a more preferred embodiment from the group consisting of L, A, V, F and in an even more preferred emobidement V or F.

X⁶ is not Fand X⁷ is not V. X⁶ and X⁷ are in a preferred embodiment independently from each other an amino acid selected from the group consisting of G, A, L, I, Y, W, P, D, E, N, Q, S, T, C, M, K, R and H, in a more preferred embodiment from the group consisting of P, L, and T and in an even more preferred embodiment X⁶ is P or L and X⁷ is L or T and in an further preferred embodiment X⁶ is L and X⁷ is T.

X¹⁵ is not G. X¹⁵ is in a preferred embodiment a blank or an amino acid selected from the group consisting of A, V, L, I, F, Y, W, P, D, E, N, Q, S, T, C, M, K, R and H, in a more preferred embodiment X¹⁵ is R or a blank, further preferred X¹⁵ is R. Blank means there is no amino acid on this position.

X²⁰ is not F. X²⁰ is in a preferred embodiment an amino acid selected from the group consisting of G, A, V, L, I, Y, W, P, D, E, N, Q, S, T, C, M, K, R and H, in a more preferred embodiment N or D and in an even more preferred embodiment D.

X²¹ is not N and X²² is not A. X²¹ and X²² are in a preferred embodiment independently from each other an amino acid selected from the group consisting of G, V, L, I, F, Y, W, P, D, E, Q, S, T, C, M, K, R and H. In a more preferred embodiment from the group consisting of D, Y, H, S, G. I in an even more preferred embodiment X²¹ is D, Y or H, further preferred Y and X²² is S or G, further preferred G.

X³⁰ is not F. X³⁰ is in a preferred embodiment an amino acid selected from the group consisting of G, A, V, L, I, Y, W, P, D, E, N, Q, S, T, C, M, K, R and H, in a more preferred embodiment X³⁰ is N or D and in an even more preferred embodiment X³⁰ is D.

X³¹ is not N and X³² is not A. X³¹ and X³² are in a preferred embodiment independently from each other an amino acid selected from the group consisting of G, V, L, I, F, Y, W, P, D, E, Q, S, T, C, M, K, R and H, in a more preferred embodiment from the group consisting of D, Y, H, S, G. In an even more preferred embodiment X³¹ is D, Y or H, further preferred Y and X³² is S or G, further preferred G.

X⁴¹ is not Y. X⁴¹ is in a preferred embodiment an amino acid selected from the group consisting of G, A, V, L, I, F, W, P, D, E, N, Q, S, T, C, M, K, R and H, in a more preferred embodiment X⁴¹ is L.

X⁴⁵ is not Q. X⁴⁵ is in a preferred embodiment an amino acid selected from the group consisting of G, A, V, L, I, F, Y, W, P, D, E, N, S, T, C, M, K, R and H, in a more preferred embodiment from the group consisting of M, K and F and in an even more preferred embodiment X⁴⁵ is F.

X⁴⁸ is not S and X⁴⁹ is not M and X⁵⁰ is not I. X⁴⁸, X⁴⁹ and X⁵⁰ are independently from each other in a preferred embodiment an amino acid selected from the group consisting of G, A, V, L, F, Y, W, P, D, E, N, Q, T, C, K, R and H, in a more preferred embodiment from the group consisting of Q, W, L and V. In an even more preferred embodiment X⁴⁸ is Q or W, further preferred X⁴⁸ is W and X⁴⁹, X⁵⁰ are independently from each other L or V, further preferred X⁴⁹, X⁵⁰ are independently from each other V.

X⁶⁷ is not N. X⁶⁷ is in a preferred embodiment an amino acid selected from the group consisting of G, A, V, L, I, F, Y, W, P, D, E, Q, S, T, C, M, K, R and H, in a more preferred embodiment X⁶⁷ is H or R and in an even more preferred embodiment X⁶⁷ is H.

X⁸⁴ is not W and X⁸⁵ is not Y and X⁸⁶ is not V. X⁸⁴, X⁸⁵ and X⁸⁶ are in a preferred embodiment independently from each other an amino acid selected from the group consisting of G, A, L, I, F, P, D, E, N, Q, S, T, C, M, K, R and H. X⁸⁴X⁸⁵X⁸⁶ are in a more preferred embodiment independently from each other selected from the group consisting of S, F, V, P, M, A, L, K and G and in an even more preferred embodiment X⁸⁴X⁸⁵X⁸⁶ are VAK.

This invention also provides an isolated nucleic acid sequence encoding a protein containing an amino acid sequence mentioned above as (of claim 1 or of claim 2) and an isolated polypeptide encoded by such a nucleic acid sequence (of claim 3).

In another embodiment of the present invention the above mentioned isolated polypeptide (of claim 1 or of claim 2) functions as a modulator of a seed storage compound in microorganisms or in plants.

In another embodiment of the present invention the above mentioned isolated polypeptide (of claim 1 or of claim 2) is used to increase the level of a oleic acid in a transgenic plant as compared to the wild type variety of the plant, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more.

In a preferred embodiment the above mentioned isolated polypeptide (of claim 1 or of claim 2) has a polypeptide sequence as disclosed in SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO:32, SEQ ID NO: 34 or SEQ ID NO: 36.

In a further embodiment the above mentioned isolated polypeptide (of claim 1 or of claim 2) is selected from the group consisting of
a. a polypeptide sequence as disclosed in SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO:32, SEQ ID NO: 34 or SEQ ID NO: 36
b. a polypeptide sequence encoded by a polynucleotide sequence as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35;
c. a polypeptide sequence having at least 70% sequence identity with the polypeptide sequence of a) or b) above.

The present invention provides moreover an isolated nucleic acid comprising a polynucleotide sequence selected from the group consisting of:
a. a polynucleotide sequence as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35;
b. a polynucleotide sequence encoding a polypeptide as disclosed in SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO:32, SEQ ID NO: 34 or SEQ ID NO: 36;
c. a polynucleotide sequence having at least 70% sequence identity with the nucleic acid of a) or b)above;
d. a polynucleotide sequence that is complementary to the nucleic acid of a) or b) above; and
e. a polynucleotide sequence that hybridizes under stringent conditions to the nucleic acid of a) or b) above.

The present invention provides furthermore an isolated polypeptide selected from the group consisting of
a. a polypeptide sequence encoded by a polynucleotide sequence as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35;
b. a polypeptide sequence as disclosed in SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO:32, SEQ ID NO: 34 or SEQ ID NO: 36;
c. a polypeptide sequence having at least 70% sequence identity with the polypeptide sequence of a) or b) above.

The present invention also provides an isolated nucleic acid from *Arabidopsis thaliana, Glycine max, Oryza sativa, Zea mays, Linum usitatissimum, Hordeum vulgare* or *Triticum aestivum* encoding a Lipid Metabolism Protein (LMP), or a portion thereof. These sequences may be used to modify or increase lipids and fatty acids, cofactors and enzymes in microorganisms and plants, e.g. by the increasing of the level of oleic acid by 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more..

Arabidopsis plants are known to produce considerable amounts of fatty acids like linoleic and linolenic acid (see, e.g., Table 2) and for their close similarity in many aspects (gene homology etc:) to the oil crop plant Brassica. Therefore, nucleic acid molecules originating from a plant like *Arabidopsis thaliana, Brassica napus, Glycine max, Oryza sativa, Zea mays, Linum usitatissimum, Hordeum vulgare* or *Triticum aestivum* or related organisms are especially suited to modify the lipid and fatty acid metabolism in a host, especially in microorganisms and plants. Furthermore, nucleic acids from the plant *Arabidopsis thaliana, Brassica napus, Glycine max, Oryza sativa,* Zea *mays, Linum usitatissimum, Hordeum vulgare* or *Triticum aestivum* or related organisms can be used to identify those DNA sequences and enzymes in other species, which are useful to modify the biosynthesis of precursor molecules of fatty acids in the respective organisms.

The present invention further provides an isolated nucleic acid comprising a fragment of at least 15 nucleotides of a nucleic acid from a plant *(Arabidopsis thaliana, Glycine max, Oryza sativa, Zea mays, Linum usitatissimum, Hordeum vulgare* or *Triticum aestivum)* encoding a LMP, or a portion thereof.

Also provided by the present invention are polypeptides encoded by the nucleic acids, and heterologous polypeptides comprising polypeptides encoded by the nucleic acids, and antibodies to those polypeptides.

Additionally, the present invention relates to and provides the use of LMP nucleic acids in the production of transgenic plants having a modified level or composition of a seed storage compound. In regard to an altered composition, the present invention can be used to, for example, increase the percentage of oleic acid relative to other plant oils, e.g. linolic acid or linoleic acid, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more. A method of producing a transgenic plant with a modified level or composition of a seed storage compound includes the steps of transforming a plant cell with an expression vector comprising a LMP nucleic acid, and generating a plant with a modified level or composition of the seed storage compound from the plant cell. In a preferred embodiment, the plant is an oil producing species selected from the group consisting of canola, linseed, soybean, sunflower, maize, oat, rye, barley, wheat, rice, pepper, tagetes, cotton, oil palm, coconut palm, flax, castor and peanut, for example.

According to the present invention, the compositions and methods described herein can be used to alter the composition of a LMP in a transgenic plant and to increase or decrease the level of a LMP in a transgenic plant comprising increasing or decreasing the expression of a LMP nucleic acid in the plant. Increased or decreased expression of the LMP nucleic acid can be achieved through transgenic overexpression, cosuppression approaches, antisense approaches and in vivo mutagenesis of the LMP nucleic acid. The present invention can also be used to increase or decrease the level of a lipid in a seed oil, by 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more, to increase or decrease the level of a fatty acid in a seed oil, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more, or to increase or decrease the level of a starch in a seed or plant, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more..

MicroRNAs (miRNAs) have emerged as evolutionarily conserved, RNA-based regulators of gene expression in plants and animals. MiRNAs (∼ 21 to 25 nt) arise from larger precursors with a stem loop structure that are transcribed from non-protein-coding genes. miRNA targets a specific mRNA to suppress gene expression at post-transcriptional (*i.e*. degrades mRNA) or translational levels (*i.e*. inhibits protein synthesis) (Bartel D 2004, Cell 116, 281-297).

MiRNA precursor (pre-miRNA) can be engineered in such way that endogenous miRNA encoded by pre-miRNA is replaced by a miRNA to target a gene-of-interest, e.g. dsRed reporter gene.

The present inventions provides furthermore a method of producing a transgenic plant having an increased level of oleic acid compared to the wildtype comprising,
a. a first step of transforming a plant cell with an RNA precursor construct, and
b. a second step of generating from the plant cell the transgenic plant,
wherein said construct contains a promoter that drives expression in a plant cell operably linked to a nucleotide sequence encoding a precursor micro RNA sequence, wherein the nucleotide sequence encoding said micro RNA precurser sequence is selected from the group consisting of
a. a nucleotide sequence as depicted in SEQ ID NO: 47
b. a polynucleotide sequence having at least 70% sequence identity with the nucleic acid of a) above;
c. a polynucleotide sequence that is complementary to the nucleic acid of a) above; and
d. a polynucleotide sequence that hybridizes under stringent conditions
to the nucleic acid of a) above.

Maize genes coding for fatty acid desaturases, are expressed in many tissues including seeds. A 19 to 21nt (e.g. ACCAGACCCCGAACGCCGC as described in SEQ ID NO: 40) complimentary to a maize desaturase coding region or 5' UTR and 3'UTR in mRNA can be used to replace Zm miR166 (5' tcggaccaggcttcattcccc 3') as described in SEQ ID NO: 37 and in SEQ ID NO: 38 in Zm miR166 precursor. The transgene can then be transformed into maize. The expression of the engineered Zm miR166 gene can be controlled by a maize seed-specific promoter (e.g. endosperm specific 10 KD Zein promoter or Glob1 embryo-specific promoter).

A microRNA (e.g. ACCAGACCCCGAACGCCGC as described in SEQ ID NO: 40) is generally generated in seeds when the engineered Zm miR166 precursor is processed. This miRNA specifically can bind to the region in a maize fatty acid desaturase mRNA complimentary to the miRNA, which can result in a reduction of this targeted maize desaturase expression at transcriptional or translational levels in seeds by gene silencing machinery. As a result, transgenic plant, preferably zea mays could have desirable fatty acid level and composition as for example low linolenic acid and/or medium or high oleic acid weight percentages in seeds.

The present inventions provides furthermore a method to alter, preferably to reduce the expression of fatty acid desaturase, especially as encoded by FAD2 orthologs, further preferred as encoded by the nucleic acids as depicted in Appendix A, in a preferred emobidement as depicted in SEQ ID NO: 5, SEQ ID NO: 7, SEQ ID NO: 9, SEQ ID NO: 11, SEQ ID NO: 13, SEQ ID NO: 15, SEQ ID NO:17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35 by producing a transgenic plant having an increased level of oleic acid compared to the wildtype comprising,
a. a first step of transforming a plant cell with an RNA precursor construct, and
b. a second step of generating from the plant cell the transgenic plant,
said construct containing a promoter that drives expression in a plant cell operably linked to a nucleotide sequence encoding a precursor micro RNA sequence, wherein the nucleotide sequence encoding said micro RNA precurser sequence is selected from the group consisting of
a. a nucleotide sequence as depicted in SEQ ID NO: 47
b. a polynucleotide sequence having at least 70% sequence identity with the nucleic acid of a) above;
c. a polynucleotide sequence that is complementary to the nucleic acid of a) above; and
d. a polynucleotide sequence that hybridizes under stringent conditions to the nucleic acid of a) above.

In a preferred embodiment the nucleotide sequence encoding a precursor micro RNA sequence has been engineered in a way that the nucleotide sequence encoding for a micro RNA as depicted in SEQ ID NO: 37 is replaced by a nucleotide sequence encoding for a micro RNA as depicted in SEQ ID NO:40.

The use of engineered micro RNA precursers and micro-RNA for modulating the expression of a gene is well known and described e.g. in US 2004/0268441, which is incorporated herein in its entirety. Engineered micro RNA precursers can be used to modulate the expression of one or of several target genes, e.g. one, two, three , four or five of the nucleotide sequences as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35. The use of engineered micro-RNA precursers and micro-RNA for modulating the expression of a gene can be combined with other methods of genetic engineering well known to the man skilled in the art.

The promoter can be ubiquitous or tissue-specific such as seed-specific and endosperm-specific. The promoter is preferably a seed specific promoter. This method can be used to efficiently increase the level of oleic acid in a seed, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more.

The use of engineered micro-RNA precursers and micro-RNA for modulating the expression of a gene can be applied to every plant, especially to the plants described herein, in a preferred embodiment to monocotyledonous plants and in a more preferred embodiment to zea mays.

A further object of the present invention is an isolated nucleic acid comprising a polynucleotide sequence selected from the group consisting of:
a. a nucleotide sequence as depicted in SEQ ID NO: 47
b. a polynucleotide sequence having at least 70% sequence identity with the nucleic acid of a) above;
c. a polynucleotide sequence that is complementary to the nucleic acid of a) above; and
e. a polynucleotide sequence that hybridizes under stringent conditions to the nucleic acid of a) above.
This nucleotide sequence can be used to modulate the expression of a gene of interest, especially to down-regulate the expression of a target gene, especially of the above mentioned nucleotide sequences.

A further object of the present invention is the micro RNA precursor encoded by a nucleotide sequence selected from the groups consisting of:
a. a nucleotide sequence as depicted in SEQ ID NO: 47
b. a polynucleotide sequence having at least 70% sequence identity with the nucleic acid of a) above;
c. a polynucleotide sequence that is complementary to the nucleic acid of a) above; and
d. a polynucleotide sequence that hybridizes under stringent conditions to the nucleic acid of a) above.

A further object of the present invention is the micro RNA as depicted in SEQ ID NO: 40.

More specifically, the present invention includes and provides a method for increasing total oil content in a seeds comprising: transforming a plant with a nucleic acid construct that comprises as operably linked components, a promoter and nucleic acid sequences capable of modulating the level of *FAD2*-like mRNA or FAD2-like protein, and growing the plant. Furthermore, the present invention includes and provides a method for increasing the level of oleic acid in a seed comprising: transforming a plant with a nucleic acid construct that comprises as operably linked components, a promoter, a structural nucleic acid sequence capable of increasing the level of oleic acid, and growing the plant

Also included herein is a seed produced by a transgenic plant transformed by a LMP DNA sequence, wherein the seed contains the LMP DNA sequence and wherein the plant is true breeding for a modified level of a seed storage compound. The present invention additionally includes a seed oil produced by the aforementioned seed.

Further provided by the present invention are vectors comprising the nucleic acids, host cells containing the vectors, and descendent plant materials produced by transforming a plant cell with the nucleic acids and/or vectors.

According to the present invention, the compounds, compositions, and methods described herein can be used to increase or decrease the relative percentages of a lipid in a seed oil, increase or decrease the level of a lipid in a seed oil, or to increase or decrease the level of a fatty acid in a seed oil, or to increase or decrease the level of a starch or other carbohydrate in a seed or plant, or to increase or decrease the level of proteins in a seed or plant, by e.g. 1 weight-%, 2,5 weight-%, 5 weight-%, 7,5 weight-%, 10 weight-%, 12,5 weight-%, 15 weight-%, 17,5 weight-%, 20 weight-%, 22,5 weight-%, 25 weight-% or more. The manipulations described herein can also be used to improve seed germination and growth of the young seedlings and plants and to enhance plant yield of seed storage compounds.

It is further provided a method of producing a higher or lower than normal or typical level of storage compound in a transgenic plant expressing a LMP nucleic acid from *Arabidopsis thaliana, Brassica napus, Glycine max, Oryza sativa,* Zea *mays, Linum usitatissimum, Hordeum vulgare* or *Triticum aestivum* in the transgenic plant, wherein the transgenic plant is *Arabidopsis thaliana, Brassica napus, Glycine max, Oryza sativa, Zea mays, Linum usitatissimum, Hordeum vulgare, Triticum aestivum, Helianthus anuus* or Beta *vulgaris* or a species different from *Arabidopsis thaliana, Brassica napus, Glycine max, Zea mays, Linum usitatissimum, Hordeum vulgare, Oryza sativa* or *Triticum aestivum.* Also included herein are compositions and methods of the modification of the efficiency of production of a seed storage compound. As used herein, where the phrase *Arabidopsis thaliana, Brassica napus, Glycine max, Oryza sativa,* Zea *mays, Linum usitatissimum, Hordeum vulgare, Triticum aestivum, Helianthus anuus* or *Beta vulgaris* is used, this also means *Arabidopsis thaliana* and/or *Brassica napus and*/*or Glycine max* and/or *Oryza sativa* and/or Zea *mays* and/or *Linum usitatissimum* and/or *Hordeum vulgare* and/or *Triticum aestivum* and/or *Helianthus anuus* and/or *Beta vulgaris.*

Accordingly, it is an object of the present invention to provide novel isolated LMP nucleic acids and isolated LMP amino acid sequences from *Glycine max, Oryza sativa, Zea mays, Linum usitatissimum, Hordeum vulgare* or *Triticum aestivum* as well as active fragments, analogs, and orthologs thereof. Those active fragments, analogs, and orthologs can also be from different plant species as one skilled in the art will appreciate that other plant species will also contain those or related nucleic acids.

It is another object of the present invention to provide transgenic plants having modified levels of seed storage compounds, and in particular, modified levels of a lipid, a fatty acid or a sugar.

The polynucleotides and polypeptides of the present invention, including agonists and/or fragments thereof, have also uses that include modulating plant growth, and potentially plant yield, preferably increasing plant growth under adverse conditions (drought, cold, light, UV). In addition, antagonists of the present invention may have uses that include modulating plant growth and/or yield, through preferably increasing plant growth and yield. In yet another embodiment, overexpression polypeptides of the present invention using a constitutive promoter may be useful for increasing plant yield under stress conditions (drought, light, cold, UV) by modulating light utilization efficiency. Moreover, polynucleotides and polypeptides of the present invention will improve seed germination and seed dormancy and, hence, will improve plant growth and/or yield of seed storage compounds.

The isolated nucleic acid molecules of the present invention may further comprise an operably linked promoter or partial promoter region. The promoter can be a constitutive promoter, an inducible promoter or a tissue-specific promoter. The constitutive promoter can be, for example, the superpromoter (Ni et al., Plant J. 7:661-676, 1995; US5955646). The tissue-specific promoter can be active in vegetative tissue or reproductive tissue. The tissue-specific promoter active in reproductive tissue can be a seed-specific promoter. The tissue-specific promoter active in vegetative tissue can be a root-specific, shoot-specific, meristem-specific or leaf-specific promoter. The isolated nucleic acid molecule of the present invention can still further comprise a 5' non-translated sequence, 3' non-translated sequence, introns, or the combination thereof.

The present invention also provides a method for increasing the number and/or size of one or more plant organs of a plant expressing an isolated nucleic acid from *Arabidopsis thaliana, Brassica napus, Glycine max, Oryza sativa, Zea mays, Linum usitatissimum, Hordeum vulgare* or *Triticum aestivum* encoding a LMP, or a portion thereof. More specifically, seed size and/or seed number and/or weight might be manipulated.

It is a further object of the present invention to provide methods for producing such aforementioned transgenic plants.

It is another object of the present invention to provide seeds and seed oils from such aforementioned transgenic plants.

These and other objects, features and advantages of the present invention will become apparent after a review of the following detailed description of the disclosed embodiments and the appended claims.

### BRIEF DESCRIPTION OF THE DRAWINGS

The invention can be more fully understood from the following detailed description and the accompanying drawings and sequence listing which form a part of this application.

Figures 1A-D. SEQ ID NO: 1-4 - Nucleic acid sequence, open reading frame of the nucleic acid and amino acid sequences of the *Arabidopsis thaliana* gene *AtFAD-01.*

Figures 2A-C. SEQ ID NO: 5-8 - Nucleic acid sequence, open reading frame of the nucleic acid and amino acid sequences of the *Glycine max* gene *GmFAD-01.*

Figures 3A-C. SEQ ID NO: 9-12 - Nucleic acid sequence, open reading frame of the nucleic acid and amino acid sequence of the *Glycine max* gene *GmFAD-02.*

Figures 4A-C. SEQ ID NO: 13-16 - Nucleic acid sequence, open reading frame of the nucleic acid and amino acid sequence of the *Glycine max* gene *GmFAD-03.*

Figures 5A-C. SEQ ID NO: 17-20 - Nucleic acid sequence, open reading frame of the nucleic acid and amino acid sequence of the *Zea mays* gene *ZmFAD-01.*

Figures 6A-C. SEQ ID NO: 21-24 - Nucleic acid sequence, open reading frame of the nucleic acid and amino acid sequence of the *Oryza sativa* gene *OsFAD-01.*

Figures 7A-C. SEQ ID NO: 25-28 - Nucleic acid sequence, open reading frame of the nucleic acid and amino acid sequence of the *Linum usitatissimum* gene *LuFAD-01.*

Figures 8A-C. SEQ ID NO: 29-32 - Nucleic acid sequence, open reading frame of the nucleic acid and amino acid sequence of the *Hordeum vulgare* gene *HvFAD-01.*

Figures 9A-C. SEQ ID NO: 33-36 - Nucleic acid sequence, open reading frame of the nucleic acid and amino acid sequence of the *Tritium aestivum* gene *TaFAD-01.*

Figure 10. T2 seed fatty acid data obtained with OsFAD-01 driven by the USP promoter and transformed into the *fad2 Arabidopsis* mutant (the genetic background of the transformed lines is Columbia-2, each bar represents the fatty acid data obtained with 5 mg bulked seeds of one individual plant).

Figure 11. T2 seed fatty acid data obtained with HvFAD-01 driven by the USP promoter and transformed into the *fad2 Arabidopsis* mutant (the genetic background of the transformed lines is Columbia-2, each bar represents the fatty acid data obtained with 5 mg bulked seeds of one individual plant).

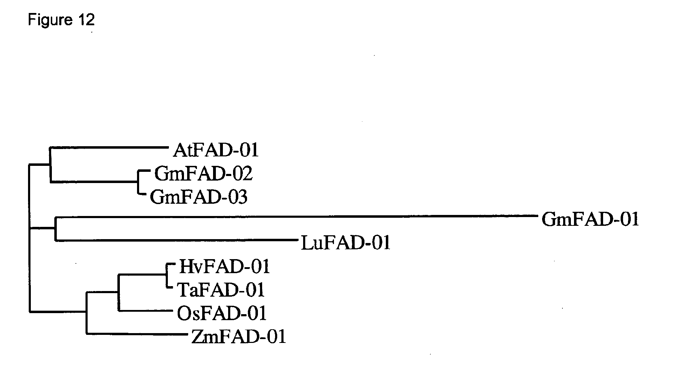

Figure 12. Diagram illustrating the relative homology among the disclosed AtFAD-01, GmFAD-01, GmFAD-02, GmFAD-03, LuFAD-01, HvFAD-01, TaFAD-01, OsFAD-01 and ZmFAD-01 amino acid sequences. The diagram was generated using Align X (Aug. 22, 2003) of Vector NTI Suite 9.0. The parameters used for the multiple alignment were as follows: Gap opening penalty: 10; Gap extension penalty: 0.05; Gap separation penalty range: 8; % identity for alignment delay: 40

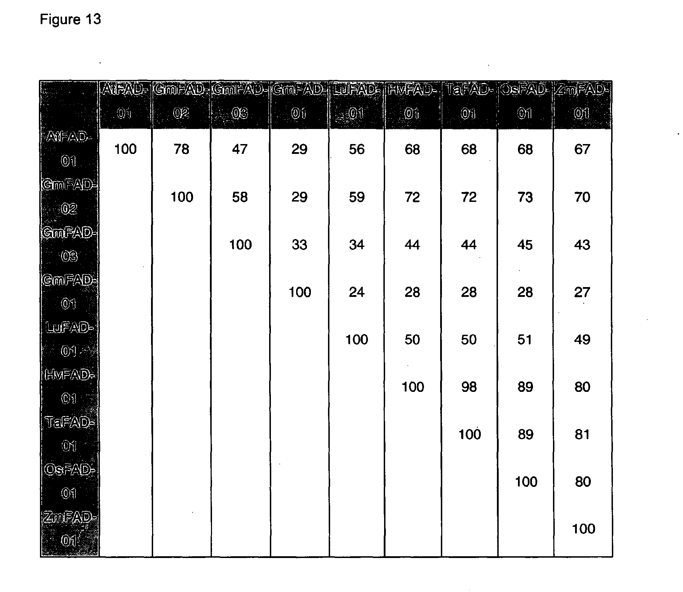

Figure 13. Table illustrating the similarity among the AtFAD-01, GmFAD-01, GmFAD-02, GmFAD-03, LuFAD-01, HvFAD-01, TaFAD-01, OsFAD-01 and ZmFAD-01 amino acid sequences. The table was generated using Align X (Aug. 22, 2003) of Vector NTI Suite 9.0. For other parameters see legend of Figure 12.

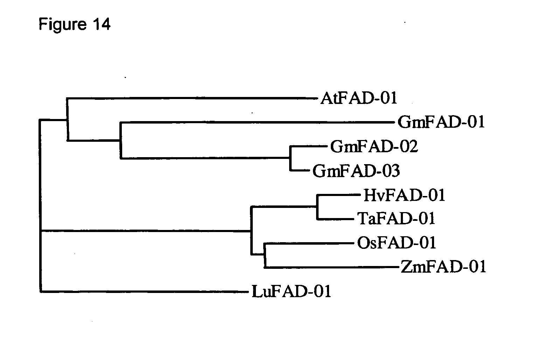

Figure 14. Diagram illustrating the relative homology among the disclosed AtFAD-01, GmFAD-01, GmFAD-02, GmFAD-03, LuFAD-01, HvFAD-01, TaFAD-01, OsFAD-01 and ZmFAD-01 nucleic acid sequences. The diagram was generated using Align X (Aug. 22, 2003) of Vector NTI Suite 9.0. The parameters used for the multiple alignment were as follows: Gap opening penalty: 15; Gap extension penalty: 6.66; Gap separation penalty range: 8; % identity for alignment delay: 40

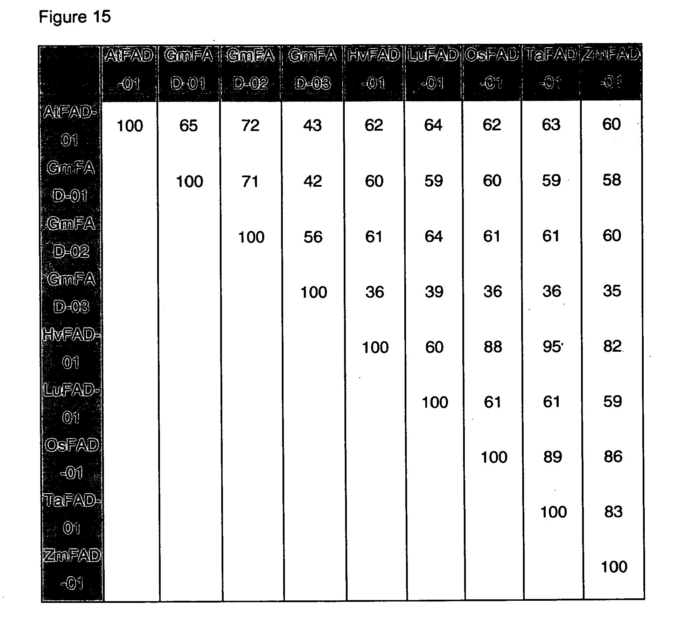

Figure 15. Table illustrating the similarity among the AtFAD-01, GmFAD-01, GmFAD-02, GmFAD-03, LuFAD-01, HvFAD-01, TaFAD-01, OsFAD-01 and ZmFAD-01 nucleic acid sequences. The table was generated using Align X (Aug. 22, 2003) of Vector NTI Suite 9.0. For other parameters see legend of Figure 14.

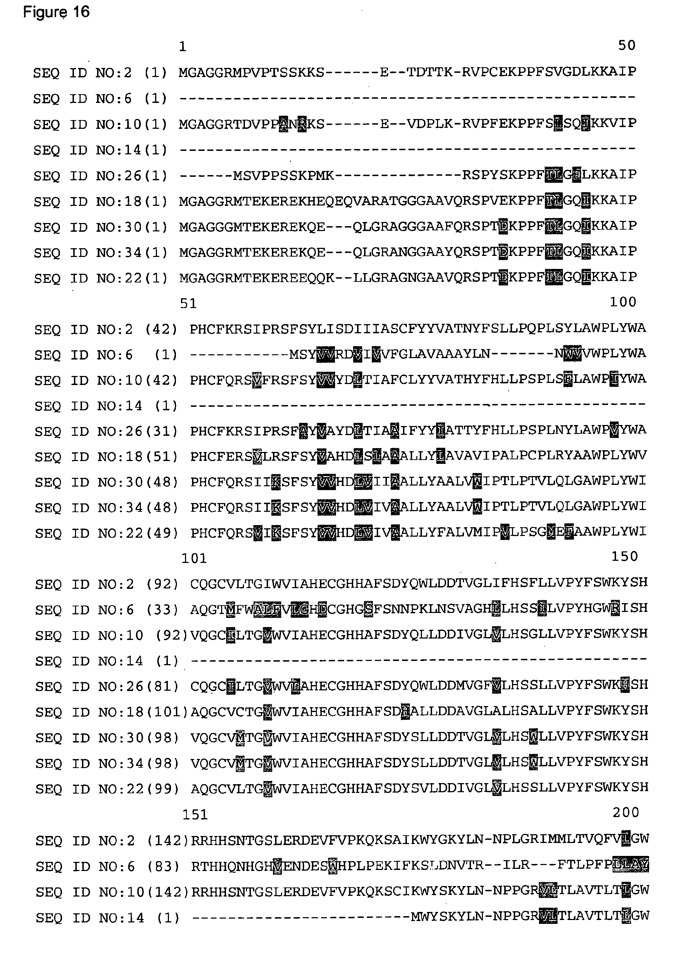

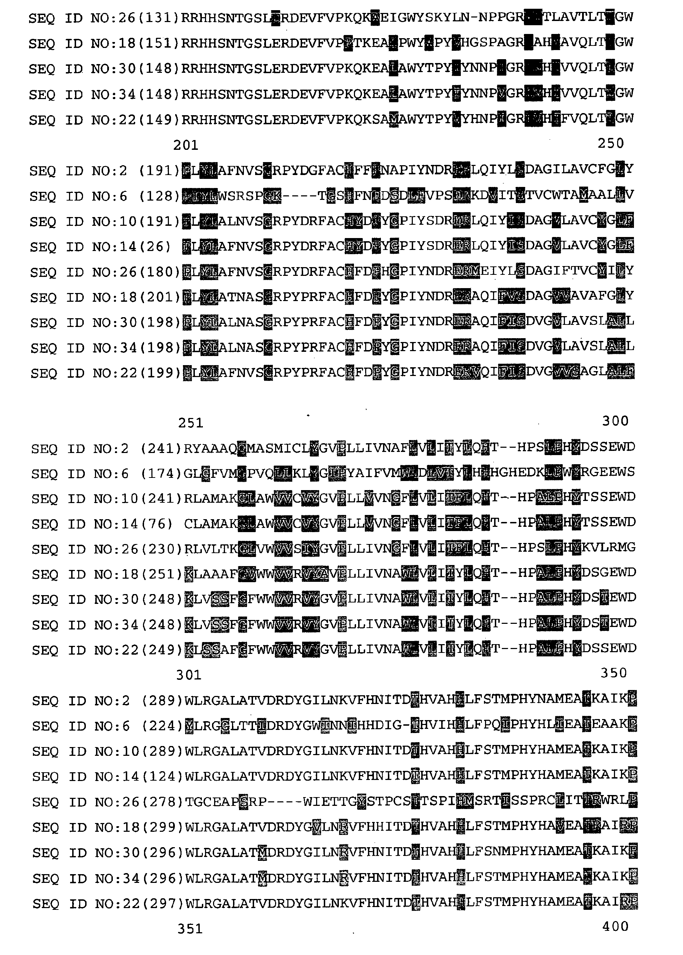

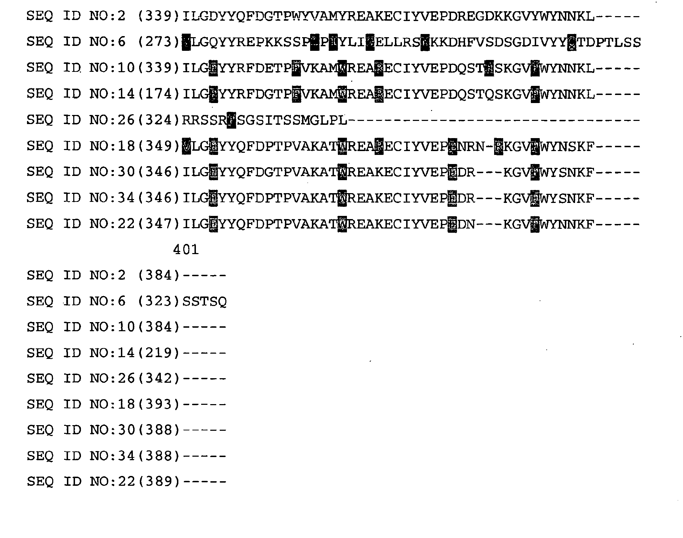

Figure 16. Sequence alignment of AtFAD-01, GmFAD-01, GmFAD-02, GmFAD-03, LuFAD-01, HvFAD-01, TaFAD-01, OsFAD-01 and ZmFAD-01 amino acid sequences. The alignment was generated using Align X (Aug. 22, 2003) of Vector NTI Suite 9.0. The parameters used for the multiple alignment were as follows: Gap opening penalty: 15; Gap extension penalty: 6.66; Gap separation penalty range: 8; % identity for alignment delay: 40

### GENERAL DEFINITIONS

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention, which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent, more preferably 5 percent up or down (higher or lower).

As used herein, the word "or" means any one member of a particular list and also includes any combination of members of that list.

As used herein, the term "amino acid sequence" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The abbreviations used herein are conventional one letter codes for the amino acids: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid (see L. Stryer, Biochemistry, 1988, W. H. Freeman and Company, New York. The letter "x" as used herein within an amino acid sequence can stand for any amino acid residue.

The term "nucleic acid" refers to deoxyribonucleotides or ribonucleotides and polymers or hybrids thereof in either single-or double-stranded, sense or antisense form.

The phrase "nucleic acid sequence" as used herein refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid, which is transcribed and translated in a sequence-specific manner to produce into a particular polypeptide or protein when placed under the control of appropriate regulatory sequences. The coding region is said to encode such a polypeptide or protein. Unless otherwise indicated, a particular nucleic acid sequence also implicitly encompasses conservatively modified variants thereof (e. g., degenerate codon substitutions) and complementary sequences, as well as the sequence explicitly indicated. The term "nucleic acid" is used interchangeably herein with "gene", "cDNA, "mRNA", "oligonucleotide," and "polynucleotide".

As used herein, the terms "complementary" or "complementarity" are used in reference to nucleotide sequences related by the base-pairing rules. For example, the sequence 5'-AGT-3' is complementary to the sequence 5'-ACT-3'. Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. A "complement" of a nucleic acid sequence as used herein refers to a nucleotide sequence whose nucleic acids show total complementarity to the nucleic acids of the nucleic acid sequence.

The term "genome" or "genomic DNA" is referring to the heritable genetic information of a host organism. Said genomic DNA comprises the DNA of the nucleus (also referred to as chromosomal DNA) but also the DNA of the plastids (e.g., chloroplasts) and other cellular organelles (e.g., mitochondria). Preferably the terms genome or genomic DNA is referring to the chromosomal DNA of the nucleus.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like *e.g.*, polymerase chain reaction (PCR) analysis, Southern blot analysis, fluorescence *in situ* hybridization (FISH), and *in situ* PCR.

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

The terms "heterologous nucleic acid sequence" or "heterologous DNA" are used interchangeably to refer to a nucleotide sequence, which is ligated to, or is manipulated to become ligated to, a nucleic acid sequence to which it is not ligated in nature, or to which it is ligated at a different location in nature. Heterologous DNA is not endogenous to the cell into which it is introduced, but has been obtained from another cell. Generally, although not necessarily, such heterologous DNA encodes RNA and proteins that are not normally produced by the cell into which it is expressed. A promoter, transcription regulating sequence or other genetic element is considered to be "heterologous" in relation to another sequence (e.g., encoding a marker sequence or am agronomically relevant trait) if said two sequences are not combined or differently operably linked their natural environment. Preferably, said sequences are not operably linked in their natural environment (i.e. come from different genes). Most preferably, said regulatory sequence is covalently joined and adjacent to a nucleic acid to which it is not adjacent in its natural environment.

The term "transgene" as used herein refers to any nucleic acid sequence, which is introduced into the genome of a cell or which has been manipulated by experimental manipulations by man. Preferably, said sequence is resulting in a genome which is different from a naturally occurring organism (*e.g.*, said sequence, if endogenous to said organism, is introduced into a location different from its natural location, or its copy number is increased or decreased). A transgene may be an "endogenous DNA sequence", "an "exogenous DNA sequence" (*e.g.*, a foreign gene), or a "heterologous DNA sequence". The term "endogenous DNA sequence" refers to a nucleotide sequence, which is naturally found in the cell into which it is introduced so long as it does not contain some modification (*e.g.*, a point mutation, the presence of a selectable marker gene, *etc.*) relative to the naturally-occurring sequence.

The term "transgenic" or "recombinant" when used in reference to a cell or an organism (e.g., with regard to a barley plant or plant cell) refers to a cell or organism which contains a transgene, or whose genome has been altered by the introduction of a transgene. A transgenic organism or tissue may comprise one or more transgenic cells. Preferably, the organism or tissue is substantially consisting of transgenic cells (i.e., more than 80%, preferably 90%, more preferably 95%, most preferably 99% of the cells in said organism or tissue are transgenic).

A "recombinant polypeptide" is a non-naturally occurring polypeptide that differs in sequence from a naturally occurring polypeptide by at least one amino acid residue. Preferred methods for producing said recombinant polypeptide and/or nucleic acid may comprise directed or non-directed mutagenesis, DNA shuffling or other methods of recursive recombination.

The term "equivalent" when made in reference to a hybridization condition as it relates to a hybridization condition of interest means that the hybridization condition and the hybridization condition of interest result in hybridization of nucleic acid sequences which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence.

In a preferred embodiment for the purposes of the invention, unless defined elsewhise, the percent sequence identity between two nucleic acid or polypeptide sequences is determined using the Vector NTI 7.0 (PC) software package (InforMax, 7600 Wisconsin Ave., Bethesda, MD 20814). A gap-opening penalty of 15 and a gap extension penalty of 6.66 are preferably used for determining the percent identity of two nucleic acids. A gap-opening penalty of 10 and a gap extension penalty of 0.1 are preferably used for determining the percent identity of two polypeptides. All other parameters are preferably set at the default settings. For purposes of a multiple alignment (Clustal W algorithm), in a preferred embodiment, the gap-opening penalty is 10, and the gap extension penalty is 0.05 with blosum62 matrix. It is to be understood that for the purposes of determining sequence identity when comparing a DNA sequence to an RNA sequence, a thymidine nucleotide sequence is equivalent to an uracil nucleotide.

When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, *etc.*) and the concentration of the salts and other components (*e.g.*, the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding, lower stringencies may be preferred to detect a larger number of nucleic acid sequences having different homologies.

The term "gene" refers to a coding region operably joined to appropriate regulatory sequences capable of regulating the expression of the polypeptide in some manner. A gene includes untranslated regulatory regions of DNA (e. g., promoters, enhancers, repressors, etc.) preceding (upstream) and following (downstream) the coding region (open reading frame, ORF) as well as, where applicable, intervening sequences (i.e., introns) between individual coding regions (i.e., exons). The term "structural gene" as used herein is intended to mean a DNA sequence that is transcribed into mRNA which is then translated into a sequence of amino acids characteristic of a specific polypeptide.

As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'side by the nucleotide triplet "ATG" which encodes the initiator methionine and on the 3'-side by one of the three triplets which specify stop codons (i.e., TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, posttranscriptional cleavage and polyadenylation.

The terms "polypeptide", "peptide", "oligopeptide", "polypeptide", "gene product", "expression product" and "protein" are used interchangeably herein to refer to a polymer or oligomer of consecutive amino acid residues.

The term "isolated" as used herein means that a material has been removed from its original environment. For example, a naturally-occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment.

The term "genetically-modified organism" or "GMO" refers to any organism that comprises transgene DNA. Exemplary organisms include plants, animals and microorganisms.

The term "cell" or "plant cell" as used herein refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronized or not synchronized. A plant cell within the meaning of this invention may be isolated (e.g., in suspension culture) or comprised in a plant tissue, plant organ or plant at any developmental stage.

The term "organ" with respect to a plant (or "plant organ") means parts of a plant and may include (but shall not limited to) for example roots, fruits, shoots, stem, leaves, anthers, sepals, petals, pollen, seeds, *etc.*

The term "tissue" with respect to a plant (or "plant tissue") means arrangement of multiple plant cells including differentiated and undifferentiated tissues of plants. Plant tissues may constitute part of a plant organ (*e.g.*, the epidermis of a plant leaf) but may also constitute tumor tissues (e.g., callus tissue) and various types of cells in culture (*e.g.*, single cells, protoplasts, embryos, calli, protocorm-like bodies, *etc.*). Plant tissue may be *in planta,* in organ culture, tissue culture, or cell culture.

The term "plant" as used herein refers to a plurality of plant cells which are largely differentiated into a structure that is present at any stage of a plant's development. Such structures include one or more plant organs including, but are not limited to, fruit, shoot, stem, leaf, flower petal, *etc.*

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like e.g., PCR analysis, Southern blot analysis, fluorescence in situ hybridization (FISH), and in situ PCR.

The term "structural gene" as used herein is intended to mean a DNA sequence that is transcribed into mRNA which is then translated into a sequence of amino acids characteristic of a specific polypeptide.

The term "expression" refers to the biosynthesis of a gene product. For example, in the case of a structural gene, expression involves transcription of the structural gene into mRNA and - optionally - the subsequent translation of mRNA into one or more polypeptides.

The term "expression cassette" or "expression construct" as used herein is intended to mean the combination of any nucleic acid sequence to be expressed in operable linkage with a promoter sequence and - optionally - additional elements (like e.g., terminator and/or polyadenylation sequences) which facilitate expression of said nucleic acid sequence.

"Promoter", "promoter element," or "promoter sequence" as used herein, refers to the nucleotide sequences at the 5' end of a nucleotide sequence which direct the initiation of transcription (i.e., is capable of controlling the transcription of the nucleotide sequence into mRNA). A promoter is typically, though not necessarily, located 5' (*i.e*., upstream) of a nucleotide sequence of interest (*e.g.*, proximal to the transcriptional start site of a structural gene) whose transcription into mRNA it controls, and provides a site for specific binding by RNA polymerase and other transcription factors for initiation of transcription. Promoter sequences are necessary, but not always sufficient, to drive the expression of a downstream gene. In general, eukaryotic promoters include a characteristic DNA sequence homologous to the consensus 5'-TATAAT-3' (TATA) box about 10-30 bp 5' to the transcription start (cap) site, which, by convention, is numbered +1. Bases 3' to the cap site are given positive numbers, whereas bases 5' to the cap site receive negative numbers, reflecting their distance from the cap site. Another promoter component, the CAAT box, is often found about 30 to 70 bp 5' to the TATA box and has homology to the canonical form 5'-CCAAT-3' (Breathnach 1981). In plants the CAAT box is sometimes replaced by a sequence known as the AGGA box, a region having adenine residues symmetrically flanking the triplet G(orT)NG (Messing 1983). Other sequences conferring regulatory influences on transcription can be found within the promoter region and extending as far as 1000 bp or more 5' from the cap site. The term "constitutive" when made in reference to a promoter means that the promoter is capable of directing transcription of an operably linked nucleic acid sequence in the absence of a stimulus (*e.g*., heat shock, chemicals, light, *etc.*). Typically, constitutive promoters are capable of directing expression of a transgene in substantially any cell and any tissue.

Regulatory Control refers to the modulation of gene expression induced by DNA sequence elements located primarily, but not exclusively, upstream of (5' to) the transcription start site. Regulation may result in an all-or-nothing response to environmental stimuli, or it may result in variations in the level of gene expression. In this invention, the heat shock regulatory elements function to enhance transiently the level of downstream gene expression in response to sudden temperature elevation.

Polyadenylation signal refers to any nucleic acid sequence capable of effecting mRNA processing, usually characterized by the addition of polyadenylic acid tracts to the 3'-ends of the mRNA precursors. The polyadenylation signal DNA segment may itself be a composite of segments derived from several sources, naturally occurring or synthetic, and may be from a genomic DNA or an RNA-derived cDNA. Polyadenylation signals are commonly recognized by the presence of homology to the canonical form 5'-AATAA-3', although variation of distance, partial "readthrough", and multiple tandem canonical sequences are not uncommon (Messing 1983). It should be recognized that a canonical "polyadenylation signal" may in fact cause transcriptional termination and not polyadenylation per se (Montell 1983).

Heat shock elements refer to DNA sequences that regulate gene expression in response to the stress of sudden temperature elevations. The response is seen as an immediate albeit transitory enhancement in level of expression of a downstream gene. The original work on heat shock genes was done with Drosophila but many other species including plants (Bamett 1980) exhibited analogous responses to stress. The essential primary component of the heat shock element was described in Drosophila to have the consensus sequence 5'-CTGGAATNTTCTAGA-3' (where N=A, T, C, or G) and to be located in the region between residues -66 through -47 bp upstream to the transcriptional start site (Pelham 1982). A chemically synthesized oligonucleotide copy of this consensus sequence can replace the natural sequence in conferring heat shock inducibility.

Leader sequence refers to a DNA sequence comprising about 100 nucleotides located between the transcription start site and the translation start site. Embodied within the leader sequence is a region that specifies the ribosome binding site.

Introns or intervening sequences refer in this work to those regions of DNA sequence that are transcribed along with the coding sequences (exons) but are then removed in the formation of the mature mRNA. Introns may occur anywhere within a transcribed sequence--between coding sequences of the same or different genes, within the coding sequence of a gene, interrupting and splitting its amino acid sequences, and within the promoter region (5' to the translation start site). introns in the primary transcript are excised and the coding sequences are simultaneously and precisely ligated to form the mature mRNA. The junctions of introns and exons form the splice sites. The base sequence of an intron begins with GU and ends with AG. The same splicing signal is found in many higher eukaryotes.

The term "operable linkage" or "operably linked" is to be understood as meaning, for example, the sequential arrangement of a regulatory element (e.g. a promoter) with a nucleic acid sequence to be expressed and, if appropriate, further regulatory elements (such as e.g., a terminator) in such a way that each of the regulatory elements can fulfill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. Operable linkage, and an expression cassette, can be generated by means of customary recombination and cloning techniques as described (e.g., in Maniatis 1989; Silhavy 1984; Ausubel 1987; Gelvin 1990). However, further sequences which, for example, act as a linker with specific cleavage sites for restriction enzymes, or as a signal peptide, may also be positioned between the two sequences. The insertion of sequences may also lead to the expression of fusion proteins. Preferably, the expression cassette, consisting of a linkage of promoter and nucleic acid sequence to be expressed, can exist in a vector-integrated form and be inserted into a plant genome, for example by transformation.

The term "transformation" as used herein refers to the introduction of genetic material (*e.g.*, a transgene) into a cell. Transformation of a cell may be stable or transient. The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA) which detects the presence of a polypeptide encoded by one or more of the transgenes. Alternatively, transient transformation may be detected by detecting the activity of the protein (*e.g.*, □-glucuronidase) encoded by the transgene (*e.g.*, the uid A gene) as demonstrated herein [*e.g.*, histochemical assay of GUS enzyme activity by staining with X-gluc which gives a blue precipitate in the presence of the GUS enzyme; and a chemiluminescent assay of GUS enzyme activity using the GUS-Light kit (Tropix)]. The term "transient transformant" refers to a cell which has transiently incorporated one or more transgenes. In contrast, the term "stable transformation" or "stably transformed" refers to the introduction and integration of one or more transgenes into the genome of a cell, preferably resulting in chromosomal integration and stable heritability through meiosis. Stable transformation of a cell may be detected by Southern blot hybridization of genomic DNA of the cell with nucleic acid sequences which are capable of binding to one or more of the transgenes. Alternatively, stable transformation of a cell may also be detected by the polymerase chain reaction of genomic DNA of the cell to amplify transgene sequences. The term "stable transformant" refers to a cell which has stably integrated one or more transgenes into the genomic DNA (including the DNA of the plastids and the nucleus), preferably integration into the chromosomal DNA of the nucleus. Thus, a stable transformant is distinguished from a transient transformant in that, whereas genomic DNA from the stable transformant contains one or more transgenes, genomic DNA from the transient transformant does not contain a transgene. Transformation also includes introduction of genetic material into plant cells in the form of plant viral vectors involving epichromosomal replication and gene expression which may exhibit variable properties with respect to meiotic stability. Transformation also includes introduction of genetic material into plant cells in the form of plant viral vectors involving epichromosomal replication and gene expression which may exhibit variable properties with respect to meiotic stability. Preferably, the term "transformation" includes introduction of genetic material into plant cells resulting in chromosomal integration and stable heritability through meiosis.

The terms "infecting" and "infection" with a bacterium refer to co-incubation of a target biological sample, (*e.g.*, cell, tissue, *etc.*) with the bacterium under conditions such that nucleic acid sequences contained within the bacterium are introduced into one or more cells of the target biological sample.

The term "*Agrobacterium*" refers to a soil-bome, Gram-negative, rod-shaped phytopathogenic bacterium which causes crown gall. The term "*Agrobacterium*" includes, but is not limited to, the strains *Agrobacterium tumefaciens,* (which typically causes crown gall in infected plants), and *Agrobacterium rhizogenes* (which causes hairy root disease in infected host plants). Infection of a plant cell with *Agrobacterium* generally results in the production of opines (*e.g.*, nopaline, agropine, octopine *etc.*) by the infected cell. Thus, *Agrobacterium* strains which cause production of nopaline (*e.g.*, strain LBA4301, C58, A208) are referred to as "nopaline-type" *Agrobacteria; Agrobacterium* strains which cause production of octopine (*e.g.*, strain LBA4404, Ach5, B6) are referred to as "octopine-type" *Agrobacteria;* and *Agrobacterium* strains which cause production of agropine (*e.g.*, strain EHA105, EHA101, A281) are referred to as "agropine-type" *Agrobacteria*.

The terms "bombarding, "bombardment," and "biolistic bombardment" refer to the process of accelerating particles towards a target biological sample (*e.g.*, cell, tissue, *etc.*) to effect wounding of the cell membrane of a cell in the target biological sample and/or entry of the particles into the target biological sample. Methods for biolistic bombardment are known in the art (*e.g.*, US 5,584,807, the contents of which are herein incorporated by reference), and are commercially available (e.g., the helium gas-driven microprojectile accelerator (PDS-1000/He) (BioRad).

The term "hybridization" as used herein includes "any process by which a strand of nucleic acid joins with a complementary strand through base pairing." (Coombs 1994). Hybridization and the strength of hybridization (*i.e*., the strength of the association between the nucleic acids) is impacted by such factors as the degree of complementarity between the nucleic acids, stringency of the conditions involved, the Tm of the formed hybrid, and the G:C ratio within the nucleic acids.

As used herein, the term "Tm" is used in reference to the "melting temperature." The melting temperature is the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0.41 (% G+C), when a nucleic acid is in aqueous solution at 1 M NaCl [see *e.g.*, Anderson and Young, Quantitative Filter Hybridization, in Nucleic Acid Hybridization (1985)]. Other references include more sophisticated computations which take structural as well as sequence characteristics into account for the calculation of Tm.

Low stringency conditions when used in reference to nucleic acid hybridization unless defined elsewhise comprise conditions equivalent to binding or hybridization at 68°C. in a solution consisting of 5x SSPE (43.8 g/L NaCl, 6.9 g/L NaH₂PO₄.H₂O and 1.85 g/L EDTA, pH adjusted to 7.4 with NaOH), 1% SDS, 5x Denhardt's reagent [50x Denhardt's contains the following per 500 mL: 5 g Ficoll (Type 400, Pharmacia), 5 g BSA (Fraction V; Sigma)] and 100 µg/mL denatured salmon sperm DNA followed by washing in a solution comprising 0.2x SSPE, and 0.1 % SDS at room temperature when a DNA probe of about 100 to about 1000 nucleotides in length is employed.

High stringency conditions when used in reference to nucleic acid hybridization comprise unless defined elsewhise conditions equivalent to binding or hybridization at 68° C. in a solution consisting of 5x SSPE, 1% SDS, 5x Denhardt's reagent and 100 µg/mL denatured salmon sperm DNA followed by washing in a solution comprising 0.1 x SSPE, and 0.1 % SDS at 68° C. when a probe of about 100 to about 1000 nucleotides in length is employed.

The term "equivalent" when made in reference to a hybridization condition as it relates to a hybridization condition of interest means that the hybridization condition and the hybridization condition of interest result in hybridization of nucleic acid sequences which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence.

When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, *etc.*) and the concentration of the salts and other components (*e.g.*, the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding, lower stringencies may be preferred to detect a larger number of nucleic acid sequences having different homologies.

### DETAILED DESCRIPTION OF THE INVENTION

The present invention may be understood more readily by reference to the following detailed description of the preferred embodiments of the invention and the Examples included therein.

Before the present compounds, compositions, and methods are disclosed and described, it is to be understood that this invention is not limited to specific nucleic acids, specific polypeptides, specific cell types, specific host cells, specific conditions, or specific methods, etc., as such may, of course, vary, and the numerous modifications and variations therein will be apparent to those skilled in the art. It is also to be understood that the terminology used herein is for the purpose of describing particular embodiments only and is not intended to be limiting. As used in the specification and in the claims, "a" or "an" can mean one or more, depending upon the context in which it is used. Thus, for example, reference to "a cell" can mean that at least one cell can be utilized.

The present invention is based, in part, on the isolation and characterization of nucleic acid molecules encoding FAD2-like LMPs from plants including *Arabidopsis thaliana,* soybean (*Glycine max*), rice (*Oryza sativa*)*,* corn (*Zea mays*)*,* linseed (*Linum usitatissimum*), barley (*Hordeum vulgare*) and wheat (*Triticum aestivum*) and other related crop species like maize, barley, linseed, sugar beat or sunflower.

In accordance with the purpose(s) of this invention, as embodied and broadly described herein, this invention, in one aspect, provides an isolated nucleic acid from a plant (*Arabidopsis thaliana, Glycine max, Zea mays, Oryza sativa, Linum usitatissimum, Hordeum vulgare* or *Triticum aestivum)* encoding a Lipid Metabolism Protein (LMP), or a portion thereof.

One aspect of the invention pertains to isolated nucleic acid molecules that encode LMP polypeptides or biologically active portions thereof, as well as nucleic acid fragments sufficient for use as hybridization probes or primers for the identification or amplification of an LMP-encoding nucleic acid (e.g., LMP DNA). As used herein, the term "nucleic acid molecule" is intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of a gene: at least about 1000 nucleotides of sequence upstream from the 5' end of the coding region and at least about 200 nucleotides of sequence downstream from the 3' end of the coding region of the gene. The nucleic acid molecule can be single-stranded or double-stranded, but preferably is double-stranded DNA. An "isolated" nucleic acid molecule is one which is substantially separated from other nucleic acid molecules which are present in the natural source of the nucleic acid. Preferably, an "isolated" nucleic acid is substantially free of sequences that naturally flank the nucleic acid (i.e., sequences located at the 5' and 3' ends of the nucleic acid) in the genomic DNA of the organism, from which the nucleic acid is derived. For example, in various embodiments, the isolated LMP nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived (e.g., a *Arabidopsis thaliana, Glycine max, Oryza sativa, Zea mays, Linum usitatissimum, Hordeum vulgare* or *Triticum aestivum* cell). Moreover, an "isolated" nucleic acid molecule, such as a cDNA molecule, can be substantially free of other cellular material, or culture medium when produced by recombinant techniques, or chemical precursors or other chemicals when chemically synthesized.

A nucleic acid molecule of the present invention, e.g., a nucleic acid molecule having a nucleotide sequence of Appendix A, in a preferred embodiment as disdosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, or a portion thereof, can be isolated using standard molecular biology techniques and the sequence information provided herein. For example, an *Arabidopsis thaliana, Glycine max, Oryza sativa, Zea mays, Linum usitatissimum, Hordeum vulgare* or *Triticum aestivum* LMP cDNA can be isolated from an *Arabidopsis thaliana, Glycine max, Oryza sativa, Zea mays, Linum usitatissimum, Hordeum vulgare* or *Triticum aestivum* library using all or portion of one of the sequences of Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, as a hybridization probe and standard hybridization techniques (e.g., as described in Sambrook et al. 1989, Molecular Cloning: A Laboratory Manual. 2nd, ed., Cold Spring Harbor Laboratory, Cold Spring Harbor Laboratory Press, Cold Spring Harbor, NY). Moreover, a nucleic acid molecule encompassing all or a portion of one of the sequences of Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, can be isolated by the polymerase chain reaction using oligonucleotide primers designed based upon this sequence (e.g., a nucleic acid molecule encompassing all or a portion of one of the sequences of Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, can be isolated by the polymerase chain reaction using oligonucleotide primers designed based upon this same sequence of Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35). For example, mRNA can be isolated from plant cells (e.g., by the guanidinium-thiocyanate extraction procedure of Chirgwin et al. 1979, Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences shown in Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35. A nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to a LMP nucleotide sequence can be prepared by standard synthetic techniques, e.g., using an automated DNA synthesizer.

In a preferred embodiment, an isolated nucleic acid of the invention comprises one of the nucleotide sequences shown in Appendix A, in a preferred embodiment as disclosed in SEQ ID NO: 5, SEQ ID NO: 9, SEQ ID NO: 13, SEQ ID NO: 17, SEQ ID NO: 21, SEQ ID NO:25, SEQ ID NO: 29, or SEQ ID NO:33. The sequences of Appendix A, in a preferred embodiment as disclosed in SEQ ID NO: 5, SEQ ID NO: 9, SEQ ID NO: 13, SEQ ID NO: 17, SEQ ID NO: 21, SEQ ID NO:25, SEQ ID NO: 29, or SEQ ID NO:33 correspond to the *Arabidopsis thaliana, Glycine max, Oryza sativa, Zea mays, Linum usitatissimum, Hordeum vulgare* or *Triticum aestivum* LMP cDNAs of the invention. These cDNAs comprise sequences encoding LMPs (i.e., the "coding region", indicated in Appendix A), as well as 5' untranslated sequences and 3' untranslated sequences. Alternatively, the nucleic acid molecules can comprise only the coding region of any of the sequences in Appendix A, in a preferred embodiment as disclosed in SEQ ID NO: 7, SEQ ID NO: 11, SEQ ID NO: 15, SEQ ID NO: 19, SEQ ID NO: 23, SEQ ID NO: 27, SEQ ID NO: 31, or SEQ ID NO: 35 or can contain whole genomic fragments isolated from genomic DNA.

For the purposes of this application, it will be understood that each of the sequences set forth in Appendix A has an identifying entry number (e.g., *TaFAD-01*). Each of these sequences may generally comprise three parts: a 5' upstream region, a coding region, and a downstream region. A coding region of these sequences is indicated as "ORF position" (Table 3).

In another preferred embodiment, an isolated nucleic acid molecule of the invention comprises a nucleic acid molecule, which is a complement of one of the nucleotide sequences shown in Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, or a portion thereof. A nucleic acid molecule which is complementary to one of the nucleotide sequences shown in Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, is one which is sufficiently complementary to one of the nucleotide sequences shown in Appendix A such that it can hybridize to one of the nucleotide sequences shown in Appendix A, thereby forming a stable duplex.

In still another preferred embodiment, an isolated nucleic acid molecule of the invention comprises a nucleotide sequence which is at least about 50-60%, preferably at least about 60-70%, more preferably at least about 70-80%, 80-90%, or 90-95%, also preferable at least about 85%, 86 %, 87 %, 88 %, 89 %, 90 %, 91 %, 92 %, 93 %, or 94 % and even more preferably at least about 95%, 96%, 97%, 98%, 99% or more homologous to a nucleotide sequence shown in Appendix A, in a preferred embodiment as disclosed in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, or a portion thereof. The nucleotide sequence homology is preferably determined using Align X (Aug. 22, 2003) of Vector NTI Suite 9.0 with following parameters: Gap opening penalty: 15; Gap extension penalty: 6.66; Gap separation penalty range: 8; % identity for alignment delay: 40.

In an additional preferred embodiment, an isolated nucleic acid molecule of the invention comprises a nucleotide sequence which hybridizes, e.g., hybridizes under stringent conditions, to one of the nucleotide sequences shown in Appendix A, or a portion thereof. These hybridization conditions include washing with a solution having a salt concentration of about 0.02 molar at pH 7 at about 60°C.

Moreover, the nucleic acid molecule of the invention can comprise only a portion of the coding region of one of the sequences in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, for example a fragment, which can be used as a probe or primer or a fragment encoding a biologically active portion of a LMP. The nucleotide sequences determined from the cloning of the LMP genes from *Arabidopsis thaliana, Glycine max, Oryza sativa, Zea mays, Linum usitatissimum, Hordeum vulgare* or *Triticum aestivum* allows for the generation of probes and primers designed for use in identifying and/or cloning LMP homologues in other cell types and organisms, as well as LMP homologues from other plants or related species. Therefore this invention also provides compounds comprising the nucleic acids disclosed herein, or fragments thereof. These compounds include the nucleic acids attached to a moiety. These moieties include, but are not limited to, detection moieties, hybridization moieties, purification moieties, delivery moieties, reaction moieties, binding moieties, and the like. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of one of the sequences set forth in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, an anti-sense sequence of one of the sequences set forth in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, or naturally occurring mutants thereof. Primers based on a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, can be used in PCR reactions to clone LMP homologues. Probes based on the LMP nucleotide sequences can be used to detect transcripts or genomic sequences encoding the same or homologous proteins. In preferred embodiments, the probe further comprises a label group attached thereto, e.g. the label group can be a radioisotope, a fluorescent compound, an enzyme, or an enzyme co-factor. Such probes can be used as a part of a genomic marker test kit for identifying cells which express a LMP, such as by measuring a level of a LMP-encoding nucleic acid in a sample of cells, e.g., detecting LMP mRNA levels or determining whether a genomic LMP gene has been mutated or deleted.

In one embodiment, the nucleic acid molecule of the invention encodes a protein or portion thereof which includes an amino acid sequence which is sufficiently homologous to an amino acid encoded by a sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, such that the protein or portion thereof maintains the same or a similar function as the wild-type protein. As used herein, the language "sufficiently homologous" refers to proteins or portions thereof which have amino acid sequences which include a minimum number of identical or equivalent (e.g., an amino acid residue, which has a similar side chain as an amino acid residue in one of the ORFs of a sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,) amino acid residues to an amino acid sequence such that the protein or portion thereof is able to participate in the metabolism of compounds necessary for the production of seed storage compounds in plants, construction of cellular membranes in microorganisms or plants, or in the transport of molecules across these membranes. Regulatory proteins, such as DNA binding proteins, transcription factors, kinases, phosphatases, or protein members of metabolic pathways such as the lipid, starch and protein biosynthetic pathways, or membrane transport systems, may play a role in the biosynthesis of seed storage compounds. Examples of such activities are described herein (see putative annotations in Table 3). Examples of LMP-encoding nucleic acid sequences are set forth in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,.

As altered or increased sugar and/or fatty acid production is a general trait wished to be inherited into a wide variety of plants like maize, wheat, rye, oat, triticale, rice, barley, soybean, peanut, cotton, canola, linseed, manihot, pepper, sunflower, sugar beet and tagetes, solanaceous plants like potato, tobacco, eggplant, and tomato, Vicia species, pea, alfalfa, bushy plants (coffee, cacao, tea), Salix species, trees (oil palm, coconut) and perennial grasses and forage crops, these crop plants are also preferred target plants for genetic engineering as one further embodiment of the present invention.

Portions of proteins encoded by the LMP nucleic acid molecules of the invention are preferably biologically active portions of one of the LMPs. As used herein, the term "biologically active portion of a LMP" is intended to include a portion, e.g., a domain/ motif, of a LMP that participates in the metabolism of compounds necessary for the biosynthesis of seed storage lipids, or the construction of cellular membranes in microorganisms or plants, or in the transport of molecules across these membranes, or has an activity as set forth in Table 3. To determine whether a LMP or a biologically active portion thereof can participate in the metabolism of compounds necessary for the production of seed storage compounds and cellular membranes, an assay of enzymatic activity may be performed. Such assay methods are well known to those skilled in the art, and as described in Example 14 of the Exemplification.

Biologically active portions of a LMP include peptides comprising amino acid sequences derived from the amino acid sequence of a LMP (e.g., an amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, or the amino acid sequence of a protein homologous to a LMP, which include fewer amino acids than a full length LMP or the full length protein which is homologous to a LMP) and exhibit at least one activity of a LMP. Typically, biologically active portions (peptides, e.g., peptides which are, for example, 5, 10, 15, 20, 30, 35, 36, 37, 38, 39, 40, 50, 100 or more amino acids in length) comprise a domain or motif with at least one activity of a LMP. Moreover, other biologically active portions, in which other regions of the protein are deleted, can be prepared by recombinant techniques and evaluated for one or more of the activities described herein. Preferably, the biologically active portions of a LMP include one or more selected domains/motifs or portions thereof having biological activity.

Additional nucleic acid fragments encoding biologically active portions of a LMP can be prepared by isolating a portion of one of the sequences, expressing the encoded portion of the LMP or peptide (e.g., by recombinant expression *in vitro)* and assessing the activity of the encoded portion of the LMP or peptide.

The invention further encompasses nucleic acid molecules that differ from one of the nucleotide sequences shown in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, (and portions thereof) due to degeneracy of the genetic code and thus encode the same LMP as that encoded by the nucleotide sequences shown in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,. In a further embodiment, the nucleic acid molecule of the invention encodes a full length protein which is substantially homologous to an amino acid sequence of a polypeptide encoded by an open reading frame shown in Appendix A. In one embodiment, the full-length nucleic acid or protein or fragment of the nucleic acid or protein is from *Arabidopsis thaliana, Glycine max, Oryza sativa, Zea mays, Linum usitatissimum, Hordeum vulgare* or *Triticum aestivum.*

In addition to the *Arabidopsis thaliana, Glycine max, Oryza sativa, Zea mays, Linum usitatissimum, Hordeum vulgare* or *Triticum aestivum* LMP nucleotide sequences shown in Appendix A, it will be appreciated by those skilled in the art that DNA sequence polymorphism that lead to changes in the amino acid sequences of LMPs may exist within a population (e.g., the *Arabidopsis thaliana, Brassica napus, Glycine max, Oryza sativa,* Zea *mays, Linum usitatissimum, Hordeum vul*gare or *Triticum aestivum* population). Such genetic polymorphism in the LMP gene may exist among individuals within a population due to natural variation. As used herein, the terms "gene" and "recombinant gene" refer to nucleic acid molecules comprising an open reading frame encoding a LMP, preferably a *Arabidopsis thaliana, Glycine max, Oryza sativa, Zea mays, Linum usitatissimum, Hordeum vulgare* or *Triticum aestivum* LMP. Such natural variations can typically result in 1-40% variance in the nucleotide sequence of the LMP gene. Any and all such nucleotide variations and resulting amino acid polymorphisms in LMP that are the result of natural variation and that do not alter the functional activity of LMPs are intended to be within the scope of the invention.

Nucleic acid molecules corresponding to natural variants and *non-Arabidopsis thaliana, Glycine max, Oryza sativa, Zea mays, Linum usitatissimum, Hordeum vulgare* or *Triticum aestivum* orthologs of the *Arabidopsis thaliana, Glycine max, Oryza sativa, Zea mays, Linum usitatissimum, Hordeum vulgare* or *Triticum aestivum* LMP cDNA of the invention can be isolated based on their homology to *Arabidopsis thaliana, Glycine max, Oryza sativa, Zea mays, Linum usitatissimum, Hordeum vulgare* or *Triticum aestivum* LMP nucleic acid disclosed herein using the *Arabidopsis thaliana, Glycine max, Oryza sativa, Zea mays, Linum usitatissimum, Hordeum vulgare* or *Triticum aestivum* cDNA, or a portion thereof, as a hybridization probe according to standard hybridization techniques under stringent hybridization conditions. As used herein, the term "orthologs" refers to two nucleic acids from different species, but that have evolved from a common ancestral gene by speciation. Normally, orthologs encode proteins having the same or similar functions. Accordingly, in another embodiment, an isolated nucleic acid molecule of the invention is at least 15 nucleotides in length and hybridizes under stringent conditions to the nucleic acid molecule comprising a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,. In other embodiments, the nucleic acid is at least 30, 50, 100, 250 or more nucleotides in length. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 65%, more preferably at least about 70%, and even more preferably at least about 75% or more homologous to each other typically remain hybridized to each other. Such stringent conditions are known to those skilled in the art and can be found in Cur rent Protocols in Molecular Biology, John Wiley & Sons, N.Y., 1989: 6.3.1-6.3.6. A preferred, nonlimiting example of stringent hybridization conditions are hybridization in 6X sodium chloride/sodium citrate (SSC) at about 45°C, followed by one or more washes in 0.I£ X SSC, 0.1% SDS at 50-65°C. Preferably, an isolated nucleic acid molecule of the invention that hybridizes under stringent conditions to a sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, corresponds to a naturally occurring nucleic acid molecule. As used herein, a "naturally-occurring" nucleic acid molecule refers to an RNA or DNA molecule having a nucleotide sequence that occurs in nature (e.g., encodes a natural protein). In one embodiment, the nucleic acid encodes a natural *Arabidopsis thaliana, Glycine max, Oryza sativa, Zea mays, Linum usitatissimum, Hordeum vulgare* or *Triticum aestivum* LMP.

In addition to naturally-occurring variants of the LMP sequence that may exist in the population, the skilled artisan will further appreciate that changes can be introduced by mutation into a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, thereby leading to changes in the amino acid sequence of the encoded LMP, without altering the functional ability of the LMP. For example, nucleotide substitutions leading to amino acid substitutions at "non-essential" amino acid residues can be made in a sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35. A "non-essential" amino acid residue is a residue that can be altered from the wild-type sequence of one of the LMPs (Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35) without altering the activity of said LMP, whereas an "essential" amino acid residue is required for LMP activity. Other amino acid residues, however, (e.g., those that are not conserved or only semi-conserved in the domain having LMP activity) may not be essential for activity and thus are likely to be amenable to alteration without altering LMP activity.

Accordingly, another aspect of the invention pertains to nucleic acid molecules encoding LMPs that contain changes in amino acid residues that are not essential for LMP activity. Such LMPs differ in amino acid sequence from a sequence yet retain at least one of the LMP activities described herein. In one embodiment, the isolated nucleic acid molecule comprises a nucleotide sequence encoding a protein, wherein the protein comprises an amino acid sequence at least about 50% homologous to an amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and is capable of participation in the metabolism of compounds necessary for the production of seed storage compounds in *Arabidopsis thaliana, Glycine max, Oryza sativa, Zea mays, Linum usitatissimum, Hordeum vulgare* or *Triticum aestivum,* or cellular membranes, or has one or more activities set forth in Table 3. Preferably, the protein encoded by the nucleic acid molecule is at least about 50-60% homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, more preferably at least about 60-70% homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, even more preferably at least about 70-80%, 80-90%, 90-95%, also preferable at least about 85%, 86 %, 87 %, 88 %, 89 %, 90 %, 91 %, 92 %, 93 %, 94 % or 95 % homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and most preferably at least about 96%, 97%, 98%, or 99% homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,. The polypeptide sequence homology is preferably determined using Align X (Aug. 22, 2003) of Vector NTI Suite 9.0 with following parameters: Gap opening penalty: 10; Gap extension penalty: 0.05; Gap separation penalty range: 8; % identity for alignment delay: 40.

To determine the percent homology of two amino acid sequences (e.g., one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and a mutant form thereof) or of two nucleic acids, the sequences are aligned for optimal comparison purposes (e.g., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence (e.g., one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35) is occupied by the same amino acid residue or nucleotide as the corresponding position in the other sequence (e.g., a mutant form of the sequence selected from the polypeptide encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,), then the molecules are homologous at that position (i.e., as used herein amino acid or nucleic acid "homology" is equivalent to amino acid or nucleic acid "identity"). The percent homology between the two sequences is a function of the number of identical positions shared by the sequences (i.e., % homology = numbers of identical positions/total numbers of positions x 100).

An isolated nucleic acid molecule encoding a LMP homologous to a protein sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, can be created by introducing one or more nucleotide substitutions, additions or deletions into a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, such that one or more amino acid substitutions, additions or deletions are introduced into the encoded protein. Mutations can be introduced into one of the sequences of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted non-essential amino acid residue in a LMP is preferably replaced with another amino acid residue from the same side chain family. Alternatively, in another embodiment, mutations can be introduced randomly along all or part of a LMP coding sequence, such as by saturation mutagenesis, and the resultant mutants can be screened for a LMP activity described herein to identify mutants that retain LMP activity. Following mutagenesis of one of the sequences of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, the encoded protein can be expressed recombinantly and the activity of the protein can be determined using, for example, assays described herein (see Examples 11-13 of the Exemplification).

LMPs are preferably produced by recombinant DNA techniques. For example, a nucleic acid molecule encoding the protein is cloned into an expression vector (as described above), the expression vector is introduced into a host cell (as described herein) and the LMP is expressed in the host cell. The LMP can then be isolated from the cells by an appropriate purification scheme using standard protein purification techniques. Alternative to recombinant expression, a LMP or peptide thereof can be synthesized chemically using standard peptide synthesis techniques. Moreover, native LMP can be isolated from cells, for example using an anti-LMP antibody, which can be produced by standard techniques utilizing a LMP or fragment thereof of this invention.

The invention also provides LMP chimeric or fusion proteins. As used herein, a LMP "chimeric protein" or "fusion protein" comprises a LMP polypeptide operatively linked to a non-LMP polypeptide. An "LMP polypeptide" refers to a polypeptide having an amino acid sequence corresponding to a LMP, whereas a "non-LMP polypeptide" refers to a polypeptide having an amino acid sequence corresponding to a protein which is not substantially homologous to the LMP, e.g., a protein which is different from the LMP and which is derived from the same or a different organism. Within the fusion protein, the term "operatively linked" is intended to indicate that the LMP polypeptide and the non-LMP polypeptide are fused to each other so that both sequences fulfill the proposed function attributed to the sequence used. The non-LMP polypeptide can be fused to the N-terminus or C-terminus of the LMP polypeptide. For example, in one embodiment, the fusion protein is a GST-LMP (glutathione S-transferase) fusion protein in which the LMP sequences are fused to the C-terminus of the GST sequences. Such fusion proteins can facilitate the purification of recombinant LMPs. In another embodiment, the fusion protein is a LMP containing a heterologous signal sequence at its N-terminus. In certain host cells (e.g., mammalian host cells), expression and/or secretion of a LMP can be increased through use of a heterologous signal sequence.

Preferably, a LMP chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments, which can subsequently be annealed and reamplified to generate a chimeric gene sequence (see, for example, Current Protocols in Molecular Biology, eds. Ausubel et al., John Wiley & Sons: 1992). Moreover, many expression vectors are commercially available that already encode a fusion moiety (e.g., a GST polypeptide). An LMP-encoding nucleic acid can be cloned into such an expression vector such that the fusion moiety is linked in-frame to the LMP.

In addition to the nucleic acid molecules encoding LMPs described above, another aspect of the invention pertains to isolated nucleic acid molecules that are antisense thereto. An "antisense" nucleic acid comprises a nucleotide sequence that is complementary to a "sense" nucleic acid encoding a protein, e.g., complementary to the coding strand of a double-stranded cDNA molecule or complementary to an mRNA sequence. Accordingly, an antisense nucleic acid can be hydrogen bond to a sense nucleic acid. The antisense nucleic acid can be complementary to an entire LMP coding strand, or to only a portion thereof. In one embodiment, an antisense nucleic acid molecule is antisense to a "coding region" of the coding strand of a nucleotide sequence encoding a LMP. The term "coding region" refers to the region of the nucleotide sequence comprising codons that are translated into amino acid residues (e.g., the entire coding region of *TaFAD-01* comprises nucleotides 165-1325). In another embodiment, the antisense nucleic acid molecule is antisense to a "noncoding region" of the coding strand of a nucleotide sequence encoding LMP. The term "noncoding region" refers to 5' and 3' sequences that flank the coding region that are not translated into amino acids (i.e., also referred to as 5' and 3' untranslated regions).

Given the coding strand sequences encoding LMP disclosed herein (e.g., the sequences set forth in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35), antisense nucleic acids of the invention can be designed according to the rules of Watson and Crick base pairing. The antisense nucleic acid molecule can be complementary to the entire coding region of LMP mRNA, but more preferably is an oligonucleotide that is antisense to only a portion of the coding or noncoding region of LMP mRNA. For example, the antisense oligonucleotide can be complementary to the region surrounding the translation start site of LMP mRNA. An antisense oligonucleotide can be, for example, about 5, 10, 15, 20, 25, 30, 35, 40, 45 or 50 nucleotides in length. An antisense or sense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylamino-methyl-2-thiouridine, 5-carboxymethylaminomethyluracil, dihydro-uracil, beta-D-galactosylqueosine, inosine, N-6-isopentenyladenine, 1-methyl-guanine, 1-methylinosine, 2,2-dimethylguanine, 2-methytadenine, 2-methylguanine, 3-methylcytosine, 5-methyl-cytosine, N-6-adenine, 7-methylguanine, 5-methylaminomethyluracil, 5-methoxyamino-methyl-2-thiouracil, beta-D-mannosyiqueosine, 5'-methoxycarboxymethyl-uracil, 5-methoxyuracil, 2-methylthio-N-6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diamino-purine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

In another variation of the antisense technology, a double-strand interfering RNA construct can be used to cause a down-regulation of the LMP mRNA level and LMP activity in transgenic plants. This requires transforming the plants with a chimeric construct containing a portion of the LMP sequence in the sense orientation fused to the antisense sequence of the same portion of the LMP sequence. A DNA linker region of variable length can be used to separate the sense and antisense fragments of LMP sequences in the construct.

The antisense nucleic acid molecules of the invention are typically administered to a cell or generated *in situ* such that they hybridize with or bind to cellular mRNA and/or genomic DNA encoding a LMP to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic including plant promoters are preferred.

In yet another embodiment, the antisense nucleic acid molecule of the invention is an anomeric nucleic acid molecule. An anomeric nucleic acid molecule forms specific double-stranded hybrids with complementary RNA in which, contrary to the usual units, the strands run parallel to each other (Gaultier et al. 1987, Nucleic Acids Res. 15:6625-6641). The antisense nucleic acid molecule can also comprise a 2'-o-methyl-ribonucleotide (Inoue et al. 1987, Nucleic Acids Res. 15:6131-6148) or a chimeric RNA-DNA analogue (Inoue et al. 1987, FEBS Lett. 215:327-330).

In still another embodiment, an antisense nucleic acid of the invention is a ribozyme. Ribozymes are catalytic RNA molecules with ribonuclease activity, which are capable of cleaving a single-stranded nucleic acid, such as an mRNA, to which they have a complementary region. Thus, ribozymes (e.g., hammerhead ribozymes (described in Haselhoff & Gerlach 1988, Nature 334:585-591)) can be used to catalytically cleave LMP mRNA transcripts to thereby inhibit translation of LMP mRNA. A ribozyme having specificity for a LMP-encoding nucleic acid can be designed based upon the nucleotide sequence of a LMP cDNA disclosed herein (i.e., Bn01 in Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35) or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in a LMP-encoding mRNA (see, e.g., Cech et al., U.S. Patent No. 4,987,071 and Cech et al., U.S. Patent No. 5,116,742). Alternatively, LMP mRNA can be used to select a catalytic RNA having a specific ribonuclease activity from a pool of RNA molecules (see, e.g., Bartel, D. & Szostak J.W. 1993, Science 261:1411-1418).

Alternatively, LMP gene expression can be inhibited by targeting nucleotide sequences complementary to the regulatory region of a LMP nucleotide sequence (e.g., a LMP promoter and/or enhancers) to form triple helical structures that prevent transcription of a LMP gene in target cells (See generally, Helene C. 1991, Anticancer Drug Des. 6:569-84; Helene C. et al. 1992, Ann. N.Y. Acad. Sci. 660:27-36; and Maher, L.J. 1992, Bioassays 14:807-15).

In still another embodiment, microRNA technology can be used (Bartel D., Cell, 116:281-297, 2004). A MicroRNA precursor can be engineered to target and down-regulate the expression of a gene-of-interest. The precursor can be predominantly expressed in seeds or in other tissues as well. miRNAs (∼ 21 to 25 nt) arise from larger precursors with a stem loop structure that are transcribed from non-protein-coding genes. miRNA targets a specific mRNA to suppress gene expression at post-transcriptional level (*i*.*e*. degrades mRNA) or at translational level (*i*.*e*. inhibits protein synthesis).

Another aspect of the invention pertains to vectors, preferably expression vectors, containing a nucleic acid encoding a LMP (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence and both sequences are fused to each other so that each fulfills its proposed function (e.g., in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; Gene Expression Technology: Methods in Enzymology 185, Academic Press, San Diego, CA (1990) or see: Gruber and Crosby, in: Methods in Plant Molecular Biology and Biotechnolgy, CRC Press, Boca Raton, Florida, eds.: Glick & Thompson, Chapter 7, 89-108 including the references therein. Regulatory sequences include those that direct constitutive expression of a nucleotide sequence in many types of host cell and those that direct expression of the nucleotide sequence only in certain host cells or under certain conditions. It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., LMPs, mutant forms of LMPs, fusion proteins, etc.).

The recombinant expression vectors of the invention can be designed for expression of LMPs in prokaryotic or eukaryotic cells. For example, LMP genes can be expressed in bacterial cells, insect cells (using baculovirus expression vectors), yeast and other fungal cells (see Romanos M.A. et al. 1992, Foreign gene expression in yeast: a review, Yeast 8:423-488; van den Hondel, C.A.M.J.J. et al. 1991, Heterologous gene expression in filamentous fungi, in: More Gene Manipulations in Fungi, Bennet & Lasure, eds., p. 396-428:Academic Press: an Diego; and van den Hondel & Punt 1991, Gene transfer systems and vector development for filamentous fungi, in: Applied Molecular Genetics of Fungi, Peberdy et al., eds., p. 1-28, Cambridge University Press: Cambridge), algae (Falciatore et al. 1999, Marine Biotechnology 1:239-251), ciliates of the types: Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella, and Stylonychia, especially of the genus Stylonychia lemnae with vectors following a transformation method as described in WO 98/01572 and multicellular plant cells (see Schmidt & Willmitzer 1988, High efficiency Agrobacterium tumefaciens-mediated transformation of Arabidopsis thaliana leaf and cotyledon plants, Plant Cell Rep.:583-586); Plant Molecular Biology and Biotechnology, C Press, Boca Raton, Florida, chapter 6/7, S.71-119 (1993); White, Jenes et al., Techniques for Gene Transfer, in: Transgenic Plants, Vol. 1, Engineering and Utilization, eds.: Kung and Wu, Academic Press 1993, 128-43; Potrykus 1991, Annu. Rev. Plant Physiol. Plant Mol. Biol. 42:205-225 (and references cited therein) or mammalian cells. Suitable host cells are discussed further in Goeddel, Gene Expression Technology: Methods in Enzymology 185, Academic Press, San Diego, CA 1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve one or more of the following purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith & Johnson 1988, Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. In one embodiment, the coding sequence of the LMP is cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. Recombinant LMP unfused to GST can be recovered by cleavage of the fusion protein with thrombin.

Examples of suitable inducible non-fusion *E. coli* expression vectors include pTrc (Amann et al. 1988, Gene 69:301-315) and pET 11d (Studier et al. 1990, Gene Expression Technology: Methods in Enzymology 185, Academic Press, San Diego, California 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21 (DE3) or HMS174 (DE3) from a resident prophage harboring a T7 gn1 gene under the transcriptional control of the lacUV 5 promoter.

One strategy to maximize recombinant protein expression is to express the protein in a host bacteria with an impaired capacity to proteolytically cleave the recombinant protein (Gottesman S. 1990, Gene Expression Technology: Methods in Enzymology 185:119-128, Academic Press, San Diego, California). Another strategy is to alter the nucleic acid sequence of the nucleic acid to be inserted into an expression vector so that the individual codons for each amino acid are those preferentially utilized in the bacterium chosen for expression (Wada et al. 1992, Nucleic Acids Res. 20:2111-2118). Such alteration of nucleic acid sequences of the invention can be carried out by standard DNA synthesis techniques.

In another embodiment, the LMP expression vector is a yeast expression vector. Examples of vectors for expression in yeast *S. cerevisiae* include pYepSecl (Baldari et al. 1987, Embo J. 6:229-234), pMFa (Kurjan & Herskowitz 1982, Cell 30:933-943), pJRY88 (Schultz et al. 1987, Gene 54:113-123), and pYES2 (Invitrogen Corporation, San Diego, CA). Vectors and methods for the construction of vectors appropriate for use in other fungi, such as the filamentous fungi, include those detailed in: van den Hondel & Punt 1991, "Gene transfer systems and vector development for filamentous fungi, in: Applied Molecular Genetics of Fungi, Peberdy et al., eds., p. 1-28, Cambridge University Press: Cambridge.

Alternatively, the LMPs of the invention can be expressed in insect cells using baculovirus expression vectors. Baculovirus vectors available for expression of proteins in cultured insect cells (e.g., Sf 9 cells) include the pAc series (Smith et al. 1983, Mol. Cell Biol. 3:2156-2165) and the pVL series (Lucklow & Summers 1989, Virology 170:31-39).

In yet another embodiment, a nucleic acid of the invention is expressed in mammalian cells using a mammalian expression vector. Examples of mammalian expression vectors include pCDM8 (Seed 1987, Nature 329:840) and pMT2PC (Kaufman et al. 1987, EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, Fritsh and Maniatis, Molecular Cloning: A Laboratory Manual. 2nd, ed., Cold Spring Harbor Laboratory, Cold Spring Harbor Laboratory Press, Cold Spring Harbor, NY, 1989.

In another embodiment, the LMPs of the invention may be expressed in uni-cellular plant cells (such as algae, see Falciatore et al. (1999, Marine Biotechnology 1:239-251 and references therein) and plant cells from higher plants (e.g., the spermatophytes, such as crop plants). Examples of plant expression vectors include those detailed in: Becker, Kemper, Schell and Masterson (1992, "New plant binary vectors with selectable markers located proximal to the left border", Plant Mol. Biol. 20:1195-1197) and Bevan (1984, "Binary Agrobacterium vectors for plant transformation, Nucleic Acids Res. 12:8711-8721; Vectors for Gene Transfer in Higher Plants; in: Transgenic Plants, Vol. 1, Engineering and Utilization, eds.: Kung und R. Wu, Academic Press, 1993, S. 15-38).

A plant expression cassette preferably contains regulatory sequences capable to drive gene expression in plant cells and which are operably linked so that each sequence can fulfil its function such as termination of transcription, including polyadenylation signals. Preferred polyadenylation signals are those originating from *Agrobacterium tumefaciens* t-DNA such as the gene 3 known as octopine synthase of the Ti-plasmid pTiACH5 (Gielen et al. 1984, EMBO J. 3:835) or functional equivalents thereof but also all other terminators functionally active in plants are suitable.

As plant gene expression is very often not limited on transcriptional levels a plant expression cassette preferably contains other operably linked sequences like translational enhancers such as the overdrive-sequence containing the 5'-untranslated leader sequence from tobacco mosaic virus enhancing the protein per RNA ratio (Gallie et al. 1987, Nucleic Acids Res. 15:8693-8711).

Plant gene expression has to be operably linked to an appropriate promoter conferring gene expression in a timely, cell or tissue specific manner. Preferred are promoters driving constitutive expression (Benfey et al. 1989, EMBO J. 8:2195-2202) like those derived from plant viruses like the 35S CAMV (Franck et al. 1980, Cell 21:285-294), the 19S CaMV (see also US 5,352,605 and WO 84/02913) or plant promoters like those from Rubisco small subunit described in US 4,962,028. Even more preferred are seed-specific promoters driving expression of LMP proteins during all or selected stages of seed development. Seed-specific plant promoters are known to those of ordinary skill in the art and are identified and characterized using seed-specific mRNA libraries and expression profiling techniques. Seed-specific promoters include the napin-gene promoter from rapeseed (US 5,608,152), the USP-promoter from *Vicia faba* (Baeumlein et al. 1991, Mol. Gen. Genetics 225:459-67), the oleosin-promoter from Arabidopsis (WO 98/45461), the phaseolin-promoter from *Phaseolus vulgaris* (US 5,504,200), the Bce4-promoter from *Brassica* (W09113980) or the legumin B4 promoter (LeB4; Baeumlein et al. 1992, Plant J. 2:233-239) as well as promoters conferring seed specific expression in monocot plants like maize, barley, wheat, rye, rice etc. Suitable promoters to note are the Ipt2 or Ipt1-gene promoter from barley (WO 95/15389 and WO 95/23230) or those described in WO 99/16890 (promoters from the barley hordein-gene, the rice glutelin gene, the rice oryzin gene, the rice prolamin gene, the wheat gliadin gene, wheat glutelin gene, the maize zein gene, the oat glutelin gene, the Sorghum kasirin-gene, and the rye secalin gene).

Plant gene expression can also be facilitated via an inducible promoter (for a review see Gatz 1997, Annu. Rev. Plant Physiol. Plant Mol. Biol. 48:89-108). Chemically inducible promoters are especially suitable if gene expression is desired in a time specific manner. Examples for such promoters are a salicylic acid inducible promoter (WO 95/19443), a tetracycline inducible promoter (Gatz et al. 1992, Plant J. 2:397-404) and an ethanol inducible promoter (WO 93/21334).

Promoters responding to biotic or abiotic stress conditions are also suitable promoters such as the pathogen inducible PRP1-gene promoter (Ward et al., 1993, Plant. Mol. Biol. 22:361-366), the heat inducible hsp80-promoter from tomato (US 5,187,267), cold inducible alpha-amylase promoter from potato (WO 96/12814) or the wound-inducible pinll-promoter (EP 375091).

Other preferred sequences for use in plant gene expression cassettes are targeting-sequences necessary to direct the gene-product in its appropriate cell compartment (for review see Kermode 1996, Crit. Rev. Plant Sci. 15:285-423 and references cited therein) such as the vacuole, the nucleus, all types of plastids like amyloplasts, chloroplasts, chromoplasts, the extracellular space, mitochondria, the endoplasmic reticulum, oil bodies, peroxisomes and other compartments of plant cells. Also especially suited are promoters that confer plastid-specific gene expression, as plastids are the compartment where precursors and some end products of lipid biosynthesis are synthesized. Suitable promoters such as the viral RNA-polymerase promoter are described in WO 95/16783 and WO 97/06250 and the clpP-promoter from *Arabidopsis* described in WO 99/46394.

The invention further provides a recombinant expression vector comprising a DNA molecule of the invention cloned into the expression vector in an antisense orientation. That is, the DNA molecule is operatively linked to a regulatory sequence in a manner that allows for expression (by transcription of the DNA molecule) of an RNA molecule that is antisense to LMP mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub et al. (1986, Antisense RNA as a molecular tool for genetic analysis, Reviews - Trends in Genetics, Vol. 1) and Mol et al. (1990, FEBS Lett. 268:427-430).

Another aspect of the invention pertains to host cells into which a recombinant expression vector of the invention has been introduced. The terms "host cell" and "recombinant host cell" are used interchangeably herein. It is to be understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic or eukaryotic cell. For example, a LMP can be expressed in bacterial cells, insect cells, fungal cells, mammalian cells (such as Chinese hamster ovary cells (CHO) or COS cells), algae, ciliates or plant cells. Other suitable host cells are known to those skilled in the art.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, natural competence, chemical-mediated transfer, or electroporation. Suitable methods for transforming or transfecting host cells including plant cells can be found in Sambrook et al. (1989, Molecular Cloning: A Laboratory Manual. 2nd, ed., Cold Spring Harbor Laboratory, Cold Spring Harbor Laboratory Press, Cold Spring Harbor, NY) and other laboratory manuals such as Methods in Molecular Biology 1995, Vol. 44, Agrobacterium protocols, ed: Gartland and Davey, Humana Press, Totowa, New Jersey.

For stable transfection of mammalian and plant cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those that confer resistance to drugs, such as G418, hygromycin, kanamycin and methotrexate or in plants that confer resistance towards an herbicide such as glyphosate or glufosinate. A nucleic acid encoding a selectable marker can be introduced into a host cell on the same vector as that encoding a LMP or can be introduced on a separate vector. Cells stably transfected with the introduced nucleic acid can be identified by, for example, drug selection (e.g., cells that have incorporated the selectable marker gene will survive, while the other cells die).

To create a homologous recombinant microorganism, a vector is prepared which contains at least a portion of a LMP gene into which a deletion, addition or substitution has been introduced to thereby alter, e.g., functionally disrupt, the LMP gene. Preferably, this LMP gene is an *Arabidopsis thaliana, Glycine max, Oryza sativa*, *Zea mays, Linum usitatissimum, Hordeum vulgare* or *Triticum aestivum* LMP gene, but it can be a homologue from a related plant or even from a mammalian, yeast, or insect source. In a preferred embodiment, the vector is designed such that, upon homologous recombination, the endogenous LMP gene is functionally disrupted (i.e., no longer encodes a functional protein; also referred to as a knock-out vector). Alternatively, the vector can be designed such that, upon homologous recombination, the endogenous LMP gene is mutated or otherwise altered but still encodes functional protein (e.g., the upstream regulatory region can be altered to thereby alter the expression of the endogenous LMP). To create a point mutation via homologous recombination, DNA-RNA hybrids can be used in a technique known as chimeraplasty (Cole-Strauss et al. 1999, Nucleic Acids Res. 27:1323-1330 and Kmiec 1999, American Scientist 87:240-247). Homologous recombination procedures in *Arabidopsis thaliana* or other crops are also weli known in the art and are contemplated for use herein.

In a homologous recombination vector, the altered portion of the LMP gene is flanked at its 5' and 3' ends by additional nucleic acid of the LMP gene to allow for homologous recombination to occur between the exogenous LMP gene carried by the vector and an endogenous LMP gene in a microorganism or plant. The additional flanking LMP nucleic acid is of sufficient length for successful homologous recombination with the endogenous gene. Typically, several hundreds of base pairs up to kilobases of flanking DNA (both at the 5' and 3' ends) are included in the vector (see e.g., Thomas & Capecchi 1987, Cell 51:503, for a description of homologous recombination vectors). The vector is introduced into a microorganism or plant cell (e.g., via polyethyleneglycol mediated DNA). Cells in which the introduced LMP gene has homologously recombined with the endogenous LMP gene are selected using art-known techniques.

In another embodiment, recombinant microorganisms can be produced which contain selected systems, which allow for regulated expression of the introduced gene. For example, inclusion of a LMP gene on a vector placing it under control of the lac operon permits expression of the LMP gene only in the presence of IPTG. Such regulatory systems are well known in the art.

A host cell of the invention, such as a prokaryotic or eukaryotic host cell in culture can be used to produce (i.e., express) a LMP. Accordingly, the invention further provides methods for producing LMPs using the host cells of the invention. In one embodiment, the method comprises culturing a host cell of the invention (into which a recombinant expression vector encoding a LMP has been introduced, or which contains a wild-type or altered LMP gene in it's genome) in a suitable medium until LMP is produced. In another embodiment, the method further comprises isolating LMPs from the medium or the host cell.

Another aspect of the invention pertains to isolated LMPs, and biologically active portions thereof. An "isolated" or "purified" protein or biologically active portion thereof is substantially free of cellular material when produced by recombinant DNA techniques, or chemical precursors or other chemicals when chemically synthesized. The language "substantially free of cellular material" includes preparations of LMP in which the protein is separated from cellular components of the cells in which it is naturally or recombinantly produced. In one embodiment, the language "substantially free of cellular material" includes preparations of LMP having less than about 30% (by dry weight) of non-LMP (also referred to herein as a "contaminating protein"), more preferably less than about 20% of non-LMP, still more preferably less than about 10% of non-LMP, and most preferably less than about 5% non-LMP. When the LMP or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations of LMP in which the protein is separated from chemical precursors or other chemicals that are involved in the synthesis of the protein. In one embodiment, the language "substantially free of chemical precursors or other chemicals" includes preparations of LMP having less than about 30% (by dry weight) of chemical precursors or non-LMP chemicals, more preferably less than about 20% chemical precursors or non-LMP chemicals, still more preferably less than about 10% chemical precursors or non-LMP chemicals, and most preferably less than about 5% chemical precursors or non-LMP chemicals. In preferred embodiments, isolated proteins or biologically active portions thereof lack contaminating proteins from the same organism from which the LMP is derived. Typically, such proteins are produced by recombinant expression of, for example, an *Arabidopsis thaliana, Glycine max, Oryza sativa*, *Zea mays, Linum usitatissimum, Hordeum vulgare* or *Triticum aestivum* LMP in other plants than *Arabidopsis thaliana, Glycine max, Oryza sativa*, Zea *mays, Linum usitatissimum, Hordeum vulgare* or *Triticum aestivum* or microorganisms, algae or fungi.

An isolated LMP or a portion thereof of the invention can participate in the metabolism of compounds necessary for the production of seed storage compounds in *Arabidopsis thaliana, Glycine max, Oryza sativa, Zea mays, Linum usitatissimum, Hordeum vulgare* or *Triticum aestivum* or of cellular membranes, or has one or more of the activities set forth in Table 3. In preferred embodiments, the protein or portion thereof comprises an amino acid sequence which is sufficiently homologous to an amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35 such that the protein or portion thereof maintains the ability to participate in the metabolism of compounds necessary for the construction of cellular membranes in *Arabidopsis thaliana, Glycine max, Oryza sativa, Zea mays, Linum usitatissimum, Hordeum vulgare* or *Triticum aestivum,* or in the transport of molecules across these membranes. The portion of the protein is preferably a biologically active portion as described herein. In another preferred embodiment, a LMP of the invention has an amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,. In yet another preferred embodiment, the LMP has an amino acid sequence which is encoded by a nucleotide sequence which hybridizes, e.g., hybridizes under stringent conditions, to a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35,. In still another preferred embodiment, the LMP has an amino acid sequence which is encoded by a nucleotide sequence that is at least about 50-60%, preferably at least about 60-70%, more preferably at least about 70-80%, 80-90%, 90-95%, also preferable at least about 85%, 86 %, 87 %, 88 %, 89 %, 90 %, 91 %, 92 %, 93 %, 94 %, 95 % homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and most preferably at least about 96%, 97%, 98%, or 99% homologous to one of the sequences encoded by a nucleic acid of Appendix A in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35. The polypeptide sequence homology is preferably determined using Align X (Aug. 22, 2003) of Vector NTI Suite 9.0 with following parameters: Gap opening penalty: 10; Gap extension penalty: 0.05; Gap separation penalty range: 8; % identity for alignment delay: 40.

The preferred LMPs of the present invention also preferably possess at least one of the LMP activities described herein. For example, a preferred LMP of the present invention includes an amino acid sequence encoded by a nucleotide sequence which hybridizes, e.g., hybridizes under stringent conditions, to a nucleotide sequence of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and which can participate in the metabolism of compounds necessary for the construction of cellular membranes in *Arabidopsis thaliana, Glycine max, Oryza sativa, Zea mays, Linum usitatissimum, Hordeum vulgare* or *Triticum aestivum,* or in the transport of molecules across these membranes, or which has one or more of the activities set forth in Table 3.

In other embodiments, the LMP is substantially homologous to an amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and retains the functional activity of the protein of one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, yet differs in amino acid sequence due to natural variation or mutagenesis, as described in detail above. Accordingly, in another embodiment, the LMP is a protein which comprises an amino acid sequence which is at least about 50-60%, preferably at least about 60-70%, and more preferably at least about 70-80, 80-90, 90-95%, also preferable at least about 85%, 86 %, 87 %, 88 %, 89 %, 90 %, 91 %, 92 %, 93 %, 94 % or 95 % homologous to one of the sequences encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35, and most preferably at least about 96%, 97%, 98%, or 99% homologous to one of the sequences encoded by a nucleic acid to an entire amino acid sequence and which has at least one of the LMP activities described herein.. The polypeptide sequence homology is preferably determined using Align X (Aug. 22, 2003) of Vector NTI Suite 9.0 with following parameters: Gap opening penalty: 10; Gap extension penalty: 0.05; Gap separation penalty range: 8; % identity for alignment delay: 40. In another embodiment, the invention pertains to a full *Arabidopsis thaliana, Glycine max, Oryza sativa* or *Triticum aestivum* protein which is substantially homologous to an entire amino acid sequence encoded by a nucleic acid of Appendix A, in a preferred embodiment as depicted in SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO:25, SEQ ID NO: 27, SEQ ID NO: 29, SEQ ID NO: 31, SEQ ID NO:33 or SEQ ID NO: 35.

Dominant negative mutations or trans-dominant suppression can be used to reduce the activity of a LMP in transgenics seeds in order to change the levels of seed storage compounds. To achieve this a mutation that abolishes the activity of the LMP is created and the inactive non-functional LMP gene is overexpressed in the transgenic plant. The inactive trans-dominant LMP protein competes with the active endogenous LMP protein for substrate or interactions with other proteins and dilutes out the activity of the active LMP. In this way the biological activity of the LMP is reduced without actually modifying the expression of the endogenous LMP gene. This strategy was used by Pontier et al to modulate the activity of plant transcription factors (Pontier D, Miao ZH, Lam E, Plant J 2001 Sep. 27(6): 529-38, Trans-dominant suppression of plant TGA factors reveals their negative and positive roles in plant defense responses).

Homologues of the LMP can be generated by mutagenesis, e.g., discrete point mutation or truncation of the LMP. As used herein, the term "homologue" refers to a variant form of the LMP that acts as an agonist or antagonist of the activity of the LMP. An agonist of the LMP can retain substantially the same, or a subset, of the biological activities of the LMP. An antagonist of the LMP can inhibit one or more of the activities of the naturally occurring form of the LMP, by, for example, competitively binding to a downstream or upstream member of the cell membrane component metabolic cascade which includes the LMP, or by binding to a LMP which mediates transport of compounds across such membranes, thereby preventing translocation from taking place.

In an alternative embodiment, homologues of the LMP can be identified by screening combinatorial libraries of mutants, e.g., truncation mutants, of the LMP for LMP agonist or antagonist activity. In one embodiment, a variegated library of LMP variants is generated by combinatorial mutagenesis at the nucleic acid level and is encoded by a variegated gene library. A variegated library of LMP variants can be produced by, for example, enzymatically ligating a mixture of synthetic oligonucleotides into gene sequences such that a degenerate set of potential LMP sequences is expressible as individual polypeptides, or alternatively, as a set of larger fusion proteins (e.g., for phage display) containing the set of LMP sequences therein. There are a variety of methods that can be used to produce libraries of potential LMP homologues from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential LMP sequences. Methods for synthesizing degenerate oligonucleotides are known in the art (see, e.g., Narang 1983, Tetrahedron 39:3; Itakura et al. 1984, Annu. Rev. Biochem. 53:323; Itakura et al. 1984, Science 198:1056; Ike et al. 1983, Nucleic Acids Res. 11:477).

In addition, libraries of fragments of the LMP coding sequences can be used to generate a variegated population of LMP fragments for screening and subsequent selection of homologues of a LMP. In one embodiment, a library of coding sequence fragments can be generated by treating a double stranded PCR fragment of a LMP coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the LMP.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of LMP homologues. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify LMP homologues (Arkin & Yourvan 1992, Proc. Natl. Acad. Sci. USA 89:7811-7815; Delgrave et al. 1993, Protein Engineering 6:327-331).

In another embodiment, cell based assays can be exploited to analyze a variegated LMP library, using methods well known in the art.

The nucleic acid molecules, proteins, protein homologues, fusion proteins, primers, vectors, and host cells described herein can be used in one or more of the following methods: identification of *Arabidopsis thaliana, Glycine max, Oryza sativa, Zea mays, Linum usitatissimum, Hordeum vulgare* or *Triticum aestivum* and related organisms; mapping of genomes of organisms related to *Arabidopsis thaliana, Glycine max, Oryza sativa, Zea mays, Linum usitatissimum, Hordeum vulgare* or *Triticum aestivum*; identification and localization of *Arabidopsis thaliana, Glycine max, Oryza sativa,* Zea *mays, Linum usitatissimum, Hordeum vulgare* or *Triticum aestivum* sequences of interest; evolutionary studies; determination of LMP regions required for function; modulation of a LMP activity; modulation of the metabolism of one or more cell functions; modulation of the transmembrane transport of one or more compounds; and modulation of seed storage compound accumulation.

The plant *Arabidopsis thaliana* represents one member of higher (or seed) plants. It is related to other plants such as *Brassica napus, Glycine max, Oryza sativa*, *Zea mays, Linum usitatissimum, Hordeum vulgare* or *Triticum aestivum* which require light to drive photosynthesis and growth. Plants like *Arabidopsis thaliana, Brassica napus, Glycine max, Oryza sativa, Zea mays, Linum usitatissimum, Hordeum vulgare* or *Triticum aestivum* share a high degree of homology on the DNA sequence and polypeptide level, allowing the use of heterologous screening of DNA molecules with probes evolving from other plants or organisms, thus enabling the derivation of a consensus sequence suitable for heterologous screening or functional annotation and prediction of gene functions in third species. The ability to identify such functions can therefore have significant relevance, e.g., prediction of substrate specificity of enzymes. Further, these nucleic acid molecules may serve as reference points for the mapping of Arabidopsis genomes, or of genomes of related organisms.

The LMP nucleic acid molecules of the invention have a variety of uses. First, the nucleic acid and protein molecules of the invention may serve as markers for specific regions of the genome. This has utility not only in the mapping of the genome, but also for functional studies of *Arabidopsis thaliana, Brassica napus, Glycine max, Oryza sativa, Zea mays, Linum usitatissimum, Hordeum vulgare* or *Triticum aestivum* proteins. For example, to identify the region of the genome to which a particular *Arabidopsis thaliana, Brassica napus, Glycine max, Oryza sativa, Zea mays, Linum usitatissimum, Hordeum vulgare* or *Triticum aestivum* DNA-binding protein binds, the *Arabidopsis thaliana, Brassica napus, Glycine max, Oryza sativa, Zea mays, Linum usitatissimum, Hordeum vulgare* or *Triticum aestivum* genome could be digested, and the fragments incubated with the DNA-binding protein. Those which bind the protein may be additionally probed with the nucleic acid molecules of the invention, preferably with readily detectable labels; binding of such a nucleic acid molecule to the genome fragment enables the localization of the fragment to the genome map of *Arabidopsis thaliana, Brassica napus, Glycine max, Oryza sativa, Zea mays, Linum usitatissimum, Hordeum vulgare* or *Triticum aestivum*, and, when performed multiple times with different enzymes, facilitates a rapid determination of the nucleic acid sequence to which the protein binds. Further, the nucleic acid molecules of the invention may be sufficiently homologous to the sequences of related species such that these nucleic acid molecules may serve as markers for the construction of a genomic map in related plants.

The LMP nucleic acid molecules of the invention are also useful for evolutionary and protein structural studies. The metabolic and transport processes in which the molecules of the invention participate are utilized by a wide variety of prokaryotic and eukaryotic cells; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Manipulation of the LMP nucleic acid molecules of the invention may result in the production of LMPs having functional differences from the wild-type LMPs. These proteins may be improved in efficiency or activity, may be present in greater numbers in the cell than is usual, or may be decreased in efficiency or activity.

There are a number of mechanisms by which the alteration of a LMP of the invention may directly affect the accumulation and/or composition of seed storage compounds. In the case of plants expressing LMPs, increased transport can lead to altered accumulation of compounds and/or solute partitioning within the plant tissue and organs which ultimately could be used to affect the accumulation of one or more seed storage compounds during seed development. An example is provided by Mitsukawa et al. (1997, Proc. Natl. Acad. Sci. USA 94:7098-7102), where overexpression of an Arabidopsis high-affinity phosphate transporter gene in tobacco cultured cells enhanced cell growth under phosphate-limited conditions. Phosphate availability also affects significantly the production of sugars and metabolic intermediates (Hurry et al. 2000, Plant J. 24:383-396) and the lipid composition in leaves and roots (Härtel et al. 2000, Proc. Natl. Acad. Sci. USA 97:10649-10654). Likewise, the activity of the plant ACCase has been demonstrated to be regulated by phosphorylation (Savage & Ohlrogge 1999, Plant J. 18:521-527) and alterations in the activity of the kinases and phosphatases (LMPs) that act on the ACCase could lead to increased or decreased levels of seed lipid accumulation. Moreover, the presence of lipid kinase activities in chloroplast envelope membranes suggests that signal transduction pathways and/or membrane protein regulation occur in envelopes (see, e.g., Müller et al. 2000, J. Biol. Chem. 275:19475-19481 and literature cited therein). The *ABI1* and *ABI2* genes encode two protein serine/threonine phosphatases 2C, which are regulators in abscisic acid signaling pathway, and thereby in early and late seed development (e.g. Merlot et al. 2001, Plant J. 25:295-303). For more examples see also the section 'background of the invention'.

The present invention also provides antibodies that specifically bind to an LMP-polypeptide, or a portion thereof, as encoded by a nucleic acid disclosed herein or as described herein.

Antibodies can be made by many well-known methods (see, e.g. Harlow and Lane, "Antibodies; A Laboratory Manual" Cold Spring Harbor Laboratory, Cold Spring Harbor, New York, 1988). Briefly, purified antigen can be injected into an animal in an amount and in intervals sufficient to elicit an immune response. Antibodies can either be purified directly, or spleen cells can be obtained from the animal. The cells can then fused with an immortal cell line and screened for antibody secretion. The antibodies can be used to screen nucleic acid clone libraries for cells secreting the antigen. Those positive clones can then be sequenced (see, for example, Kelly et al. 1992, Bio/Technology 10:163-167; Bebbington et al. 1992, Bio/Technology 10:169-175).

The phrase "selectively binds" with the polypeptide refers to a binding reaction which is determinative of the presence of the protein in a heterogeneous population of proteins and other biologics. Thus, under designated immunoassay conditions, the specified antibodies bound to a particular protein do not bind in a significant amount to other proteins present in the sample. Selective binding to an antibody under such conditions may require an antibody that is selected for its specificity for a particular protein. A variety of immunoassay formats may be used to select antibodies that selectively bind with a particular protein. For example, solid-phase ELISA immuno-assays are routinely used to select antibodies selectively immunoreactive with a protein. See Harlow and Lane "Antibodies, A Laboratory Manual" Cold Spring Harbor Publications, New York (1988), for a description of immunoassay formats and conditions that could be used to determine selective binding.

In some instances, it is desirable to prepare monoclonal antibodies from various hosts. A description of techniques for preparing such monoclonal antibodies may be found in Stites et al., editors, "Basic and Clinical Immunology," (Lange Medical Publications, Los Altos, Calif., Fourth Edition) and references cited therein, and in Harlow and Lane ("Antibodies, A Laboratory Manual" Cold Spring Harbor Publications, New York, 1988).

Throughout this application, various publications are referenced. The disclosures of all of these publications and those references cited within those publications in their entireties are hereby incorporated by reference into this application in order to more fully describe the state of the art to which this invention pertains.

It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and Examples be considered as exemplary only, with a true scope and spirit of the invention being indicated by the claims included herein.

### EXAMPLES

### Example 1: General Processes

### General Cloning Processes:

Cloning processes such as, for example, restriction cleavages, agarose gel electrophoresis, purification of DNA fragments, transfer of nucleic acids to nitrocellulose and nylon membranes, linkage of DNA fragments, transformation of Escherichia coli and yeast cells, growth of bacteria and sequence analysis of recombinant DNA were carried out as described in Sambrook et al. (1989, Cold Spring Harbor Laboratory Press: ISBN 0-87969-309-6) or Kaiser, Michaelis and Mitchell (1994, "Methods in Yeast Genetics", Cold Spring Harbor Laboratory Press: ISBN 0-87969-451-3).

### b) Chemicals:

The chemicals used were obtained, if not mentioned otherwise in the text, in p.a. quality from the companies Fluka (Neu-Ulm), Merck (Darmstadt), Roth (Karlsruhe), Serva (Heidelberg) and Sigma (Deisenhofen). Solutions were prepared using purified, pyrogen-free water, designated as H2O in the following text, from a Milli-Q water system water purification plant (Millipore, Eschbom). Restriction endonucleases, DNA-modifying enzymes and molecular biology kits were obtained from the companies AGS (Heidelberg), Amersham (Braunschweig), Biometra (Göttingen), Boehringer (Mannheim), Genomed (Bad Oeynnhausen), New England Biolabs (Schwalbach/ Taunus), Novagen (Madison, Wisconsin, USA), Perkin-Elmer (Weiterstadt), Pharmacia (Freiburg), Qiagen (Hilden) and Stratagene (Amsterdam, Netherlands). They were used, if not mentioned otherwise, according to the manufacturer's instructions.

### c) Plant Material and Growth:

### Arabidopsis plants

For this study, root material, leaves, siliques and seeds of wild-type and *fad2* mutant (as described in Miquel & Browse, 1992, J Biol Chem 267: 1502-1509) plants of *Arabidopsis thaliana* were used. Wild type and *fad2* mutant Arabidopsis seeds were preincubated for three days in the dark at 4°C before placing them into an incubator (AR-75, Percival Scientific, Boone, IA) at a photon flux density of 60-80 µmol m⁻² s⁻¹ and a light period of 16 hours (22°C), and a dark period of 8 hours (18°C). All plants were started on half-strength MS medium (Murashige & Skoog, 1962, Physiol. Plant. 15, 473-497), pH 6.2, 2% sucrose and 1.2% agar. Seeds were sterilized for 20 minutes in 20% bleach 0.5% triton X100 and rinsed 6 times with excess sterile water. Plants were either grown as described above or on soil under standard conditions as described in Klaus et ai. (2002, Plant Physioi. 128:885-895).

### Glycine max

*Glycine max* cv. Resnick was used for this study to create cDNA libraries. Seed, seed pod, flower, leaf, stem and root tissues were collected from plants that were in some cases dark-, salt-, heat- and drought-treated. In some cases plants have been nematode infected as well. However, this study focused on the use of seed and seed pod tissues for cDNA libraries. Plants were tagged to harvest seeds at the set days after anthesis: 5-15, 15-25, 25-35, & 33-50.

### Oryza sativa

*Oryza sativa* ssp. Japonica cv. Nippon-barre was used for this study to create cDNA libraries. Seed, seed pod, flower, leaf, stem and root tissues were collected from plants that were in some cases dark-, salt-, heat- and drought-treated. This study focused on the use of seed embryo tissues for cDNA libraries. Embryo and endosperm were collected separately in case endosperm tissue might interfere with RNA extraction. Plants have been grown in the greenhouse on Wisconsin soil (has high organic matter) at 85°F under a 14-h photoperiod. Rice embryos were dissected out of the developing seeds.

### Zea mays

*Zea mays* hybrid B73 x Mo17 and B73 inbred (the female inbred parent of the hybrid B73 x Mo17) were used to generate cDNA libraries. Fruit or Seed (Fertilized ovules/ young kernels at stage 1 and 9 d post pollination; kernels at milk stage [R3, early starch production], 23 d post pollination; kernels at early dough stage (R4), developing starch grains and well-formed embryo present, 30 d post pollination of filed-grown plants; very young kernels at blister stage [R2, watery endosperm]; kernels at early dent stage (R5), endosperm becoming firm, 36 d post pollination; B73 inbreds, kernels at 9 and 19 d post pollination), flowers (tassel development: from 6 cm tassel (V10) up to and including anthesis, 44 to 70 dap; ear development: ear shoots from 2 cm (V13) up to and including silking (unpollinated), 51 to 70 dap), leaves/shoot/rosettes (mixed ages, all prior to seed-fill; includes leaves of a) 3-leaf plants(V3), b) 6-leaf plants (V6),and c) an older source leaf (3rd from the ground), just before tassel emergence in the field), stem (located underground of 2 to 5-leaf plants; roots and most leaf tissue removed, 13 to 29 dap of field-grown plants; Stem tissue near the ear at tassel emergence and during seed-fill (milk stage), 56 to 84 dap, field-grown plants) and root tissues (from young to mid-age plants: from seedlings, 6-leaf plants, and 9-leaf plants; 12 to 35 dap) were collected from plants.

### Linum usitatissimum

*Linum usitatissimum* cv 00-44427 and cv 00-44338 (of the Svalöf Weibull collection) was used for this study to create cDNA libraries. Plants have been grown in 2 liter pots with potting soil containing 5 ml Osmocote/liter soil in a cooled greenhouse chamber at 19°C. Material from developing seeds has been collected at 15daa (embryo is in a stage of intensive elongation, filling of about 2/3 of the seed; embryo is green in late torpedo stage), 25 daa (embryo fully elongated and increased in width, whole seed is filled out; embryo is still fully green) and 33 daa (seed is starting to get mature; color of embryo is changing to lighter green and the tip is yellow).

### Hordeum vulgare

*Hordeum vulgare* cv. Morex was used for this study to create cDNA libraries. Plants have been grown in the greenhouse in metromix under a 15-h photoperiod at 23°C during the day period and 18°C during the night period. Grain was at the watery ripe to late milk stage. The mid to upper seedhead primarily was harvested. Seed material was collected 75 days after planting.

### Triticum aestivum

*Triticum aestivum* cv. Galeon was used for this study to create cDNA libraries. Seed, flower, fruits, leaf, stem and root tissues were collected from plants that were in some cases dark-, salt-, heat- and drought-treated. Plants have been grown in the greenhouse in metromix under a 12-h photoperiod at 72°F during the day period and 65°F during the night period.

### Example 2: Total DNA Isolation from Plants

The details for the isolation of total DNA relate to the working up of one gram fresh weight of plant material.

CTAB buffer: 2% (w/v) N-cethyl-N,N,N-trimethylammonium bromide (CTAB); 100 mM Tris HCl pH 8.0; 1.4 M NaCl; 20 mM EDTA. N-Lauryisarcosine buffer: 10% (w/v) N-laurylsarcosine; 100 mM Tris HCl pH 8.0; 20 mM EDTA.

The plant material was triturated under liquid nitrogen in a mortar to give a fine powder and transferred to 2 ml Eppendorf vessels. The frozen plant material was then covered with a layer of 1 ml of decomposition buffer (1 ml CTAB buffer, 100 µl of N-lauryisarcosine buffer, 20 µl of β-mercaptoethanol and 10 µl of proteinase K solution, 10 mg/ml) and incubated at 60°C for one hour with continuous shaking. The homogenate obtained was distributed into two Eppendorf vessels (2 ml) and extracted twice by shaking with the same volume of chloroform/isoamyl alcohol (24:1). For phase separation, centrifugation was carried out at 8000g and RT for 15 min in each case. The DNA was then precipitated at -70°C for 30 min using ice-cold isopropanol. The precipitated DNA was sedimented at 4°C and 10,000 g for 30 min and resuspended in 180 µl of TE buffer (Sambrook et al. 1989, Cold Spring Harbor Laboratory Press: ISBN 0-87969-309-6). For further purification, the DNA was treated with NaCl (1.2 M final concentration) and precipitated again at -70°C for 30 min using twice the volume of absolute ethanol. After a washing step with 70% ethanol, the DNA was dried and subsequently taken up in 50 µl of H₂O + RNAse (50 mg/ml final concentration). The DNA was dissolved overnight at 4°C and the RNAse digestion was subsequently carried out at 37°C for 1 h. Storage of the DNA took place at 4°C.

### Example 3: Isolation of Total RNA and poly-(A)+ RNA from Plants

### Arabidopsis thaliana

For the investigation of transcripts, both total RNA and poly-(A)+ RNA were isolated. RNA is isolated from siliques of Arabidopsis plants according to the following procedure:

### RNA preparation from Arabidopsis seeds - "hot" extraction:

### Buffers, enzymes and solution

2M KCI

Proteinase K

Phenol (for RNA)

Chloroform:Isoamylalchol

(Phenol:choloroform 1:1; pH adjusted for RNA)

4 M LiCl, DEPC-treated

DEPC-treated water

3M NaOAc, pH 5, DEPC-treated

Isopropanol

70% ethanol (made up with DEPC-treated water)

Resuspension buffer:0.5% SDS, 10 mM Tris pH 7.5, 1 mM EDTA made up with

DEPC-treated water as this solution can not be DEPC-treated

Extraction Buffer:

0.2M Na Borate

30 mM EDTA

30 mM EGTA

1 % SDS (250µl of 10% SDS-solution for 2.5ml buffer)

1 % Deoxycholate (25mg for 2,5ml buffer)

2% PVPP (insoluble - 50mg for 2.5ml buffer)

2% PVP 40K (50mg for 2.5ml buffer)

10 mM DTT

100 mM β-Mercaptoethanol (fresh, handle under fume hood - use 35µl of 14.3M solution for 5ml buffer)

### Extraction

Heat extraction buffer up to 80°C. Grind tissue in liquid nitrogen-cooled mortar, transfer tissue powder to 1.5ml tube. Tissue should kept frozen until buffer is added so transfer the sample with pre-cooled spatula and keep the tube in liquid nitrogen all time. Add 350µl preheated extraction buffer (here for 100mg tissue, buffer volume can be as much as 500µl for bigger samples) to tube, vortex and heat tube to 80°C for -1 min. Keep then on ice. Vortex sample, grind additionally with electric mortar.

### Digestion

**Add Proteinase K (0.15mg/100mg tissue), vortex and keep at 37°C for one hour. First Purification**

Add 27µl 2M KCI. Chill on ice for 10 min. Centrifuge at 12.000 rpm for 10 minutes at room temperature. Transfer supernatant to fresh, RNAase-free tube and do one phenol extraction, followed by a chloroform:isoamylalcohol extraction. Add 1 vol. isopropanol to supernatant and chill on ice for 10 min Pellet RNA by centrifugation (7000 rpm for 10 min at RT). Resolve pellet in 1ml 4M LiCl by 10 to 15min vortexing. Pellet RNA by 5min centrifugation.

### Second Purification

Resuspend pellet in 500µl Resuspension buffer. Add 500µl phenol and vortex. Add 250µl chloroform:isoamylalcohol and vortex. Spin for 5 min. and transfer supernatant to fresh tube. Repeat chloform:isoamylalcohol extraction until interface is clear. Transfer supernatant to fresh tube and add 1/10 vol 3M NaOAc, pH 5 and 600µl isopropanol. Keep at -20 for 20 min or longer. Pellet RNA by 10 min centrifugation. Wash pellet once with 70% ethanol. Remove all remaining alcohol before resolving pellet with 15 to 20µl DEPC-water. Determine quantity and quality by measuring the absorbance of a 1:200 dilution at 260 and 280nm. 40µg RNA/ml = 1OD260

RNA from wild-type of Arabidopsis is isolated as described (Hosein, 2001, Plant Mol. Biol. Rep., 19, 65a-65e; Ruuska, S.A., Girke, T., Benning, C., & Ohlrogge, J.B., 2002, Plant Cell, 14, 1191-1206).

The mRNA is prepared from total RNA, using the Amersham Pharmacia Biotech mRNA purification kit, which utilizes oligo(dT)-cellulose columns.

Isolation of Poly-(A)+ RNA was isolated using Dyna BeadsR (Dynal, Oslo, Norway) following the instructions of the manufacturer's protocol. After determination of the concentration of the RNA or of the poly(A)+ RNA, the RNA was precipitated by addition of 1/10 volumes of 3 M sodium acetate pH 4.6 and 2 volumes of ethanol and stored at -70°C.

### Glycine max, Oryza sativa, Zea mays, Linum usitatissimum, Hordeum vulgare and Triticum aestivum

*Glycine max* and *Linum usitatissimum* seeds were separated from pods to create homogeneous materials for seed and seed pod cDNA libraries. Tissues were ground into fine powder under liquid N₂ using a mortar and pestle and transferred to a 50 ml tube. Tissue samples were stored at -80 °C until extractions could be performed.

In the case of *Oryza sativa*, 5K - 10K embryos and endosperm were isolated through dissection. Tissues were placed in small tubes or petri dishes on ice during dissection. Containers were placed on dry ice, then stored at -80°C.

In the case of *Zea mays*, tissues were ground into fine powder under liquid N₂ using a mortar and pestle and transferred to a 50 ml tube. Tissue samples were stored at -80 °C until extractions could be performed.

In the case of *Hordeum vulgare* seed heads were cut (arrox 2 inch sections) to have florets at the indicated stage. All of the awns were trimmed away. The stage chosen was early/mid seed fill. Seed tissue cDNA libraries grains were either in watery ripe or in milk stage.

In the case of *Triticum aestivum,* seed germination samples of Galeon wheat seeds were planted at a depth of 2" in metromix in a 20" x 12" flat. The soil was soaked liberally with water and then watered twice daily. 3-4 days later when the coleopiles were ∼1 cm, the seedlings were washed with water and blotted. To create flower cDNA libraries an equal number of heads are collected at 30%, 60% and 100% head emergence from the sheath on each of two days. There were no anthers showing yet. In order to generate seed tissue cDNA libraries grains were either watery ripe or in milk stage depending on he position of grains in the head; for later seed developmental stages only the seed heads were harvested. For the root libraries, only roots were harvested. Plants had one main stem and three strong tillers. Plants were grown in pots, the medium was washed off and the roots were saved for this sample. Plants were untreated.

Total RNA was extracted from tissues using RNeasy Maxi kit (Qiagen) according to manufacture's protocol and mRNA was processed from total RNA using Oligotex mRNA Purification System kit (Qiagen), also according to manufacture's protocol. mRNA was sent to Hyseq Pharmaceuticals Incorporated (Sunnyville, CA) for further processing of mRNA from each tissue type into cDNA libraries and for use in their proprietary processes in which similar inserts in plasmids are clustered based on hybridization patterns.

### Example 4: cDNA Library Construction

For cDNA library construction, first strand synthesis was achieved using Murine Leukemia Virus reverse transcriptase (Roche, Mannheim, Germany) and oligo-d(T)-primers, second strand synthesis by incubation with DNA polymerase I, Klenow enzyme and RNAseH digestion at 12°C (2 h), 16°C (1 h) and 22°C (1 h). The reaction was stopped by incubation at 65°C (10 min) and subsequently transferred to ice. Double stranded DNA molecules were blunted by T4-DNA-polymerase (Roche, Mannheim) at 37°C (30 min). Nucleotides were removed by phenol/chloroform extraction and Sephadex G50 spin columns. EcoRI adapters (Pharmacia, Freiburg, Germany) were ligated to the cDNA ends by T4-DNA-ligase (Roche, 12°C, overnight) and phosphorylated by incubation with polynucleotide kinase (Roche, 37°C, 30 min). This mixture was subjected to separation on a low melting agarose gel. DNA molecules larger than 300 base pairs were eluted from the gel, phenol extracted, concentrated on Elutip-D-columns (Schleicher and Schuell, Dassel, Germany) and were ligated to vector arms and packed into lambda ZAPII phages or lambda ZAP-Express phages using the Gigapack Gold Kit (Stratagene, Amsterdam, Netherlands) using material and following the instructions of the manufacturer.

*Glycine max, Oryza sativa, Zea mays, Linum usitatissimum, Hordeum vulgare* and *Triticum aestivum* cDNA libraries were generated at Hyseq Pharmaceuticals Incorporated (Sunnyville, CA) No amplification steps were used in the library production to retain expression information. Hyseq's genomic approach involves grouping the genes into clusters and then sequencing representative members from each cluster. cDNA libraries were generated from oligo dT column purified mRNA. Colonies from transformation of the cDNA library into *E*.coli were randomly picked and the cDNA insert were amplified by PCR and spotted on nylon membranes. A set of ³³⁻P radiolabeled oligonucleotides were hybridized to the clones and the resulting hybridization pattern determined to which cluster a particular clone belonged. cDNA clones and their DNA sequences were obtained for use in overexpression in transgenic plants and in other molecular biology processes described herein.

### Example 5: Identification of LMP Genes of Interest that Are FAD2-like Arabidopsis thaliana

Arabidopsis wild type and the Arabidopsis *fad2* mutant were used to identify LMP-encoding genes. The *FAD2* gene has been cloned and described (J Okuley, J Lightner, K Feldmann, N Yadav, E Lark, & J Browse, Plant Cell 6:147-158, 1994). *FAD2* encodes the microsomal fatty acid _{□}6-desaturase enzyme that inserts a double bond at the _{□}12 position of oleic acid (C18:1^{□9}) bound to phosphatidylcholine to produce linoleic acid (C18:2^{□9, 12}) and, for this reason, is also referred to as a 12-desaturase. *FAD2* is present as a single gene in the Arabidopsis genome.

### Glycine max, Oryza sativa, Zea mays, Linum usitatissimum, Hordeum vulgare and Triticum aestivum

This example illustrates how cDNA clones encoding FAD2-like polypeptide of *Glycine max, Oryza sativa, Zea mays, Linum usitatissimum, Hordeum vulgare* and *Triticum aestivum* were identified and isolated.

In order to identify *FAD2*-like genes in propriety databases, a similarity analysis using BLAST software (Basic Local Alignment Search Tool, version 2.2.6, Altschul et al., 1997, Nucleic Acid Res. 25: 3389-3402) was carry out. The default settings were used except for e-value cut-off (1e-10) and all protein searches were done using the BLOSUM62 matrix. The amino acid sequence of the *Arabidopsis* FAD2 polypeptide was used as a query to search and align DNA databases from *Glycine max, Oryza sativa, Zea mays, Linum usitatissimum, Hordeum vulgare* and *Triticum aestivum* that were translated in all six reading frames, using the TBLASTN algorithm. Such similarity analysis of the BPS in-house databases resulted in the identification of numerous ESTs and cDNA contigs.

RNA expression profile data obtained from the Hyseq clustering process were used to determine organ-specificity. Clones showing a greater expression in seed libraries compared to the other tissue libraries were selected as LMP candidate genes. The *Glycine max, Oryza sativa, Zea mays, Linum usitatissimum, Hordeum vulgare* and *Triticum aestivum* clones were selected for overexpression in Arabidopsis and specific crop plants based on their expression profile.

### Example 6: Cloning of full-length cDNAs and orthologs of identified LMP genes

Clones corresponding to full-length sequences and partial cDNAs from *Arabidopsis thaliana, Glycine max, Oryza sativa, Zea mays, Linum usitatissimum, Hordeum vulgare* or *Triticum aestivum* had been identified in the in-house proprietary Hyseq databases. The Hyseq clones of *Glycine max, Oryza sativa, Zea mays, Linum usitatissimum, Hordeum vulgare* and *Triticum aestivum* genes were sequenced at DNA Landmarks using a ABI 377 slab gel sequencer and BigDye Terminator Ready Reaction kits (PE Biosystems, Foster City, CA). Sequence algingments were done to determine whether the Hyseq clones were full-length or partial clones. In cases where the Hyseq clones were determined to be partial cDNAs the following procedure was used to isolate the full-length sequences. Full-length cDNAs were isolated by RACE PCR using the SMART RACE cDNA amplification kit from Clontech allowing both 5'- and 3' rapid amplification of cDNA ends (RACE). The RACE PCR primers were designed based on the Hyseq clone sequences. The isolation of full-length cDNAs and the RACE PCR protocol used were based on the manufacturer's conditions. The RACE product fragments were extracted from agarose gels with a QIAquick Gel Extraction Kit (Qiagen) and ligated into the TOPO pCR 2.1 vector (Invitrogen) following manufacturer's instructions. Recombinant vectors were transformed into TOP10 cells (Invitrogen) using standard conditions (Sambrook et al. 1989). Transformed cells were grown overnight at 37°C on LB agar containing 50 µg/ml kanamycin and spread with 40µl of a 40 mg/ml stock solution of X-gal in dimethylformamide for blue-white selection. Single white colonies were selected and used to inoculate 3 ml of liquid LB containing 50 µg/ml kanamycin and grown overnight at 37°C. Plasmid DNA is extracted using the QIAprep Spin Miniprep Kit (Qiagen) following manufacturer's instructions. Subsequent analyses of clones and restriction mapping was performed according to standard molecular biology techniques (Sambrook et al. 1989).

Full-length cDNAs were isolated and cloned into binary vectors by using the following procedure: Gene specific primers were designed using the full-length sequences obtained from Hyseq clones or subsequent RACE amplification products. Full-length sequences and genes were amplified utilizing Hyseq clones or cDNA libraries as DNA template using touch-down PCR. In some cases, primers were designed to add an "AACA" Kozak-like sequence just upstream of the gene start codon and two bases downstream were, in some cases, changed to GC to facilitate increased gene expression levels (Chandrashekhar et al. 1997, Plant Molecular Biology 35:993-1001). PCR reaction cycles were: 94 °C, 5 min; 9 cycles of 94 °C, 1 min, 65 °C, 1 min, 72 °C, 4 min and in which the anneal temperature was lowered by 1 °C each cycle; 20 cycles of 94 °C, 1 min, 55 °C, 1 min, 72 °C, 4 min; and the PCR cycle was ended with 72 °C, 10 min. Amplified PCR products were gel purified from 1% agarose gels using GenElute -EtBr spin columns (Sigma) and after standard enzymatic digestion, were ligated into the plant binary vector pBPS-GB1 for transformation of Arabidopsis. The binary vector was amplified by overnight growth in *E. coli* DH5 in LB media and appropriate antibiotic and plasmid was prepared for downstream steps using Qiagen MiniPrep DNA preparation kit. The insert was verified throughout the various cloning steps by determining its size through restriction digest and inserts were sequenced to ensure the expected gene was used in Arabidopsis transformation.

Gene sequences can be used to identify homologous or heterologous genes (orthologs, the same LMP gene from another plant) from cDNA or genomic libraries. This can be done by designing PCR primers to conserved sequences identified by multiple sequence alignments. Orthologs are often identified by designing degenerate primers to full-length or partial sequences of genes of interest.

Gene sequences can be used to identify homologues or orthologs from cDNA or genomic libraries. Homologous genes (e. g. full-length cDNA clones) can be isolated via nucleic acid hybridization using for example cDNA libraries: Depending on the abundance of the gene of interest, 100,000 up to 1,000,000 recombinant bacteriophages are plated and transferred to nylon membranes. After denaturation with alkali, DNA is immobilized on the membrane by e. g. UV cross linking. Hybridization is carried out at high stringency conditions. Aqueous solution hybridization and washing is performed at an ionic strength of 1 M NaCl and a temperature of 68°C. Hybridization probes are generated by e. g. radioactive (32P) nick transcription labeling (High Prime, Roche, Mannheim, Germany). Signals are detected by autoradiography.

Partially homologous or heterologous genes that are related but not identical can be identified in a procedure analogous to the above-described procedure using low stringency hybridization and washing conditions. For aqueous hybridization, the ionic strength is normally kept at 1 M NaCl while the temperature is progressively lowered from 68 to 42°C.

Isolation of gene sequences with homologies (or sequence identity/similarity) only in a distinct domain of (for example 10-20 amino acids) can be carried out by using synthetic radio labeled oligonucleotide probes. Radio labeled oligonucleotides are prepared by phosphorylation of the 5-prime end of two complementary oligonucleotides with T4 polynucleotide kinase. The complementary oligonucleotides are annealed and ligated to form concatemers. The double stranded concatemers are than radiolabeled by for example nick transcription. Hybridization is normally performed at low stringency conditions using high oligonucleotide concentrations.

*Oligonucleotide hybridization solution:*

6 x SSC

M sodium phosphate

mM EDTA (pH 8)

0.5 % SDS

100 µg/ml denaturated salmon sperm DNA

% nonfat dried milk

During hybridization, temperature is lowered stepwise to 5-10°C below the estimated oligonucleotide Tm or down to room temperature followed by washing steps and autoradiography. Washing is performed with low stringency such as 3 washing steps using 4x SSC. Further details are described by Sambrook et al. (1989, "Molecular Cloning: A Laboratory Manual", Cold Spring Harbor Laboratory Press) or Ausubel et al. (1994, "Current Protocols in Molecular Biology", John Wiley & Sons).

### Example 7: Identification of Genes of Interest by Screening Expression Libraries with Antibodies

c-DNA clones can be used to produce recombinant protein for example in *E. coli* (e. g. Qiagen QIAexpress pQE system). Recombinant proteins are then normally affinity purified via Ni-NTA affinity chromatography (Qiagen). Recombinant proteins can be used to produce specific antibodies for example by using standard techniques for rabbit immunization. Antibodies are affinity purified using a Ni-NTA column saturated with the recombinant antigen as described by Gu et al. (1994, BioTechniques 17:257-262). The antibody can then be used to screen expression cDNA libraries to identify homologous or heterologous genes via an immunological screening (Sambrook et al. 1989, Molecular Cloning: A Laboratory Manual", Cold Spring Harbor Laboratory Press or Ausubel et al. 1994, "Current Protocols in Molecular Biology", John Wiley & Sons).

### Example 8: Northern-Hybridization

For RNA hybridization, 20 µg of total RNA or 1 µg of poly-(A)+ RNA is separated by gel electrophoresis in 1.25% agarose gels using formaldehyde as described in Amasino (1986, Anal. Biochem. 152:304), transferred by capillary attraction using 10 x SSC to positively charged nylon membranes (Hybond N+, Amersham, Braunschweig), immobilized by UV light and pre-hybridized for 3 hours at 68°C using hybridization buffer (10% dextran sulfate w/v, 1 M NaCl, 1% SDS, 100 µg/ml of herring sperm DNA). The labeling of the DNA probe with the Highprime DNA labeling kit (Roche, Mannheim, Germany) is carried out during the pre-hybridization using alpha-32P dCTP (Amersham, Braunschweig, Germany). Hybridization is carried out after addition of the labeled DNA probe in the same buffer at 68°C overnight. The washing steps are carried out twice for 15 min using 2 x SSC and twice for 30 min using 1 x SSC, 1% SDS at 68°C. The exposure of the sealed filters is carried out at - 70°C for a period of 1 day to 14 days.

### Example 9: DNA Sequencing and Computational Functional Analysis

cDNA libraries can be used for DNA sequencing according to standard methods, in particular by the chain termination method using the ABI PRISM Big Dye Terminator Cycle Sequencing Ready Reaction Kit (Perkin-Elmer, Weiterstadt, Germany). Random sequencing can be carried out subsequent to preparative plasmid recovery from cDNA libraries via *in vivo* mass excision, retransformation, and subsequent plating of DH10B on agar plates (material and protocol details from Stratagene, Amsterdam, Netherlands). Plasmid DNA can be prepared from overnight grown *E. coli* cultures grown in Luria-Broth medium containing ampicillin (see Sambrook et al. (1989, Cold Spring Harbor Laboratory Press: ISBN 0-87969-309-6) on a Qiagene DNA preparation robot (Qiagen, Hilden) according to the manufacturer's protocols). Sequences can be processed and annotated using the software package EST-MAX commercially provided by Bio-Max (Munich, Germany). The program incorporates bioinformatics methods important for functional and structural characterization of protein sequences. For reference see *http:*//*pedant.mips.biochem. mpg.de.*

The most important algorithms incorporated in EST-MAX are: FASTA: Very sensitive protein sequence database searches with estimates of statistical significance (Pearson W.R. 1990, Rapid and sensitive sequence comparison with FASTP and FASTA. Methods Enzymol. 183:63-98). BLAST: Very sensitive protein sequence database searches with estimates of statistical significance (Altschul S.F., Gish W., Miller W., Myers E.W. and Lipman D.J. Basic local alignment search tool. J. Mol. Biol. 215:403-410). PREDATOR: High-accuracy secondary structure prediction from single and multiple sequences. (Frishman & Argos 1997, 75% accuracy in protein secondary structure prediction. Proteins 27:329-335). CLUSTALW: Multiple sequence alignment (Thompson, J.D., Higgins, D.G. and Gibson, T.J. 1994, CLUSTAL W: improving the sensitivity of progressive multiple sequence alignment through sequence weighting, positions-specific gap penalties and weight matrix choice, Nucleic Acids Res. 22:4673-4680). TMAP: Transmembrane region prediction from multiply aligned sequences (Persson B. & Argos P. 1994, Prediction of transmembrane segments in proteins utilizing multiple sequence alignments, J. Mol. Biol. 237:182-192). ALOM2:Transmembrane region prediction from single sequences (Klein P., Kanehisa M., and DeLisi C. 1984, Prediction of protein function from sequence properties: A discriminant analysis of a database. Biochim. Biophys. Acta 787:221-226. Version 2 by Dr. K. Nakai). PROSEARCH: Detection of PROSITE protein sequence patterns. Kolakowski L.F. Jr., Leunissen J.A.M. and Smith J.E. 1992, ProSearch: fast searching of protein sequences with regular expression patterns related to protein structure and function. Biotechniques 13:919-921). BLIMPS: Similarity searches against a database of ungapped blocks (Wallace & Henikoff 1992, PATMAT: A searching and extraction program for sequence, pattern and block queries and databases, CABIOS 8:249-254. Written by Bill Alford).

### Example 10: Plasmids for Plant Transformation

For plant transformation binary vectors such as pBinAR can be used (Höfgen & Willmitzer 1990, Plant Sci. 66:221-230). Construction of the binary vectors can be performed by ligation of the cDNA in sense or antisense orientation into the T-DNA. 5-prime to the cDNA a plant promoter activates transcription of the cDNA. A polyadenylation sequence is located 3'-prime to the cDNA. Tissue-specific expression can be achieved by using a tissue specific promoter. For example, seed-specific expression can be achieved by cloning the napin or LeB4 or USP promoter 5-prime to the cDNA. Also any other seed specific promoter element can be used. For constitutive expression within the whole plant the CaMV 35S promoter can be used. The expressed protein can be targeted to a cellular compartment using a signal peptide, for example for plastids, mitochondria or endoplasmic reticulum (Kermode 1996, Crit. Rev. Plant Sci. 15:285-423). The signal peptide is cloned 5-prime in frame to the cDNA to achieve subcellular localization of the fusion protein.

Plant binary vectors used for example are the pBPS-GB007, pSUN2-GW or pBPS-GB047 vectors into which the LMP gene candidates are cloned. These binary vectors contain an antibiotic resistance gene driven under the control of the AtAct2-I promoter and a USP or other seed-specific promoter or a constitutive promoter in front of the candidate gene with the NOSpA terminator or the OCS terminator. Partial or full-length LMP cDNA are cloned into the multiple cloning site of the plant binary vector in sense or antisense orientation behind the USP or seed-specific or other seed-specific or constitutive promoters. Further promoters that can be used for different crop species are alo mentioned in example 11.

The recombinant vector containing the gene of interest is transformed into Top10 cells (Invitrogen) using standard conditions. Transformed cells are selected for on LB agar containing 50 µg/ml kanamycin grown overnight at 37°C. Plasmid DNA is extracted using the QIAprep Spin Miniprep Kit (Qiagen) following manufacturer's instructions. Analysis of subsequent clones and restriction mapping is performed according to standard molecular biology techniques (Sambrook et al. 1989, Molecular Cloning, A Laboratory Manual. 2nd Edition. Cold Spring Harbor Laboratory Press. Cold Spring Harbor, NY).

### Example 11: Agrobacterium Mediated Plant Transformation

*Agrobacterium* mediated plant transformation with the LMP nucleic acids described herein can be performed using standard transformation and regeneration techniques (Gelvin, Stanton B. & Schilperoort R.A, Plant Molecular Biology Manual, 2nd ed. Kluwer Academic Publ., Dordrecht 1995 in Sect., Ringbuc Zentrale Signatur:BT11-P; Glick, Bernard R. and Thompson, John E. Methods in Plant Molecular Biology and Biotechnology, S. 360, CRC Press, Boca Raton 1993). For example, *Agrobacterium* mediated transformation can be performed using the GV3 (pMP90) (Koncz & Schell, 1986, Mol. Gen. Genet. 204:383-396) or LBA4404 (Clontech) *Agrobacterium tumefaciens* strain.

*Arabidopsis thaliana* can be grown and transformed according to standard conditions (Bechtold 1993, Acad. Sci. Paris. 316:1194-1199; Bent et al. 1994, Science 265:1856-1860). Additionally, rapeseed can be transformed with the LMR nucleic acids of the present invention via cotyledon or hypocotyl transformation (Moloney et al. 1989, Plant Cell Report 8:238-242; De Block et al. 1989, Plant Physiol. 91:694-701). Use of antibiotic for *Agrobacterium* and plant selection depends on the binary vector and the *Agrobacterium* strain used for transformation. Rapeseed selection is normally performed using a selectable plant marker. Additionally, *Agrobacterium* mediated gene transfer to flax can be performed using, for example, a technique described by Mlynarova et al. (1994, Plant Cell Report 13:282-285).

The Arabidopsis *FAD2* or *FAD2*-like gene may be cloned into a binary vector, transformed and expressed either with a constitutive promoter like the superpromoter (Stanton B. Gelvin, USP# 5,428,147 and USP#5,217,903) or seed-specific promoters like USP (unknown seed protein) from *Vicia faba* (Baeumlein et al. 1991, Mol. Gen. Genetics 225:459-67), or the legumin B4 promoter (LeB4; Baeumlein et al. 1992, Plant J. 2:233-239) as well as promoters conferring seed-specific expression in monocot plants like maize, barley, wheat, rye, rice, etc.

The Arabidopsis AHAS (AtAHAS) gene could be used as a selectable marker in these constructs.

Transformation of soybean can be performed using for example a technique described in EP 0424 047, U.S. Patent No. 5,322,783 (Pioneer Hi-Bred International) or in EP 0397 687, U.S. Patent No. 5,376,543 or U.S. Patent No. 5,169,770 (University Toledo), or by any of a number of other transformation procedures known in the art. Soybean seeds are surface sterilized with 70% ethanol for 4 minutes at room temperature with continuous shaking, followed by 20% (v/v) Clorox supplemented with 0.05% (v/v) tween for 20 minutes with continuous shaking. Then the seeds are rinsed 4 times with distilled water and placed on moistened sterile filter paper in a Petri dish at room temperature for 6 to 39 hours. The seed coats are peeled off, and cotyledons are detached from the embryo axis. The embryo axis is examined to make sure that the meristematic region is not damaged. The excised embryo axes are collected in a half-open sterile Petri dish and air-dried to a moisture content less than 20% (fresh weight) in a sealed Petri dish until further use.

The method of plant transformation is also applicable to *Brassica napus, Linum usitatissimum* and other crops. In particular, seeds of canola are surface sterilized with 70% ethanol for 4 minutes at room temperature with continuous shaking, followed by 20% (v/v) Clorox supplemented with 0.05 % (v/v) Tween for 20 minutes, at room temperature with continuous shaking. Then, the seeds are rinsed 4 times with distilled water and placed on moistened sterile filter paper in a Petri dish at room temperature for 18 hours. The seed coats are removed and the seeds are air dried overnight in a half-open sterile Petri dish. During this period, the seeds lose approximately 85% of their water content. The seeds are then stored at room temperature in a sealed Petri dish until further

*Agrobacterium tumefaciens* culture is prepared from a single colony in LB solid medium plus appropriate antibiotics (e.g. 100 mg/l streptomycin, 50 mg/l kanamycin) followed by growth of the single colony in liquid LB medium to an optical density at 600 nm of 0.8. Then, the bacteria culture is pelleted at 7000 rpm for 7 minutes at room temperature, and re-suspended in MS (Murashige & Skoog 1962, Physiol. Plant. 15:473-497) medium supplemented with 100 mM acetosyringone. Bacteria cultures are incubated in this pre-induction medium for 2 hours at room temperature before use. The axis of soybean zygotic seed embryos at approximately 44% moisture content are imbibed for 2 h at room temperature with the pre-induced *Agrobacterium* suspension culture. (The imbibition of dry embryos with a culture of *Agrobacterium* is also applicable to maize embryo axes). The embryos are removed from the imbibition culture and are transferred to Petri dishes containing solid MS medium supplemented with 2% sucrose and incubated for 2 days, in the dark at room temperature. Alternatively, the embryos are placed on top of moistened (liquid MS medium) sterile filter paper in a Petri dish and incubated under the same conditions described above. After this period, the embryos are transferred to either solid or liquid MS medium supplemented with 500 mg/l carbenicillin or 300 mg/l cefotaxime to kill the agrobacteria. The liquid medium is used to moisten the sterile filter paper. The embryos are incubated during 4 weeks at 25°C, under 440 µmol m⁻²s⁻¹ and 12 hours photoperiod. Once the seedlings have produced roots, they are transferred to sterile metromix soil. The medium of the *in vitro* plants is washed off before transferring the plants to soil. The plants are kept under a plastic cover for 1 week to favor the acclimatization process. Then the plants are transferred to a growth room where they are incubated at 25°C, under 440 µmol m⁻²s⁻¹ light intensity and 12 h photoperiod for about 80 days.

Samples of the primary transgenic plants (T₀) are analyzed by PCR to confirm the presence of T-DNA. These results are confirmed by Southern hybridization wherein DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labeled probe by PCR as recommended by the manufacturer.

As an example for monocot transformation, a constitutive or seed-specific promoter in combination with maize Ubiquitin intron and *FAD2* or *FAD*2-like nucleic acid molecules may be used. For example, the *PtxA-FAD2* ortholog gene construct in pUC is digested with *Pac*I and *Xma*I. pBPSMM348 is digested with *Pac*I and *Xma*I to isolate maize Ubiquitin intron (ZmUbi intron) followed by electrophoresis and the QIAEX II Gel Extraction Kit (cat# 20021). The ZmUbi intron is ligated into the PtxA-*FAD*2 or *FAD2*-like nucleic acid molecule in pUC to generate pUC based PtxA-ZmUbi intron-*FAD2* or *FAD*2-like nucleic acid molecule construct followed by restriction enzyme digestion with Afel and *Pme*I. PtxA-ZmUbi intron *FAD2* or *FAD*2-like gene cassette will be cut out of a Seaplaque low melting temperature agarose gel (SeaPlaque® GTG® Agarose catalog No. 50110) after electrophoresis. A monocotyledonous base vector containing a selectable marker cassette (Monocot base vector) is digested with *Pme*I. The *FAD2* or *FAD*2-like nucleic acid molecule expression cassette containing a seed specific promoter-ZmUbi intron is ligated into the Monocot base vector. Subsequently, the construct is transformed into a recombinant LBA4404 strain containing pSB1 (super *vir* plasmid) using electroporation following a general protocol in the art. *Agrobacteriu*m-mediated transformation in maize is performed using immature embryo following a protocol described in US 5,591,616. An imidazolinone herbicide selection is applied to obtain transgenic maize lines.

Examples for promoters used in corn are also the zeins, which are a group of storage proteins found in corn endosperm. Genomic clones for zein genes have been isolated (Pedersen et al., Cell 29:1015-1026, 1982) and the promoters from these clones, including the 15 kD, 16 kD, 19 kD, 22 kD, 27 kD genes, could also be used. Other promoters know to function in corn are starch synthases, branching enzymes, debranching enzymes, oleosins, glutelins and sucrose synthases. A particularly preferred promoter for corn endosperm expression is the promoter for the glutelin gene from rice, more particularly the Osgt-1 promoter (Zheng et al., Mole. Cell Biol. 13:5829-5842, 1993).

Examples of promoters suitable for expression in wheat include those promoters for the ADP glucose pyrosynthase subunits, the granule bound and other starch synthase, the branching and debranching enzymes, the embryogenesis-abundant proteins, the gliadins and the glutenins.

Examples of promoters suitable for expression in barley include those promoters for the ADP glucose pyrosynthase subunits, the granule bound and other starch synthase, the branching and debranching enzymessucrose synthases, the hordeins, the embryo globulins and the aleurone specific proteins.

Examples of promoters suitable for expression in soybean include promoters already mentioned herein. Yet, other promoters that can be used are a soybean 7S promoter and the soybean 7S□' beta conglycinin promoter.

In general, a rice (or other monocot) *FAD2* gene or *FAD2*-like gene under a plant promoter like USP could be transformed into corn, or another crop plant, to generate effects of monocot *FAD2* genes in other monocots, or dicot *FAD2* genes in other dicots, or monocot genes in dicots, or vice versa. The plasmids containing these *FAD2* or *FAD2*-like coding sequences, 5' of a promoter and 3' of a terminator would be constructed in a manner similar to those described for construction of other plasmids herein. Examples of promoters suitable for expression in rice include those promoters for the ADP glucose pyrosynthase subunits, the granule bound and other starch synthase, the branching enzymes, the debranching enzymes, sucrose synthases and the glutelins. A particularly preferred promoter is the promoter for rice glutelin, Osgt-1.

### Example 12: In vivo Mutagenesis

*In vivo* mutagenesis of microorganisms can be performed by incorporation and passage of the plasmid (or other vector) DNA through *E. coli* or other microorganisms (e.g. *Bacillus* spp. or yeasts such as *Saccharomyces cerevisiae*) that are impaired in their capabilities to maintain the integrity of their genetic information. Typical mutator strains have mutations in the genes for the DNA repair system (e.g., mutHLS, mutD, mutT, etc.; for reference, see Rupp W.D. 1996, DNA repair mechanisms, in: Escherichia coli and Salmonella, p. 2277-2294, ASM: Washington.) Such strains are well known to those skilled in the art. The use of such strains is illustrated, for example, in Greener and Callahan 1994, Strategies 7:32-34. Transfer of mutated DNA molecules into plants is preferably done after selection and testing in microorganisms. Transgenic plants are generated according to various examples within the exemplification of this document.

### Example 13: Assessment of the mRNA Expression and Activity of a Recombinant Gene Product in the Transformed Organism

The activity of a recombinant gene product in the transformed host organism can be measured on the transcriptional or/and on the translational level. A useful method to ascertain the level of transcription of the gene (an indicator of the amount of mRNA available for translation to the gene product) is to perform a Northern blot (for reference see, for example, Ausubel et al. 1988, Current Protocols in Molecular Biology, Wiley: New York), in which a primer designed to bind to the gene of interest is labeled with a detectable tag (usually radioactive or chemiluminescent), such that when the total RNA of a culture of the organism is extracted, run on gel, transferred to a stable matrix and incubated with this probe, the binding and quantity of binding of the probe indicates the presence and also the quantity of mRNA for this gene. This information at least partially demonstrates the degree of transcription of the transformed gene. Total cellular RNA can be prepared from plant cells, tissues or organs by several methods, all well-known in the art, such as that described in Bormann et al. (1992, Mol. Microbiol. 6:317-326).

To assess the presence or relative quantity of protein translated from this mRNA, standard techniques, such as a Western blot, may be employed (see, for example, Ausubel et al. 1988, Current Protocols in Molecular Biology, Wiley: New York). In this process, total cellular proteins are extracted, separated by gel electrophoresis, transferred to a matrix such as nitrocellulose, and incubated with a probe, such as an antibody, which specifically binds to the desired protein. This probe is generally tagged with a chemiluminescent or colorimetric label, which may be readily detected. The presence and quantity of label observed indicates the presence and quantity of the desired mutant protein present in the cell.

The activity of LMPs that bind to DNA can be measured by several well-established methods, such as DNA band-shift assays (also called gel retardation assays). The effect of such LMP on the expression of other molecules can be measured using reporter gene assays (such as that described in Kolmar H. et al. 1995, EMBO J. 14:3895-3904 and references cited therein). Reporter gene test systems are well known and established for applications in both prokaryotic and eukaryotic cells, using enzymes such as beta-galactosidase, green fluorescent protein, and several others.

The determination of activity of lipid metabolism membrane-transport proteins can be performed according to techniques such as those described in Gennis R.B. (1989 Pores, Channels and Transporters, in Biomembranes, Molecular Structure and Function, Springer: Heidelberg, pp. 85-137, 199-234 and 270-322).

### Example 14: In vitro Analysis of the Function of Arabidopsis thaliana, Glycine max, Oryza sativa, Zea mays, Linum usitatissimum, Hordeum vulgare or Triticum aestivum FAD2 or FAD2-like Genes in Transgenic Plants

The determination of activities and kinetic parameters of enzymes is well established in the art. Experiments to determine the activity of any given altered enzyme must be tailored to the specific activity of the wild-type enzyme, which is well within the ability of one skilled in the art. Overviews about enzymes in general, as well as specific details concerning structure, kinetics, principles, methods, applications and examples for the determination of many enzyme activities may be found, for example, in the following references: Dixon, M. & Webb, E.C. 1979, Enzymes. Longmans: London; Fersht, (1985) Enzyme Structure and Mechanism. Freeman: New York; Walsh (1979) Enzymatic Reaction Mechanisms. Freeman: San Francisco; Price, N.C., Stevens, L. (1982) Fundamentals of Enzymology. Oxford Univ. Press: Oxford; Boyer, P.D., ed. (1983) The Enzymes, 3rd ed. Academic Press: New York; Bisswanger, H., (1994) Enzymkinetik, 2nd ed. VCH: Weinheim (ISBN 3527300325); Bergmeyer, H.U., Bergmeyer, J., GraßI, M., eds. (1983-1986) Methods of Enzymatic Analysis, 3rd ed., vol. I-XII, Verlag Chemie: Weinheim; and Ullmann's Encyclopedia of Industrial Chemistry (1987) vol. A9, Enzymes. VCH: Weinheim, p. 352-363.

### Example 15: Analysis of the Impact of Recombinant Proteins on the Production of a Desired Seed Storage Compound

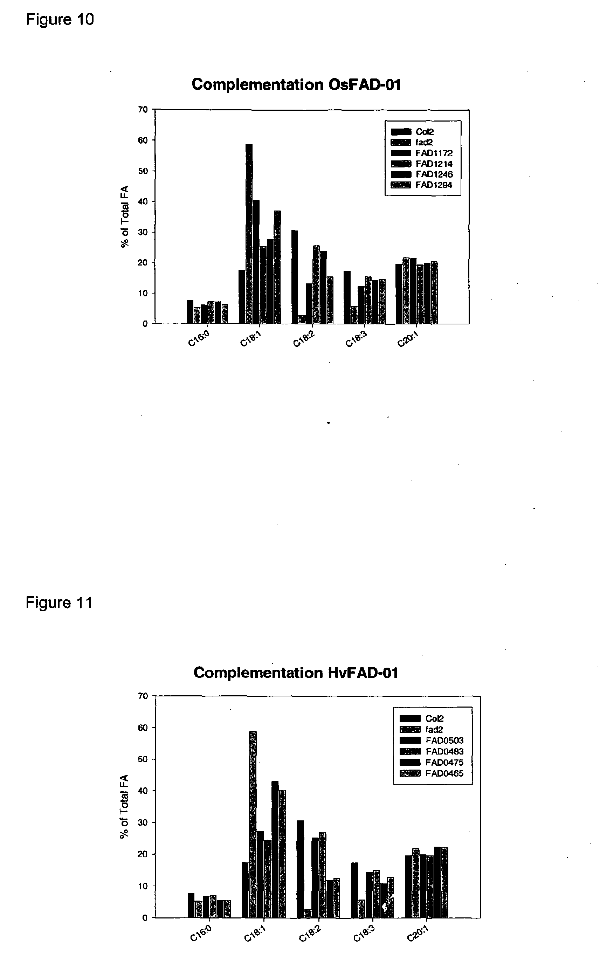

As an example for seed oil changes, seeds from transformed *Arabidopsis thaliana* plants were analyzed by gas chromatography (GC) for fatty acid profiles. Each bar in Figures 10 and 11 represents the value obtained with 5 mg bulked seeds of one plant of wild-type or *fad2* mutant or of one independent transgenic event in a *fad2* mutant background. As illustrated in Figure 10, the *fad2* mutant of *Arbidopsis thaliana* showed an increase in the content of oleic acid (C18:1) from 16% in the wild type to about 60% in the *fad2* mutant. Likewise there is a strong reduction in the proportion of linoleic acid (C18:2) from about 31% in the wild type to 2% in the *fad2* mutant. Arabidopsis *fad2* mutant seeds transformed with the *Oryza sativa* gene OsFAD-01 (Seq ID No. 17) showed a restoration of the fatty acid pattern in several independent transgenic lines in the *fad2* mutant background (see events FAD1172, FAD1214, FAD1246 and FAD1294 in Fig. 10) towards the wild type composition even in a segregating T2 seed population. A similar response was obtained with the *Hordeum vulgare* gene HvFAD-01 (Seq ID No. 23) as shown in Figure 11 for the events FAD0503, FAD0483, FAD0475 and FAD0465. This result indicates that both the *Oryza sativa* (OsFAD-01) and the *Hordeum vulgare* (HvFAD-01) genes are capable of complementing the *fad2 Arabidopsis* mutant with regard to the seed fatty acid profile, indicating their function as fatty acid desaturases. All other fatty acid desaturase genes from different crop plants indicated in this application exhibited a similar response in a *fad2* Arabidopsis mutant background as well (data not shown).

The effect of the genetic modification in plants on a desired seed storage compound (such as a sugar, lipid or fatty acid) can be assessed by growing the modified plant under suitable conditions and analyzing the seeds or any other plant organ for increased production of the desired product (i.e., a lipid or a fatty acid). Such analysis techniques are well known to one skilled in the art, and include spectroscopy, thin layer chromatography, staining methods of various kinds, enzymatic and microbiological methods, and analytical chromatography such as high performance liquid chromatography (see, for example, Ullman 1985, Encyclopedia of Industrial Chemistry, vol. A2, pp. 89-90 and 443-613, VCH: Weinheim; Fallon, A. et al. 1987, Applications of HPLC in Biochemistry in: Laboratory Techniques in Biochemistry and Molecular Biology, vol. 17; Rehm et al., 1993 Product recovery and purification, Biotechnology, vol. 3, Chapter III, pp. 469-714, VCH: Weinheim; Belter, P.A. et al., 1988 Bioseparations: downstream processing for biotechnology, John Wiley & Sons; Kennedy J.F. & Cabral J.M.S. 1992, Recovery processes for biological materials, John Wiley and Sons; Shaeiwitz J.A. & Henry J.D. 1988, Biochemical separations in: Ulmann's Encyclopedia of Industrial Chemistry, Separation and purification techniques in biotechnology, vol. B3, Chapter 11, pp. 1-27, VCH: Weinheim; and Dechow F.J. 1989).

Besides the above-mentioned methods, plant lipids are extracted from plant material as described by Cahoon et al. (1999, Proc. Natl. Acad. Sci. USA 96, 22:12935-12940) and Browse et al. (1986, Anal. Biochemistry 442:141-145). Qualitative and quantitative lipid or fatty acid analysis is described in Christie, William W., Advances in Lipid Methodology. Ayr/Scotiand:Oily Press. - (Oily Press Lipid Library; Christie, William W., Gas Chromatography and Lipids. A Practical Guide - Ayr, Scotland:Oily Press, 1989 Repr. 1992. - IX, 307 S. - (Oily Press Lipid Library; and "Progress in Lipid Research, Oxford :Pergamon Press, 1 (1952) -16 (1977) Progress in the Chemistry of Fats and Other Lipids CODEN.

Unequivocal proof of the presence of fatty acid products can be obtained by the analysis of transgenic plants following standard analytical procedures: GC, GC-MS or TLC as variously described by Christie and references therein (1997 in: Advances on Lipid Methodology 4th ed.: Christie, Oily Press, Dundee, pp. 119-169; 1998). Detailed methods are described for leaves by Lemieux et al. (1990, Theor. Appl. Genet. 80:234-240) and for seeds by Focks & Benning (1998, Plant Physiol. 118:91-101).

Positional analysis of the fatty acid composition at the sn-1, sn-2 or sn-3 positions of the glycerol backbone is determined by lipase digestion (see, e.g., Siebertz & Heinz 1977, Z. Naturforsch. 32c:193-205, and Christie 1987, Lipid Analysis 2nd Edition, Pergamon Press, Exeter, ISBN 0-08-023791-6).

Total seed oil levels can be measured by any appropriate method. Quantitation of seed oil contents is often performed with conventional methods, such as near infrared analysis (NIR) or nuclear magnetic resonance imaging (NMR). NIR spectroscopy has become a standard method for screening seed samples whenever the samples of interest have been amenable to this technique. Samples studied include canola, soybean, maize, wheat, rice, and others. NIR analysis of single seeds can be used (see e.g. Velasco et al., 'Estimation of seed weight, oil content and fatty acid composition in intact single seeds of rapeseed (Brassica napus L.) by near-infrared reflectance spectroscopy, 'Euphytica, Vol. 106, 1999, pp. 79-85). NMR has also been used to analyze oil content in seeds (see e.g. Robertson & Morrison, "Analysis of oil content of sunflower seed by wide-line NMR, "Journal of the American Oil Chemists Society, 1979, Vol. 56, 1979, pp. 961-964, which is herein incorporated by reference in its entirety).

A typical way to gather information regarding the influence of increased or decreased protein activities on lipid and sugar biosynthetic pathways is for example via analyzing the carbon fluxes by labeling studies with leaves or seeds using ¹⁴C-acetate or ¹⁴C-pyruvate (see, e.g. Focks & Benning 1998, Plant Physiol. 118:91-101; Eccleston & Ohlrogge 1998, Plant Cell 10:613-621). The distribution of carbon-14 into lipids and aqueous soluble components can be determined by liquid scintillation counting after the respective separation (for example on TLC plates) including standards like ¹⁴C-sucrose and ¹⁴C-malate (Eccleston & Ohlrogge 1998, Plant Cell 10:613-621).

Material to be analyzed can be disintegrated via sonification, glass milling, liquid nitrogen and grinding or via other applicable methods. The material has to be centrifuged after disintegration. The sediment is re-suspended in distilled water, heated for 10 minutes at 100°C, cooled on ice and centrifuged again followed by extraction in 0.5 M sulfuric acid in methanol containing 2% dimethoxypropane for 1 hour at 90°C leading to hydrolyzed oil and lipid compounds resulting in transmethylated lipids. These fatty acid methyl esters are extracted in petrolether and finally subjected to GC analysis using a capillary column (Chrompack, WCOT Fused Silica, CP-Wax-52 CB, 25 m, 0.32 mm) at a temperature gradient between 170°C and 240°C for 20 minutes and 5 min. at 240°C. The identity of resulting fatty acid methylesters is defined by the use of standards available form commercial sources (i.e., Sigma).

In case of fatty acids where standards are not available, molecule identity is shown via derivatization and subsequent GC-MS analysis. For example, the localization of triple bond fatty acids is shown via GC-MS after derivatization via 4,4-Dimethoxy-oxazolin-Derivaten (Christie, Oily Press, Dundee, 1998).

A common standard method for analyzing sugars, especially starch, is published by Stitt M., Lilley R.Mc.C., Gerhardt R. and Heldt M.W. (1989, "Determination of metabolite levels in specific cells and subcellular compartments of plant leaves" Methods Enzymol. 174:518-552; for other methods see also Härtel et al. 1998, Plant Physiol. Biochem. 36:407-417 and Focks & Benning 1998, Plant Physiol. 118:91-101).

For the extraction of soluble sugars and starch, 50 seeds are homogenized in 500 µl of 80% (v/v) ethanol in a 1.5-ml polypropylene test tube and incubated at 70°C for 90 min. Following centrifugation at 16,000 g for 5 min, the supernatant is transferred to a new test tube. The pellet is extracted twice with 500 µl of 80% ethanol. The solvent of the combined supernatants is evaporated at room temperature under a vacuum. The residue is dissolved in 50 µl of water, representing the soluble carbohydrate fraction. The pellet left from the ethanol extraction, which contains the insoluble carbohydrates including starch, is homogenized in 200 µl of 0.2 N KOH, and the suspension is incubated at 95°C for 1 h to dissolve the starch. Following the addition of 35 µl of 1 N acetic acid and centrifugation for 5 min at 16,000 g, the supernatant is used for starch quantification.

To quantify soluble sugars, 10 µl of the sugar extract is added to 990 µl of reaction buffer containing 100 mM imidazole, pH 6.9, 5 mM MgCl₂, 2 mM NADP, 1 mM ATP, and 2 units 2 ml⁻¹ of Glucose-6-P-dehydrogenase. For enzymatic determination of glucose, fructose and sucrose, 4.5 units of hexokinase, 1 unit of phosphoglucoisomerase, and 2 µl of a saturated fructosidase solution are added in succession. The production of NADPH is photometrically monitored at a wavelength of 340 nm. Similarly, starch is assayed in 30 µl of the insoluble carbohydrate fraction with a kit from Boehringer Mannheim.

An example for analyzing the protein content in leaves and seeds can be found by Bradford M.M. (1976, "A rapid and sensitive method for the quantification of microgram quantities of protein using the principle of protein dye binding" Anal. Biochem. 72:248-254). For quantification of total seed protein, 15-20 seeds are homogenized in 250 µl of acetone in a 1.5-ml polypropylene test tube. Following centrifugation at 16,000 g, the supernatant is discarded and the vacuum-dried pellet is resuspended in 250 µl of extraction buffer containing 50 mM Tris-HCl, pH 8.0, 250 mM NaCl, 1 mM EDTA, and 1% (w/v) SDS. Following incubation for 2 h at 25°C, the homogenate is centrifuged at 16,000 g for 5 min and 200 ml of the supernatant will be used for protein measurements. In the assay, γ-globulin is used for calibration. For protein measurements, Lowry DC protein assay (Bio-Rad) or Bradford-assay (Bio-Rad) is used.

Enzymatic assays of hexokinase and fructokinase are performed spectrophotometrically according to Renz et al. (1993, Planta 190:156-165), of phosphogluco-isomerase, ATP-dependent 6-phosphofructokinase, pyrophosphate-dependent 6-phospho-fructokinase, Fructose-1,6-bisphosphate aldolase, triose phosphate isomerase, glyceral-3-P dehydrogenase, phosphoglycerate kinase, phosphoglycerate mutase, enolase and pyruvate kinase are performed according to Burrell et al. (1994, Planta 194:95-101) and of UDP-Glucose-pyrophosphorylase according to Zrenner et al. (1995, Plant J. 7:97-107).

Intermediates of the carbohydrate metabolism, like Glucose-1-phosphate, Glucose-6-phosphate, Fructose-6-phosphate, Phosphoenolpyruvate, Pyruvate, and ATP are measured as described in Härtel et al. (1998, Plant Physiol. Biochem. 36:407-417) and metabolites are measured as described in Jelitto et al. (1992, Planta 188:238-244).

In addition to the measurement of the final seed storage compound (i.e., lipid, starch or storage protein) it is also possible to analyze other components of the metabolic pathways utilized for the production of a desired seed storage compound, such as intermediates and side-products, to determine the overall efficiency of production of the compound (Fiehn et al. 2000, Nature Biotech. 18:1447-1161).

For example, yeast expression vectors comprising the nucleic acids disclosed herein, or fragments thereof, can be constructed and transformed into *Saccharomyces cerevisiae* using standard protocols. The resulting transgenic cells can then be assayed for alterations in sugar, oil, lipid or fatty acid contents.

Similarly, plant expression vectors comprising the nucleic acids disclosed herein, or fragments thereof, can be constructed and transformed into an appropriate plant cell such as Arabidopsis, soybean, rapeseed, rice, maize, wheat, *Medicago truncatula*, etc., using standard protocols. The resulting transgenic cells and/or plants derived there from can then be assayed for alterations in sugar, oil, lipid or fatty acid contents.

Additionally, the sequences disclosed herein, or fragments thereof, can be used to generate knockout mutations in the genomes of various organisms, such as bacteria, mammalian cells, yeast cells, and plant cells (Girke at al. 1998, Plant J. 15:39-48). The resultant knockout cells can then be evaluated for their composition and content in seed storage compounds, and the effect on the phenotype and/or genotype of the mutation. For other methods of gene inactivation include US 6004804 "Non-Chimeric Mutational Vectors" and Puttaraju et al. (1999, "Spliceosome-mediated RNA trans-splicing as a tool for gene therapy" Nature Biotech. 17:246-252).

### Example 16: Purification of the Desired Product from Transformed Organisms

An LMP can be recovered from plant material by various methods well known in the art. Organs of plants can be separated mechanically from other tissue or organs prior to isolation of the seed storage compound from the plant organ. Following homogenization of the tissue, cellular debris is removed by centrifugation and the supernatant fraction containing the soluble proteins is retained for further purification of the desired compound. If the product is secreted from cells grown in culture, then the cells are removed from the culture by low-speed centrifugation and the supemate fraction is retained for further purification.

The supernatant fraction from either purification method is subjected to chromatography with a suitable resin, in which the desired molecule is either retained on a chromatography resin while many of the impurities in the sample are not, or where the impurities are retained by the resin, while the sample is not. Such chromatography steps may be repeated as necessary, using the same or different chromatography resins. One skilled in the art would be well-versed in the selection of appropriate chromatography resins and in their most efficacious application for a particular molecule to be purified. The purified product may be concentrated by filtration or ultrafiltration, and stored at a temperature at which the stability of the product is maximized.

There is a wide array of purification methods known to the art and the preceding method of purification is not meant to be limiting. Such purification techniques are described, for example, in Balley J.E. & Ollis D.F. 1986, Biochemical Engineering Fundamentals, McGraw-Hill:New York).

The identity and purity of the isolated compounds may be assessed by techniques standard in the art. These include high-performance liquid chromatography (HPLC), spectroscopic methods, staining methods, thin layer chromatography, analytical chromatography such as high performance liquid chromatography, NIRS, enzymatic assay, or microbiologically. Such analysis methods are reviewed in: Patek et al. (1994, Appl. Environ. Microbiol. 60:133-140), Malakhova et al. (1996, Biotekhnologiya 11:27-32) and Schmidt et al. (1998, Bioprocess Engineer 19:67-70), Ulmann's Encyclopedia of Industrial Chemistry (1996, Vol. A27, VCH: Weinheim, p. 89-90, p. 521-540, p. 540-547, p. 559-566, 575-581 and p. 581-587) and Michal G. (1999, Biochemical Pathways: An Atlas of Biochemistry and Molecular Biology, John Wiley and Sons; Fallon, A. et al. 1987, Applications of HPLC in Biochemistry in: Laboratory Techniques in Biochemistry and Molecular Biology, vol. 17).

### Example 17. Down regulation of gene expression by engineering microRNA precursor

MicroRNAs (miRNAs) have emerged as evolutionarily conserved, RNA-based regulators of gene expression in plants and animals. MiRNAs (∼ 21 to 25 nt) arise from larger precursors with a stem loop structure that are transcribed from non-protein-coding genes. miRNA targets a specific mRNA to suppress gene expression at post-transcriptional (*i*.*e*. degrades mRNA) or translational levels (*i*.*e*. inhibits protein synthesis) (Bartel D 2004, Cell 116, 281-297).

MiRNA precursor (pre-miRNA) can be engineered in such way that endogenous miRNA encoded by pre-miRNA is replaced by a miRNA to target a gene-of-interest, e.g. dsRed reporter gene.

Maize miR166 precursor was selected for engineering. The nucleotide sequence encoding the miR166 precurser is depicted in SEQ ID NO: 47. Two binary expression constructs were generated through multi-site Gateway cloning approach (Invitrogen, Carlsbad, CA). RLM323 as described in SEQ ID NO: 41 was a control, i.e. native maize miR166 expression under the control of ScBV (sugarcane bacilliform badnavirus) promoter and NOS (nopaline synthase) terminator. RLM325 as descirbed by SEQ ID NO: 42 was identical to RLM323 except native miR166 (5' tcggaccaggcttcattcccc 3') as described in SEQ ID NO: 37 and in SEQ ID NO: 38 within the precursor was replaced by a miRNA targeting dsRed (5' ttgtagatgaagcagccgtcc 3') as described in SEQ ID NO: 39. MiR dsRed is complementary to 3' region of dsRed mRNA.

RLM323 and RLM325 were transformed via *Agrobacteria* into homozygote maize SDM10828 which already carries a binary vector, RLM185 to express dsRed under control of ScBV promoter and NOS terminator. Leaf samples from 3 independent T0 events carrying RLM323 and 29 independent T0 events carrying RLM325 were collected. The samples were then analyzed using Typhoon 9400 (General Engineering), an image system under settings to detect dsRed fluorescence. Fluorescence intensity from RLM325 events was reduced over 90% comparing to the intensity from the control, RLM323 events.

The production of miR dsRed in RLM325 events was confirmed in Northern blotting analysis. A specific band of -21 nt was detected in RLM325, but not RLM323 (control) events using a radioactive labelled probe complementary to miR dsRed. The reduction (nearly 90%) of dsRed mRNA in RLM325 events was confirmed by qRT-PCR comparing to the control RLM323. Taken together, these data demonstrated miRNA precursor can be engineered to target a gene-of-interest.

Maize genes coding for fatty acid desaturases, are expressed in many tissues including seeds. A 19 to 21nt (e.g. ACCAGACCCCGAACGCCGC as described in SEQ ID NO: 40) complimentary to a maize desaturase coding region or 5' UTR and 3'UTR in mRNA is used to replace Zm miR166 (5' tcggaccaggcttcattcccc 3') as described in SEQ ID NO: 37 and in SEQ ID NO: 38 in Zm miR166 precursor. The transgene will then be transformed into maize. The expression of the engineered Zm miR166 gene will be controlled by a maize seed-specific promoter (e.g. endosperm specific 10 KD Zein promoter or Glob1 embryo-specific promoter).

A microRNA (e.g. ACCAGACCCCGAACGCCGC as described in SEQ ID NO: 40) is generated in seeds when the engineered Zm miR166 precursor is processed. This miRNA specifically binds to the region in a maize fatty acid desaturase mRNA complimentary to the miRNA, which results in a reduction of this targeted maize desaturase expression at transcriptional or translational levels in seeds by gene silencing machinery. As a result, transgenic maize could have desirable fatty acid level and composition as for example low linolenic acid and/or medium or high oleic acid levels in seeds.

### Example 18 - Screening for Increased Seed Size

The conditional expression of *FAD2* and of the crop *FAD2*-like genes can result in an increased seed size. Transgenic *Arabidopsis* or crop plants expressing *FAD2* or *FAD2*-like genes will be produced. Transgenic plants with seeds larger than the wild-type will be identified by using a microscope. In addition, the seed weight will be measured in transgenic lines. For example, *fad2* mutant seeds showed a 20% reduction in seed weight as compared with the wild type. In the segregating T2 seed generation of the independent Arabidopsis transgenic lines pFAD2RT-7 and pFAD2RT-5 the weight of 100 seeds was increased by 30 and 40%, respectively. In homozygous T3 seeds the seed weight was increased up to 60% as compared with the empty vector control (data not shown). Increased seed weight was reflected in an increased seed size of *FAD2* gene overexpressors in Arabidopsis. Increased seed size leads to greater yield in many economically important crop plants. Therefore, increased seed size is one goal of genetically engineering and selection using *FAD2* or *FAD2-*like nucleic acid molecules as described in this application.

**Table 1.**

| **Plant Lipid Classes** | |
|---|---|
| Neutral Lipids | Triacylglycerol (TAG) |
| | Diacylglycerol (DAG) |
| | Monoacylglycerol (MAG) |
| Polar Lipids | Monogalactosyldiacylglycerol (MGDG) |
| | Digalactosyldiacylglycerol (DGDG) |
| | Phosphatidylglycerol (PG) |
| | Phosphatidylcholine (PC) |
| | Phosphatidylethanolamine (PE) |
| | Phosphatidylinositol (PI) |
| | Phosphatidylserine (PS) |
| | Sulfoquinovosyldiacylglycerol |

**Table 2.**

| Common Plant Fatty Acids | |
|---|---|
| 16:0 | Palmitic acid |
| 16:1 | Palmitoleic acid |
| 16:3 | Palmitolenic acid |
| 18:0 | Stearic acid |
| 18:1 | Oleic acid |
| 18:2 | Linoleic acid |
| 18:3 | Linolenic acid |
| γ-18:3 | Gamma-linolenic acid* |
| 20:0 | Arachidic acid |
| 20:1 | Eicosenoic acid |
| 22:6 | Docosahexanoic acid (DHA) * |
| 20:2 | Eicosadienoic acid |
| 20:4 | Arachidonic acid (AA) * |
| 20:5 | Eicosapentaenoic acid (EPA) * |
| 22:1 | Erucic acid |

These fatty acids do not normally occur in plant seed oils, but their production in transgenic plant seed oil is of importance in plant biotechnology.

**Table 3.**

| **A table of the putative functions of the *FAD2*-like LMPs (the full length nucleic acid sequences can be found in Appendix A using the sequence codes)** | | | | |
|---|---|---|---|---|
| **SEQ ID NO:** | **Sequence name** | **Species** | **Function** | **ORF Position** |
| 1 | *AtFAD-01* | *Arabidopsis thaliana* | omega-6 fatty acid desaturase, endoplasmic reticulum (FAD2) / delta-12 desaturase | 157-1305 |
| 5 | *GmFAD-01* | *Glycine max* | omega-6 fatty acid desaturase, endoplasmic reticulum (FAD2) / delta-12 desaturase | 115-1275 |
| 9 | *GmFAD-02* | *Glycine max* | omega-6 fatty acid desaturase, endoplasmic reticulum (FAD2) / delta-12 desaturase | 96-1244 |
| | | | omega-6 fatty acid desaturase, endoplasmic reticulum (FAD2) / | |
| 13 | *GmFAD-03* | *Glycine max* | delta-12 desaturase | 96-749 |
| 17 | *ZmFAD-01* | *Zea mays* | Corn delta- 12 desaturase fad2-2 | 176-1351 |
| 21 | *OsFAD-01* | *Oryza sativa* | Putative delta-12 oleate desaturase | 150-1313 |
| 25 | *LuFAD-01* | *Linum usitatissimum* | Delta-12 fatty acid desaturase | 48-1070 |
| 29 | *HvFAD-01* | *Hordeum vulgare* | Putative delta-12 oleate desaturase | 25-1185 |
| 33 | *TaFAD-01* | *Triticum aestivum* | Putative delta-12 oleate desaturase | 165-1325 |
| | | | | |

Those skilled in the art will recognize, or will be able to ascertain using no more than routine experimentation, many equivalents to the specific embodiments of the invention described herein. Such equivalents are intended to be encompassed by the claims to the invention disclosed and claimed herein.

### Appendix A

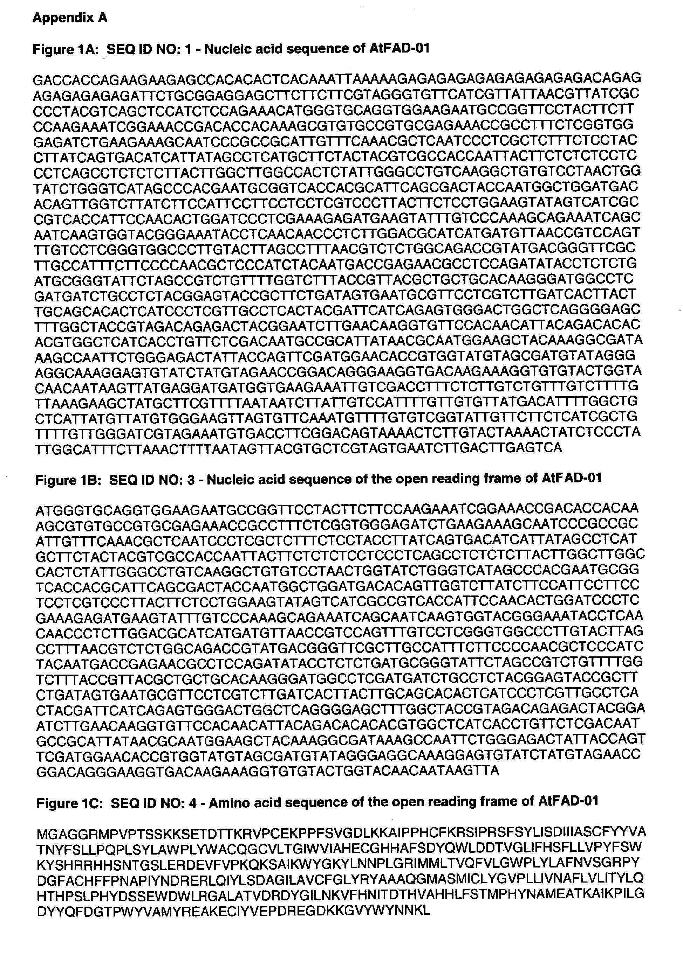

Figure 1A: SEQ ID NO: 1 - Nucleic acid sequence of AtFAD-01

Figure 1B: SEQ ID NO: 3 - Nucleic acid sequence of the open reading frame of AtFAD-01

Figure 1C: SEQ ID NO: 4 - Amino acid sequence of the open reading frame of AtFAD-01

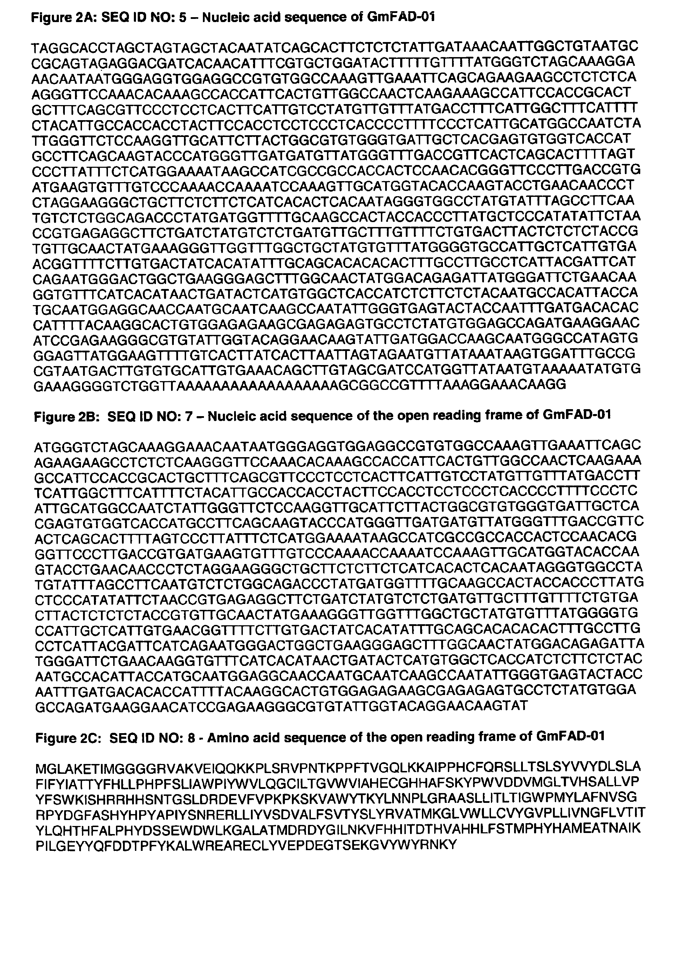

Figure 2A: SEQ ID NO: 5 - Nucleic acid sequence of GmFAD-01

Figure 2B: SEQ ID NO: 7 - Nucleic acid sequence of the open reading frame of GmFAD-01

Figure 2C: SEQ ID NO: 8 - Amino acid sequence of the open reading frame of GmFAD-01

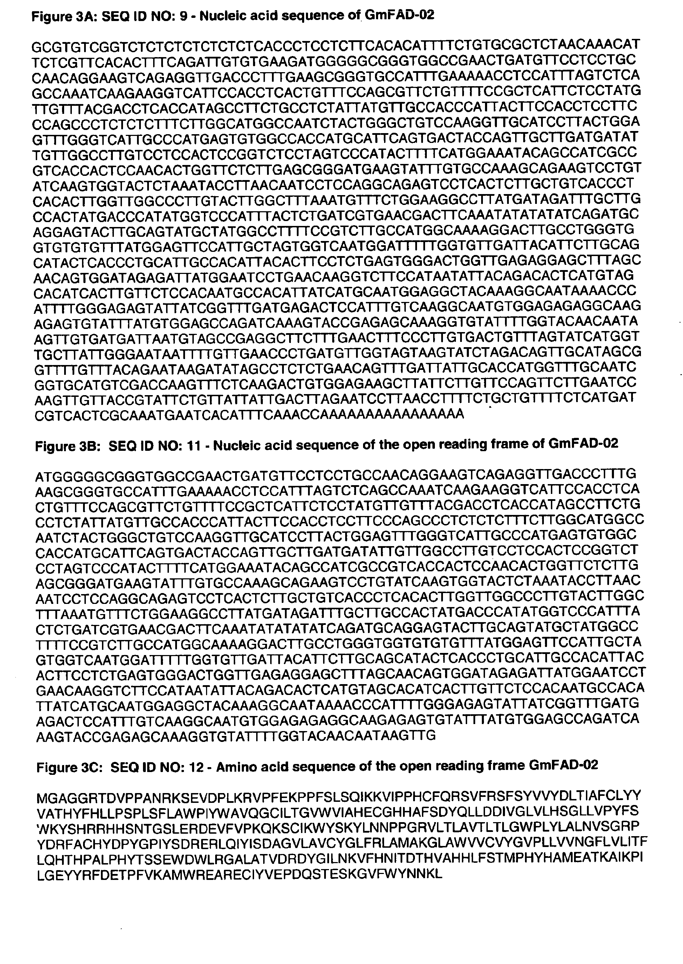

Figure 3A: SEQ ID NO: 9 - Nucleic acid sequence of GmFAD-02

Figure 3B: SEQ ID NO: 11 - Nucleic acid sequence of the open reading frame of GmFAD-02

Figure 3C: SEQ ID NO: 12 - Amino acid sequence of the open reading frame GmFAD-02

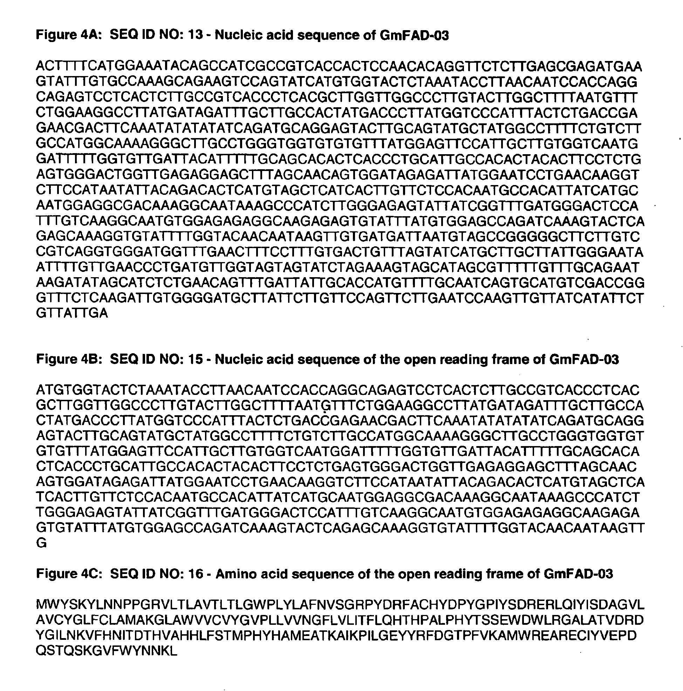

Figure 4A: SEQ ID NO: 12 - Nucleic acid sequence of GmFAD-03

Figure 4B: SEQ ID NO: 15 - Nucleic acid sequence of the open reading frame of GmFAD-03

Figure 4C: SEQ ID NO: 16 - Amino acid sequence of the open reading frame of GmFAD-03

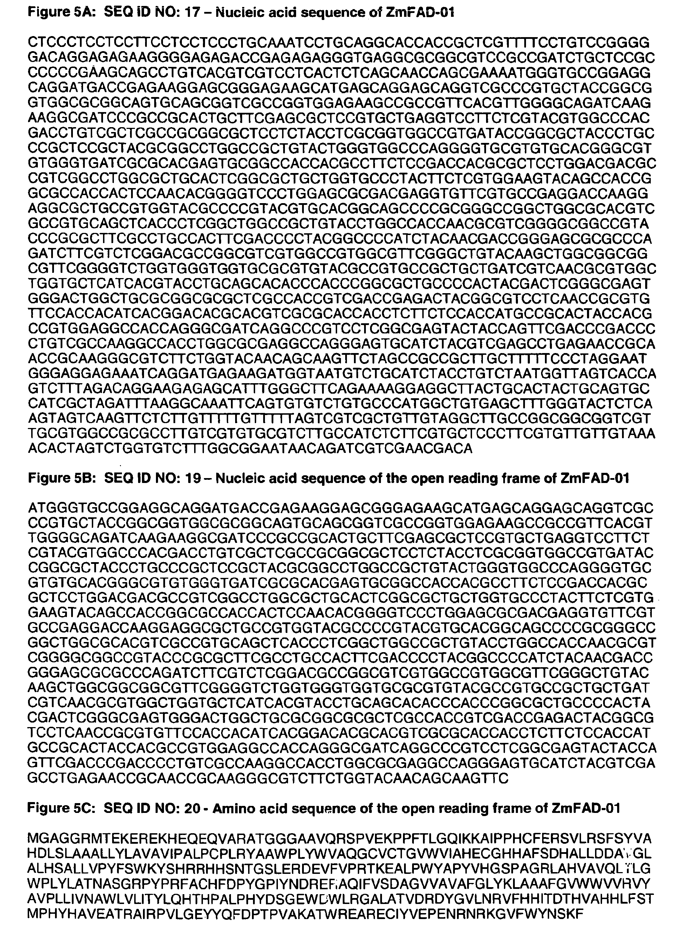

Figure 5A: SEQ ID NO: 17 - Nucleic acid sequence of ZmFAD-01

Figure 5B: SEQ ID NO: 19 - Nucleic acid sequence of the open reading frame of ZmFAD-01

Figure 5C: SEQ ID NO: 20 - Amino acid sequence of the open reading frame of ZmFAD-01

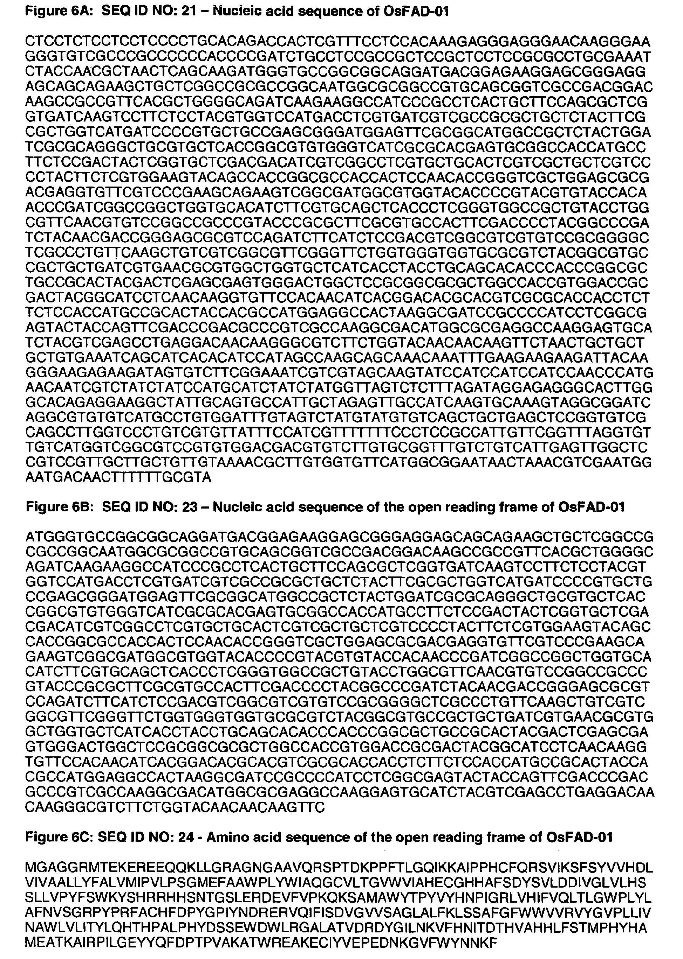

Figure 6A: SEQ ID NO: 21 - Nucleic acid sequence of OsFAD-01

Figure 6B: SEQ ID NO: 23 - Nucleic acid sequence of the open reading frame of OsFAD-01

Figure 6C: SEQ ID NO: 24 - Amino acid sequence of the open reading frame of OsFAD-01

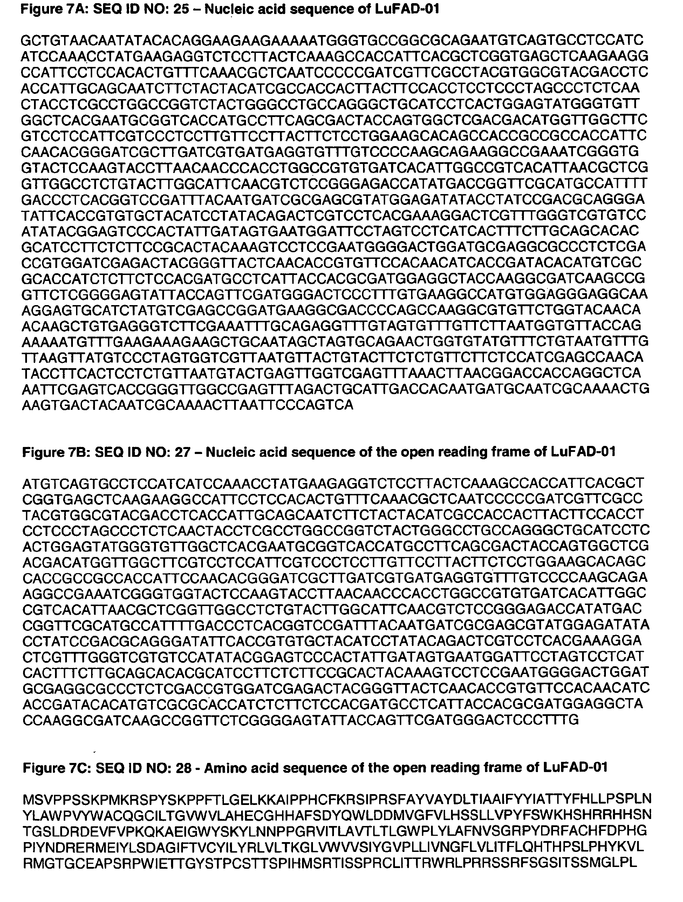

Figure 7A: SEQ ID NO: 25 - Nucleic acid sequence of LuFAD-01

Figure 7B: SEQ ID NO: 27 - Nucleic acid sequence of the open reading frame of LuFAD-01

Figure 7C: SEQ ID NO: 28 - Amino acid sequence of the open reading frame of LuFAD-01

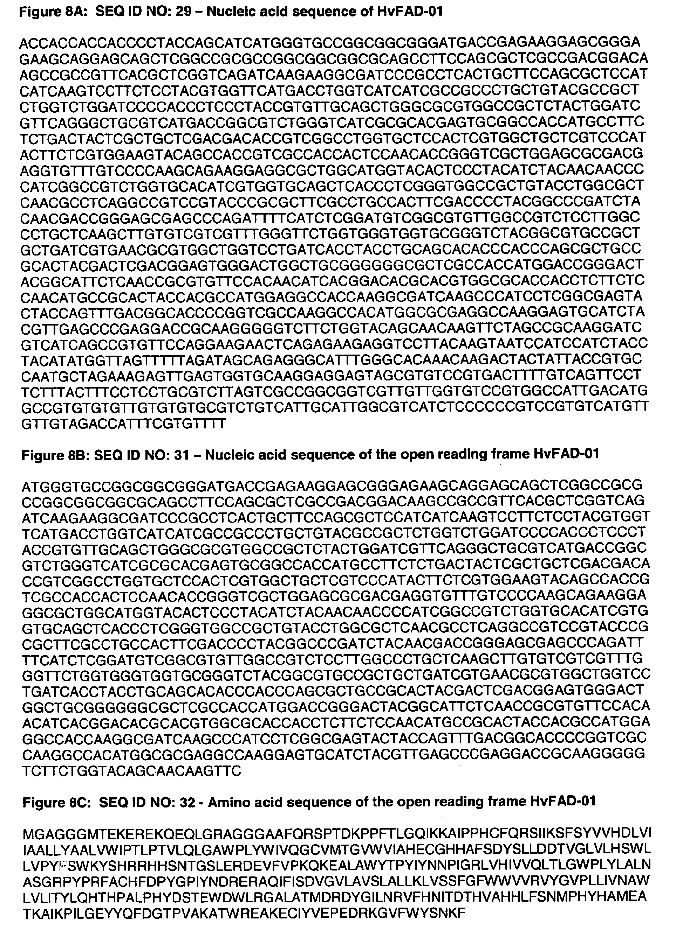

Figure 8A: SEQ ID NO: 29 - Nucleic acid sequence of HvFAD-01

Figure 8B: SEQ ID NO: 31 - Nucleic acid sequence of the open reading frame HvFAD-01

Figure 8C: SEQ ID NO: 32 - Amino acid sequence of the open reading frame HvFAD-01

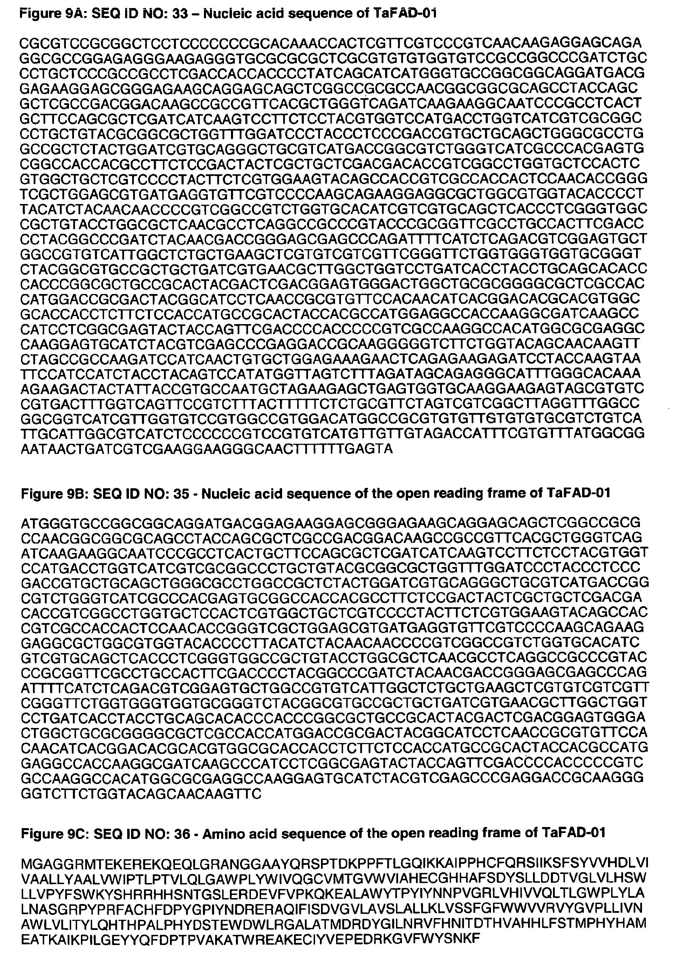

Figure 9A: SEQ ID NO: 33 - Nucleic acid sequence of TaFAD-01

Figure 9B: SEQ ID NO: 35 - Nucleic acid sequence of the open reading frame of TaFAD-01

Figure 9C: SEQ ID NO: 36 - Amino acid sequence of the open reading frame of TaFAD-01

## Claims

1. An isolated nucleic acid comprising a polynucleotide sequence selected from the group consisting of:
a polynucleotide sequence as depicted in SEQ ID NO: 21 and SEQ ID NO: 23;
a polynucleotide sequence encoding a polypeptide as depicted in SEQ ID NO: 24;
a polynucleotide sequence having at least 70% sequence identity with the nucleic acid of a) or b) above;
a polynucleotide sequence that is complementary to the nucleic acid of a) or b) above; and
a polynucleotide sequence that hybridizes under stringent conditions to the
nucleic acid of a) or b)above.

2. An isolated polypeptide encoded by a polynucleotide sequence as claimed in claim 1.

3. The isolated nucleic acid of claim 1, wherein the isolated nucleic acid encodes a polypeptide that functions as a modulator of a seed storage compound in plants.

4. The isolated polypeptide of claim 2, wherein the isolated LMP polypeptide sequence functions as a modulator of a seed storage compound in plants.

5. An expression vector containing the nucleic acid of Claims 1 or 3, wherein the nucleic acid is operatively linked to a promoter selected from the group consisting of a seed-specific promoter, a root-specific promoter and a non-tissue-specific promoter.

6. A method of producing a transgenic plant having a modified level of a seed storage compound weight percentage compared to the wildtype comprising,
a. a first step of introduction into a plant cell of an expression vector containing a nucleic acid and
b. a second step of generating from the plant cell the transgenic plant,
wherein the nucleic acid encodes a polypeptide that functions as a modulator of a seed storage compound in the plant, and wherein the nucleic acid comprises a polynucleotide sequence selected from the group consisting of:
a. a polynucleotide sequence as depicted in SEQ ID NO: 21;
b. a polynucleotide sequence encoding a polypeptide as depicted in SEQ ID NO: 24;
c. a polynucleotide sequence having at least 70% sequence identity with the nucleic acid of a) or b) above;
d. a polynucleotide sequence that is complementary to the nucleic acid of a) or b) above; and
e. a polynucleotide sequence that hybridizes under stringent conditions to the nucleic acid of a) or b) above.

7. The method of Claim 6, wherein the nucleic acid comprises a polynucleotide sequence having at least 90% sequence identity with the polynucleotide sequence of a) or b) of Claim 1.

8. The method of Claim 6, wherein the level of oleic acid is increased in the transgenic plant as compared to a wild type variety of the plant.

9. A method of modulating the level of a seed storage compound weight percentage in a plant, comprising
a. a first step of introduction into a plant cell of an expression vector containing a nucleic acid, and
b. a second step of generating from the plant cell the transgenic plant,
wherein the nucleic acid encodes a polypeptide that functions as a modulator of a seed storage compound in the plant wherein the nucleic acid comprises the polynucleotide sequence of claim 1.

10. The method of Claim 9, wherein the level of oleic acid weight percentage is modified.

11. A transgenic plant made by the method of claims 6 or 9.

12. The transgenic plant of Claim 11, wherein the level of oleic acid weight percentage is increased in the transgenic plant as compared to the wild type variety of the plant.

13. The transgenic plant of Claim 11, wherein the plant is selected from the group consisting of rapeseed, canola, linseed, soybean, sunflower, maize, oat, rye, barley, wheat, pepper, tagetes, cotton, oil palm, coconut palm, flax, castor, sugarbeet, rice and peanut.

14. A seed produced by the transgenic plant of Claim 11, wherein the plant expresses the polypeptide that functions as a modulator of a seed storage compound and wherein the plant is true breeding for a modified level of seed storage compound weight percentage as compared to a wild type variety of the plant.


SEQUENCE LISTING

<110>  BASF Plant Science GmbH
       Haertel, Heiko
       Gibson, Jermaine
       Ren, Peifeng

<120>  Nucleic acid molecules encoding fatty acid desaturase genes from
       plants and methods of use

<130>  PF2063015115

<150>  US 60/637531
<151>  2004-12-20

<160>  47

<170>  PatentIn version 3.3

<210>  1
<211>  1615
<212>  DNA
<213>  Arabidopsis thaliana

<220>
<221>  misc_feature
<222>  (1)..(156)

<220>
<221>  CDS
<222>  (157)..(1305)

<220>
<221>  misc_feature
<222>  (1306)..(1615)

<400>  1
gaccaccaga agaagagcca cacactcaca aattaaaaag agagagagag agagagagac      60

agagagagag agagattctg cggaggagct tcttcttcgt agggtgttca tcgttattaa     120

cgttatcgcc cctacgtcag ctccatctcc agaaac atg ggt gca ggt gga aga      174
                                        Met Gly Ala Gly Gly Arg
                                        1               5

atg ccg gtt cct act tct tcc aag aaa tcg gaa acc gac acc aca aag      222
Met Pro Val Pro Thr Ser Ser Lys Lys Ser Glu Thr Asp Thr Thr Lys
            10                  15                  20

cgt gtg ccg tgc gag aaa ccg cct ttc tcg gtg gga gat ctg aag aaa      270
Arg Val Pro Cys Glu Lys Pro Pro Phe Ser Val Gly Asp Leu Lys Lys
        25                  30                  35

gca atc ccg ccg [?]at tgt ttc aaa cgc tca atc cct cgc tct ttc tcc      318
Ala Ile Pro Pro His Cys Phe Lys Arg Ser Ile Pro Arg Ser Phe Ser
    40                  45                  50

tac ctt atc agt gac atc att ata gcc tca tgc ttc tac tac gtc gcc      366
Tyr Leu Ile Ser Asp Ile Ile Ile Ala Ser Cys Phe Tyr Tyr Val Ala
55                  60                  65                  70

acc aat tac ttc tct ctc ctc cct cag cct ctc tct tac ttg gct tgg 414
Thr Asn Tyr Phe Ser Leu Leu Pro Gln Pro Leu Ser Tyr Leu Ala Trp
                75                  80                  85

cca ctc tat tgg gcc tgt caa ggc tgt gtc cta act ggt atc tgg gtc 462
Pro Leu Tyr Trp Ala Cys Gln Gly Cys Val Leu Thr Gly Ile Trp Val
            90                  95                  100

ata gcc cac gaa tgc ggt cac cac gca ttc agc gac tac caa tgg ctg 510
Ile Ala His Glu Cys Gly His His Ala Phe Ser Asp Tyr Gln Trp Leu
        105                 110                 115

gat gac aca gtt ggt ctt atc ttc cat tcc ttc ctc ctc gtc cct tac 558
Asp Asp Thr Val Gly Leu Ile Phe His Ser Phe Leu Leu Val Pro Tyr
    120                 125                 130

ttc tcc tgg aag tat agt cat cgc cgt cac cat tcc aac act gga tcc 606
Phe Ser Trp Lys Tyr Ser His Arg Arg His His Ser Asn Thr Gly Ser
135                 140                 145                 150

ctc gaa aga gat gaa gta ttt gtc cca aag cag aaa tca gca atc aag 654
Leu Glu Arg Asp Glu Val Phe Val Pro Lys Gln Lys Ser Ala Ile Lys
                155                 160                 165

tgg tac ggg aaa tac ctc aac aac cct ctt gga cgc atc atg atg tta 702
Trp Tyr Gly Lys Tyr Leu Asn Asn Pro Leu Gly Arg Ile Met Met Leu
            170                 175                 180

acc gtc cag ttt gtc ctc ggg tgg ccc ttg tac tta gcc ttt aac gtc 750
Thr Val Gln Phe Val Leu Gly Trp Pro Leu Tyr Leu Ala Phe Asn Val
        185                 190                 195

tct ggc aga ccg tat gac ggg ttc gct tgc cat ttc ttc ccc aac gct 798
Ser Gly Arg Pro Tyr Asp Gly Phe Ala Cys His Phe Phe Pro Asn Ala
    200                 205                 210

ccc atc tac aat gac cga gaa cgc ctc cag ata tac ctc tct gat gcg 846
Pro Ile Tyr Asn Asp Arg Glu Arg Leu Gln Ile Tyr Leu Ser Asp Ala
215                 220                 225                 230

ggt att cta gcc gtc tgt ttt ggt ctt tac cgt tac gct gct gca caa 894
Gly Ile Leu Ala Val Cys Phe Gly Leu Tyr Arg Tyr Ala Ala Ala Gln
                235                 240                 245

ggg atg gcc tcg atg atc tgc ctc tac gga gta ccg ctt ctg ata gtg 942
Gly Met Ala Ser Met Ile Cys Leu Tyr Gly Val Pro Leu Leu Ile Val
            250                 255                 260

aat gcg ttc ctc gtc ttg atc act tac ttg cag cac act cat ccc tcg 990
Asn Ala Phe Leu Val Leu Ile Thr Tyr Leu Gln His Thr His Pro Ser
        265                 270                 275

ttg cct cac tac gat tca tca gag tgg gac tgg ctc agg gga gct ttg 1038
Leu Pro His Tyr Asp Ser Ser Glu Trp Asp Trp Leu Arg Gly Ala Leu
    280                 285                 290

gct acc gta gac aga gac tac gga atc ttg aac aag gtg ttc cac aac 1086
Ala Thr Val Asp Arg Asp Tyr Gly Ile Leu Asn Lys Val Phe His Asn
295                 300                 305                 310

att aca gac aca cac gtg gct cat cac ctg ttc tcg aca atg ccg cat 1134

Ile Thr Asp Thr His Val Ala His His Leu Phe Ser Thr Met Pro His
                315                 320                 325

tat aac gca atg gaa gct aca aag gcg ata aag cca att ctg gga gac      1182
Tyr Asn Ala Met Glu Ala Thr Lys Ala Ile Lys Pro Ile Leu Gly Asp
            330                 335                 340

tat tac cag ttc gat gga aca ccg tgg tat gta gcg atg tat agg gag      1230
Tyr Tyr Gln Phe Asp Gly Thr Pro Trp Tyr Val Ala Met Tyr Arg Glu
        345                 350                 355

gca aag gag tgt atc tat gta gaa ccg gac agg gaa ggt gac aag aaa      1278
Ala Lys Glu Cys Ile Tyr Val Glu Pro Asp Arg Glu Gly Asp Lys Lys
    360                 365                 370

ggt gtg tac tgg tac aac aat aag tta tgaggatgat ggtgaagaaa            1325
Gly Val Tyr Trp Tyr Asn Asn Lys Leu
375                 380

ttgtcgacct ttctcttgtc tgtttgtctt ttgttaaaga agctatgctt cgttttaata    1385

atcttattgt ccattttgtt gtgttatgac attttggctg ctcattatgt tatgtgggaa    1445

gttagtgttc aaatgttttg tgtcggtatt gttcttctca tcgctgtttt gttgggatcg    1505

tagaaatgtg accttcggac agtaaaactc ttgtactaaa actatctccc tattggcatt    1565

tcttaaactt ttaatagtta cgtgctcgta gtgaatcttg acttgagtca               1615

<210>  2
<211>  383
<212>  PRT
<213>  Arabidopsis thaliana

<400>  2

Met Gly Ala Gly Gly Arg Met Pro Val Pro Thr Ser Ser Lys Lys Ser
1               5                   10                  15

Glu Thr Asp Thr Thr Lys Arg Val Pro Cys Glu Lys Pro Pro Phe Ser
            20                  25                  30

Val Gly Asp Leu Lys Lys Ala Ile Pro Pro His Cys Phe Lys Arg Ser
        35                  40                  45

Ile Pro Arg Ser Phe Ser Tyr Leu Ile Ser Asp Ile Ile Ile Ala Ser
    50                  55                  60

Cys Phe Tyr Tyr Val Ala Thr Asn Tyr Phe Ser Leu Leu Pro Gln Pro
65                  70                  75                  80

Leu Ser Tyr Leu Ala Trp Pro Leu Tyr Trp Ala Cys Gln Gly Cys Val
                85                  90                  95

Leu Thr Gly Ile Trp Val Ile Ala His Glu Cys Gly His His Ala Phe

            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Gln Lys Ser Ala Ile Lys Trp Tyr Gly Lys Tyr Leu Asn Asn Pro Leu
                165                 170                 175

Gly Arg Ile Met Met Leu Thr Val Gln Phe Val Leu Gly Trp Pro Leu
            180                 185                 190

Tyr Leu Ala Phe Asn Val Ser Gly Arg Pro Tyr Asp Gly Phe Ala Cys
        195                 200                 205

His Phe Phe Pro Asn Ala Pro Ile Tyr Asn Asp Arg Glu Arg Leu Gln
    210                 215                 220

Ile Tyr Leu Ser Asp Ala Gly Ile Leu Ala Val Cys Phe Gly Leu Tyr
225                 230                 235                 240

Arg Tyr Ala Ala Ala Gln Gly Met Ala Ser Met Ile Cys Leu Tyr Gly
                245                 250                 255

Val Pro Leu Leu Ile Val Asn Ala Phe Leu Val Leu Ile Thr Tyr Leu
            260                 265                 270

Gln His Thr His Pro Ser Leu Pro His Tyr Asp Ser Ser Glu Trp Asp
        275                 280                 285

Trp Leu Arg Gly Ala Leu Ala Thr Val Asp Arg Asp Tyr Gly Ile Leu
    290                 295                 300

Asn Lys Val Phe His Asn Ile Thr Asp Thr His Val Ala His His Leu
305                 310                 315                 320

Phe Ser Thr Met Pro His Tyr Asn Ala Met Glu Ala Thr Lys Ala Ile
                325                 330                 335

Lys Pro Ile Leu Gly Asp Tyr Tyr Gln Phe Asp Gly Thr Pro Trp Tyr
            340                 345                 350

Val Ala Met Tyr Arg Glu Ala Lys Glu Cys Ile Tyr Val Glu Pro Asp
        355                 360                 365

Arg Glu Gly Asp Lys Lys Gly Val Tyr Trp Tyr Asn Asn Lys Leu
    370                 375                 380

<210> 3
<211> 1149
<212> DNA
<213> Arabidopsis thaliana

<220>
<221> CDS
<222> (1)..(1149)

<400> 3
atg ggt gca ggt gga aga atg ccg gtt cct act tct tcc aag aaa tcg 48
Met Gly Ala Gly Gly Arg Met Pro Val Pro Thr Ser Ser Lys Lys Ser
1               5                   10                  15

gaa acc gac acc aca aag cgt gtg ccg tgc gag aaa ccg cct ttc tcg 96
Glu Thr Asp Thr Thr Lys Arg Val Pro Cys Glu Lys Pro Pro Phe Ser
            20                  25                  30

gtg gga gat ctg aag aaa gca atc ccg ccg cat tgt ttc aaa cgc tca 144
Val Gly Asp Leu Lys Lys Ala Ile Pro Pro His Cys Phe Lys Arg Ser
        35                  40                  45

atc cct cgc tct ttc tcc tac ctt atc agt gac atc att ata gcc tca 192
Ile Pro Arg Ser Phe Ser Tyr Leu Ile Ser Asp Ile Ile Ile Ala Ser
    50                  55                  60

tgc ttc tac tac gtc gcc acc aat tac ttc tct ctc ctc cct cag cct 240
Cys Phe Tyr Tyr Val Ala Thr Asn Tyr Phe Ser Leu Leu Pro Gln Pro
65                  70                  75                  80

ctc tct tac ttg gct tgg cca ctc tat tgg gcc tgt caa ggc tgt gtc 288
Leu Ser Tyr Leu Ala Trp Pro Leu Tyr Trp Ala Cys Gln Gly Cys Val
                85                  90                  95

cta act ggt atc tgg gtc ata gcc cac gaa tgc ggt cac cac gca ttc 336
Leu Thr Gly Ile Trp Val Ile Ala His Glu Cys Gly His His Ala Phe
            100                 105                 110

agc gac tac caa tgg ctg gat gac aca gtt ggt ctt atc ttc cat tcc 384
Ser Asp Tyr Gln Trp Leu Asp Asp Thr Val Gly Leu Ile Phe His Ser
        115                 120                 125

ttc ctc ctc gtc cct tac ttc tcc tgg aag tat agt cat cgc cgt cac 432
Phe Leu Leu Val Pro Tyr Phe Ser Trp Lys Tyr Ser His Arg Arg His
    130                 135                 140

cat tcc aac act gga tcc ctc gaa aga gat gaa gta ttt gtc cca aag 480
His Ser Asn Thr Gly Ser Leu Glu Arg Asp Glu Val Phe Val Pro Lys
145                 150                 155                 160

cag aaa tca gca atc aag tgg tac ggg aaa tac ctc aac aac cct ctt 528
Gln Lys Ser Ala Ile Lys Trp Tyr Gly Lys Tyr Leu Asn Asn Pro Leu

165                 170                 175

gga cgc atc atg atg tta acc gtc cag ttt gtc ctc ggg tgg ccc ttg     576
Gly Arg Ile Met Met Leu Thr Val Gln Phe Val Leu Gly Trp Pro Leu
        180                 185                 190

tac tta gcc ttt aac gtc tct ggc aga ccg tat gac ggg ttc gct tgc     624
Tyr Leu Ala Phe Asn Val Ser Gly Arg Pro Tyr Asp Gly Phe Ala Cys
    195                 200                 205

cat ttc ttc ccc aac gct ccc atc tac aat gac cga gaa cgc ctc cag     672
His Phe Phe Pro Asn Ala Pro Ile Tyr Asn Asp Arg Glu Arg Leu Gln
    210                 215                 220

ata tac ctc tct gat gcg ggt att cta gcc gtc tgt ttt ggt ctt tac     720
Ile Tyr Leu Ser Asp Ala Gly Ile Leu Ala Val Cys Phe Gly Leu Tyr
225                 230                 235                 240

cgt tac gct gct gca caa ggg atg gcc tcg atg atc tgc ctc tac gga     768
Arg Tyr Ala Ala Ala Gln Gly Met Ala Ser Met Ile Cys Leu Tyr Gly
                245                 250                 255

gta ccg ctt ctg ata gtg aat gcg ttc ctc gtc ttg atc act tac ttg     816
Val Pro Leu Leu Ile Val Asn Ala Phe Leu Val Leu Ile Thr Tyr Leu
            260                 265                 270

cag cac act cat ccc tcg ttg cct cac tac gat tca tca gag tgg gac     864
Gln His Thr His Pro Ser Leu Pro His Tyr Asp Ser Ser Glu Trp Asp
        275                 280                 285

tgg ctc agg gga gct ttg gct acc gta gac aga gac tac gga atc ttg     912
Trp Leu Arg Gly Ala Leu Ala Thr Val Asp Arg Asp Tyr Gly Ile Leu
    290                 295                 300

aac aag gtg ttc cac aac att aca gac aca cac gtg gct cat cac ctg     960
Asn Lys Val Phe His Asn Ile Thr Asp Thr His Val Ala His His Leu
305                 310                 315                 320

ttc tcg aca atg ccg cat tat aac gca atg gaa gct aca aag gcg ata     1008
Phe Ser Thr Met Pro His Tyr Asn Ala Met Glu Ala Thr Lys Ala Ile
                325                 330                 335

aag cca att ctg gga gac tat tac cag ttc gat gga aca ccg tgg tat     1056
Lys Pro Ile Leu Gly Asp Tyr Tyr Gln Phe Asp Gly Thr Pro Trp Tyr
            340                 345                 350

gta gcg atg tat agg gag gca aag gag tgt atc tat gta gaa ccg gac     1104
Val Ala Met Tyr Arg Glu Ala Lys Glu Cys Ile Tyr Val Glu Pro Asp
        355                 360                 365

agg gaa ggt gac aag aaa ggt gtg tac tgg tac aac aat aag tta         1149
Arg Glu Gly Asp Lys Lys Gly Val Tyr Trp Tyr Asn Asn Lys Leu
    370                 375                 380

<210>  4
<211>  383
<212>  PRT
<213>  Arabidopsis thaliana

<400>  4

Met Gly Ala Gly Gly Arg Met Pro Val Pro Thr Ser Ser Lys Lys Ser
1               5                   10                  15

Glu Thr Asp Thr Thr Lys Arg Val Pro Cys Glu Lys Pro Pro Phe Ser
            20                  25                  30

Val Gly Asp Leu Lys Lys Ala Ile Pro Pro His Cys Phe Lys Arg Ser
        35                  40                  45

Ile Pro Arg Ser Phe Ser Tyr Leu Ile Ser Asp Ile Ile Ile Ala Ser
    50                  55                  60

Cys Phe Tyr Tyr Val Ala Thr Asn Tyr Phe Ser Leu Leu Pro Gln Pro
65                  70                  75                  80

Leu Ser Tyr Leu Ala Trp Pro Leu Tyr Trp Ala Cys Gln Gly Cys Val
                85                  90                  95

Leu Thr Gly Ile Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Gln Lys Ser Ala Ile Lys Trp Tyr Gly Lys Tyr Leu Asn Asn Pro Leu
                165                 170                 175

Gly Arg Ile Met Met Leu Thr Val Gln Phe Val Leu Gly Trp Pro Leu
            180                 185                 190

Tyr Leu Ala Phe Asn Val Ser Gly Arg Pro Tyr Asp Gly Phe Ala Cys
        195                 200                 205

His Phe Phe Pro Asn Ala Pro Ile Tyr Asn Asp Arg Glu Arg Leu Gln
    210                 215                 220

Ile Tyr Leu Ser Asp Ala Gly Ile Leu Ala Val Cys Phe Gly Leu Tyr
225                 230                 235                 240

Arg Tyr Ala Ala Ala Gln Gly Met Ala Ser Met Ile Cys Leu Tyr Gly
                245                 250                 255

Val Pro Leu Leu Ile Val Asn Ala Phe Leu Val Leu Ile Thr Tyr Leu
            260                 265                 270

Gln His Thr His Pro Ser Leu Pro His Tyr Asp Ser Ser Glu Trp Asp
        275                 280                 285

Trp Leu Arg Gly Ala Leu Ala Thr Val Asp Arg Asp Tyr Gly Ile Leu
    290                 295                 300

Asn Lys Val Phe His Asn Ile Thr Asp Thr His Val Ala His His Leu
305                 310                 315                 320

Phe Ser Thr Met Pro His Tyr Asn Ala Met Glu Ala Thr Lys Ala Ile
                325                 330                 335

Lys Pro Ile Leu Gly Asp Tyr Tyr Gln Phe Asp Gly Thr Pro Trp Tyr
            340                 345                 350

Val Ala Met Tyr Arg Glu Ala Lys Glu Cys Ile Tyr Val Glu Pro Asp
        355                 360                 365

Arg Glu Gly Asp Lys Lys Gly Val Tyr Trp Tyr Asn Asn Lys Leu
    370                 375                 380

<210>  5
<211>  1491
<212>  DNA
<213>  Glycine max

<220>
<221>  misc_feature
<222>  (1)..(114)

<220>
<221>  CDS
<222>  (115)..(1275)

<220>
<221>  misc_feature
<222>  (1276)..(1491)

<400>  5
taggcaccta gctagtagct acaatatcag cacttctctc tattgataaa caattggctg      60

taatgccgca gtagaggacg atcacaacat ttcgtgctgg atactttttg tttt atg      117
                                                          Met
                                                          1

ggt cta gca aag gaa aca ata atg gga ggt gga ggc cgt gtg gcc aaa      165
Gly Leu Ala Lys Glu Thr Ile Met Gly Gly Gly Gly Arg Val Ala Lys
            5                   10                  15

gtt gaa att cag cag aag aag cct ctc tca agg gtt cca aac aca aag 213
Val Glu Ile Gln Gln Lys Lys Pro Leu Ser Arg Val Pro Asn Thr Lys
        20                  25                  30

cca cca ttc act gtt ggc caa ctc aag aaa gcc att cca ccg cac tgc 261
Pro Pro Phe Thr Val Gly Gln Leu Lys Lys Ala Ile Pro Pro His Cys
    35                  40                  45

ttt cag cgt tcc ctc ctc act tca ttg tcc tat gtt gtt tat gac ctt 309
Phe Gln Arg Ser Leu Leu Thr Ser Leu Ser Tyr Val Val Tyr Asp Leu
50                  55                  60                  65

tca ttg gct ttc att ttc tac att gcc acc acc tac ttc cac ctc ctc 357
Ser Leu Ala Phe Ile Phe Tyr Ile Ala Thr Thr Tyr Phe His Leu Leu
                70                  75                  80

cct cac ccc ttt tcc ctc att gca tgg cca atc tat tgg gtt ctc caa 405
Pro His Pro Phe Ser Leu Ile Ala Trp Pro Ile Tyr Trp Val Leu Gln
            85                  90                  95

ggt tgc att ctt act ggc gtg tgg gtg att gct cac gag tgt ggt cac 453
Gly Cys Ile Leu Thr Gly Val Trp Val Ile Ala His Glu Cys Gly His
        100                 105                 110

cat gcc ttc agc aag tac cca tgg gtt gat gat gtt atg ggt ttg acc 501
His Ala Phe Ser Lys Tyr Pro Trp Val Asp Asp Val Met Gly Leu Thr
    115                 120                 125

gtt cac tca gca ctt tta gtc cct tat ttc tca tgg aaa ata agc cat 549
Val His Ser Ala Leu Leu Val Pro Tyr Phe Ser Trp Lys Ile Ser His
130                 135                 140                 145

cgc cgc cac cac tcc aac acg ggt tcc ctt gac cgt gat gaa gtg ttt 597
Arg Arg His His Ser Asn Thr Gly Ser Leu Asp Arg Asp Glu Val Phe
                150                 155                 160

gtc cca aaa cca aaa tcc aaa gtt gca tgg tac acc aag tac ctg aac 645
Val Pro Lys Pro Lys Ser Lys Val Ala Trp Tyr Thr Lys Tyr Leu Asn
            165                 170                 175

aac cct cta gga agg gct gct tct ctt ctc atc aca ctc aca ata ggg 693
Asn Pro Leu Gly Arg Ala Ala Ser Leu Leu Ile Thr Leu Thr Ile Gly
        180                 185                 190

tgg cct atg tat tta gcc ttc aat gtc tct ggc aga ccc tat gat ggt 741
Trp Pro Met Tyr Leu Ala Phe Asn Val Ser Gly Arg Pro Tyr Asp Gly
    195                 200                 205

ttt gca agc cac tac cac cct tat gct ccc ata tat tct aac cgt gag 789
Phe Ala Ser His Tyr His Pro Tyr Ala Pro Ile Tyr Ser Asn Arg Glu
210                 215                 220                 225

agg ctt ctg atc tat gtc tct gat gtt gct ttg ttt tct gtg act tac 837
Arg Leu Leu Ile Tyr Val Ser Asp Val Ala Leu Phe Ser Val Thr Tyr
                230                 235                 240

tct ctc tac cgt gtt gca act atg aaa ggg ttg gtt tgg ctg cta tgt 885
Ser Leu Tyr Arg Val Ala Thr Met Lys Gly Leu Val Trp Leu Leu Cys
            245                 250                 255

gtt tat ggg gtg cca ttg ctc att gtg aac ggt ttt ctt gtg act atc 933

Val Tyr Gly Val Pro Leu Leu Ile Val Asn Gly Phe Leu Val Thr Ile
        260                 265                 270

aca tat ttg cag cac aca cac ttt gcc ttg cct cat tac gat tca tca      981
Thr Tyr Leu Gln His Thr His Phe Ala Leu Pro His Tyr Asp Ser Ser
    275                 280                 285

gaa tgg gac tgg ctg aag gga gct ttg gca act atg gac aga gat tat      1029
Glu Trp Asp Trp Leu Lys Gly Ala Leu Ala Thr Met Asp Arg Asp Tyr
290                 295                 300                 305

ggg att ctg aac aag gtg ttt cat cac ata act gat act cat gtg gct      1077
Gly Ile Leu Asn Lys Val Phe His His Ile Thr Asp Thr His Val Ala
                310                 315                 320

cac cat ctc ttc tct aca atg cca cat tac cat gca atg gag gca acc      1125
His His Leu Phe Ser Thr Met Pro His Tyr His Ala Met Glu Ala Thr
            325                 330                 335

aat gca atc aag cca ata ttg ggt gag tac tac caa ttt gat gac aca      1173
Asn Ala Ile Lys Pro Ile Leu Gly Glu Tyr Tyr Gln Phe Asp Asp Thr
        340                 345                 350

cca ttt tac aag gca ctg tgg aga gaa gcg aga gag tgc ctc tat gtg      1221
Pro Phe Tyr Lys Ala Leu Trp Arg Glu Ala Arg Glu Cys Leu Tyr Val
    355                 360                 365

gag cca gat gaa gga aca tcc gag aag ggc gtg tat tgg tac agg aac      1269
Glu Pro Asp Glu Gly Thr Ser Glu Lys Gly Val Tyr Trp Tyr Arg Asn
370                 375                 380                 385

aag tat tgatggacca agcaatgggc catagtggga gttatggaag ttttgtcact      1325
Lys Tyr

tatcacttaa ttagtagaat gttataaata agtggatttg ccgcgtaatg acttgtgtgc   1385

attgtgaaac agcttgtagc gatccatggt tataatgtaa aaatatgtgg aaaggggtct   1445

ggttaaaaaa aaaaaaaaaa aagcggccgt tttaaaggaa acaagg                  1491

<210>  6
<211>  387
<212>  PRT
<213>  Glycine max

<400>  6

Met Gly Leu Ala Lys Glu Thr Ile Met Gly Gly Gly Gly Arg Val Ala
1               5                   10                  15

Lys Val Glu Ile Gln Gln Lys Lys Pro Leu Ser Arg Val Pro Asn Thr
            20                  25                  30

Lys Pro Pro Phe Thr Val Gly Gln Leu Lys Lys Ala Ile Pro Pro His
        35                  40                  45

Cys Phe Gln Arg Ser Leu Leu Thr Ser Leu Ser Tyr Val Val Tyr Asp

50                  55                  60

Leu Ser Leu Ala Phe Ile Phe Tyr Ile Ala Thr Thr Tyr Phe His Leu
65                  70                  75                  80

Leu Pro His Pro Phe Ser Leu Ile Ala Trp Pro Ile Tyr Trp Val Leu
                85                  90                  95

Gln Gly Cys Ile Leu Thr Gly Val Trp Val Ile Ala His Glu Cys Gly
            100                 105                 110

His His Ala Phe Ser Lys Tyr Pro Trp Val Asp Asp Val Met Gly Leu
        115                 120                 125

Thr Val His Ser Ala Leu Leu Val Pro Tyr Phe Ser Trp Lys Ile Ser
    130                 135                 140

His Arg Arg His His Ser Asn Thr Gly Ser Leu Asp Arg Asp Glu Val
145                 150                 155                 160

Phe Val Pro Lys Pro Lys Ser Lys Val Ala Trp Tyr Thr Lys Tyr Leu
                165                 170                 175

Asn Asn Pro Leu Gly Arg Ala Ala Ser Leu Leu Ile Thr Leu Thr Ile
            180                 185                 190

Gly Trp Pro Met Tyr Leu Ala Phe Asn Val Ser Gly Arg Pro Tyr Asp
        195                 200                 205

Gly Phe Ala Ser His Tyr His Pro Tyr Ala Pro Ile Tyr Ser Asn Arg
    210                 215                 220

Glu Arg Leu Leu Ile Tyr Val Ser Asp Val Ala Leu Phe Ser Val Thr
225                 230                 235                 240

Tyr Ser Leu Tyr Arg Val Ala Thr Met Lys Gly Leu Val Trp Leu Leu
                245                 250                 255

Cys Val Tyr Gly Val Pro Leu Leu Ile Val Asn Gly Phe Leu Val Thr
            260                 265                 270

Ile Thr Tyr Leu Gln His Thr His Phe Ala Leu Pro His Tyr Asp Ser
        275                 280                 285

Ser Glu Trp Asp Trp Leu Lys Gly Ala Leu Ala Thr Met Asp Arg Asp
    290                 295                 300

Tyr Gly Ile Leu Asn Lys Val Phe His His Ile Thr Asp Thr His Val
305                 310                 315                 320

Ala His His Leu Phe Ser Thr Met Pro His Tyr His Ala Met Glu Ala
                325                 330                 335

Thr Asn Ala Ile Lys Pro Ile Leu Gly Glu Tyr Tyr Gln Phe Asp Asp
            340                 345                 350

Thr Pro Phe Tyr Lys Ala Leu Trp Arg Glu Ala Arg Glu Cys Leu Tyr
        355                 360                 365

Val Glu Pro Asp Glu Gly Thr Ser Glu Lys Gly Val Tyr Trp Tyr Arg
    370                 375                 380

Asn Lys Tyr
385

<210>  7
<211>  1161
<212>  DNA
<213>  Glycine max

<220>
<221>  CDS
<222>  (1)..(1161)

<400>  7
atg ggt cta gca aag gaa aca ata atg gga ggt gga ggc cgt gtg gcc      48
Met Gly Leu Ala Lys Glu Thr Ile Met Gly Gly Gly Gly Arg Val Ala
1               5                   10                  15

aaa gtt gaa att cag cag aag aag cct ctc tca agg gtt cca aac aca      96
Lys Val Glu Ile Gln Gln Lys Lys Pro Leu Ser Arg Val Pro Asn Thr
            20                  25                  30

aag cca cca ttc act gtt ggc caa ctc aag aaa gcc att cca ccg cac      144
Lys Pro Pro Phe Thr Val Gly Gln Leu Lys Lys Ala Ile Pro Pro His
        35                  40                  45

tgc ttt cag cgt tcc ctc ctc act tca ttg tcc tat gtt gtt tat gac      192
Cys Phe Gln Arg Ser Leu Leu Thr Ser Leu Ser Tyr Val Val Tyr Asp
    50                  55                  60

ctt tca ttg gct ttc att ttc tac att gcc acc acc tac ttc cac ctc      240
Leu Ser Leu Ala Phe Ile Phe Tyr Ile Ala Thr Thr Tyr Phe His Leu
65                  70                  75                  80

ctc cct cac ccc ttt tcc ctc att gca tgg cca atc tat tgg gtt ctc      288
Leu Pro His Pro Phe Ser Leu Ile Ala Trp Pro Ile Tyr Trp Val Leu
                85                  90                  95

caa ggt tgc att ctt act ggc gtg tgg gtg att gct cac gag tgt ggt      336
Gln Gly Cys Ile Leu Thr Gly Val Trp Val Ile Ala His Glu Cys Gly

            100                 105                 110

cac cat gcc ttc agc aag tac cca tgg gtt gat gat gtt atg ggt ttg 384
His His Ala Phe Ser Lys Tyr Pro Trp Val Asp Asp Val Met Gly Leu
        115                 120                 125

acc gtt cac tca gca ctt tta gtc cct tat ttc tca tgg aaa ata agc 432
Thr Val His Ser Ala Leu Leu Val Pro Tyr Phe Ser Trp Lys Ile Ser
    130                 135                 140

cat cgc cgc cac cac tcc aac acg ggt tcc ctt gac cgt gat gaa gtg 480
His Arg Arg His His Ser Asn Thr Gly Ser Leu Asp Arg Asp Glu Val
145                 150                 155                 160

ttt gtc cca aaa cca aaa tcc aaa gtt gca tgg tac acc aag tac ctg 528
Phe Val Pro Lys Pro Lys Ser Lys Val Ala Trp Tyr Thr Lys Tyr Leu
                165                 170                 175

aac aac cct cta gga agg gct gct tct ctt ctc atc aca ctc aca ata 576
Asn Asn Pro Leu Gly Arg Ala Ala Ser Leu Leu Ile Thr Leu Thr Ile
            180                 185                 190

ggg tgg cct atg tat tta gcc ttc aat gtc tct ggc aga ccc tat gat 624
Gly Trp Pro Met Tyr Leu Ala Phe Asn Val Ser Gly Arg Pro Tyr Asp
        195                 200                 205

ggt ttt gca agc cac tac cac cct tat gct ccc ata tat tct aac cgt 672
Gly Phe Ala Ser His Tyr His Pro Tyr Ala Pro Ile Tyr Ser Asn Arg
    210                 215                 220

gag agg ctt ctg atc tat gtc tct gat gtt gct ttg ttt tct gtg act 720
Glu Arg Leu Leu Ile Tyr Val Ser Asp Val Ala Leu Phe Ser Val Thr
225                 230                 235                 240

tac tct ctc tac cgt gtt gca act atg aaa ggg ttg gtt tgg ctg cta 768
Tyr Ser Leu Tyr Arg Val Ala Thr Met Lys Gly Leu Val Trp Leu Leu
                245                 250                 255

tgt gtt tat ggg gtg cca ttg ctc att gtg aac ggt ttt ctt gtg act 816
Cys Val Tyr Gly Val Pro Leu Leu Ile Val Asn Gly Phe Leu Val Thr
            260                 265                 270

atc aca tat ttg cag cac aca cac ttt gcc ttg cct cat tac gat tca 864
Ile Thr Tyr Leu Gln His Thr His Phe Ala Leu Pro His Tyr Asp Ser
        275                 280                 285

tca gaa tgg gac tgg ctg aag gga gct ttg gca act atg gac aga gat 912
Ser Glu Trp Asp Trp Leu Lys Gly Ala Leu Ala Thr Met Asp Arg Asp
    290                 295                 300

tat ggg att ctg aac aag gtg ttt cat cac ata act gat act cat gtg 960
Tyr Gly Ile Leu Asn Lys Val Phe His His Ile Thr Asp Thr His Val
305                 310                 315                 320

gct cac cat ctc ttc tct aca atg cca cat tac cat gca atg gag gca 1008
Ala His His Leu Phe Ser Thr Met Pro His Tyr His Ala Met Glu Ala
                325                 330                 335

acc aat gca atc aag cca ata ttg ggt gag tac tac caa ttt gat gac 1056
Thr Asn Ala Ile Lys Pro Ile Leu Gly Glu Tyr Tyr Gln Phe Asp Asp
            340                 345                 350

aca cca ttt tac aag gca ctg tgg aga gaa gcg aga gag tgc ctc tat     1104
Thr Pro Phe Tyr Lys Ala Leu Trp Arg Glu Ala Arg Glu Cys Leu Tyr
        355                 360                 365

gtg gag cca gat gaa gga aca tcc gag aag ggc gtg tat tgg tac agg     1152
Val Glu Pro Asp Glu Gly Thr Ser Glu Lys Gly Val Tyr Trp Tyr Arg
    370                 375                 380

aac aag tat                                                         1161
Asn Lys Tyr
385

<210>  8
<211>  387
<212>  PRT
<213>  Glycine max

<400>  8

Met Gly Leu Ala Lys Glu Thr Ile Met Gly Gly Gly Gly Arg Val Ala
1               5                   10                  15

Lys Val Glu Ile Gln Gln Lys Lys Pro Leu Ser Arg Val Pro Asn Thr
            20                  25                  30

Lys Pro Pro Phe Thr Val Gly Gln Leu Lys Lys Ala Ile Pro Pro His
        35                  40                  45

Cys Phe Gln Arg Ser Leu Leu Thr Ser Leu Ser Tyr Val Val Tyr Asp
    50                  55                  60

Leu Ser Leu Ala Phe Ile Phe Tyr Ile Ala Thr Thr Tyr Phe His Leu
65                  70                  75                  80

Leu Pro His Pro Phe Ser Leu Ile Ala Trp Pro Ile Tyr Trp Val Leu
                85                  90                  95

Gln Gly Cys Ile Leu Thr Gly Val Trp Val Ile Ala His Glu Cys Gly
            100                 105                 110

His His Ala Phe Ser Lys Tyr Pro Trp Val Asp Asp Val Met Gly Leu
        115                 120                 125

Thr Val His Ser Ala Leu Leu Val Pro Tyr Phe Ser Trp Lys Ile Ser
    130                 135                 140

His Arg Arg His His Ser Asn Thr Gly Ser Leu Asp Arg Asp Glu Val
145                 150                 155                 160

Phe Val Pro Lys Pro Lys Ser Lys Val Ala Trp Tyr Thr Lys Tyr Leu
                165                 170                 175

Asn Asn Pro Leu Gly Arg Ala Ala Ser Leu Leu Ile Thr Leu Thr Ile
            180                 185                 190

Gly Trp Pro Met Tyr Leu Ala Phe Asn Val Ser Gly Arg Pro Tyr Asp
        195                 200                 205

Gly Phe Ala Ser His Tyr His Pro Tyr Ala Pro Ile Tyr Ser Asn Arg
    210                 215                 220

Glu Arg Leu Leu Ile Tyr Val Ser Asp Val Ala Leu Phe Ser Val Thr
225                 230                 235                 240

Tyr Ser Leu Tyr Arg Val Ala Thr Met Lys Gly Leu Val Trp Leu Leu
                245                 250                 255

Cys Val Tyr Gly Val Pro Leu Leu Ile Val Asn Gly Phe Leu Val Thr
            260                 265                 270

Ile Thr Tyr Leu Gln His Thr His Phe Ala Leu Pro His Tyr Asp Ser
        275                 280                 285

Ser Glu Trp Asp Trp Leu Lys Gly Ala Leu Ala Thr Met Asp Arg Asp
    290                 295                 300

Tyr Gly Ile Leu Asn Lys Val Phe His His Ile Thr Asp Thr His Val
305                 310                 315                 320

Ala His His Leu Phe Ser Thr Met Pro His Tyr His Ala Met Glu Ala
                325                 330                 335

Thr Asn Ala Ile Lys Pro Ile Leu Gly Glu Tyr Tyr Gln Phe Asp Asp
            340                 345                 350

Thr Pro Phe Tyr Lys Ala Leu Trp Arg Glu Ala Arg Glu Cys Leu Tyr
        355                 360                 365

Val Glu Pro Asp Glu Gly Thr Ser Glu Lys Gly Val Tyr Trp Tyr Arg
    370                 375                 380

Asn Lys Tyr
385

<210>  9
<211>  1616
<212>  DNA
<213>  Glycine max

<220>
<221>  misc_feature
<222>  (1)..(95)

<220>
<221>  CDS
<222>  (96)..(1244)

<220>
<221>  misc_feature
<222>  (1245)..(1616)

<400>  9
gcgtgtcggt ctctctctct ctctcaccct cctcttcaca cattttctgt gcgctctaac      60

aaacattctc gttcacactt tcagattgtg tgaag atg ggg gcg ggt ggc cga      113
                                       Met Gly Ala Gly Gly Arg
                                       1               5

act gat gtt cct cct gcc aac agg aag tca gag gtt gac cct ttg aag      161
Thr Asp Val Pro Pro Ala Asn Arg Lys Ser Glu Val Asp Pro Leu Lys
            10                  15                  20

cgg gtg cca ttt gaa aaa cct cca ttt agt ctc agc caa atc aag aag      209
Arg Val Pro Phe Glu Lys Pro Pro Phe Ser Leu Ser Gln Ile Lys Lys
        25                  30                  35

gtc att cca cct cac tgt ttc cag cgt tct gtt ttc cgc tca ttc tcc      257
Val Ile Pro Pro His Cys Phe Gln Arg Ser Val Phe Arg Ser Phe Ser
    40                  45                  50

tat gtt gtt tac gac ctc acc ata gcc ttc tgc ctc tat tat gtt gcc      305
Tyr Val Val Tyr Asp Leu Thr Ile Ala Phe Cys Leu Tyr Tyr Val Ala
55                  60                  65                  70

acc cat tac ttc cac ctc ctt ccc agc cct ctc tct ttc ttg gca tgg      353
Thr His Tyr Phe His Leu Leu Pro Ser Pro Leu Ser Phe Leu Ala Trp
                75                  80                  85

cca atc tac tgg gct gtc caa ggt tgc atc ctt act gga gtt tgg gtc      401
Pro Ile Tyr Trp Ala Val Gln Gly Cys Ile Leu Thr Gly Val Trp Val
            90                  95                  100

att gcc cat gag tgt ggc cac cat gca ttc agt gac tac cag ttg ctt      449
Ile Ala His Glu Cys Gly His His Ala Phe Ser Asp Tyr Gln Leu Leu
        105                 110                 115

gat gat att gtt ggc ctt gtc ctc cac tcc ggt ctc cta gtc cca tac      497
Asp Asp Ile Val Gly Leu Val Leu His Ser Gly Leu Leu Val Pro Tyr
    120                 125                 130

ttt tca tgg aaa tac agc cat cgc cgt cac cac tcc aac act ggt tct      545
Phe Ser Trp Lys Tyr Ser His Arg Arg His His Ser Asn Thr Gly Ser
135                 140                 145                 150

ctt gag cgg gat gaa gta ttt gtg cca aag cag aag tcc tgt atc aag      593
Leu Glu Arg Asp Glu Val Phe Val Pro Lys Gln Lys Ser Cys Ile Lys
                155                 160                 165

tgg tac tct aaa tac ctt aac aat cct cca ggc aga gtc ctc act ctt      641

Trp Tyr Ser Lys Tyr Leu Asn Asn Pro Pro Gly Arg Val Leu Thr Leu
            170                 175                 180

gct gtc acc ctc aca ctt ggt tgg ccc ttg tac ttg gct tta aat gtt 689
Ala Val Thr Leu Thr Leu Gly Trp Pro Leu Tyr Leu Ala Leu Asn Val
        185                 190                 195

tct gga agg cct tat gat aga ttt gct tgc cac tat gac cca tat ggt 737
Ser Gly Arg Pro Tyr Asp Arg Phe Ala Cys His Tyr Asp Pro Tyr Gly
    200                 205                 210

ccc att tac tct gat cgt gaa cga ctt caa ata tat ata tca gat gca 785
Pro Ile Tyr Ser Asp Arg Glu Arg Leu Gln Ile Tyr Ile Ser Asp Ala
215                 220                 225                 230

gga gta ctt gca gta tgc tat ggc ctt ttc cgt ctt gcc atg gca aaa 833
Gly Val Leu Ala Val Cys Tyr Gly Leu Phe Arg Leu Ala Met Ala Lys
                235                 240                 245

gga ctt gcc tgg gtg gtg tgt gtt tat gga gtt cca ttg cta gtg gtc 881
Gly Leu Ala Trp Val Val Cys Val Tyr Gly Val Pro Leu Leu Val Val
            250                 255                 260

aat gga ttt ttg gtg ttg att aca ttc ttg cag cat act cac cct gca 929
Asn Gly Phe Leu Val Leu Ile Thr Phe Leu Gln His Thr His Pro Ala
        265                 270                 275

ttg cca cat tac act tcc tct gag tgg gac tgg ttg aga gga gct tta 977
Leu Pro His Tyr Thr Ser Ser Glu Trp Asp Trp Leu Arg Gly Ala Leu
    280                 285                 290

gca aca gtg gat aga gat tat gga atc ctg aac aag gtc ttc cat aat 1025
Ala Thr Val Asp Arg Asp Tyr Gly Ile Leu Asn Lys Val Phe His Asn
295                 300                 305                 310

att aca gac act cat gta gca cat cac ttg ttc tcc aca atg cca cat 1073
Ile Thr Asp Thr His Val Ala His His Leu Phe Ser Thr Met Pro His
                315                 320                 325

tat cat gca atg gag gct aca aag gca ata aaa ccc att ttg gga gag 1121
Tyr His Ala Met Glu Ala Thr Lys Ala Ile Lys Pro Ile Leu Gly Glu
            330                 335                 340

tat tat cgg ttt gat gag act cca ttt gtc aag gca atg tgg aga gag 1169
Tyr Tyr Arg Phe Asp Glu Thr Pro Phe Val Lys Ala Met Trp Arg Glu
        345                 350                 355

gca aga gag tgt att tat gtg gag cca gat caa agt acc gag agc aaa 1217
Ala Arg Glu Cys Ile Tyr Val Glu Pro Asp Gln Ser Thr Glu Ser Lys
    360                 365                 370

ggt gta ttt tgg tac aac aat aag ttg tgatgattaa tgtagccgag 1264
Gly Val Phe Trp Tyr Asn Asn Lys Leu
375                 380

gcttctttga actttccctt gtgactgttt agtatcatgg ttgcttattg ggaataattt 1324

tgttgaaccc tgatgttggt agtaagtatc tagacagttg catagcggtt ttgtttacag 1384

aataagatat agcctctctg aacagtttga ttattgcacc atggtttgca atcggtgcat 1444

gtcgaccaag tttctcaaga ctgtggagaa gcttattctt gttccagttc ttgaatccaa 1504

gttgttaccg tattctgtta ttattgactt agaatcctta accttttctg ctgttttctc 1564

atgatcgtca ctcgcaaatg aatcacattt caaaccaaaa aaaaaaaaaa aa 1616

<210> 10
<211> 383
<212> PRT
<213> Glycine max

<400> 10

Met Gly Ala Gly Gly Arg Thr Asp Val Pro Pro Ala Asn Arg Lys Ser
1               5                   10                  15

Glu Val Asp Pro Leu Lys Arg Val Pro Phe Glu Lys Pro Pro Phe Ser
            20                  25                  30

Leu Ser Gln Ile Lys Lys Val Ile Pro Pro His Cys Phe Gln Arg Ser
        35                  40                  45

Val Phe Arg Ser Phe Ser Tyr Val Val Tyr Asp Leu Thr Ile Ala Phe
    50                  55                  60

Cys Leu Tyr Tyr Val Ala Thr His Tyr Phe His Leu Leu Pro Ser Pro
65                  70                  75                  80

Leu Ser Phe Leu Ala Trp Pro Ile Tyr Trp Ala Val Gln Gly Cys Ile
                85                  90                  95

Leu Thr Gly Val Trp Val Ile Ala His Glu Cys Gly His His Ala Phe
            100                 105                 110

Ser Asp Tyr Gln Leu Leu Asp Asp Ile Val Gly Leu Val Leu His Ser
        115                 120                 125

Gly Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Gln Lys Ser Cys Ile Lys Trp Tyr Ser Lys Tyr Leu Asn Asn Pro Pro
                165                 170                 175

Gly Arg Val Leu Thr Leu Ala Val Thr Leu Thr Leu Gly Trp Pro Leu
            180                 185                 190

Tyr Leu Ala Leu Asn Val Ser Gly Arg Pro Tyr Asp Arg Phe Ala Cys

195                    200                    205

His Tyr Asp Pro Tyr Gly Pro Ile Tyr Ser Asp Arg Glu Arg Leu Gln
    210                    215                    220

Ile Tyr Ile Ser Asp Ala Gly Val Leu Ala Val Cys Tyr Gly Leu Phe
225                    230                    235                    240

Arg Leu Ala Met Ala Lys Gly Leu Ala Trp Val Val Cys Val Tyr Gly
                245                    250                    255

Val Pro Leu Leu Val Val Asn Gly Phe Leu Val Leu Ile Thr Phe Leu
            260                    265                    270

Gln His Thr His Pro Ala Leu Pro His Tyr Thr Ser Ser Glu Trp Asp
        275                    280                    285

Trp Leu Arg Gly Ala Leu Ala Thr Val Asp Arg Asp Tyr Gly Ile Leu
    290                    295                    300

Asn Lys Val Phe His Asn Ile Thr Asp Thr His Val Ala His His Leu
305                    310                    315                    320

Phe Ser Thr Met Pro His Tyr His Ala Met Glu Ala Thr Lys Ala Ile
                325                    330                    335

Lys Pro Ile Leu Gly Glu Tyr Tyr Arg Phe Asp Glu Thr Pro Phe Val
            340                    345                    350

Lys Ala Met Trp Arg Glu Ala Arg Glu Cys Ile Tyr Val Glu Pro Asp
        355                    360                    365

Gln Ser Thr Glu Ser Lys Gly Val Phe Trp Tyr Asn Asn Lys Leu
    370                    375                    380

<210>  11<211>  1149
<212>  DNA
<213>  Glycine max

<220>
<221>  CDS
<222>  (1)..(1149)

<400>  11
atg ggg gcg ggt ggc cga act gat gtt cct cct gcc aac agg aag tca        48
Met Gly Ala Gly Gly Arg Thr Asp Val Pro Pro Ala Asn Arg Lys Ser
1               5                   10                  15

gag gtt gac cct ttg aag cgg gtg cca ttt gaa aaa cct cca ttt agt        96

Glu Val Asp Pro Leu Lys Arg Val Pro Phe Glu Lys Pro Pro Phe Ser
            20                  25                  30

ctc agc caa atc aag aag gtc att cca cct cac tgt ttc cag cgt tct      144
Leu Ser Gln Ile Lys Lys Val Ile Pro Pro His Cys Phe Gln Arg Ser
        35                  40                  45

gtt ttc cgc tca ttc tcc tat gtt gtt tac gac ctc acc ata gcc ttc      192
Val Phe Arg Ser Phe Ser Tyr Val Val Tyr Asp Leu Thr Ile Ala Phe
    50                  55                  60

tgc ctc tat tat gtt gcc acc cat tac ttc cac ctc ctt ccc agc cct      240
Cys Leu Tyr Tyr Val Ala Thr His Tyr Phe His Leu Leu Pro Ser Pro
65                  70                  75                  80

ctc tct ttc ttg gca tgg cca atc tac tgg gct gtc caa ggt tgc atc      288
Leu Ser Phe Leu Ala Trp Pro Ile Tyr Trp Ala Val Gln Gly Cys Ile
                85                  90                  95

ctt act gga gtt tgg gtc att gcc cat gag tgt ggc cac cat gca ttc      336
Leu Thr Gly Val Trp Val Ile Ala His Glu Cys Gly His His Ala Phe
            100                 105                 110

agt gac tac cag ttg ctt gat gat att gtt ggc ctt gtc ctc cac tcc      384
Ser Asp Tyr Gln Leu Leu Asp Asp Ile Val Gly Leu Val Leu His Ser
        115                 120                 125

ggt ctc cta gtc cca tac ttt tca tgg aaa tac agc cat cgc cgt cac      432
Gly Leu Leu Val Pro Tyr Phe Ser Trp Lys Tyr Ser His Arg Arg His
    130                 135                 140

cac tcc aac act ggt tct ctt gag cgg gat gaa gta ttt gtg cca aag      480
His Ser Asn Thr Gly Ser Leu Glu Arg Asp Glu Val Phe Val Pro Lys
145                 150                 155                 160

cag aag tcc tgt atc aag tgg tac tct aaa tac ctt aac aat cct cca      528
Gln Lys Ser Cys Ile Lys Trp Tyr Ser Lys Tyr Leu Asn Asn Pro Pro
                165                 170                 175

ggc aga gtc ctc act ctt gct gtc acc ctc aca ctt ggt tgg ccc ttg      576
Gly Arg Val Leu Thr Leu Ala Val Thr Leu Thr Leu Gly Trp Pro Leu
            180                 185                 190

tac ttg gct tta aat gtt tct gga agg cct tat gat aga ttt gct tgc      624
Tyr Leu Ala Leu Asn Val Ser Gly Arg Pro Tyr Asp Arg Phe Ala Cys
        195                 200                 205

cac tat gac cca tat ggt ccc att tac tct gat cgt gaa cga ctt caa      672
His Tyr Asp Pro Tyr Gly Pro Ile Tyr Ser Asp Arg Glu Arg Leu Gln
    210                 215                 220

ata tat ata tca gat gca gga gta ctt gca gta tgc tat ggc ctt ttc      720
Ile Tyr Ile Ser Asp Ala Gly Val Leu Ala Val Cys Tyr Gly Leu Phe
225                 230                 235                 240

cgt ctt gcc atg gca aaa gga ctt gcc tgg gtg gtg tgt gtt tat gga      768
Arg Leu Ala Met Ala Lys Gly Leu Ala Trp Val Val Cys Val Tyr Gly
                245                 250                 255

gtt cca ttg cta gtg gtc aat gga ttt ttg gtg ttg att aca ttc ttg      816
Val Pro Leu Leu Val Val Asn Gly Phe Leu Val Leu Ile Thr Phe Leu
            260                 265                 270

cag cat act cac cct gca ttg cca cat tac act tcc tct gag tgg gac 864
Gln His Thr His Pro Ala Leu Pro His Tyr Thr Ser Ser Glu Trp Asp
        275                 280                 285

tgg ttg aga gga gct tta gca aca gtg gat aga gat tat gga atc ctg 912
Trp Leu Arg Gly Ala Leu Ala Thr Val Asp Arg Asp Tyr Gly Ile Leu
    290                 295                 300

aac aag gtc ttc cat aat att aca gac act cat gta gca cat cac ttg 960
Asn Lys Val Phe His Asn Ile Thr Asp Thr His Val Ala His His Leu
305                 310                 315                 320

ttc tcc aca atg cca cat tat cat gca atg gag gct aca aag gca ata 1008
Phe Ser Thr Met Pro His Tyr His Ala Met Glu Ala Thr Lys Ala Ile
                325                 330                 335

aaa ccc att ttg gga gag tat tat cgg ttt gat gag act cca ttt gtc 1056
Lys Pro Ile Leu Gly Glu Tyr Tyr Arg Phe Asp Glu Thr Pro Phe Val
            340                 345                 350

aag gca atg tgg aga gag gca aga gag tgt att tat gtg gag cca gat 1104
Lys Ala Met Trp Arg Glu Ala Arg Glu Cys Ile Tyr Val Glu Pro Asp
        355                 360                 365

caa agt acc gag agc aaa ggt gta ttt tgg tac aac aat aag ttg 1149
Gln Ser Thr Glu Ser Lys Gly Val Phe Trp Tyr Asn Asn Lys Leu
    370                 375                 380

<210> 12
<211> 383
<212> PRT
<213> Glycine max

<400> 12

Met Gly Ala Gly Gly Arg Thr Asp Val Pro Pro Ala Asn Arg Lys Ser
1               5                   10                  15

Glu Val Asp Pro Leu Lys Arg Val Pro Phe Glu Lys Pro Pro Phe Ser
            20                  25                  30

Leu Ser Gln Ile Lys Lys Val Ile Pro Pro His Cys Phe Gln Arg Ser
        35                  40                  45

Val Phe Arg Ser Phe Ser Tyr Val Val Tyr Asp Leu Thr Ile Ala Phe
    50                  55                  60

Cys Leu Tyr Tyr Val Ala Thr His Tyr Phe His Leu Leu Pro Ser Pro
65                  70                  75                  80

Leu Ser Phe Leu Ala Trp Pro Ile Tyr Trp Ala Val Gln Gly Cys Ile
                85                  90                  95

Leu Thr Gly Val Trp Val Ile Ala His Glu Cys Gly His His Ala Phe

            100                 105                 110

Ser Asp Tyr Gln Leu Leu Asp Asp Ile Val Gly Leu Val Leu His Ser
        115                 120                 125

Gly Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Gln Lys Ser Cys Ile Lys Trp Tyr Ser Lys Tyr Leu Asn Asn Pro Pro
                165                 170                 175

Gly Arg Val Leu Thr Leu Ala Val Thr Leu Thr Leu Gly Trp Pro Leu
            180                 185                 190

Tyr Leu Ala Leu Asn Val Ser Gly Arg Pro Tyr Asp Arg Phe Ala Cys
        195                 200                 205

His Tyr Asp Pro Tyr Gly Pro Ile Tyr Ser Asp Arg Glu Arg Leu Gln
    210                 215                 220

Ile Tyr Ile Ser Asp Ala Gly Val Leu Ala Val Cys Tyr Gly Leu Phe
225                 230                 235                 240

Arg Leu Ala Met Ala Lys Gly Leu Ala Trp Val Val Cys Val Tyr Gly
                245                 250                 255

Val Pro Leu Leu Val Val Asn Gly Phe Leu Val Leu Ile Thr Phe Leu
            260                 265                 270

Gln His Thr His Pro Ala Leu Pro His Tyr Thr Ser Ser Glu Trp Asp
        275                 280                 285

Trp Leu Arg Gly Ala Leu Ala Thr Val Asp Arg Asp Tyr Gly Ile Leu
    290                 295                 300

Asn Lys Val Phe His Asn Ile Thr Asp Thr His Val Ala His His Leu
305                 310                 315                 320

Phe Ser Thr Met Pro His Tyr His Ala Met Glu Ala Thr Lys Ala Ile
                325                 330                 335

Lys Pro Ile Leu Gly Glu Tyr Tyr Arg Phe Asp Glu Thr Pro Phe Val
            340                 345                 350

Lys Ala Met Trp Arg Glu Ala Arg Glu Cys Ile Tyr Val Glu Pro Asp
        355                 360                 365

Gln Ser Thr Glu Ser Lys Gly Val Phe Trp Tyr Asn Asn Lys Leu
    370                 375                 380

<210>  13
<211>  1053
<212>  DNA
<213>  Glycine max

<220>
<221>  misc_feature
<222>  (1)..(95)

<220>
<221>  CDS
<222>  (96)..(749)

<220>
<221>  misc_feature
<222>  (750)..(1053)

<400>  13
acttttcatg gaaatacagc catcgccgtc accactccaa cacaggttct cttgagcgag      60

atgaagtatt tgtgccaaag cagaagtcca gtatc atg tgg tac tct aaa tac      113
                                       Met Trp Tyr Ser Lys Tyr
                                       1               5

ctt aac aat cca cca ggc aga gtc ctc act ctt gcc gtc acc ctc acg      161
Leu Asn Asn Pro Pro Gly Arg Val Leu Thr Leu Ala Val Thr Leu Thr
            10                  15                  20

ctt ggt tgg ccc ttg tac ttg gct ttt aat gtt tct gga agg cct tat      209
Leu Gly Trp Pro Leu Tyr Leu Ala Phe Asn Val Ser Gly Arg Pro Tyr
        25                  30                  35

gat aga ttt gct tgc cac tat gac cct tat ggt ccc att tac tct gac      257
Asp Arg Phe Ala Cys His Tyr Asp Pro Tyr Gly Pro Ile Tyr Ser Asp
    40                  45                  50

cga gaa cga ctt caa ata tat ata tca gat gca gga gta ctt gca gta      305
Arg Glu Arg Leu Gln Ile Tyr Ile Ser Asp Ala Gly Val Leu Ala Val
55                  60                  65                  70

tgc tat ggc ctt ttc tgt ctt gcc atg gca aaa ggg ctt gcc tgg gtg      353
Cys Tyr Gly Leu Phe Cys Leu Ala Met Ala Lys Gly Leu Ala Trp Val
                75                  80                  85

gtg tgt gtt tat gga gtt cca ttg ctt gtg gtc aat gga ttt ttg gtg      401
Val Cys Val Tyr Gly Val Pro Leu Leu Val Val Asn Gly Phe Leu Val
            90                  95                  100

ttg att aca ttt ttg cag cac act cac cct gca ttg cca cac tac act      449
Leu Ile Thr Phe Leu Gln His Thr His Pro Ala Leu Pro His Tyr Thr
        105                 110                 115

tcc tct gag tgg gac tgg ttg aga gga gct tta gca aca gtg gat aga     497
Ser Ser Glu Trp Asp Trp Leu Arg Gly Ala Leu Ala Thr Val Asp Arg
    120                 125                 130

gat tat gga atc ctg aac aag gtc ttc cat aat att aca gac act cat     545
Asp Tyr Gly Ile Leu Asn Lys Val Phe His Asn Ile Thr Asp Thr His
135                 140                 145                 150

gta gct cat cac ttg ttc tcc aca atg cca cat tat cat gca atg gag     593
Val Ala His His Leu Phe Ser Thr Met Pro His Tyr His Ala Met Glu
                155                 160                 165

gcg aca aag gca ata aag ccc atc ttg gga gag tat tat cgg ttt gat     641
Ala Thr Lys Ala Ile Lys Pro Ile Leu Gly Glu Tyr Tyr Arg Phe Asp
            170                 175                 180

ggg act cca ttt gtc aag gca atg tgg aga gag gca aga gag tgt att     689
Gly Thr Pro Phe Val Lys Ala Met Trp Arg Glu Ala Arg Glu Cys Ile
        185                 190                 195

tat gtg gag cca gat caa agt act cag agc aaa ggt gta ttt tgg tac     737
Tyr Val Glu Pro Asp Gln Ser Thr Gln Ser Lys Gly Val Phe Trp Tyr
    200                 205                 210

aac aat aag ttg tgatgattaa tgtagccggg ggcttcttgt ccgtcaggtg     789
Asn Asn Lys Leu
215

ggatggtttg aactttcctt tgtgactgtt tagtatcatg cttgcttatt gggaataatt     849

ttgttgaacc ctgatgttgg tagtagtatc tagaaagtag catagcgttt ttgtttgcag     909

aataagatat agcatctctg aacagtttga ttattgcacc atgttttgca atcagtgcat     969

gtcgaccggg tttctcaaga ttgtggggat gcttattctt gttccagttc ttgaatccaa     1029

gttgttatca tattctgtta ttga     1053

<210>  14
<211>  218
<212>  PRT
<213>  Glycine max

<400>  14

Met Trp Tyr Ser Lys Tyr Leu Asn Asn Pro Pro Gly Arg Val Leu Thr
1               5                   10                  15

Leu Ala Val Thr Leu Thr Leu Gly Trp Pro Leu Tyr Leu Ala Phe Asn
            20                  25                  30

Val Ser Gly Arg Pro Tyr Asp Arg Phe Ala Cys His Tyr Asp Pro Tyr
        35                  40                  45

Gly Pro Ile Tyr Ser Asp Arg Glu Arg Leu Gln Ile Tyr Ile Ser Asp
    50                  55                  60

Ala Gly Val Leu Ala Val Cys Tyr Gly Leu Phe Cys Leu Ala Met Ala
65                  70                  75                  80

Lys Gly Leu Ala Trp Val Val Cys Val Tyr Gly Val Pro Leu Leu Val
                85                  90                  95

Val Asn Gly Phe Leu Val Leu Ile Thr Phe Leu Gln His Thr His Pro
            100                 105                 110

Ala Leu Pro His Tyr Thr Ser Ser Glu Trp Asp Trp Leu Arg Gly Ala
        115                 120                 125

Leu Ala Thr Val Asp Arg Asp Tyr Gly Ile Leu Asn Lys Val Phe His
    130                 135                 140

Asn Ile Thr Asp Thr His Val Ala His His Leu Phe Ser Thr Met Pro
145                 150                 155                 160

His Tyr His Ala Met Glu Ala Thr Lys Ala Ile Lys Pro Ile Leu Gly
                165                 170                 175

Glu Tyr Tyr Arg Phe Asp Gly Thr Pro Phe Val Lys Ala Met Trp Arg
            180                 185                 190

Glu Ala Arg Glu Cys Ile Tyr Val Glu Pro Asp Gln Ser Thr Gln Ser
        195                 200                 205

Lys Gly Val Phe Trp Tyr Asn Asn Lys Leu
    210                 215

<210>  15
<211>  654
<212>  DNA
<213>  Glycine max

<220>
<221>  CDS
<222>  (1)..(654)

<400>  15
atg tgg tac tct aaa tac ctt aac aat cca cca ggc aga gtc ctc act      48
Met Trp Tyr Ser Lys Tyr Leu Asn Asn Pro Pro Gly Arg Val Leu Thr
1               5                   10                  15

ctt gcc gtc acc ctc acg ctt ggt tgg ccc ttg tac ttg gct ttt aat      96
Leu Ala Val Thr Leu Thr Leu Gly Trp Pro Leu Tyr Leu Ala Phe Asn
            20                  25                  30

gtt tct gga agg cct tat gat aga ttt gct tgc cac tat gac cct tat      144
Val Ser Gly Arg Pro Tyr Asp Arg Phe Ala Cys His Tyr Asp Pro Tyr
        35                  40                  45

ggt ccc att tac tct gac cga gaa cga ctt caa ata tat ata tca gat 192
Gly Pro Ile Tyr Ser Asp Arg Glu Arg Leu Gln Ile Tyr Ile Ser Asp
    50                  55                  60

gca gga gta ctt gca gta tgc tat ggc ctt ttc tgt ctt gcc atg gca 240
Ala Gly Val Leu Ala Val Cys Tyr Gly Leu Phe Cys Leu Ala Met Ala
65                  70                  75                  80

aaa ggg ctt gcc tgg gtg gtg tgt gtt tat gga gtt cca ttg ctt gtg 288
Lys Gly Leu Ala Trp Val Val Cys Val Tyr Gly Val Pro Leu Leu Val
                85                  90                  95

gtc aat gga ttt ttg gtg ttg att aca ttt ttg cag cac act cac cct 336
Val Asn Gly Phe Leu Val Leu Ile Thr Phe Leu Gln His Thr His Pro
            100                 105                 110

gca ttg cca cac tac act tcc tct gag tgg gac tgg ttg aga gga gct 384
Ala Leu Pro His Tyr Thr Ser Ser Glu Trp Asp Trp Leu Arg Gly Ala
        115                 120                 125

tta gca aca gtg gat aga gat tat gga atc ctg aac aag gtc ttc cat 432
Leu Ala Thr Val Asp Arg Asp Tyr Gly Ile Leu Asn Lys Val Phe His
    130                 135                 140

aat att aca gac act cat gta gct cat cac ttg ttc tcc aca atg cca 480
Asn Ile Thr Asp Thr His Val Ala His His Leu Phe Ser Thr Met Pro
145                 150                 155                 160

cat tat cat gca atg gag gcg aca aag gca ata aag ccc atc ttg gga 528
His Tyr His Ala Met Glu Ala Thr Lys Ala Ile Lys Pro Ile Leu Gly
                165                 170                 175

gag tat tat cgg ttt gat ggg act cca ttt gtc aag gca atg tgg aga 576
Glu Tyr Tyr Arg Phe Asp Gly Thr Pro Phe Val Lys Ala Met Trp Arg
            180                 185                 190

gag gca aga gag tgt att tat gtg gag cca gat caa agt act cag agc 624
Glu Ala Arg Glu Cys Ile Tyr Val Glu Pro Asp Gln Ser Thr Gln Ser
        195                 200                 205

aaa ggt gta ttt tgg tac aac aat aag ttg 654
Lys Gly Val Phe Trp Tyr Asn Asn Lys Leu
    210                 215

<210> 16
<211> 218
<212> PRT
<213> Glycine max

<400> 16

Met Trp Tyr Ser Lys Tyr Leu Asn Asn Pro Pro Gly Arg Val Leu Thr
1               5                   10                  15

Leu Ala Val Thr Leu Thr Leu Gly Trp Pro Leu Tyr Leu Ala Phe Asn
            20                  25                  30

Val Ser Gly Arg Pro Tyr Asp Arg Phe Ala Cys His Tyr Asp Pro Tyr

35                  40                  45

Gly Pro Ile Tyr Ser Asp Arg Glu Arg Leu Gln Ile Tyr Ile Ser Asp
    50                  55                  60

Ala Gly Val Leu Ala Val Cys Tyr Gly Leu Phe Cys Leu Ala Met Ala
65                  70                  75                  80

Lys Gly Leu Ala Trp Val Val Cys Val Tyr Gly Val Pro Leu Leu Val
                85                  90                  95

Val Asn Gly Phe Leu Val Leu Ile Thr Phe Leu Gln His Thr His Pro
            100                 105                 110

Ala Leu Pro His Tyr Thr Ser Ser Glu Trp Asp Trp Leu Arg Gly Ala
        115                 120                 125

Leu Ala Thr Val Asp Arg Asp Tyr Gly Ile Leu Asn Lys Val Phe His
    130                 135                 140

Asn Ile Thr Asp Thr His Val Ala His His Leu Phe Ser Thr Met Pro
145                 150                 155                 160

His Tyr His Ala Met Glu Ala Thr Lys Ala Ile Lys Pro Ile Leu Gly
                165                 170                 175

Glu Tyr Tyr Arg Phe Asp Gly Thr Pro Phe Val Lys Ala Met Trp Arg
            180                 185                 190

Glu Ala Arg Glu Cys Ile Tyr Val Glu Pro Asp Gln Ser Thr Gln Ser
        195                 200                 205

Lys Gly Val Phe Trp Tyr Asn Asn Lys Leu
    210                 215

<210>  17
<211>  1750
<212>  DNA
<213>  Zea mays

<220>
<221>  misc_feature
<222>  (1)..(175)

<220>
<221>  CDS
<222>  (176)..(1351)

<220>

<221>  misc_feature
<222>  (1352)..(1750)

<400>  17
ctccctcctc cttcctcctc cctgcaaatc ctgcaggcac caccgctcgt tttcctgtcc      60
gggggacagg agagaagggg agagaccgag agagggtgag gcgcggcgtc cgccgatctg     120

ctccgccccc cgaagcagcc tgtcacgtcg tcctcactct cagcaaccag cgaaa atg      178
                                                             Met
                                                             1

ggt gcc gga ggc agg atg acc gag aag gag cgg gag aag cat gag cag      226
Gly Ala Gly Gly Arg Met Thr Glu Lys Glu Arg Glu Lys His Glu Gln
            5                   10                  15

gag cag gtc gcc cgt gct acc ggc ggt ggc gcg gca gtg cag cgg tcg      274
Glu Gln Val Ala Arg Ala Thr Gly Gly Gly Ala Ala Val Gln Arg Ser
        20                  25                  30

ccg gtg gag aag ccg ccg ttc acg ttg ggg cag atc aag aag gcg atc      322
Pro Val Glu Lys Pro Pro Phe Thr Leu Gly Gln Ile Lys Lys Ala Ile
    35                  40                  45

ccg ccg cac tgc ttc gag cgc tcc gtg ctg agg tcc ttc tcg tac gtg      370
Pro Pro His Cys Phe Glu Arg Ser Val Leu Arg Ser Phe Ser Tyr Val
50                  55                  60                  65

gcc cac gac ctg tcg ctc gcc gcg gcg ctc ctc tac ctc gcg gtg gcc      418
Ala His Asp Leu Ser Leu Ala Ala Ala Leu Leu Tyr Leu Ala Val Ala
                70                  75                  80

gtg ata ccg gcg cta ccc tgc ccg ctc cgc tac gcg gcc tgg ccg ctg      466
Val Ile Pro Ala Leu Pro Cys Pro Leu Arg Tyr Ala Ala Trp Pro Leu
            85                  90                  95

tac tgg gtg gcc cag ggg tgc gtg tgc acg ggc gtg tgg gtg atc gcg      514
Tyr Trp Val Ala Gln Gly Cys Val Cys Thr Gly Val Trp Val Ile Ala
        100                 105                 110

cac gag tgc ggc cac cac gcc ttc tcc gac cac gcg ctc ctg gac gac      562
His Glu Cys Gly His His Ala Phe Ser Asp His Ala Leu Leu Asp Asp
    115                 120                 125

gcc gtc ggc ctg gcg ctg cac tcg gcg ctg ctg gtg ccc tac ttc tcg      610
Ala Val Gly Leu Ala Leu His Ser Ala Leu Leu Val Pro Tyr Phe Ser
130                 135                 140                 145

tgg aag tac agc cac cgg cgc cac cac tcc aac acg ggg tcc ctg gag      658
Trp Lys Tyr Ser His Arg Arg His His Ser Asn Thr Gly Ser Leu Glu
                150                 155                 160

cgc gac gag gtg ttc gtg ccg agg acc aag gag gcg ctg ccg tgg tac      706
Arg Asp Glu Val Phe Val Pro Arg Thr Lys Glu Ala Leu Pro Trp Tyr
            165                 170                 175

gcc ccg tac gtg cac ggc agc ccc gcg ggc cgg ctg gcg cac gtc gcc      754
Ala Pro Tyr Val His Gly Ser Pro Ala Gly Arg Leu Ala His Val Ala
        180                 185                 190

gtg cag ctc acc ctc ggc tgg ccg ctg tac ctg gcc acc aac gcg tcg      802
Val Gln Leu Thr Leu Gly Trp Pro Leu Tyr Leu Ala Thr Asn Ala Ser
    195                 200                 205

ggg cgg ccg tac ccg cgc ttc gcc tgc cac ttc gac ccc tac ggc ccc 850
Gly Arg Pro Tyr Pro Arg Phe Ala Cys His Phe Asp Pro Tyr Gly Pro
210                 215                 220                 225

atc tac aac gac cgg gag cgc gcc cag atc ttc gtc tcg gac gcc ggc 898
Ile Tyr Asn Asp Arg Glu Arg Ala Gln Ile Phe Val Ser Asp Ala Gly
                230                 235                 240

gtc gtg gcc gtg gcg ttc ggg ctg tac aag ctg gcg gcg gcg ttc ggg 946
Val Val Ala Val Ala Phe Gly Leu Tyr Lys Leu Ala Ala Ala Phe Gly
            245                 250                 255

gtc tgg tgg gtg gtg cgc gtg tac gcc gtg ccg ctg ctg atc gtc aac 994
Val Trp Trp Val Val Arg Val Tyr Ala Val Pro Leu Leu Ile Val Asn
        260                 265                 270

gcg tgg ctg gtg ctc atc acg tac ctg cag cac acc cac ccg gcg ctg 1042
Ala Trp Leu Val Leu Ile Thr Tyr Leu Gln His Thr His Pro Ala Leu
    275                 280                 285

ccc cac tac gac tcg ggc gag tgg gac tgg ctg cgc ggc gcg ctc gcc 1090
Pro His Tyr Asp Ser Gly Glu Trp Asp Trp Leu Arg Gly Ala Leu Ala
290                 295                 300                 305

acc gtc gac cga gac tac ggc gtc ctc aac cgc gtg ttc cac cac atc 1138
Thr Val Asp Arg Asp Tyr Gly Val Leu Asn Arg Val Phe His His Ile
                310                 315                 320

acg gac acg cac gtc gcg cac cac ctc ttc tcc acc atg ccg cac tac 1186
Thr Asp Thr His Val Ala His His Leu Phe Ser Thr Met Pro His Tyr
            325                 330                 335

cac gcc gtg gag gcc acc agg gcg atc agg ccc gtc ctc ggc gag tac 1234
His Ala Val Glu Ala Thr Arg Ala Ile Arg Pro Val Leu Gly Glu Tyr
        340                 345                 350

tac cag ttc gac ccg acc cct gtc gcc aag gcc acc tgg cgc gag gcc 1282
Tyr Gln Phe Asp Pro Thr Pro Val Ala Lys Ala Thr Trp Arg Glu Ala
    355                 360                 365

agg gag tgc atc tac gtc gag cct gag aac cgc aac cgc aag ggc gtc 1330
Arg Glu Cys Ile Tyr Val Glu Pro Glu Asn Arg Asn Arg Lys Gly Val
370                 375                 380                 385

ttc tgg tac aac agc aag ttc tagccgccgc ttgcttttc cctaggaatg 1381
Phe Trp Tyr Asn Ser Lys Phe
                390

ggaggagaaa tcaggatgag aagatggtaa tgtctgcatc tacctgtcta atggttagtc 1441

accagtcttt agacaggaag agagcatttg ggcttcagaa aaggaggctt actgcactac 1501

tgcagtgcca tcgctagatt taaggcaaat tcagtgtgtc tgtgcccatg gctgtgagct 1561

ttgggtactc tcaagtagtc aagttctctt gtttttgttt tta[?]tcgtcg ctgttgtagg 1621

cttgccggcg gc[?]gtcgttg cgtggccgcg ccttgtcgtg tgcgtcttgc catctcttcg 1681

tgctcccttc gtgttgttgt aaaacactag tctggtgtct ttggcggaat aacagatcgt 1741

cgaacgaca 1750

<210>  18
<211>  392
<212>  PRT
<213>  Zea mays

<400>  18

Met Gly Ala Gly Gly Arg Met Thr Glu Lys Glu Arg Glu Lys His Glu
1               5                   10                  15

Gln Glu Gln Val Ala Arg Ala Thr Gly Gly Gly Ala Ala Val Gln Arg
            20                  25                  30

Ser Pro Val Glu Lys Pro Pro Phe Thr Leu Gly Gln Ile Lys Lys Ala
        35                  40                  45

Ile Pro Pro His Cys Phe Glu Arg Ser Val Leu Arg Ser Phe Ser Tyr
    50                  55                  60

Val Ala His Asp Leu Ser Leu Ala Ala Ala Leu Leu Tyr Leu Ala Val
65                  70                  75                  80

Ala Val Ile Pro Ala Leu Pro Cys Pro Leu Arg Tyr Ala Ala Trp Pro
                85                  90                  95

Leu Tyr Trp Val Ala Gln Gly Cys Val Cys Thr Gly Val Trp Val Ile
            100                 105                 110

Ala His Glu Cys Gly His His Ala Phe Ser Asp His Ala Leu Leu Asp
        115                 120                 125

Asp Ala Val Gly Leu Ala Leu His Ser Ala Leu Leu Val Pro Tyr Phe
    130                 135                 140

Ser Trp Lys Tyr Ser His Arg Arg His His Ser Asn Thr Gly Ser Leu
145                 150                 155                 160

Glu Arg Asp Glu Val Phe Val Pro Arg Thr Lys Glu Ala Leu Pro Trp
                165                 170                 175

Tyr Ala Pro Tyr Val His Gly Ser Pro Ala Gly Arg Leu Ala His Val
            180                 185                 190

Ala Val Gln Leu Thr Leu Gly Trp Pro Leu Tyr Leu Ala Thr Asn Ala
        195                 200                 205

Ser Gly Arg Pro Tyr Pro Arg Phe Ala Cys His Phe Asp Pro Tyr Gly

210                 215                 220

Pro Ile Tyr Asn Asp Arg Glu Arg Ala Gln Ile Phe Val Ser Asp Ala
225                 230                 235                 240

Gly Val Val Ala Val Ala Phe Gly Leu Tyr Lys Leu Ala Ala Ala Phe
                245                 250                 255

Gly Val Trp Trp Val Val Arg Val Tyr Ala Val Pro Leu Leu Ile Val
            260                 265                 270

Asn Ala Trp Leu Val Leu Ile Thr Tyr Leu Gln His Thr His Pro Ala
        275                 280                 285

Leu Pro His Tyr Asp Ser Gly Glu Trp Asp Trp Leu Arg Gly Ala Leu
    290                 295                 300

Ala Thr Val Asp Arg Asp Tyr Gly Val Leu Asn Arg Val Phe His His
305                 310                 315                 320

Ile Thr Asp Thr His Val Ala His His Leu Phe Ser Thr Met Pro His
                325                 330                 335

Tyr His Ala Val Glu Ala Thr Arg Ala Ile Arg Pro Val Leu Gly Glu
            340                 345                 350

Tyr Tyr Gln Phe Asp Pro Thr Pro Val Ala Lys Ala Thr Trp Arg Glu
        355                 360                 365

Ala Arg Glu Cys Ile Tyr Val Glu Pro Glu Asn Arg Asn Arg Lys Gly
    370                 375                 380

Val Phe Trp Tyr Asn Ser Lys Phe
385                 390

<210>  19
<211>  1176
<212>  DNA
<213>  Zea mays

<220>
<221>  CDS
<222>  (1)..(1176)

<400>  19
atg ggt gcc gga ggc agg atg acc gag aag gag cgg gag aag cat gag      48
Met Gly Ala Gly Gly Arg Met Thr Glu Lys Glu Arg Glu Lys His Glu
1               5                   10                  15

cag gag cag gtc gcc cgt gct acc ggc ggt ggc gcg gca gtg cag cgg 96
Gln Glu Gln Val Ala Arg Ala Thr Gly Gly Gly Ala Ala Val Gln Arg
            20                  25                  30

tcg ccg gtg gag aag ccg ccg ttc acg ttg ggg cag atc aag aag gcg 144
Ser Pro Val Glu Lys Pro Pro Phe Thr Leu Gly Gln Ile Lys Lys Ala
        35                  40                  45

atc ccg ccg cac tgc ttc gag cgc tcc gtg ctg agg tcc ttc tcg tac 192
Ile Pro Pro His Cys Phe Glu Arg Ser Val Leu Arg Ser Phe Ser Tyr
    50                  55                  60

gtg gcc cac gac ctg tcg ctc gcc gcg gcg ctc ctc tac ctc gcg gtg 240
Val Ala His Asp Leu Ser Leu Ala Ala Ala Leu Leu Tyr Leu Ala Val
65                  70                  75                  80

gcc gtg ata ccg gcg cta ccc tgc ccg ctc cgc tac gcg gcc tgg ccg 288
Ala Val Ile Pro Ala Leu Pro Cys Pro Leu Arg Tyr Ala Ala Trp Pro
                85                  90                  95

ctg tac tgg gtg gcc cag ggg tgc gtg tgc acg ggc gtg tgg gtg atc 336
Leu Tyr Trp Val Ala Gln Gly Cys Val Cys Thr Gly Val Trp Val Ile
            100                 105                 110

gcg cac gag tgc ggc cac cac gcc ttc tcc gac cac gcg ctc ctg gac 384
Ala His Glu Cys Gly His His Ala Phe Ser Asp His Ala Leu Leu Asp
        115                 120                 125

gac gcc gtc ggc ctg gcg ctg cac tcg gcg ctg ctg gtg ccc tac ttc 432
Asp Ala Val Gly Leu Ala Leu His Ser Ala Leu Leu Val Pro Tyr Phe
    130                 135                 140

tcg tgg aag tac agc cac cgg cgc cac cac tcc aac acg ggg tcc ctg 480
Ser Trp Lys Tyr Ser His Arg Arg His His Ser Asn Thr Gly Ser Leu
145                 150                 155                 160

gag cgc gac gag gtg ttc gtg ccg agg acc aag gag gcg ctg ccg tgg 528
Glu Arg Asp Glu Val Phe Val Pro Arg Thr Lys Glu Ala Leu Pro Trp
                165                 170                 175

tac gcc ccg tac gtg cac ggc agc ccc gcg ggc cgg ctg gcg cac gtc 576
Tyr Ala Pro Tyr Val His Gly Ser Pro Ala Gly Arg Leu Ala His Val
            180                 185                 190

gcc gtg cag ctc acc ctc ggc tgg ccg ctg tac ctg gcc acc aac gcg 624
Ala Val Gln Leu Thr Leu Gly Trp Pro Leu Tyr Leu Ala Thr Asn Ala
        195                 200                 205

tcg ggg cgg ccg tac ccg cgc ttc gcc tgc cac ttc gac ccc tac ggc 672
Ser Gly Arg Pro Tyr Pro Arg Phe Ala Cys His Phe Asp Pro Tyr Gly
    210                 215                 220

ccc atc tac aac gac cgg gag cgc gcc cag atc ttc gtc tcg gac gcc 720
Pro Ile Tyr Asn Asp Arg Glu Arg Ala Gln Ile Phe Val Ser Asp Ala
225                 230                 235                 240

ggc gtc gtg gcc gtg gcg ttc ggg ctg tac aag ctg gcg gcg gcg ttc 768
Gly Val Val Ala Val Ala Phe Gly Leu Tyr Lys Leu Ala Ala Ala Phe
                245                 250                 255

ggg gtc tgg tgg gtg gtg cgc gtg tac gcc gtg ccg ctg ctg atc gtc 816
Gly Val Trp Trp Val Val Arg Val Tyr Ala Val Pro Leu Leu Ile Val

260                 265                 270

aac gcg tgg ctg gtg ctc atc acg tac ctg cag cac acc cac ccg gcg      864
Asn Ala Trp Leu Val Leu Ile Thr Tyr Leu Gln His Thr His Pro Ala
    275                 280                 285

ctg ccc cac tac gac tcg ggc gag tgg gac tgg ctg cgc ggc gcg ctc      912
Leu Pro His Tyr Asp Ser Gly Glu Trp Asp Trp Leu Arg Gly Ala Leu
    290                 295                 300

gcc acc gtc gac cga gac tac ggc gtc ctc aac cgc gtg ttc cac cac      960
Ala Thr Val Asp Arg Asp Tyr Gly Val Leu Asn Arg Val Phe His His
305                 310                 315                 320

atc acg gac acg cac gtc gcg cac cac ctc ttc tcc acc atg ccg cac      1008
Ile Thr Asp Thr His Val Ala His His Leu Phe Ser Thr Met Pro His
                325                 330                 335

tac cac gcc gtg gag gcc acc agg gcg atc agg ccc gtc ctc ggc gag      1056
Tyr His Ala Val Glu Ala Thr Arg Ala Ile Arg Pro Val Leu Gly Glu
            340                 345                 350

tac tac cag ttc gac ccg acc cct gtc gcc aag gcc acc tgg cgc gag      1104
Tyr Tyr Gln Phe Asp Pro Thr Pro Val Ala Lys Ala Thr Trp Arg Glu
        355                 360                 365

gcc agg gag tgc atc tac gtc gag cct gag aac cgc aac cgc aag ggc      1152
Ala Arg Glu Cys Ile Tyr Val Glu Pro Glu Asn Arg Asn Arg Lys Gly
    370                 375                 380

gtc ttc tgg tac aac agc aag ttc                                      1176
Val Phe Trp Tyr Asn Ser Lys Phe
385                 390


<210>  20
<211>  392
<212>  PRT
<213>  Zea mays

<400>  20

Met Gly Ala Gly Gly Arg Met Thr Glu Lys Glu Arg Glu Lys His Glu
1               5                   10                  15


Gln Glu Gln Val Ala Arg Ala Thr Gly Gly Gly Ala Ala Val Gln Arg
            20                  25                  30


Ser Pro Val Glu Lys Pro Pro Phe Thr Leu Gly Gln Ile Lys Lys Ala
        35                  40                  45


Ile Pro Pro His Cys Phe Glu Arg Ser Val Leu Arg Ser Phe Ser Tyr
    50                  55                  60


Val Ala His Asp Leu Ser Leu Ala Ala Ala Leu Leu Tyr Leu Ala Val
65                  70                  75                  80

Ala Val Ile Pro Ala Leu Pro Cys Pro Leu Arg Tyr Ala Ala Trp Pro
                85                  90                  95

Leu Tyr Trp Val Ala Gln Gly Cys Val Cys Thr Gly Val Trp Val Ile
            100                 105                 110

Ala His Glu Cys Gly His His Ala Phe Ser Asp His Ala Leu Leu Asp
        115                 120                 125

Asp Ala Val Gly Leu Ala Leu His Ser Ala Leu Leu Val Pro Tyr Phe
    130                 135                 140

Ser Trp Lys Tyr Ser His Arg Arg His His Ser Asn Thr Gly Ser Leu
145                 150                 155                 160

Glu Arg Asp Glu Val Phe Val Pro Arg Thr Lys Glu Ala Leu Pro Trp
                165                 170                 175

Tyr Ala Pro Tyr Val His Gly Ser Pro Ala Gly Arg Leu Ala His Val
            180                 185                 190

Ala Val Gln Leu Thr Leu Gly Trp Pro Leu Tyr Leu Ala Thr Asn Ala
        195                 200                 205

Ser Gly Arg Pro Tyr Pro Arg Phe Ala Cys His Phe Asp Pro Tyr Gly
    210                 215                 220

Pro Ile Tyr Asn Asp Arg Glu Arg Ala Gln Ile Phe Val Ser Asp Ala
225                 230                 235                 240

Gly Val Val Ala Val Ala Phe Gly Leu Tyr Lys Leu Ala Ala Ala Phe
                245                 250                 255

Gly Val Trp Trp Val Val Arg Val Tyr Ala Val Pro Leu Leu Ile Val
            260                 265                 270

Asn Ala Trp Leu Val Leu Ile Thr Tyr Leu Gln His Thr His Pro Ala
        275                 280                 285

Leu Pro His Tyr Asp Ser Gly Glu Trp Asp Trp Leu Arg Gly Ala Leu
    290                 295                 300

Ala Thr Val Asp Arg Asp Tyr Gly Val Leu Asn Arg Val Phe His His
305                 310                 315                 320

Ile Thr Asp Thr His Val Ala His His Leu Phe Ser Thr Met Pro His
                325                 330                 335

Tyr His Ala Val Glu Ala Thr Arg Ala Ile Arg Pro Val Leu Gly Glu
            340                 345                 350

Tyr Tyr Gln Phe Asp Pro Thr Pro Val Ala Lys Ala Thr Trp Arg Glu
        355                 360                 365

Ala Arg Glu Cys Ile Tyr Val Glu Pro Glu Asn Arg Asn Arg Lys Gly
    370                 375                 380

Val Phe Trp Tyr Asn Ser Lys Phe
385                 390

<210>  21
<211>  1863
<212>  DNA
<213>  Oryza sativa

<220>
<221>  misc_feature
<222>  (1)..(149)

<220>
<221>  CDS
<222>  (150)..(1313)

<220>
<221>  misc_feature
<222>  (1314)..(1863)

<400>  21
ctcctctcct cctcccctgc acagaccact cgtttcctcc acaaagaggg agggaacaag      60

ggaagggtgt cgcccgcccc ccaccccgat ctgcctccgc cgctccgctc ctccgcgcct     120

gcgaaatcta ccaacgctaa ctcagcaag atg ggt gcc ggc ggc agg atg acg       173
                                Met Gly Ala Gly Gly Arg Met Thr
                                1               5

gag aag gag cgg gag gag cag cag aag ctg ctc ggc cgc gcc ggc aat       221
Glu Lys Glu Arg Glu Glu Gln Gln Lys Leu Leu Gly Arg Ala Gly Asn
    10                  15                  20

ggc gcg gcc gtg cag cgg tcg ccg acg gac aag ccg ccg ttc acg ctg       269
Gly Ala Ala Val Gln Arg Ser Pro Thr Asp Lys Pro Pro Phe Thr Leu
25                  30                  35                  40

ggg cag atc aag aag gcc atc ccg cct cac tgc ttc cag cgc tcg gtg       317
Gly Gln Ile Lys Lys Ala Ile Pro Pro His Cys Phe Gln Arg Ser Val
                45                  50                  55

atc aag tcc ttc tcc tac gtg gtc cat gac ctc gtg atc gtc gcc gcg       365
Ile Lys Ser Phe Ser Tyr Val Val His Asp Leu Val Ile Val Ala Ala
            60                  65                  70

ctg ctc tac ttc gcg ctg gtc atg atc ccc gtg ctg ccg agc ggg atg       413

Leu Leu Tyr Phe Ala Leu Val Met Ile Pro Val Leu Pro Ser Gly Met
        75                  80                  85

gag ttc gcg gca tgg ccg ctc tac tgg atc gcg cag ggc tgc gtg ctc      461
Glu Phe Ala Ala Trp Pro Leu Tyr Trp Ile Ala Gln Gly Cys Val Leu
    90                  95                  100

acc ggc gtg tgg gtc atc gcg cac gag tgc ggc cac cat gcc ttc tcc      509
Thr Gly Val Trp Val Ile Ala His Glu Cys Gly His His Ala Phe Ser
105                 110                 115                 120

gac tac tcg gtg ctc gac gac atc gtc ggc ctc gtg ctg cac tcg tcg      557
Asp Tyr Ser Val Leu Asp Asp Ile Val Gly Leu Val Leu His Ser Ser
                125                 130                 135

ctg ctc gtc ccc tac ttc tcg tgg aag tac agc cac cgg cgc cac cac      605
Leu Leu Val Pro Tyr Phe Ser Trp Lys Tyr Ser His Arg Arg His His
            140                 145                 150

tcc aac acc ggg tcg ctg gag cgc gac gag gtg ttc gtc ccg aag cag      653
Ser Asn Thr Gly Ser Leu Glu Arg Asp Glu Val Phe Val Pro Lys Gln
        155                 160                 165

aag tcg gcg atg gcg tgg tac acc ccg tac gtg tac cac aac ccg atc      701
Lys Ser Ala Met Ala Trp Tyr Thr Pro Tyr Val Tyr His Asn Pro Ile
    170                 175                 180

ggc cgg ctg gtg cac atc ttc gtg cag ctc acc ctc ggg tgg ccg ctg      749
Gly Arg Leu Val His Ile Phe Val Gln Leu Thr Leu Gly Trp Pro Leu
185                 190                 195                 200

tac ctg gcg ttc aac gtg tcc ggc cgc ccg tac ccg cgc ttc gcg tgc      797
Tyr Leu Ala Phe Asn Val Ser Gly Arg Pro Tyr Pro Arg Phe Ala Cys
                205                 210                 215

cac ttc gac ccc tac ggc ccg atc tac aac gac cgg gag cgc gtc cag      845
His Phe Asp Pro Tyr Gly Pro Ile Tyr Asn Asp Arg Glu Arg Val Gln
            220                 225                 230

atc ttc atc tcc gac gtc ggc gtc gtg tcc gcg ggg ctc gcc ctg ttc      893
Ile Phe Ile Ser Asp Val Gly Val Val Ser Ala Gly Leu Ala Leu Phe
        235                 240                 245

aag ctg tcg tcg gcg ttc ggg ttc tgg tgg gtg gtg cgc gtc tac ggc      941
Lys Leu Ser Ser Ala Phe Gly Phe Trp Trp Val Val Arg Val Tyr Gly
    250                 255                 260

gtg ccg ctg ctg atc gtg aac gcg tgg ctg gtg ctc atc acc tac ctg      989
Val Pro Leu Leu Ile Val Asn Ala Trp Leu Val Leu Ile Thr Tyr Leu
265                 270                 275                 280

cag cac acc cac ccg gcg ctg ccg cac tac gac tcg agc gag tgg gac     1037
Gln His Thr His Pro Ala Leu Pro His Tyr Asp Ser Ser Glu Trp Asp
                285                 290                 295

tgg ctc cgc ggc gcg ctg gcc acc gtg gac cgc gac tac ggc atc ctc     1085
Trp Leu Arg Gly Ala Leu Ala Thr Val Asp Arg Asp Tyr Gly Ile Leu
            300                 305                 310

aac aag gtg ttc cac aac atc acg gac acg cac gtc gcg cac cac ctc     1133
Asn Lys Val Phe His Asn Ile Thr Asp Thr His Val Ala His His Leu
        315                 320                 325

ttc tcc acc atg ccg cac tac cac gcc atg gag gcc act aag gcg atc 1181
Phe Ser Thr Met Pro His Tyr His Ala Met Glu Ala Thr Lys Ala Ile
    330                 335                 340

cgc ccc atc ctc ggc gag tac tac cag ttc gac ccg acg ccc gtc gcc 1229
Arg Pro Ile Leu Gly Glu Tyr Tyr Gln Phe Asp Pro Thr Pro Val Ala
345                 350                 355                 360

aag gcg aca tgg cgc gag gcc aag gag tgc atc tac gtc gag cct gag 1277
Lys Ala Thr Trp Arg Glu Ala Lys Glu Cys Ile Tyr Val Glu Pro Glu
                365                 370                 375

gac aac aag ggc gtc ttc tgg tac aac aac aag ttc taactgctgc 1323
Asp Asn Lys Gly Val Phe Trp Tyr Asn Asn Lys Phe
            380                 385

tgctgtgaaa tcagcatcac acatccatag ccaagcagca aacaaatttg aagaagaaga 1383

ttacaaggga agagaagata gtgtcttcgg aaatcgtcgt agcaagtatc catccatcca 1443

tccaacccat gaacaatcgt ctatctatcc atgcatctat ctatggttag tctctttaga 1503

taggagaggg cacttgggca cagaggaagg ctattgcagt gccattgcta gagttgccat 1563

caagtgcaaa gtaggcggat caggcgtgtg tcatgcctgt ggatttgtag tctatgtatg 1623

tgtcagctgc tgagctccgg tgtcgcagcc ttggtccctg tcgtgttatt tccatcgttt 1683

ttttccctcc gccattgttc ggtttaggtg ttgtcatggt cggcgtccgt gtggacgacg 1743

tgtcttgtgc ggtttgtctg tcattgagtt ggctccgtcc gttgcttgct gttgtaaaac 1803

gcttgtggtg ttcatggcgg aataactaaa cgtcgaatgg aatgacaact tttttgcgta 1863

<210> 22
<211> 388
<212> PRT
<213> Oryza sativa

<400> 22

Met Gly Ala Gly Gly Arg Met Thr Glu Lys Glu Arg Glu Glu Gln Gln
1               5                   10                  15

Lys Leu Leu Gly Arg Ala Gly Asn Gly Ala Ala Val Gln Arg Ser Pro
            20                  25                  30

Thr Asp Lys Pro Pro Phe Thr Leu Gly Gln Ile Lys Lys Ala Ile Pro
        35                  40                  45

Pro His Cys Phe Gln Arg Ser Val Ile Lys Ser Phe Ser Tyr Val Val
    50                  55                  60

His Asp Leu Val Ile Val Ala Ala Leu Leu Tyr Phe Ala Leu Val Met
65                  70                  75                  80

Ile Pro Val Leu Pro Ser Gly Met Glu Phe Ala Ala Trp Pro Leu Tyr
                85                  90                  95

Trp Ile Ala Gln Gly Cys Val Leu Thr Gly Val Trp Val Ile Ala His
            100                 105                 110

Glu Cys Gly His His Ala Phe Ser Asp Tyr Ser Val Leu Asp Asp Ile
        115                 120                 125

Val Gly Leu Val Leu His Ser Ser Leu Leu Val Pro Tyr Phe Ser Trp
    130                 135                 140

Lys Tyr Ser His Arg Arg His His Ser Asn Thr Gly Ser Leu Glu Arg
145                 150                 155                 160

Asp Glu Val Phe Val Pro Lys Gln Lys Ser Ala Met Ala Trp Tyr Thr
                165                 170                 175

Pro Tyr Val Tyr His Asn Pro Ile Gly Arg Leu Val His Ile Phe Val
            180                 185                 190

Gln Leu Thr Leu Gly Trp Pro Leu Tyr Leu Ala Phe Asn Val Ser Gly
        195                 200                 205

Arg Pro Tyr Pro Arg Phe Ala Cys His Phe Asp Pro Tyr Gly Pro Ile
    210                 215                 220

Tyr Asn Asp Arg Glu Arg Val Gln Ile Phe Ile Ser Asp Val Gly Val
225                 230                 235                 240

Val Ser Ala Gly Leu Ala Leu Phe Lys Leu Ser Ser Ala Phe Gly Phe
                245                 250                 255

Trp Trp Val Val Arg Val Tyr Gly Val Pro Leu Leu Ile Val Asn Ala
            260                 265                 270

Trp Leu Val Leu Ile Thr Tyr Leu Gln His Thr His Pro Ala Leu Pro
        275                 280                 285

His Tyr Asp Ser Ser Glu Trp Asp Trp Leu Arg Gly Ala Leu Ala Thr
    290                 295                 300

Val Asp Arg Asp Tyr Gly Ile Leu Asn Lys Val Phe His Asn Ile Thr
305                 310                 315                 320

Asp Thr His Val Ala His His Leu Phe Ser Thr Met Pro His Tyr His

325                 330                 335

Ala Met Glu Ala Thr Lys Ala Ile Arg Pro Ile Leu Gly Glu Tyr Tyr
        340                 345                 350

Gln Phe Asp Pro Thr Pro Val Ala Lys Ala Thr Trp Arg Glu Ala Lys
    355                 360                 365

Glu Cys Ile Tyr Val Glu Pro Glu Asp Asn Lys Gly Val Phe Trp Tyr
    370                 375                 380

Asn Asn Lys Phe
385

<210> 23
<211> 1164
<212> DNA
<213> Oryza sativa

<220>
<221> CDS
<222> (1)..(1164)

<400> 23
atg ggt gcc ggc ggc agg atg acg gag aag gag cgg gag gag cag cag      48
Met Gly Ala Gly Gly Arg Met Thr Glu Lys Glu Arg Glu Glu Gln Gln
1               5                   10                  15

aag ctg ctc ggc cgc gcc ggc aat ggc gcg gcc gtg cag cgg tcg ccg      96
Lys Leu Leu Gly Arg Ala Gly Asn Gly Ala Ala Val Gln Arg Ser Pro
            20                  25                  30

acg gac aag ccg ccg ttc acg ctg ggg cag atc aag aag gcc atc ccg      144
Thr Asp Lys Pro Pro Phe Thr Leu Gly Gln Ile Lys Lys Ala Ile Pro
        35                  40                  45

cct cac tgc ttc cag cgc tcg gtg atc aag tcc ttc tcc tac gtg gtc      192
Pro His Cys Phe Gln Arg Ser Val Ile Lys Ser Phe Ser Tyr Val Val
    50                  55                  60

cat gac ctc gtg atc gtc gcc gcg ctg ctc tac ttc gcg ctg gtc atg      240
His Asp Leu Val Ile Val Ala Ala Leu Leu Tyr Phe Ala Leu Val Met
65                  70                  75                  80

atc ccc gtg ctg ccg agc ggg atg gag ttc gcg gca tgg ccg ctc tac      288
Ile Pro Val Leu Pro Ser Gly Met Glu Phe Ala Ala Trp Pro Leu Tyr
                85                  90                  95

tgg atc gcg cag ggc tgc gtg ctc acc ggc gtg tgg gtc atc gcg cac      336
Trp Ile Ala Gln Gly Cys Val Leu Thr Gly Val Trp Val Ile Ala His
            100                 105                 110

gag tgc ggc cac cat gcc ttc tcc gac tac tcg gtg ctc gac gac atc      384
Glu Cys Gly His His Ala Phe Ser Asp Tyr Ser Val Leu Asp Asp Ile
        115                 120                 125

gtc ggc ctc gtg ctg cac tcg tcg ctg ctc gtc ccc tac ttc tcg tgg 432
Val Gly Leu Val Leu His Ser Ser Leu Leu Val Pro Tyr Phe Ser Trp
    130                 135                 140

aag tac agc cac cgg cgc cac cac tcc aac acc ggg tcg ctg gag cgc 480
Lys Tyr Ser His Arg Arg His His Ser Asn Thr Gly Ser Leu Glu Arg
145                 150                 155                 160

gac gag gtg ttc gtc ccg aag cag aag tcg gcg atg gcg tgg tac acc 528
Asp Glu Val Phe Val Pro Lys Gln Lys Ser Ala Met Ala Trp Tyr Thr
                165                 170                 175

ccg tac gtg tac cac aac ccg atc ggc cgg ctg gtg cac atc ttc gtg 576
Pro Tyr Val Tyr His Asn Pro Ile Gly Arg Leu Val His Ile Phe Val
            180                 185                 190

cag ctc acc ctc ggg tgg ccg ctg tac ctg gcg ttc aac gtg tcc ggc 624
Gln Leu Thr Leu Gly Trp Pro Leu Tyr Leu Ala Phe Asn Val Ser Gly
        195                 200                 205

cgc ccg tac ccg cgc ttc gcg tgc cac ttc gac ccc tac ggc ccg atc 672
Arg Pro Tyr Pro Arg Phe Ala Cys His Phe Asp Pro Tyr Gly Pro Ile
    210                 215                 220

tac aac gac cgg gag cgc gtc cag atc ttc atc tcc gac gtc ggc gtc 720
Tyr Asn Asp Arg Glu Arg Val Gln Ile Phe Ile Ser Asp Val Gly Val
225                 230                 235                 240

gtg tcc gcg ggg ctc gcc ctg ttc aag ctg tcg tcg gcg ttc ggg ttc 768
Val Ser Ala Gly Leu Ala Leu Phe Lys Leu Ser Ser Ala Phe Gly Phe
                245                 250                 255

tgg tgg gtg gtg cgc gtc tac ggc gtg ccg ctg ctg atc gtg aac gcg 816
Trp Trp Val Val Arg Val Tyr Gly Val Pro Leu Leu Ile Val Asn Ala
            260                 265                 270

tgg ctg gtg ctc atc acc tac ctg cag cac acc cac ccg gcg ctg ccg 864
Trp Leu Val Leu Ile Thr Tyr Leu Gln His Thr His Pro Ala Leu Pro
        275                 280                 285

cac tac gac tcg agc gag tgg gac tgg ctc cgc ggc gcg ctg gcc acc 912
His Tyr Asp Ser Ser Glu Trp Asp Trp Leu Arg Gly Ala Leu Ala Thr
    290                 295                 300

gtg gac cgc gac tac ggc atc ctc aac aag gtg ttc cac aac atc acg 960
Val Asp Arg Asp Tyr Gly Ile Leu Asn Lys Val Phe His Asn Ile Thr
305                 310                 315                 320

gac acg cac gtc gcg cac cac ctc ttc tcc acc atg ccg cac tac cac 1008
Asp Thr His Val Ala His His Leu Phe Ser Thr Met Pro His Tyr His
                325                 330                 335

gcc atg gag gcc act aag gcg atc cgc ccc atc ctc ggc gag tac tac 1056
Ala Met Glu Ala Thr Lys Ala Ile Arg Pro Ile Leu Gly Glu Tyr Tyr
            340                 345                 350

cag ttc gac ccg acg ccc gtc gcc aag gcg aca tgg cgc gag gcc aag 1104
Gln Phe Asp Pro Thr Pro Val Ala Lys Ala Thr Trp Arg Glu Ala Lys
        355                 360                 365

gag tgc atc tac gtc gag cct gag gac aac aag ggc gtc ttc tgg tac 1152
Glu Cys Ile Tyr Val Glu Pro Glu Asp Asn Lys Gly Val Phe Trp Tyr

370                 375                 380

aac aac aag ttc                                                    1164
Asn Asn Lys Phe
385


<210>  24
<211>  388
<212>  PRT
<213>  Oryza sativa

<400>  24

Met Gly Ala Gly Gly Arg Met Thr Glu Lys Glu Arg Glu Glu Gln Gln
1               5                   10                  15

Lys Leu Leu Gly Arg Ala Gly Asn Gly Ala Ala Val Gln Arg Ser Pro
            20                  25                  30

Thr Asp Lys Pro Pro Phe Thr Leu Gly Gln Ile Lys Lys Ala Ile Pro
        35                  40                  45

Pro His Cys Phe Gln Arg Ser Val Ile Lys Ser Phe Ser Tyr Val Val
    50                  55                  60

His Asp Leu Val Ile Val Ala Ala Leu Leu Tyr Phe Ala Leu Val Met
65                  70                  75                  80

Ile Pro Val Leu Pro Ser Gly Met Glu Phe Ala Ala Trp Pro Leu Tyr
                85                  90                  95

Trp Ile Ala Gln Gly Cys Val Leu Thr Gly Val Trp Val Ile Ala His
            100                 105                 110

Glu Cys Gly His His Ala Phe Ser Asp Tyr Ser Val Leu Asp Asp Ile
        115                 120                 125

Val Gly Leu Val Leu His Ser Ser Leu Leu Val Pro Tyr Phe Ser Trp
    130                 135                 140

Lys Tyr Ser His Arg Arg His His Ser Asn Thr Gly Ser Leu Glu Arg
145                 150                 155                 160

Asp Glu Val Phe Val Pro Lys Gln Lys Ser Ala Met Ala Trp Tyr Thr
                165                 170                 175

Pro Tyr Val Tyr His Asn Pro Ile Gly Arg Leu Val His Ile Phe Val
            180                 185                 190

Gln Leu Thr Leu Gly Trp Pro Leu Tyr Leu Ala Phe Asn Val Ser Gly
        195                 200                 205

Arg Pro Tyr Pro Arg Phe Ala Cys His Phe Asp Pro Tyr Gly Pro Ile
    210                 215                 220

Tyr Asn Asp Arg Glu Arg Val Gln Ile Phe Ile Ser Asp Val Gly Val
225                 230                 235                 240

Val Ser Ala Gly Leu Ala Leu Phe Lys Leu Ser Ser Ala Phe Gly Phe
                245                 250                 255

Trp Trp Val Val Arg Val Tyr Gly Val Pro Leu Leu Ile Val Asn Ala
            260                 265                 270

Trp Leu Val Leu Ile Thr Tyr Leu Gln His Thr His Pro Ala Leu Pro
        275                 280                 285

His Tyr Asp Ser Ser Glu Trp Asp Trp Leu Arg Gly Ala Leu Ala Thr
    290                 295                 300

Val Asp Arg Asp Tyr Gly Ile Leu Asn Lys Val Phe His Asn Ile Thr
305                 310                 315                 320

Asp Thr His Val Ala His His Leu Phe Ser Thr Met Pro His Tyr His
                325                 330                 335

Ala Met Glu Ala Thr Lys Ala Ile Arg Pro Ile Leu Gly Glu Tyr Tyr
            340                 345                 350

Gln Phe Asp Pro Thr Pro Val Ala Lys Ala Thr Trp Arg Glu Ala Lys
        355                 360                 365

Glu Cys Ile Tyr Val Glu Pro Glu Asp Asn Lys Gly Val Phe Trp Tyr
    370                 375                 380

Asn Asn Lys Phe
385

<210>  25
<211>  1519
<212>  DNA
<213>  Linum usitatissimum

<220>
<221>  misc_feature
<222>  (1)..(47)

<220>
<221> CDS
<222> (48)..(1070)

<220>
<221> misc_feature
<222> (1071)..(1519)

<400> 25
gctgtaacaa tatacacagg aagaagaaaa atgggtgccg gcgcaga atg tca gtg 56
Met Ser Val
1

cct cca tca tcc aaa cct atg aag agg tct cct tac tca aag cca cca 104
Pro Pro Ser Ser Lys Pro Met Lys Arg Ser Pro Tyr Ser Lys Pro Pro
    5                   10                  15

ttc acg ctc ggt gag ctc aag aag gcc att cct cca cac tgt ttc aaa 152
Phe Thr Leu Gly Glu Leu Lys Lys Ala Ile Pro Pro His Cys Phe Lys
20                  25                  30                  35

cgc tca atc ccc cga tcg ttc gcc tac gtg gcg tac gac ctc acc att 200
Arg Ser Ile Pro Arg Ser Phe Ala Tyr Val Ala Tyr Asp Leu Thr Ile
                40                  45                  50

gca gca atc ttc tac tac atc gcc acc act tac ttc cac ctc ctc cct 248
Ala Ala Ile Phe Tyr Tyr Ile Ala Thr Thr Tyr Phe His Leu Leu Pro
            55                  60                  65

agc cct ctc aac tac ctc gcc tgg ccg gtc tac tgg gcc tgc cag ggc 296
Ser Pro Leu Asn Tyr Leu Ala Trp Pro Val Tyr Trp Ala Cys Gln Gly
        70                  75                  80

tgc atc ctc act gga gta tgg gtg ttg gct cac gaa tgc ggt cac cat 344
Cys Ile Leu Thr Gly Val Trp Val Leu Ala His Glu Cys Gly His His
    85                  90                  95

gcc ttc agc gac tac cag tgg ctc gac gac atg gtt ggc ttc gtc ctc 392
Ala Phe Ser Asp Tyr Gln Trp Leu Asp Asp Met Val Gly Phe Val Leu
100                 105                 110                 115

cat tcg tcc ctc ctt gtt cct tac ttc tcc tgg aag cac agc cac cgc 440
His Ser Ser Leu Leu Val Pro Tyr Phe Ser Trp Lys His Ser His Arg
                120                 125                 130

cgc cac cat tcc aac acg gga tcg ctt gat cgt gat gag gtg ttt gtc 488
Arg His His Ser Asn Thr Gly Ser Leu Asp Arg Asp Glu Val Phe Val
            135                 140                 145

ccc aag cag aag gcc gaa atc ggg tgg tac tcc aag tac ctt aac aac 536
Pro Lys Gln Lys Ala Glu Ile Gly Trp Tyr Ser Lys Tyr Leu Asn Asn
        150                 155                 160

cca cct ggc cgt gtg atc aca ttg gcc gtc aca tta acg ctc ggt tgg 584
Pro Pro Gly Arg Val Ile Thr Leu Ala Val Thr Leu Thr Leu Gly Trp
    165                 170                 175

cct ctg tac ttg gca ttc aac gtc tcc ggg aga cca tat gac cgg ttc 632
Pro Leu Tyr Leu Ala Phe Asn Val Ser Gly Arg Pro Tyr Asp Arg Phe
180                 185                 190                 195

gca tgc cat ttt gac cct cac ggt ccg att tac aat gat cgc gag cgt 680

Ala Cys His Phe Asp Pro His Gly Pro Ile Tyr Asn Asp Arg Glu Arg
                200                 205                 210

atg gag ata tac cta tcc gac gca ggg ata ttc acc gtg tgc tac atc 728
Met Glu Ile Tyr Leu Ser Asp Ala Gly Ile Phe Thr Val Cys Tyr Ile
            215                 220                 225

cta tac aga ctc gtc ctc acg aaa gga ctc gtt tgg gtc gtg tcc ata 776
Leu Tyr Arg Leu Val Leu Thr Lys Gly Leu Val Trp Val Val Ser Ile
        230                 235                 240

tac gga gtc cca cta ttg ata gtg aat gga ttc cta gtc ctc atc act 824
Tyr Gly Val Pro Leu Leu Ile Val Asn Gly Phe Leu Val Leu Ile Thr
    245                 250                 255

ttc ttg cag cac acg cat cct tct ctt ccg cac tac aaa gtc ctc cga 872
Phe Leu Gln His Thr His Pro Ser Leu Pro His Tyr Lys Val Leu Arg
260                 265                 270                 275

atg ggg act gga tgc gag gcg ccc tct cga ccg tgg atc gag act acg 920
Met Gly Thr Gly Cys Glu Ala Pro Ser Arg Pro Trp Ile Glu Thr Thr
                280                 285                 290

ggt tac tca aca ccg tgt tcc aca aca tca ccg ata cac atg tcg cgc 968
Gly Tyr Ser Thr Pro Cys Ser Thr Thr Ser Pro Ile His Met Ser Arg
            295                 300                 305

acc atc tct tct cca cga tgc ctc att acc acg cga tgg agg cta cca 1016
Thr Ile Ser Ser Pro Arg Cys Leu Ile Thr Thr Arg Trp Arg Leu Pro
        310                 315                 320

agg cga tca agc cgg ttc tcg ggg agt att acc agt tcg atg gga ctc 1064
Arg Arg Ser Ser Arg Phe Ser Gly Ser Ile Thr Ser Ser Met Gly Leu
    325                 330                 335

cct ttg tgaaggccat gtggagggag gcaaaggagt gcatctatgt cgagccggat 1120
Pro Leu
340

gaaggcgacc ccagccaagg cgtgttctgg tacaacaaca agctgtgagg gtcttcgaaa 1180

tttgcagagg tttgtagtgt ttgttcttaa tggtgttacc agaaaaatgt ttgaagaaag 1240

aagctgcaat agctagtgca gaactggtgt atgtttctgt aatgtttgtt aagttatgtc 1300

cctagtggtc gttaatgtta ctgtacttct ctgttcttct ccatcgagcc aacatacctt 1360

cactcctctg ttaatgtact gagttggtcg agtttaaact taacggacca ccaggctcaa 1420

attcgagtca ccgggttggc cgagtttaga ctgcattgac cacaatgatg caatcgcaaa 1480

actgaagtga ctacaatcgc aaaacttaat tcccagtca 1519

<210> 26
<211> 341
<212> PRT
<213> Linum usitatissimum

<400> 26

Met Ser Val Pro Pro Ser Ser Lys Pro Met Lys Arg Ser Pro Tyr Ser

1               5                   10                  15

Lys Pro Pro Phe Thr Leu Gly Glu Leu Lys Lys Ala Ile Pro Pro His
            20                  25                  30

Cys Phe Lys Arg Ser Ile Pro Arg Ser Phe Ala Tyr Val Ala Tyr Asp
        35                  40                  45

Leu Thr Ile Ala Ala Ile Phe Tyr Tyr Ile Ala Thr Thr Tyr Phe His
    50                  55                  60

Leu Leu Pro Ser Pro Leu Asn Tyr Leu Ala Trp Pro Val Tyr Trp Ala
65                  70                  75                  80

Cys Gln Gly Cys Ile Leu Thr Gly Val Trp Val Leu Ala His Glu Cys
                85                  90                  95

Gly His His Ala Phe Ser Asp Tyr Gln Trp Leu Asp Asp Met Val Gly
            100                 105                 110

Phe Val Leu His Ser Ser Leu Leu Val Pro Tyr Phe Ser Trp Lys His
        115                 120                 125

Ser His Arg Arg His His Ser Asn Thr Gly Ser Leu Asp Arg Asp Glu
    130                 135                 140

Val Phe Val Pro Lys Gln Lys Ala Glu Ile Gly Trp Tyr Ser Lys Tyr
145                 150                 155                 160

Leu Asn Asn Pro Pro Gly Arg Val Ile Thr Leu Ala Val Thr Leu Thr
                165                 170                 175

Leu Gly Trp Pro Leu Tyr Leu Ala Phe Asn Val Ser Gly Arg Pro Tyr
            180                 185                 190

Asp Arg Phe Ala Cys His Phe Asp Pro His Gly Pro Ile Tyr Asn Asp
        195                 200                 205

Arg Glu Arg Met Glu Ile Tyr Leu Ser Asp Ala Gly Ile Phe Thr Val
    210                 215                 220

Cys Tyr Ile Leu Tyr Arg Leu Val Leu Thr Lys Gly Leu Val Trp Val
225                 230                 235                 240

Val Ser Ile Tyr Gly Val Pro Leu Leu Ile Val Asn Gly Phe Leu Val
                245                 250                 255

Leu Ile Thr Phe Leu Gln His Thr His Pro Ser Leu Pro His Tyr Lys
            260                 265                 270

Val Leu Arg Met Gly Thr Gly Cys Glu Ala Pro Ser Arg Pro Trp Ile
        275                 280                 285

Glu Thr Thr Gly Tyr Ser Thr Pro Cys Ser Thr Thr Ser Pro Ile His
    290                 295                 300

Met Ser Arg Thr Ile Ser Ser Pro Arg Cys Leu Ile Thr Thr Arg Trp
305                 310                 315                 320

Arg Leu Pro Arg Arg Ser Ser Arg Phe Ser Gly Ser Ile Thr Ser Ser
                325                 330                 335

Met Gly Leu Pro Leu
            340

<210> 27
<211> 1023
<212> DNA
<213> Linum usitatissimum

<220>
<221> CDS
<222> (1)..(1023)

<400> 27
atg tca gtg cct cca tca tcc aaa cct atg aag agg tct cct tac tca 48
Met Ser Val Pro Pro Ser Ser Lys Pro Met Lys Arg Ser Pro Tyr Ser
1               5                   10                  15

aag cca cca ttc acg ctc ggt gag ctc aag aag gcc att cct cca cac 96
Lys Pro Pro Phe Thr Leu Gly Glu Leu Lys Lys Ala Ile Pro Pro His
            20                  25                  30

tgt ttc aaa cgc tca atc ccc cga tcg ttc gcc tac gtg gcg tac gac 144
Cys Phe Lys Arg Ser Ile Pro Arg Ser Phe Ala Tyr Val Ala Tyr Asp
        35                  40                  45

ctc acc att gca gca atc ttc tac tac atc gcc acc act tac ttc cac 192
Leu Thr Ile Ala Ala Ile Phe Tyr Tyr Ile Ala Thr Thr Tyr Phe His
    50                  55                  60

ctc ctc cct agc cct ctc aac tac ctc gcc tgg ccg gtc tac tgg gcc 240
Leu Leu Pro Ser Pro Leu Asn Tyr Leu Ala Trp Pro Val Tyr Trp Ala
65                  70                  75                  80

tgc cag ggc tgc atc ctc act gga gta tgg gtg ttg gct cac gaa tgc 288
Cys Gln Gly Cys Ile Leu Thr Gly Val Trp Val Leu Ala His Glu Cys
                85                  90                  95

ggt cac cat gcc ttc agc gac tac cag tgg ctc gac gac atg gtt ggc 336
Gly His His Ala Phe Ser Asp Tyr Gln Trp Leu Asp Asp Met Val Gly

            100                 105                 110

ttc gtc ctc cat tcg tcc ctc ctt gtt cct tac ttc tcc tgg aag cac 384
Phe Val Leu His Ser Ser Leu Leu Val Pro Tyr Phe Ser Trp Lys His
        115                 120                 125

agc cac cgc cgc cac cat tcc aac acg gga tcg ctt gat cgt gat gag 432
Ser His Arg Arg His His Ser Asn Thr Gly Ser Leu Asp Arg Asp Glu
    130                 135                 140

gtg ttt gtc ccc aag cag aag gcc gaa atc ggg tgg tac tcc aag tac 480
Val Phe Val Pro Lys Gln Lys Ala Glu Ile Gly Trp Tyr Ser Lys Tyr
145                 150                 155                 160

ctt aac aac cca cct ggc cgt gtg atc aca ttg gcc gtc aca tta acg 528
Leu Asn Asn Pro Pro Gly Arg Val Ile Thr Leu Ala Val Thr Leu Thr
                165                 170                 175

ctc ggt tgg cct ctg tac ttg gca ttc aac gtc tcc ggg aga cca tat 576
Leu Gly Trp Pro Leu Tyr Leu Ala Phe Asn Val Ser Gly Arg Pro Tyr
            180                 185                 190

gac cgg ttc gca tgc cat ttt gac cct cac ggt ccg att tac aat gat 624
Asp Arg Phe Ala Cys His Phe Asp Pro His Gly Pro Ile Tyr Asn Asp
        195                 200                 205

cgc gag cgt atg gag ata tac cta tcc gac gca ggg ata ttc acc gtg 672
Arg Glu Arg Met Glu Ile Tyr Leu Ser Asp Ala Gly Ile Phe Thr Val
    210                 215                 220

tgc tac atc cta tac aga ctc gtc ctc acg aaa gga ctc gtt tgg gtc 720
Cys Tyr Ile Leu Tyr Arg Leu Val Leu Thr Lys Gly Leu Val Trp Val
225                 230                 235                 240

gtg tcc ata tac gga gtc cca cta ttg ata gtg aat gga ttc cta gtc 768
Val Ser Ile Tyr Gly Val Pro Leu Leu Ile Val Asn Gly Phe Leu Val
                245                 250                 255

ctc atc act ttc ttg cag cac acg cat cct tct ctt ccg cac tac aaa 816
Leu Ile Thr Phe Leu Gln His Thr His Pro Ser Leu Pro His Tyr Lys
            260                 265                 270

gtc ctc cga atg ggg act gga tgc gag gcg ccc tct cga ccg tgg atc 864
Val Leu Arg Met Gly Thr Gly Cys Glu Ala Pro Ser Arg Pro Trp Ile
        275                 280                 285

gag act acg ggt tac tca aca ccg tgt tcc aca aca tca ccg ata cac 912
Glu Thr Thr Gly Tyr Ser Thr Pro Cys Ser Thr Thr Ser Pro Ile His
    290                 295                 300

atg tcg cgc acc atc tct tct cca cga tgc ctc att acc acg cga tgg 960
Met Ser Arg Thr Ile Ser Ser Pro Arg Cys Leu Ile Thr Thr Arg Trp
305                 310                 315                 320

agg cta cca agg cga tca agc cgg ttc tcg ggg agt att acc agt tcg 1008
Arg Leu Pro Arg Arg Ser Ser Arg Phe Ser Gly Ser Ile Thr Ser Ser
                325                 330                 335

atg gga ctc cct ttg 1023
Met Gly Leu Pro Leu
            340

<210>  28
<211>  341
<212>  PRT
<213>  Linum usitatissimum

<400>  28

Met Ser Val Pro Pro Ser Ser Lys Pro Met Lys Arg Ser Pro Tyr Ser
1               5                   10                  15

Lys Pro Pro Phe Thr Leu Gly Glu Leu Lys Lys Ala Ile Pro Pro His
            20                  25                  30

Cys Phe Lys Arg Ser Ile Pro Arg Ser Phe Ala Tyr Val Ala Tyr Asp
        35                  40                  45

Leu Thr Ile Ala Ala Ile Phe Tyr Tyr Ile Ala Thr Thr Tyr Phe His
    50                  55                  60

Leu Leu Pro Ser Pro Leu Asn Tyr Leu Ala Trp Pro Val Tyr Trp Ala
65                  70                  75                  80

Cys Gln Gly Cys Ile Leu Thr Gly Val Trp Val Leu Ala His Glu Cys
                85                  90                  95

Gly His His Ala Phe Ser Asp Tyr Gln Trp Leu Asp Asp Met Val Gly
            100                 105                 110

Phe Val Leu His Ser Ser Leu Leu Val Pro Tyr Phe Ser Trp Lys His
        115                 120                 125

Ser His Arg Arg His His Ser Asn Thr Gly Ser Leu Asp Arg Asp Glu
    130                 135                 140

Val Phe Val Pro Lys Gln Lys Ala Glu Ile Gly Trp Tyr Ser Lys Tyr
145                 150                 155                 160

Leu Asn Asn Pro Pro Gly Arg Val Ile Thr Leu Ala Val Thr Leu Thr
                165                 170                 175

Leu Gly Trp Pro Leu Tyr Leu Ala Phe Asn Val Ser Gly Arg Pro Tyr
            180                 185                 190

Asp Arg Phe Ala Cys His Phe Asp Pro His Gly Pro Ile Tyr Asn Asp
        195                 200                 205

Arg Glu Arg Met Glu Ile Tyr Leu Ser Asp Ala Gly Ile Phe Thr Val
    210                 215                 220

Cys Tyr Ile Leu Tyr Arg Leu Val Leu Thr Lys Gly Leu Val Trp Val
225                 230                 235                 240

Val Ser Ile Tyr Gly Val Pro Leu Leu Ile Val Asn Gly Phe Leu Val
                245                 250                 255

Leu Ile Thr Phe Leu Gln His Thr His Pro Ser Leu Pro His Tyr Lys
            260                 265                 270

Val Leu Arg Met Gly Thr Gly Cys Glu Ala Pro Ser Arg Pro Trp Ile
        275                 280                 285

Glu Thr Thr Gly Tyr Ser Thr Pro Cys Ser Thr Thr Ser Pro Ile His
    290                 295                 300

Met Ser Arg Thr Ile Ser Ser Pro Arg Cys Leu Ile Thr Thr Arg Trp
305                 310                 315                 320

Arg Leu Pro Arg Arg Ser Ser Arg Phe Ser Gly Ser Ile Thr Ser Ser
                325                 330                 335

Met Gly Leu Pro Leu
            340

<210>  29
<211>  1545
<212>  DNA
<213>  Hordeum vulgare

<220>
<221>  misc_feature
<222>  (1)..(24)

<220>
<221>  CDS
<222>  (25)..(1185)

<220>
<221>  misc_feature
<222>  (1186)..(1545)

<400>  29
accaccacca cccctaccag catc atg ggt gcc ggc ggc ggg atg acc gag        51
                           Met Gly Ala Gly Gly Gly Met Thr Glu
                           1               5

aag gag cgg gag aag cag gag cag ctc ggc cgc gcc ggc ggc ggc gca        99
Lys Glu Arg Glu Lys Gln Glu Gln Leu Gly Arg Ala Gly Gly Gly Ala
10                  15                  20                  25

gcc ttc cag cgc tcg ccg acg gac aag ccg ccg ttc acg ctc ggt cag       147

Ala Phe Gln Arg Ser Pro Thr Asp Lys Pro Pro Phe Thr Leu Gly Gln
                30                  35                  40

atc aag aag gcg atc ccg cct cac tgc ttc cag cgc tcc atc atc aag 195
Ile Lys Lys Ala Ile Pro Pro His Cys Phe Gln Arg Ser Ile Ile Lys
            45                  50                  55

tcc ttc tcc tac gtg gtt cat gac ctg gtc atc atc gcc gcc ctg ctg 243
Ser Phe Ser Tyr Val Val His Asp Leu Val Ile Ile Ala Ala Leu Leu
        60                  65                  70

tac gcc gct ctg gtc tgg atc ccc acc ctc cct acc gtg ttg cag ctg 291
Tyr Ala Ala Leu Val Trp Ile Pro Thr Leu Pro Thr Val Leu Gln Leu
    75                  80                  85

ggc gcg tgg ccg ctc tac tgg atc gtt cag ggc tgc gtc atg acc ggc 339
Gly Ala Trp Pro Leu Tyr Trp Ile Val Gln Gly Cys Val Met Thr Gly
90                  95                  100                 105

gtc tgg gtc atc gcg cac gag tgc ggc cac cat gcc ttc tct gac tac 387
Val Trp Val Ile Ala His Glu Cys Gly His His Ala Phe Ser Asp Tyr
                110                 115                 120

tcg ctg ctc gac gac acc gtc ggc ctg gtg ctc cac tcg tgg ctg ctc 435
Ser Leu Leu Asp Asp Thr Val Gly Leu Val Leu His Ser Trp Leu Leu
            125                 130                 135

gtc cca tac ttc tcg tgg aag tac agc cac cgt cgc cac cac tcc aac 483
Val Pro Tyr Phe Ser Trp Lys Tyr Ser His Arg Arg His His Ser Asn
        140                 145                 150

acc ggg tcg ctg gag cgc gac gag gtg ttt gtc ccc aag cag aag gag 531
Thr Gly Ser Leu Glu Arg Asp Glu Val Phe Val Pro Lys Gln Lys Glu
    155                 160                 165

gcg ctg gca tgg tac act ccc tac atc tac aac aac ccc atc ggc cgt 579
Ala Leu Ala Trp Tyr Thr Pro Tyr Ile Tyr Asn Asn Pro Ile Gly Arg
170                 175                 180                 185

ctg gtg cac atc gtg gtg cag ctc acc ctc ggg tgg ccg ctg tac ctg 627
Leu Val His Ile Val Val Gln Leu Thr Leu Gly Trp Pro Leu Tyr Leu
                190                 195                 200

gcg ctc aac gcc tca ggc cgt ccg tac ccg cgc ttc gcc tgc cac ttc 675
Ala Leu Asn Ala Ser Gly Arg Pro Tyr Pro Arg Phe Ala Cys His Phe
            205                 210                 215

gac ccc tac ggc ccg atc tac aac gac cgg gag cga gcc cag att ttc 723
Asp Pro Tyr Gly Pro Ile Tyr Asn Asp Arg Glu Arg Ala Gln Ile Phe
        220                 225                 230

atc tcg gat gtc ggc gtg ttg gcc gtc tcc ttg gcc ctg ctc aag ctt 771
Ile Ser Asp Val Gly Val Leu Ala Val Ser Leu Ala Leu Leu Lys Leu
    235                 240                 245

gtg tcg tcg ttt ggg ttc tgg tgg gtg gtg cgg gtc tac ggc gtg ccg 819
Val Ser Ser Phe Gly Phe Trp Trp Val Val Arg Val Tyr Gly Val Pro
250                 255                 260                 265

ctg ctg atc gtg aac gcg tgg ctg gtc ctg atc acc tac ctg cag cac 867
Leu Leu Ile Val Asn Ala Trp Leu Val Leu Ile Thr Tyr Leu Gln His
                270                 275                 280

acc cac cca gcg ctg ccg cac tac gac tcg acg gag tgg gac tgg ctg     915
Thr His Pro Ala Leu Pro His Tyr Asp Ser Thr Glu Trp Asp Trp Leu
            285                 290                 295

cgg ggg gcg ctc gcc acc atg gac cgg gac tac ggc att ctc aac cgc     963
Arg Gly Ala Leu Ala Thr Met Asp Arg Asp Tyr Gly Ile Leu Asn Arg
        300                 305                 310

gtg ttc cac aac atc acg gac acg cac gtg gcg cac cac ctc ttc tcc     1011
Val Phe His Asn Ile Thr Asp Thr His Val Ala His His Leu Phe Ser
    315                 320                 325

aac atg ccg cac tac cac gcc atg gag gcc acc aag gcg atc aag ccc     1059
Asn Met Pro His Tyr His Ala Met Glu Ala Thr Lys Ala Ile Lys Pro
330                 335                 340                 345

atc ctc ggc gag tac tac cag ttt gac ggc acc ccg gtc gcc aag gcc     1107
Ile Leu Gly Glu Tyr Tyr Gln Phe Asp Gly Thr Pro Val Ala Lys Ala
                350                 355                 360

aca tgg cgc gag gcc aag gag tgc atc tac gtt gag ccc gag gac cgc     1155
Thr Trp Arg Glu Ala Lys Glu Cys Ile Tyr Val Glu Pro Glu Asp Arg
            365                 370                 375

aag ggg gtc ttc tgg tac agc aac aag ttc tagccgcaag gatcgtcatc     1205
Lys Gly Val Phe Trp Tyr Ser Asn Lys Phe
        380                 385

agccgtgttc caggaagaac tcagagaaga ggtccttaca agtaatccat ccatctacct     1265

acatatggtt agtttttaga tagcagaggg catttgggca caaacaagac tactattacc     1325

gtgccaatgc tagaaagagt tgagtggtgc aaggaggagt agcgtgtccg tgacttttgt     1385

cagttccttc tttactttcc tcctgcgtct tagtcgccgg cggtcgttgt tggtgtccgt     1445

ggccattgac atggccgtgt gtgttgtgtg tgcgtctgtc attgcattgg cgtcatctcc     1505

ccccgtccgt gtcatgttgt tgtagaccat ttcgtgtttt     1545

<210>  30
<211>  387
<212>  PRT
<213>  Hordeum vulgare

<400>  30

Met Gly Ala Gly Gly Gly Met Thr Glu Lys Glu Arg Glu Lys Gln Glu
1               5                   10                  15

Gln Leu Gly Arg Ala Gly Gly Gly Ala Ala Phe Gln Arg Ser Pro Thr
            20                  25                  30

Asp Lys Pro Pro Phe Thr Leu Gly Gln Ile Lys Lys Ala Ile Pro Pro
        35                  40                  45

His Cys Phe Gln Arg Ser Ile Ile Lys Ser Phe Ser Tyr Val Val His

50                  55                  60

Asp Leu Val Ile Ile Ala Ala Leu Leu Tyr Ala Ala Leu Val Trp Ile
65                  70                  75                  80

Pro Thr Leu Pro Thr Val Leu Gln Leu Gly Ala Trp Pro Leu Tyr Trp
                85                  90                  95

Ile Val Gln Gly Cys Val Met Thr Gly Val Trp Val Ile Ala His Glu
            100                 105                 110

Cys Gly His His Ala Phe Ser Asp Tyr Ser Leu Leu Asp Asp Thr Val
        115                 120                 125

Gly Leu Val Leu His Ser Trp Leu Leu Val Pro Tyr Phe Ser Trp Lys
    130                 135                 140

Tyr Ser His Arg Arg His His Ser Asn Thr Gly Ser Leu Glu Arg Asp
145                 150                 155                 160

Glu Val Phe Val Pro Lys Gln Lys Glu Ala Leu Ala Trp Tyr Thr Pro
                165                 170                 175

Tyr Ile Tyr Asn Asn Pro Ile Gly Arg Leu Val His Ile Val Val Gln
            180                 185                 190

Leu Thr Leu Gly Trp Pro Leu Tyr Leu Ala Leu Asn Ala Ser Gly Arg
        195                 200                 205

Pro Tyr Pro Arg Phe Ala Cys His Phe Asp Pro Tyr Gly Pro Ile Tyr
    210                 215                 220

Asn Asp Arg Glu Arg Ala Gln Ile Phe Ile Ser Asp Val Gly Val Leu
225                 230                 235                 240

Ala Val Ser Leu Ala Leu Leu Lys Leu Val Ser Ser Phe Gly Phe Trp
                245                 250                 255

Trp Val Val Arg Val Tyr Gly Val Pro Leu Leu Ile Val Asn Ala Trp
            260                 265                 270

Leu Val Leu Ile Thr Tyr Leu Gln His Thr His Pro Ala Leu Pro His
        275                 280                 285

Tyr Asp Ser Thr Glu Trp Asp Trp Leu Arg Gly Ala Leu Ala Thr Met
    290                 295                 300

Asp Arg Asp Tyr Gly Ile Leu Asn Arg Val Phe His Asn Ile Thr Asp
305                 310                 315                 320

Thr His Val Ala His His Leu Phe Ser Asn Met Pro His Tyr His Ala
                325                 330                 335

Met Glu Ala Thr Lys Ala Ile Lys Pro Ile Leu Gly Glu Tyr Tyr Gln
            340                 345                 350

Phe Asp Gly Thr Pro Val Ala Lys Ala Thr Trp Arg Glu Ala Lys Glu
        355                 360                 365

Cys Ile Tyr Val Glu Pro Glu Asp Arg Lys Gly Val Phe Trp Tyr Ser
    370                 375                 380

Asn Lys Phe
385

<210>  31
<211>  1161
<212>  DNA
<213>  Hordeum vulgare

<220>
<221>  CDS
<222>  (1)..(1161)

<400>  31
atg ggt gcc ggc ggc ggg atg acc gag aag gag cgg gag aag cag gag      48
Met Gly Ala Gly Gly Gly Met Thr Glu Lys Glu Arg Glu Lys Gln Glu
1               5                   10                  15

cag ctc ggc cgc gcc ggc ggc ggc gca gcc ttc cag cgc tcg ccg acg      96
Gln Leu Gly Arg Ala Gly Gly Gly Ala Ala Phe Gln Arg Ser Pro Thr
            20                  25                  30

gac aag ccg ccg ttc acg ctc ggt cag atc aag aag gcg atc ccg cct      144
Asp Lys Pro Pro Phe Thr Leu Gly Gln Ile Lys Lys Ala Ile Pro Pro
        35                  40                  45

cac tgc ttc cag cgc tcc atc atc aag tcc ttc tcc tac gtg gtt cat      192
His Cys Phe Gln Arg Ser Ile Ile Lys Ser Phe Ser Tyr Val Val His
    50                  55                  60

gac ctg gtc atc atc gcc gcc ctg ctg tac gcc gct ctg gtc tgg atc      240
Asp Leu Val Ile Ile Ala Ala Leu Leu Tyr Ala Ala Leu Val Trp Ile
65                  70                  75                  80

ccc acc ctc cct acc gtg ttg cag ctg ggc gcg tgg ccg ctc tac tgg      288
Pro Thr Leu Pro Thr Val Leu Gln Leu Gly Ala Trp Pro Leu Tyr Trp
                85                  90                  95

atc gtt cag ggc tgc gtc atg acc ggc gtc tgg gtc atc gcg cac gag      336
Ile Val Gln Gly Cys Val Met Thr Gly Val Trp Val Ile Ala His Glu

            100                 105                 110

tgc ggc cac cat gcc ttc tct gac tac tcg ctg ctc gac gac acc gtc 384
Cys Gly His His Ala Phe Ser Asp Tyr Ser Leu Leu Asp Asp Thr Val
        115                 120                 125

ggc ctg gtg ctc cac tcg tgg ctg ctc gtc cca tac ttc tcg tgg aag 432
Gly Leu Val Leu His Ser Trp Leu Leu Val Pro Tyr Phe Ser Trp Lys
    130                 135                 140

tac agc cac cgt cgc cac cac tcc aac acc ggg tcg ctg gag cgc gac 480
Tyr Ser His Arg Arg His His Ser Asn Thr Gly Ser Leu Glu Arg Asp
145                 150                 155                 160

gag gtg ttt gtc ccc aag cag aag gag gcg ctg gca tgg tac act ccc 528
Glu Val Phe Val Pro Lys Gln Lys Glu Ala Leu Ala Trp Tyr Thr Pro
                165                 170                 175

tac atc tac aac aac ccc atc ggc cgt ctg gtg cac atc gtg gtg cag 576
Tyr Ile Tyr Asn Asn Pro Ile Gly Arg Leu Val His Ile Val Val Gln
            180                 185                 190

ctc acc ctc ggg tgg ccg ctg tac ctg gcg ctc aac gcc tca ggc cgt 624
Leu Thr Leu Gly Trp Pro Leu Tyr Leu Ala Leu Asn Ala Ser Gly Arg
        195                 200                 205

ccg tac ccg cgc ttc gcc tgc cac ttc gac ccc tac ggc ccg atc tac 672
Pro Tyr Pro Arg Phe Ala Cys His Phe Asp Pro Tyr Gly Pro Ile Tyr
    210                 215                 220

aac gac cgg gag cga gcc cag att ttc atc tcg gat gtc ggc gtg ttg 720
Asn Asp Arg Glu Arg Ala Gln Ile Phe Ile Ser Asp Val Gly Val Leu
225                 230                 235                 240

gcc gtc tcc ttg gcc ctg ctc aag ctt gtg tcg tcg ttt ggg ttc tgg 768
Ala Val Ser Leu Ala Leu Leu Lys Leu Val Ser Ser Phe Gly Phe Trp
                245                 250                 255

tgg gtg gtg cgg gtc tac ggc gtg ccg ctg ctg atc gtg aac gcg tgg 816
Trp Val Val Arg Val Tyr Gly Val Pro Leu Leu Ile Val Asn Ala Trp
            260                 265                 270

ctg gtc ctg atc acc tac ctg cag cac acc cac cca gcg ctg ccg cac 864
Leu Val Leu Ile Thr Tyr Leu Gln His Thr His Pro Ala Leu Pro His
        275                 280                 285

tac gac tcg acg gag tgg gac tgg ctg cgg ggg gcg ctc gcc acc atg 912
Tyr Asp Ser Thr Glu Trp Asp Trp Leu Arg Gly Ala Leu Ala Thr Met
    290                 295                 300

gac cgg gac tac ggc att ctc aac cgc gtg ttc cac aac atc acg gac 960
Asp Arg Asp Tyr Gly Ile Leu Asn Arg Val Phe His Asn Ile Thr Asp
305                 310                 315                 320

acg cac gtg gcg cac cac ctc ttc tcc aac atg ccg cac tac cac gcc 1008
Thr His Val Ala His His Leu Phe Ser Asn Met Pro His Tyr His Ala
                325                 330                 335

atg gag gcc acc aag gcg atc aag ccc atc ctc ggc gag tac tac cag 1056
Met Glu Ala Thr Lys Ala Ile Lys Pro Ile Leu Gly Glu Tyr Tyr Gln
            340                 345                 350

ttt gac ggc acc ccg gtc gcc aag gcc aca tgg cgc gag gcc aag gag 1104
Phe Asp Gly Thr Pro Val Ala Lys Ala Thr Trp Arg Glu Ala Lys Glu
        355                 360                 365

tgc atc tac gtt gag ccc gag gac cgc aag ggg gtc ttc tgg tac agc 1152
Cys Ile Tyr Val Glu Pro Glu Asp Arg Lys Gly Val Phe Trp Tyr Ser
    370                 375                 380

aac aag ttc 1161
Asn Lys Phe
385

<210> 32
<211> 387
<212> PRT
<213> Hordeum vulgare

<400> 32

Met Gly Ala Gly Gly Gly Met Thr Glu Lys Glu Arg Glu Lys Gln Glu
1               5                   10                  15

Gln Leu Gly Arg Ala Gly Gly Gly Ala Ala Phe Gln Arg Ser Pro Thr
            20                  25                  30

Asp Lys Pro Pro Phe Thr Leu Gly Gln Ile Lys Lys Ala Ile Pro Pro
        35                  40                  45

His Cys Phe Gln Arg Ser Ile Ile Lys Ser Phe Ser Tyr Val Val His
    50                  55                  60

Asp Leu Val Ile Ile Ala Ala Leu Leu Tyr Ala Ala Leu Val Trp Ile
65                  70                  75                  80

Pro Thr Leu Pro Thr Val Leu Gln Leu Gly Ala Trp Pro Leu Tyr Trp
                85                  90                  95

Ile Val Gln Gly Cys Val Met Thr Gly Val Trp Val Ile Ala His Glu
            100                 105                 110

Cys Gly His His Ala Phe Ser Asp Tyr Ser Leu Leu Asp Asp Thr Val
        115                 120                 125

Gly Leu Val Leu His Ser Trp Leu Leu Val Pro Tyr Phe Ser Trp Lys
    130                 135                 140

Tyr Ser His Arg Arg His His Ser Asn Thr Gly Ser Leu Glu Arg Asp
145                 150                 155                 160

Glu Val Phe Val Pro Lys Gln Lys Glu Ala Leu Ala Trp Tyr Thr Pro
                165                 170                 175

Tyr Ile Tyr Asn Asn Pro Ile Gly Arg Leu Val His Ile Val Val Gln
            180                 185                 190

Leu Thr Leu Gly Trp Pro Leu Tyr Leu Ala Leu Asn Ala Ser Gly Arg
        195                 200                 205

Pro Tyr Pro Arg Phe Ala Cys His Phe Asp Pro Tyr Gly Pro Ile Tyr
    210                 215                 220

Asn Asp Arg Glu Arg Ala Gln Ile Phe Ile Ser Asp Val Gly Val Leu
225                 230                 235                 240

Ala Val Ser Leu Ala Leu Leu Lys Leu Val Ser Ser Phe Gly Phe Trp
                245                 250                 255

Trp Val Val Arg Val Tyr Gly Val Pro Leu Leu Ile Val Asn Ala Trp
            260                 265                 270

Leu Val Leu Ile Thr Tyr Leu Gln His Thr His Pro Ala Leu Pro His
        275                 280                 285

Tyr Asp Ser Thr Glu Trp Asp Trp Leu Arg Gly Ala Leu Ala Thr Met
    290                 295                 300

Asp Arg Asp Tyr Gly Ile Leu Asn Arg Val Phe His Asn Ile Thr Asp
305                 310                 315                 320

Thr His Val Ala His His Leu Phe Ser Asn Met Pro His Tyr His Ala
                325                 330                 335

Met Glu Ala Thr Lys Ala Ile Lys Pro Ile Leu Gly Glu Tyr Tyr Gln
            340                 345                 350

Phe Asp Gly Thr Pro Val Ala Lys Ala Thr Trp Arg Glu Ala Lys Glu
        355                 360                 365

Cys Ile Tyr Val Glu Pro Glu Asp Arg Lys Gly Val Phe Trp Tyr Ser
    370                 375                 380

Asn Lys Phe
385

<210>  33
<211>  1749
<212>  DNA
<213>  Triticum aestivum

<220>
<221> misc_feature
<222> (1)..(164)

<220>
<221> CDS
<222> (165)..(1325)

<220>
<221> misc_feature
<222> (1326)..(1749)

<400> 33
cgcgtccgcg gctcctcccc cccgcacaaa ccactcgttc gtcccgtcaa caagaggagc 60

agaggcgccg gagagggaag agggtgcgcg cgctcgcgtg tgtggtgtcc gccggcccga 120

tctgccctgc tcccgccgcc tcgaccacca cccctatcag catc atg ggt gcc ggc 176
Met Gly Ala Gly
1

ggc agg atg acg gag aag gag cgg gag aag cag gag cag ctc ggc cgc 224
Gly Arg Met Thr Glu Lys Glu Arg Glu Lys Gln Glu Gln Leu Gly Arg
5                   10                  15                  20

gcc aac ggc ggc gca gcc tac cag cgc tcg ccg acg gac aag ccg ccg 272
Ala Asn Gly Gly Ala Ala Tyr Gln Arg Ser Pro Thr Asp Lys Pro Pro
                25                  30                  35

ttc acg ctg ggt cag atc aag aag gca atc ccg cct cac tgc ttc cag 320
Phe Thr Leu Gly Gln Ile Lys Lys Ala Ile Pro Pro His Cys Phe Gln
            40                  45                  50

cgc tcg atc atc aag tcc ttc tcc tac gtg gtc cat gac ctg gtc atc 368
Arg Ser Ile Ile Lys Ser Phe Ser Tyr Val Val His Asp Leu Val Ile
        55                  60                  65

gtc gcg gcc ctg ctg tac gcg gcg ctg gtt tgg atc cct acc ctc ccg 416
Val Ala Ala Leu Leu Tyr Ala Ala Leu Val Trp Ile Pro Thr Leu Pro
    70                  75                  80

acc gtg ctg cag ctg ggc gcc tgg ccg ctc tac tgg atc gtg cag ggc 464
Thr Val Leu Gln Leu Gly Ala Trp Pro Leu Tyr Trp Ile Val Gln Gly
85                  90                  95                  100

tgc gtc atg acc ggc gtc tgg gtc atc gcc cac gag tgc ggc cac cac 512
Cys Val Met Thr Gly Val Trp Val Ile Ala His Glu Cys Gly His His
                105                 110                 115

gcc ttc tcc gac tac tcg ctg ctc gac gac acc gtc ggc ctg gtg ctc 560
Ala Phe Ser Asp Tyr Ser Leu Leu Asp Asp Thr Val Gly Leu Val Leu
            120                 125                 130

cac tcg t[?]g ctg ctc gtc ccc tac ttc tcg tgg aag tac agc cac cgt 608
His Ser Trp Leu Leu Val Pro Tyr Phe Ser Trp Lys Tyr Ser His Arg
        135                 140                 145

cgc cac cac tcc aac acc ggg tcg ctg gag cgt gat gag gtg ttc gtc 656
Arg His His Ser Asn Thr Gly Ser Leu Glu Arg Asp Glu Val Phe Val
    150                 155                 160

ccc aag cag aag gag gcg ctg gcg tgg tac acc cct tac atc tac aac     704
Pro Lys Gln Lys Glu Ala Leu Ala Trp Tyr Thr Pro Tyr Ile Tyr Asn
165                 170                 175                 180

aac ccc gtc ggc cgt ctg gtg cac atc gtc gtg cag ctc acc ctc ggg     752
Asn Pro Val Gly Arg Leu Val His Ile Val Val Gln Leu Thr Leu Gly
                185                 190                 195

tgg ccg ctg tac ctg gcg ctc aac gcc tca ggc cgc ccg tac ccg cgg     800
Trp Pro Leu Tyr Leu Ala Leu Asn Ala Ser Gly Arg Pro Tyr Pro Arg
            200                 205                 210

ttc gcc tgc cac ttc gac ccc tac ggc ccg atc tac aac gac cgg gag     848
Phe Ala Cys His Phe Asp Pro Tyr Gly Pro Ile Tyr Asn Asp Arg Glu
        215                 220                 225

cga gcc cag att ttc atc tca gac gtc gga gtg ctg gcc gtg tca ttg     896
Arg Ala Gln Ile Phe Ile Ser Asp Val Gly Val Leu Ala Val Ser Leu
    230                 235                 240

gct ctg ctg aag ctc gtg tcg tcg ttc ggg ttc tgg tgg gtg gtg cgg     944
Ala Leu Leu Lys Leu Val Ser Ser Phe Gly Phe Trp Trp Val Val Arg
245                 250                 255                 260

gtc tac ggc gtg ccg ctg ctg atc gtg aac gct tgg ctg gtc ctg atc     992
Val Tyr Gly Val Pro Leu Leu Ile Val Asn Ala Trp Leu Val Leu Ile
                265                 270                 275

acc tac ctg cag cac acc cac ccg gcg ctg ccg cac tac gac tcg acg     1040
Thr Tyr Leu Gln His Thr His Pro Ala Leu Pro His Tyr Asp Ser Thr
            280                 285                 290

gag tgg gac tgg ctg cgc ggg gcg ctc gcc acc atg gac cgc gac tac     1088
Glu Trp Asp Trp Leu Arg Gly Ala Leu Ala Thr Met Asp Arg Asp Tyr
        295                 300                 305

ggc atc ctc aac cgc gtg ttc cac aac atc acg gac acg cac gtg gcg     1136
Gly Ile Leu Asn Arg Val Phe His Asn Ile Thr Asp Thr His Val Ala
    310                 315                 320

cac cac ctc ttc tcc acc atg ccg cac tac cac gcc atg gag gcc acc     1184
His His Leu Phe Ser Thr Met Pro His Tyr His Ala Met Glu Ala Thr
325                 330                 335                 340

aag gcg atc aag ccc atc ctc ggc gag tac tac cag ttc gac ccc acc     1232
Lys Ala Ile Lys Pro Ile Leu Gly Glu Tyr Tyr Gln Phe Asp Pro Thr
                345                 350                 355

ccc gtc gcc aag gcc aca tgg cgc gag gcc aag gag tgc atc tac gtc     1280
Pro Val Ala Lys Ala Thr Trp Arg Glu Ala Lys Glu Cys Ile Tyr Val
            360                 365                 370

gag ccc gag gac cgc aag ggg gtc ttc tgg tac agc aac aag ttc         1325
Glu Pro Glu Asp Arg Lys Gly Val Phe Trp Tyr Ser Asn Lys Phe
        375                 380                 385

tagccgccaa gatccatcaa ctgtgctgga gaaagaactc agagaagaga tcctaccaag     1385

taattccatc catctaccta cagtccatat ggttagtctt tagatagcag agggcatttg     1445

ggcacaaaag aagactacta ttaccgtgcc aatgctagaa gagctgagtg gtgcaaggaa     1505

gagtagcgtg tccgtgactt tggtcagttc cgtctttact ttttctctgc gttctagtcg 1565

tcggcttagg tttggccggc ggtcatcgtt ggtgtccgtg gccgtggaca tggccgcgtg 1625

tgttgtgtgt gcgtctgtca ttgcattggc gtcatctccc cccgtccgtg tcatgttgtt 1685

gtagaccatt tcgtgtttat ggcggaataa ctgatcgtcg aaggaagggc aacttttttg 1745

agta 1749

<210> 34
<211> 387
<212> PRT
<213> Triticum aestivum

<400> 34

Met Gly Ala Gly Gly Arg Met Thr Glu Lys Glu Arg Glu Lys Gln Glu
1               5                   10                  15

Gln Leu Gly Arg Ala Asn Gly Gly Ala Ala Tyr Gln Arg Ser Pro Thr
            20                  25                  30

Asp Lys Pro Pro Phe Thr Leu Gly Gln Ile Lys Lys Ala Ile Pro Pro
        35                  40                  45

His Cys Phe Gln Arg Ser Ile Ile Lys Ser Phe Ser Tyr Val Val His
    50                  55                  60

Asp Leu Val Ile Val Ala Ala Leu Leu Tyr Ala Ala Leu Val Trp Ile
65                  70                  75                  80

Pro Thr Leu Pro Thr Val Leu Gln Leu Gly Ala Trp Pro Leu Tyr Trp
                85                  90                  95

Ile Val Gln Gly Cys Val Met Thr Gly Val Trp Val Ile Ala His Glu
            100                 105                 110

Cys Gly His His Ala Phe Ser Asp Tyr Ser Leu Leu Asp Asp Thr Val
        115                 120                 125

Gly Leu Val Leu His Ser Trp Leu Leu Val Pro Tyr Phe Ser Trp Lys
    130                 135                 140

Tyr Ser His Arg Arg His His Ser Asn Thr Gly Ser Leu Glu Arg Asp
145                 150                 155                 160

Glu Val Phe Val Pro Lys Gln Lys Glu Ala Leu Ala Trp Tyr Thr Pro
                165                 170                 175

Tyr Ile Tyr Asn Asn Pro Val Gly Arg Leu Val His Ile Val Val Gln
            180                 185                 190

Leu Thr Leu Gly Trp Pro Leu Tyr Leu Ala Leu Asn Ala Ser Gly Arg
        195                 200                 205

Pro Tyr Pro Arg Phe Ala Cys His Phe Asp Pro Tyr Gly Pro Ile Tyr
    210                 215                 220

Asn Asp Arg Glu Arg Ala Gln Ile Phe Ile Ser Asp Val Gly Val Leu
225                 230                 235                 240

Ala Val Ser Leu Ala Leu Leu Lys Leu Val Ser Ser Phe Gly Phe Trp
                245                 250                 255

Trp Val Val Arg Val Tyr Gly Val Pro Leu Leu Ile Val Asn Ala Trp
            260                 265                 270

Leu Val Leu Ile Thr Tyr Leu Gln His Thr His Pro Ala Leu Pro His
        275                 280                 285

Tyr Asp Ser Thr Glu Trp Asp Trp Leu Arg Gly Ala Leu Ala Thr Met
    290                 295                 300

Asp Arg Asp Tyr Gly Ile Leu Asn Arg Val Phe His Asn Ile Thr Asp
305                 310                 315                 320

Thr His Val Ala His His Leu Phe Ser Thr Met Pro His Tyr His Ala
                325                 330                 335

Met Glu Ala Thr Lys Ala Ile Lys Pro Ile Leu Gly Glu Tyr Tyr Gln
            340                 345                 350

Phe Asp Pro Thr Pro Val Ala Lys Ala Thr Trp Arg Glu Ala Lys Glu
        355                 360                 365

Cys Ile Tyr Val Glu Pro Glu Asp Arg Lys Gly Val Phe Trp Tyr Ser
    370                 375                 380

Asn Lys Phe
385

<210>  35
<211>  1161
<212>  DNA
<213>  Triticum aestivum

<220>
<221> CDS
<222> (1)..(1161)

<400> 35
atg ggt gcc ggc ggc agg atg acg gag aag gag cgg gag aag cag gag 48
Met Gly Ala Gly Gly Arg Met Thr Glu Lys Glu Arg Glu Lys Gln Glu
1               5                   10                  15

cag ctc ggc cgc gcc aac ggc ggc gca gcc tac cag cgc tcg ccg acg 96
Gln Leu Gly Arg Ala Asn Gly Gly Ala Ala Tyr Gln Arg Ser Pro Thr
            20                  25                  30

gac aag ccg ccg ttc acg ctg ggt cag atc aag aag gca atc ccg cct 144
Asp Lys Pro Pro Phe Thr Leu Gly Gln Ile Lys Lys Ala Ile Pro Pro
        35                  40                  45

cac tgc ttc cag cgc tcg atc atc aag tcc ttc tcc tac gtg gtc cat 192
His Cys Phe Gln Arg Ser Ile Ile Lys Ser Phe Ser Tyr Val Val His
    50                  55                  60

gac ctg gtc atc gtc gcg gcc ctg ctg tac gcg gcg ctg gtt tgg atc 240
Asp Leu Val Ile Val Ala Ala Leu Leu Tyr Ala Ala Leu Val Trp Ile
65                  70                  75                  80

cct acc ctc ccg acc gtg ctg cag ctg ggc gcc tgg ccg ctc tac tgg 288
Pro Thr Leu Pro Thr Val Leu Gln Leu Gly Ala Trp Pro Leu Tyr Trp
                85                  90                  95

atc gtg cag ggc tgc gtc atg acc ggc gtc tgg gtc atc gcc cac gag 336
Ile Val Gln Gly Cys Val Met Thr Gly Val Trp Val Ile Ala His Glu
            100                 105                 110

tgc ggc cac cac gcc ttc tcc gac tac tcg ctg ctc gac gac acc gtc 384
Cys Gly His His Ala Phe Ser Asp Tyr Ser Leu Leu Asp Asp Thr Val
        115                 120                 125

ggc ctg gtg ctc cac tcg tgg ctg ctc gtc ccc tac ttc tcg tgg aag 432
Gly Leu Val Leu His Ser Trp Leu Leu Val Pro Tyr Phe Ser Trp Lys
    130                 135                 140

tac agc cac cgt cgc cac cac tcc aac acc ggg tcg ctg gag cgt gat 480
Tyr Ser His Arg Arg His His Ser Asn Thr Gly Ser Leu Glu Arg Asp
145                 150                 155                 160

gag gtg ttc gtc ccc aag cag aag gag gcg ctg gcg tgg tac acc cct 528
Glu Val Phe Val Pro Lys Gln Lys Glu Ala Leu Ala Trp Tyr Thr Pro
                165                 170                 175

tac atc tac aac aac ccc gtc ggc cgt ctg gtg cac atc gtc gtg cag 576
Tyr Ile Tyr Asn Asn Pro Val Gly Arg Leu Val His Ile Val Val Gln
            180                 185                 190

ctc acc ctc ggg tgg ccg ctg tac ctg gcg ctc aac gcc tca ggc cgc 624
Leu Thr Leu Gly Trp Pro Leu Tyr Leu Ala Leu Asn Ala Ser Gly Arg
        195                 200                 205

ccg tac ccg cgg ttc gcc tgc cac ttc gac ccc tac ggc ccg atc tac 672
Pro Tyr Pro Arg Phe Ala Cys His Phe Asp Pro Tyr Gly Pro Ile Tyr
    210                 215                 220

aac gac cgg gag cga gcc cag att ttc atc tca gac gtc gga gtg ctg 720
Asn Asp Arg Glu Arg Ala Gln Ile Phe Ile Ser Asp Val Gly Val Leu
225                 230                 235                 240

gcc gtg tca ttg gct ctg ctg aag ctc gtg tcg tcg ttc ggg ttc tgg 768
Ala Val Ser Leu Ala Leu Leu Lys Leu Val Ser Ser Phe Gly Phe Trp
                245                 250                 255

tgg gtg gtg cgg gtc tac ggc gtg ccg ctg ctg atc gtg aac gct tgg 816
Trp Val Val Arg Val Tyr Gly Val Pro Leu Leu Ile Val Asn Ala Trp
            260                 265                 270

ctg gtc ctg atc acc tac ctg cag cac acc cac ccg gcg ctg ccg cac 864
Leu Val Leu Ile Thr Tyr Leu Gln His Thr His Pro Ala Leu Pro His
        275                 280                 285

tac gac tcg acg gag tgg gac tgg ctg cgc ggg gcg ctc gcc acc atg 912
Tyr Asp Ser Thr Glu Trp Asp Trp Leu Arg Gly Ala Leu Ala Thr Met
    290                 295                 300

gac cgc gac tac ggc atc ctc aac cgc gtg ttc cac aac atc acg gac 960
Asp Arg Asp Tyr Gly Ile Leu Asn Arg Val Phe His Asn Ile Thr Asp
305                 310                 315                 320

acg cac gtg gcg cac cac ctc ttc tcc acc atg ccg cac tac cac gcc 1008
Thr His Val Ala His His Leu Phe Ser Thr Met Pro His Tyr His Ala
                325                 330                 335

atg gag gcc acc aag gcg atc aag ccc atc ctc ggc gag tac tac cag 1056
Met Glu Ala Thr Lys Ala Ile Lys Pro Ile Leu Gly Glu Tyr Tyr Gln
            340                 345                 350

ttc gac ccc acc ccc gtc gcc aag gcc aca tgg cgc gag gcc aag gag 1104
Phe Asp Pro Thr Pro Val Ala Lys Ala Thr Trp Arg Glu Ala Lys Glu
        355                 360                 365

tgc atc tac gtc gag ccc gag gac cgc aag ggg gtc ttc tgg tac agc 1152
Cys Ile Tyr Val Glu Pro Glu Asp Arg Lys Gly Val Phe Trp Tyr Ser
    370                 375                 380

aac aag ttc 1161
Asn Lys Phe
385

<210> 36
<211> 387
<212> PRT
<213> Triticum aestivum

<400> 36

Met Gly Ala Gly Gly Arg Met Thr Glu Lys Glu Arg Glu Lys Gln Glu
1               5                   10                  15

Gln Leu Gly Arg Ala Asn Gly Gly Ala Ala Tyr Gln Arg Ser Pro Thr
            20                  25                  30

Asp Lys Pro Pro Phe Thr Leu Gly Gln Ile Lys Lys Ala Ile Pro Pro
        35                  40                  45

His Cys Phe Gln Arg Ser Ile Ile Lys Ser Phe Ser Tyr Val Val His
    50                  55                  60

Asp Leu Val Ile Val Ala Ala Leu Leu Tyr Ala Ala Leu Val Trp Ile
65                  70                  75                  80

Pro Thr Leu Pro Thr Val Leu Gln Leu Gly Ala Trp Pro Leu Tyr Trp
                85                  90                  95

Ile Val Gln Gly Cys Val Met Thr Gly Val Trp Val Ile Ala His Glu
            100                 105                 110

Cys Gly His His Ala Phe Ser Asp Tyr Ser Leu Leu Asp Asp Thr Val
        115                 120                 125

Gly Leu Val Leu His Ser Trp Leu Leu Val Pro Tyr Phe Ser Trp Lys
    130                 135                 140

Tyr Ser His Arg Arg His His Ser Asn Thr Gly Ser Leu Glu Arg Asp
145                 150                 155                 160

Glu Val Phe Val Pro Lys Gln Lys Glu Ala Leu Ala Trp Tyr Thr Pro
                165                 170                 175

Tyr Ile Tyr Asn Asn Pro Val Gly Arg Leu Val His Ile Val Val Gln
            180                 185                 190

Leu Thr Leu Gly Trp Pro Leu Tyr Leu Ala Leu Asn Ala Ser Gly Arg
        195                 200                 205

Pro Tyr Pro Arg Phe Ala Cys His Phe Asp Pro Tyr Gly Pro Ile Tyr
    210                 215                 220

Asn Asp Arg Glu Arg Ala Gln Ile Phe Ile Ser Asp Val Gly Val Leu
225                 230                 235                 240

Ala Val Ser Leu Ala Leu Leu Lys Leu Val Ser Ser Phe Gly Phe Trp
                245                 250                 255

Trp Val Val Arg Val Tyr Gly Val Pro Leu Leu Ile Val Asn Ala Trp
            260                 265                 270

Leu Val Leu Ile Thr Tyr Leu Gln His Thr His Pro Ala Leu Pro His
        275                 280                 285

Tyr Asp Ser Thr Glu Trp Asp Trp Leu Arg Gly Ala Leu Ala Thr Met
    290                 295                 300

Asp Arg Asp Tyr Gly Ile Leu Asn Arg Val Phe His Asn Ile Thr Asp
305                 310                 315                 320

Thr His Val Ala His His Leu Phe Ser Thr Met Pro His Tyr His Ala
                325                 330                 335

Met Glu Ala Thr Lys Ala Ile Lys Pro Ile Leu Gly Glu Tyr Tyr Gln
            340                 345                 350

Phe Asp Pro Thr Pro Val Ala Lys Ala Thr Trp Arg Glu Ala Lys Glu
        355                 360                 365

Cys Ile Tyr Val Glu Pro Glu Asp Arg Lys Gly Val Phe Trp Tyr Ser
    370                 375                 380

Asn Lys Phe
385

<210>  37
<211>  21
<212>  DNA
<213>  Zea mays

<400>  37
tcggaccagg cttcattccc c                                                21

<210>  38<211>  21
<212>  DNA
<213>  Synthetic

<220>
<221>  misc_feature
<222>  (1)..(21)
<223>  micro RNA

<220>
<221>  misc_feature
<222>  (1)..(21)
<223>  DNA sequence encoding micro RNA

<400>  38
tcggaccagg cttcattccc c                                                21

<210>  39
<211>  21
<212>  DNA
<213>  Artificial Sequence

<220>

<223>  DNA sequence endoding micro RNA

<220>
<221>  misc_feature
<222>  (1)..(21)
<223>  Micro RNA

<220>
<221>  misc_feature
<222>  (1)..(21)
<223>  DNA sequence encoding Micro RNA

<220>
<221>  misc_feature
<222>  (1)..(21)
<223>  DNA sequence ecoding Micro RNA

<400>  39
ttgtagatga agcagccgtc c                                                21

<210>  40
<211>  19
<212>  RNA
<213>  Artificial

<220>
<223>  microRNA

<220>
<221>  misc_feature
<222>  (1)..(19)
<223>  micro RNA

<400>  40
accagacccc gaacgccgc                                                   19

<210>  41
<211>  11722
<212>  DNA
<213>  Artificial

<220>
<223>  A binary vector with maize miR166 precursor

<220>
<221>  misc_feature
<222>  (171)..(195)
<223>  Left T-DNA Border repeat region

<220>
<221>  promoter
<222>  (286)..(2273)
<223>  miaze ubiquitin promoter

<220>
<221>  CDS
<222>  (2316)..(3644)

<223>  E.coli D-serine dehydratase [dsdA] gene

<220>
<221>  terminator
<222>  (3784)..(4394)
<223>  Octapine Synthase 3 terminator

<220>
<221>  promoter
<222>  (4486)..(5906)
<223>  Sugarcane bacilliform virus [ScBV] promoter

<220>
<221>  precursor_RNA
<222>  (5977)..(6669)
<223>  Maize miR166 precursor

<220>
<221>  terminator
<222>  (6732)..(6984)
<223>  3'UTR of Nopaline Synthase from Agrobacterium tumefaciens T-DNA
      used as terminator

<220>
<221>  misc_feature
<222>  (7079)..(7102)
<223>  Right T-DNA Border repeat region

<220>
<221>  promoter
<222>  (7695)..(7793)
<223>  [bla] beta-lactamase promoter

<220>
<221>  rep_origin
<222>  (9460)..(10141)
<223>  ColE1 ecoli origin of replication for propigation.

<220>
<221>  CDS
<222>  (10600)..(11415)
<223>  Kanamycin Resistance selection gene

<400>  41
ttcaaacccg gcagcttagt tgccgttctt ccgaatagca tcggtaacat gagcaaagtc      60

tgccgcctta caacggctct cccgctgacg ccgtcccgga ctgatgggct gcctgtatcg     120

agtggtgatt ttgtgccgag ctgccggtcg gggagctgtt ggctggctgg tggcaggata     180

tattgtggtg taaacaaatt gacgcttaga caacttaata acacattgcg gacgttttta     240

atgtactgaa ttggatccgc ccgggcggta ccaagcttcc gcggctgcag tgcagcgtga     300

cccggtcgtg cccctctcta gagataatga gcattgcatg tctaagttat aaaaaattac     360

cacatatttt ttttgtcaca cttgtttgaa gtgcagttta tctatcttta tacatatatt     420

taaactttac tctacgaata atataatcta tagtactaca ataatatcag tgttttagag     480

aatcatataa atgaacagtt agacatggtc taaaggacaa ttgagtattt tgacaacagg     540

actctacagt tttatctttt tagtgtgcat gtgttctcct ttttttttgc aaatagcttc 600

acctatataa tacttcatcc attttattag tacatccatt tagggtttag ggttaatggt 660

ttttatagac taattttttt agtacatcta ttttattcta ttttagcctc taaattaaga 720

aaactaaaac tctattttag tttttttatt taatagttta gatataaaat agaataaaat 780

aaagtgacta aaaattaaac aaataccctt taagaaatta aaaaaactaa ggaaacattt 840

ttcttgtttc gagtagataa tgccagcctg ttaaacgccg tcgacgagtc taacggacac 900

caaccagcga accagcagcg tcgcgtcggg ccaagcgaag cagacggcac ggcatctctg 960

tcgctgcctc tggacccctc tcgagagttc cgctccaccg ttggacttgc tccgctgtcg 1020

gcatccagaa attgcgtggc ggagcggcag acgtgagccg gcacggcagg cggcctcctc 1080

ctcctctcac ggcaccggca gctacggggg attcctttcc caccgctcct tcgctttccc 1140

ttcctcgccc gccgtaataa atagacaccc cctccacacc ctctttcccc aacctcgtgt 1200

tgttcggagc gcacacacac acaaccagat ctcccccaaa tccacccgtc ggcacctccg 1260

cttcaaggta cgccgctcgt cctccccccc cccccccctc tctaccttct ctagatcggc 1320

gttccggtcc atggttaggg cccggtagtt ctacttctgt tcatgtttgt gttagatccg 1380

tgtttgtgtt agatccgtgc tgctagcgtt cgtacacgga tgcgacctgt acgtcagaca 1440

cgttctgatt gctaacttgc cagtgtttct ctttggggaa tcctgggatg gctctagccg 1500

ttccgcagac gggatcgatt tcatgatttt ttttgtttcg ttgcataggg tttggtttgc 1560

ccttttcctt tatttcaata tatgccgtgc acttgtttgt cgggtcatct tttcatgctt 1620

ttttttgtct tggttgtgat gatgtggtct ggttgggcgg tcgttctaga tcggagtaga 1680

attctgtttc aaactacctg gtggatttat taattttgga tctgtatgtg tgtgccatac 1740

atattcatag ttacgaattg aagatgatgg atggaaatat cgatctagga taggtataca 1800

tgttgatgcg ggttttactg atgcatatac agagatgctt tttgttcgct tggttgtgat 1860

gatgtggtgt ggttgggcgg tcgttcattc gttctagatc ggagtagaat actgtttcaa 1920
actacctggt gtatttatta attttggaac tgtatgtgtg tgtcatacat cttcatagtt 1980

acgagtttaa gatggatgga aatatcgatc taggataggt atacatgttg atgtgggttt 2040

tactgatgca tatacatgat ggcatatgca gcatctattc atatgctcta accttgagta 2100

cctatctatt ataataaaca agtatgtttt ataattattt cgatcttgat atacttggat 2160

gatggcatat gcagcagcta tatgtggatt tttttagccc tgccttcata cgctatttat 2220

ttgcttggta ctgtttcttt tgtcgatgct caccctgttg tttggtgtta cttctgcagg 2280

gtacggatcc tcatctaagc gcaaagagac gtact atg gaa aac gct aaa atg 2333
Met Glu Asn Ala Lys Met
1               5

aac tcg ctc atc gcc cag tat ccg ttg gta aag gat ctg gtt gct ctt 2381

Asn Ser Leu Ile Ala Gln Tyr Pro Leu Val Lys Asp Leu Val Ala Leu
            10                  15                  20

aaa gaa acc acc tgg ttt aat cct ggc acg acc tca ttg gct gaa ggt 2429
Lys Glu Thr Thr Trp Phe Asn Pro Gly Thr Thr Ser Leu Ala Glu Gly
        25                  30                  35

tta cct tat gtt ggc ctg acc gaa cag gat gtt cag gac gcc cat gcg 2477
Leu Pro Tyr Val Gly Leu Thr Glu Gln Asp Val Gln Asp Ala His Ala
    40                  45                  50

cgc tta tcc cgt ttt gca ccc tat ctg gca aaa gca ttt cct gaa act 2525
Arg Leu Ser Arg Phe Ala Pro Tyr Leu Ala Lys Ala Phe Pro Glu Thr
55                  60                  65                  70

gct gcc act ggg ggg att att gaa tca gaa ctg gtt gcc att cca gct 2573
Ala Ala Thr Gly Gly Ile Ile Glu Ser Glu Leu Val Ala Ile Pro Ala
                75                  80                  85

atg caa aaa cgg ctg gaa aaa gaa tat cag caa ccg atc agc ggg caa 2621
Met Gln Lys Arg Leu Glu Lys Glu Tyr Gln Gln Pro Ile Ser Gly Gln
            90                  95                  100

ctg tta ctg aaa aaa gat agc cat ttg ccc att tcc ggc tcc ata aaa 2669
Leu Leu Leu Lys Lys Asp Ser His Leu Pro Ile Ser Gly Ser Ile Lys
        105                 110                 115

gca cgc ggc ggg att tat gaa gtc ctg gca cac gca gaa aaa ctg gct 2717
Ala Arg Gly Gly Ile Tyr Glu Val Leu Ala His Ala Glu Lys Leu Ala
    120                 125                 130

ctg gaa gcg ggg ttg ctg acg ctt gat gat gac tac agc aaa ctg ctt 2765
Leu Glu Ala Gly Leu Leu Thr Leu Asp Asp Asp Tyr Ser Lys Leu Leu
135                 140                 145                 150

tct ccg gag ttt aaa cag ttc ttt agc caa tac agc att gct gtg ggc 2813
Ser Pro Glu Phe Lys Gln Phe Phe Ser Gln Tyr Ser Ile Ala Val Gly
                155                 160                 165

tca acc gga aat ctg ggg tta tca atc ggc att atg agc gcc cgc att 2861
Ser Thr Gly Asn Leu Gly Leu Ser Ile Gly Ile Met Ser Ala Arg Ile
            170                 175                 180

ggc ttt aag gtg aca gtt cat atg tct gct gat gcc cgg gca tgg aaa 2909
Gly Phe Lys Val Thr Val His Met Ser Ala Asp Ala Arg Ala Trp Lys
        185                 190                 195

aaa gcg aaa ctg cgc agc cat ggc gtt acg gtc gtg gaa tat gag caa 2957
Lys Ala Lys Leu Arg Ser His Gly Val Thr Val Val Glu Tyr Glu Gln
    200                 205                 210

gat tat ggt gtt gcc gtc gag gaa gga cgt aaa gca gcg cag tct gac 3005
Asp Tyr Gly Val Ala Val Glu Glu Gly Arg Lys Ala Ala Gln Ser Asp
215                 220                 225                 230

ccg aac tgt ttc ttt att gat gac gaa aat tcc cgc acg ttg ttc ctt 3053
Pro Asn Cys Phe Phe Ile Asp Asp Glu Asn Ser Arg Thr Leu Phe Leu
                235                 240                 245

ggg tat tcc gtc gct ggc cag cgt ctt aaa gcg caa ttt gcc cag caa 3101
Gly Tyr Ser Val Ala Gly Gln Arg Leu Lys Ala Gln Phe Ala Gln Gln
            250                 255                 260

ggc cgt atc gtc gat gct gat aac cct ctg ttt gtc tat ctg ccg tgt 3149
Gly Arg Ile Val Asp Ala Asp Asn Pro Leu Phe Val Tyr Leu Pro Cys
        265                 270                 275

ggt gtt ggc ggt ggt cct ggt ggc gtc gca ttc ggg ctt aaa ctg gcg 3197
Gly Val Gly Gly Gly Pro Gly Gly Val Ala Phe Gly Leu Lys Leu Ala
    280                 285                 290

ttt ggc gat cat gtt cac tgc ttt ttt gcc gaa cca acg cac tcc cct 3245
Phe Gly Asp His Val His Cys Phe Phe Ala Glu Pro Thr His Ser Pro
295                 300                 305                 310

tgt atg ttg tta ggc gtc cat aca gga tta cac gat cag att tct gtt 3293
Cys Met Leu Leu Gly Val His Thr Gly Leu His Asp Gln Ile Ser Val
                315                 320                 325

cag gat att ggt atc gac aac ctt acc gca gcg gat ggc ctt gca gtt 3341
Gln Asp Ile Gly Ile Asp Asn Leu Thr Ala Ala Asp Gly Leu Ala Val
            330                 335                 340

ggt cgc gca tca ggc ttt gtc ggg cgg gca atg gag cgt ctg ctg gat 3389
Gly Arg Ala Ser Gly Phe Val Gly Arg Ala Met Glu Arg Leu Leu Asp
        345                 350                 355

ggc ttc tat acc ctt agc gat caa acc atg tat gac atg ctt ggc tgg 3437
Gly Phe Tyr Thr Leu Ser Asp Gln Thr Met Tyr Asp Met Leu Gly Trp
    360                 365                 370

ctg gcg cag gaa gaa ggt att cgt ctt gaa cct tcg gca ctg gcg ggt 3485
Leu Ala Gln Glu Glu Gly Ile Arg Leu Glu Pro Ser Ala Leu Ala Gly
375                 380                 385                 390

atg gcc gga cct cag cgc gtg tgt gca tca gta agt tac caa cag atg 3533
Met Ala Gly Pro Gln Arg Val Cys Ala Ser Val Ser Tyr Gln Gln Met
                395                 400                 405

cac ggt ttc agc gca gaa caa ctg cgt aat acc act cat ctg gtg tgg 3581
His Gly Phe Ser Ala Glu Gln Leu Arg Asn Thr Thr His Leu Val Trp
            410                 415                 420

gcg acg gga ggt gga atg gtg ccg gaa gaa gag atg aat caa tat ctg 3629
Ala Thr Gly Gly Gly Met Val Pro Glu Glu Glu Met Asn Gln Tyr Leu
        425                 430                 435

gca aaa ggc cgt taa taacgtttca acgcagcatg gatcgtaccg agctcaatcg 3684
Ala Lys Gly Arg
    440

atcctgcttt aatgagatat gcgagacgcc tatgatcgca tgatatttgc tttcaattct 3744

gttgtgcacg ttgtaaaaaa cctgagcatg tgtagctcag atccttaccg ccggtttcgg 3804

ttcattctaa tgaatatatc acccgttact atcgtatttt tatgaataat att[?]tccgtt 3864

caatttactg attgtaccct actacttata tgtacaatat taaaatgaaa acaatatatt 3924

gtgctgaata ggtttatagc gacatctatg atagagcgcc acaataacaa acaa[?]tgcgt 3984

tttattatta caaatccaat tttaaaaaaa gcggcagaac cggtcaaacc taaaagactg 4044

attacataaa tcttattcaa atttcaaaag tgccccaggg gctagtatct acgacacacc 4104

gagcggcgaa ctaataacgc tcactgaagg gaactccggt tccccgccgg cgcgcatggg 4164
tgagattcct tgaagttgag tattggccgt ccgctctacc gaaagttacg ggcaccattc 4224
aacccggtcc agcacggcgg ccgggtaacc gacttgctgc cccgagaatt atgcagcatt 4284
tttttggtgt atgtgggccc caaatgaagt gcaggtcaaa ccttgacagt gacgacaaat 4344
cgttgggcgg gtccagggcg aattttgcga caacatgtcg aggctcagca ggatgggccc 4404
aggtacagaa ttcgcggccg tacaacgcgt accggttaat taaattacgc caagctatca 4464
actttgtata gaaaagttga ggaagttgaa gacaaagaag gtcttaaatc ctggctagca 4524
acactgaact atgccagaaa ccacatcaaa gatatgggca agcttcttgg cccattatat 4584
ccaaagacct cagagaaagg tgagcgaagg ctcaattcag aagattggaa gctgatcaat 4644
aggatcaaga caatggtgag aacgcttcca aatctcacta ttccaccaga agatgcatac 4704
attatcattg aaacagatgc atgtgcaact ggatggggag cagtatgcaa gtggaagaaa 4764
aacaaggcag acccaagaaa tacagagcaa atctgtaggt atgccagtgg aaaatttgat 4824
aagccaaaag gaacctgtga tgcagaaatc tatggggtta tgaatggctt agaaaagatg 4884
agattgttct acttggacaa aagagagatc acagtcagaa ctgacagtag tgcaatcgaa 4944
aggttctaca acaagagtgc tgaacacaag ccttctgaga tcagatggat caggttcatg 5004
gactacatca ctggtgcagg accagagata gtcattgaac acataaaagg gaagagcaat 5064
ggtttagctg acatcttgtc caggctcaaa gccaaattag ctcagaatga accaacggaa 5124
gagatgatcc tgcttacaca agccataagg gaagtaattc cttatccaga tcatccatac 5184
actgagcaac tcagagaatg gggaaacaaa attctggatc cattccccac attcaagaag 5244
gacatgttcg aaagaacaga gcaagctttt atgctaacag aggaaccagt tctactctgt 5304
gcatgcagga agcctgcaat tcagttagtg tccagaacat ctgccaaccc aggaaggaaa 5364
ttcttcaagt gcgcaatgaa caaatgccat tgctggtact gggcagatct cattgaagaa 5424
cacattcaag acagaattga tgaatttctc aagaatcttg aagttctgaa gaccggtggc 5484
gtgcaaacaa tggaggagga acttatgaag gaagtcacca agctgaagat agaagagcag 5544
gagttcgagg aataccaggc cacaccaagg gctatgtcgc cagtagccgc agaagatgtg 5604
ctagatctcc aagacgtaag caatgacgat tgaggaggca ttgacgtcag ggatgaccgc 5664
agcggagagt actgggccca ttcagtggat gctccactga gttgtattat tgtgtgcttt 5724
tcggacaagt gtgctgtcca ctttcttttg gcacctgtgc cactttattc cttgtctgcc 5784
acgatgcctt tgcttagctt gtaagcaagg atcgcagtgc gtgtgtgaca ccacccccct 5844
tccgacgctc tgcctatata aggcaccgtc tgtaagctct tacgatcatc ggtagttcac 5904
cacaagtttg tacaaaaaag caggctccga attcgccctt ggaattctta attaactgca 5964

gatatcggta ccgcagcgtc ggtggttgct tcatggctgt cgaggggaat gacgtccggt 6024

ccgaacaagc cacggctgct gctgcgctac cgccgcggct tcggaccagg cttcattccc 6084

cacgactcac catggaaacg gcagcgaggt gcgagcttcc agtgcgcgca gtgtttgatc 6144

agcaaacacc tgcgtggccg aggctgagcc ttgcaagggc aggcgtccat gttctcgtca 6204

gcaagcgtct tcttcacatg tctgaggtct gagcaattgc gggtggacat cgatggatga 6264

gcattgccgc tgcaagggaa gatcgtccgg ctcacacttg agttgggata ccatgcacta 6324

ctcttgttgt agttagatta gataccatgc gcttgaaccg tgcaatagca tagcgaggaa 6384

gaggaaccca gagaggactc agaaagagat gttgtctatt tcatccagtg ctcatctagt 6444

catctagtct gcctgcgggt gtctgcaatg aacgctgacg ctgctgccgg agacctactc 6504

tgctactggt gtggagtgga caaacaggga gggaaggcgt ctgctagaac ctagaagacg 6564

gacacaggga ggaccaacag gactaaagac cgtcgtacgt gttgaagcgg aaggactttc 6624

ttattacgtg tggcgcttcg aattaaagcc agcggttaga acggcccggg ccggccaagc 6684
ttgggaaggg cgaattcgac ccagctttct tgtacaaagt ggagctcgat cgttcaaaca 6744

tttggcaata aagtttctta agattgaatc ctgttgccgg tcttgcgatg attatcatat 6804

aatttctgtt gaattacgtt aagcatgtaa taattaacat gtaatgcatg acgttattta 6864

tgagatgggt ttttatgatt agagtcccgc aattatacat ttaatacgcg atagaaaaca 6924

aaatatagcg cgcaaactag gataaattat cgcgcgcggt gtcatctatg ttactagatc 6984

ggccggccaa ctttattata catagttgat aattcactgg gccggccaga tcttgattgt 7044

cgtttcccgc cttcagttta aactatcagt gtttgacagg atatattggc gggtaaacct 7104

aagagaaaag agcgtttatt agaataatcg gatatttaaa agggcgtgaa aaggtttatc 7164

cgttcgtcca tttgtatgtg catgccaacc acagggttcc cctcgggagt gcttggcatt 7224

ccgtgcgata atgacttctg ttcaaccacc caaacgtcgg aaagcctgac gacggagcag 7284

cattccaaaa agatcccttg gctcgtctgg gtcggctaga aggtcgagtg ggctgctgtg 7344

gcttgatccc tcaacgcggt cgcggacgta gcgcagcgcc gaaaaatcct cgatcgcaaa 7404

tccgacgctg tcgaaaagcg tgatctgctt gtcgctcttt cggccgacgt cctggccagt 7464

catcacgcgc caaagttccg tcacaggatg atctggcgcg agttgctgga tctcgccttc 7524

aatccgggtc tgtggcggga actccacgaa aatatccgaa cgcagcaaga tcgtcgacca 7584

attcttgaag acgaaagggc ctcgtgatac gcctattttt ataggttaat gtcatgataa 7644

taatggtttc ttagacgtca ggtggcactt ttcggggaaa tgtgcgcgga acccctattt 7704

gtttattttt ctaaatacat tcaaatatgt atccgctcat gagacaataa ccctgataaa 7764

tgcttcaata atattgaaaa aggaagagta tgagtattca acatttccgt gtcgccctta 7824

ttcccttttt tgcggcattt tgccttcctg tttttgctca cccagaaacg ctggtgaaag 7884

taaaagatgc tgaagatcag ttgggtgcac gagtgggtta catcgaactg gatctcaaca 7944

gcggtaagat ccttgagagt tttcgccccg aagaacgttt tccaatgatg agcactttta 8004

aagttctgct atgtggcgcg gtattatccc gtgttgacgc cgggcaagag caactcggtc 8064

gccgcataca ctattctcag aatgacttgg ttgagtactc accagtcaca gaaaagcatc 8124

ttacggatgg catgacagta agagaattat gcagtgctgc cataaccatg agtgataaca 8184

ctgcggccaa cttacttctg acaacgatcg gaggaccgaa ggagctaacc gcttttttgc 8244

acaacatggg ggatcatgta actcgccttg atcgttggga accggagctg aatgaagcca 8304

taccaaacga cgagcgtgac accacgatgc cggggggggg ggggggggac atgaggttgc 8364

cccgtattca gtgtcgctga tttgtattgt ctgaagttgt ttttacgtta agttgatgca 8424

gatcaattaa tacgatacct gcgtcataat tgattatttg acgtggtttg atggcctcca 8484

cgcacgttgt gatatgtaga tgataatcat tatcacttta cgggtccttt ccggtgatcc 8544

gacaggttac ggggcggcga cctcgcgggt tttcgctatt tatgaaaatt ttccggttta 8604

aggcgtttcc gttcttcttc gtcataactt aatgttttta tttaaaatac cctctgaaaa 8664

gaaaggaaac gacaggtgct gaaagcgagc tttttggcct ctgtcgtttc ctttctctgt 8724

ttttgtccgt ggaatgaaca atggaacccc cccccccccc ccctgcagca atggcaacaa 8784

cgttgcgcaa actattaact ggcgaactac ttactctagc ttcccggcaa caattaatag 8844

actggatgga ggcggataaa gttgcaggac cacttctgcg ctcggccctt ccggctggct 8904

ggtttattgc tgataaatct ggagccggtg agcgtgggtc tcgcggtatc attgcagcac 8964

tggggccaga tggtaagccc tcccgtatcg tagttatcta cacgacgggg agtcaggcaa 9024

ctatggatga acgaaataga cagatcgctg agataggtgc ctcactgatt aagcattggt 9084

aactgtcaga ccaagtttac tcatatatac tttagattga tttaaaactt catttttaat 9144

ttaaaaggat ctaggtgaag atcctttttg ataatctcat gaccaaaatc ccttaacgtg 9204

agttttcgtt ccactgagcg tcagaccccg tagaaaagat caaaggatct tcttgagatc 9264

ctttttttct gcgcgtaatc tgctgcttgc aaacaaaaaa accaccgcta ccagcggtgg 9324

tttgtttgcc ggatcaagag ctaccaactc tttttccgaa ggtaactggc ttcagcagag 9384

cgcagatacc aaatactgtc cttctagtgt agccgtagtt aggccaccac ttcaagaact 9444

ctgtagcacc gcctacatac ctcgctctgc taatcctgtt accagtggct gctgccagtg 9504

gcgataagtc gtgtcttacc gggttggact caagacgata gttaccggat aaggcgcagc 9564

ggtcgggctg aacggggggt tcgtgcacac agcccagctt ggagcgaacg acctacaccg 9624

aactgagata cctacagcgt gagctatgag aaagcgccac gcttcccgaa gggagaaagg 9684

cggacaggta tccggtaagc ggcagggtcg gaacaggaga gcgcacgagg gagcttccag 9744

ggggaaacgc ctggtatctt tatagtcctg tcgggtttcg ccacctctga cttgagcgtc 9804

gatttttgtg atgctcgtca ggggggcgga gcctatggaa aaacgccagc aacgcggcct 9864

ttttacggtt cctggccttt tgctggcctt ttgctcacat gttctttcct gcgttatccc 9924

ctgattctgt ggataaccgt attaccgcct ttgagtgagc tgataccgct cgccgcagcc 9984

gaacgaccga gcgcagcgag tcagtgagcg aggaagcgga agagcgcctg atgcggtatt 10044

ttctccttac gcatctgtgc ggtatttcac accgcatatg gtgcactctc agtacaatct 10104

gctctgatgc cgcatagtta agccagtata cactccgcta tcgctacgtg actgggtcat 10164

ggctgcgccc cgacacccgc caacacccgc tgacgcgccc tgacgggctt gtctgctccc 10224

ggcatccgct tacagacaag ctgtgaccgt ctccgggagc tgcatgtgtc agaggttttc 10284

accgtcatca ccgaaacgcg cgaggcagca gatcccccga tcaagtagat acactacata 10344

tatctacaat agacatcgag ccggaaggtg atgtttactt tcctgaaatc cccagcaatt 10404

ttaggccagt ttttacccaa gacttcgcct ctaacataaa ttatagttac caaatctggc 10464

aaaagggttg accggggggg gggggaaagc cacgttgtgt ctcaaaatct ctgatgttac 10524
attgcacaag ataaaaatat atcatcatga acaataaaac tgtctgctta cataaacagt 10584

aatacaaggg gtgtt atg agc cat att caa cgg gaa acg tct tgc tcg agg 10635
Met Ser His Ile Gln Arg Glu Thr Ser Cys Ser Arg
        445                 450

ccg cga tta aat tcc aac atg gat gct gat tta tat ggg tat aaa tgg 10683
Pro Arg Leu Asn Ser Asn Met Asp Ala Asp Leu Tyr Gly Tyr Lys Trp
455                 460                 465                 470

gct cgc gat aat gtc ggg caa tca ggt gcg aca atc tat cga ttg tat 10731
Ala Arg Asp Asn Val Gly Gln Ser Gly Ala Thr Ile Tyr Arg Leu Tyr
                475                 480                 485

ggg aag ccc gat gcg cca gag ttg ttt ctg aaa cat ggc aaa ggt agc 10779
Gly Lys Pro Asp Ala Pro Glu Leu Phe Leu Lys His Gly Lys Gly Ser
            490                 495                 500

gtt gcc aat gat gtt aca gat gag atg gtc aga cta aac tgg ctg acg 10827
Val Ala Asn Asp Val Thr Asp Glu Met Val Arg Leu Asn Trp Leu Thr
        505                 510                 515

gaa ttt atg cct ctt ccg acc atc aag cat ttt atc cgt act cct gat 10875
Glu Phe Met Pro Leu Pro Thr Ile Lys His Phe Ile Arg Thr Pro Asp
    520                 525                 530

gat gca tgg tta ctc acc act gcg atc ccc ggg aaa aca gca ttc cag 10923
Asp Ala Trp Leu Leu Thr Thr Ala Ile Pro Gly Lys Thr Ala Phe Gln
535                 540                 545                 550

gta tta gaa gaa tat cct gat tca ggt gaa aat att gtt gat gcg ctg 10971
Val Leu Glu Glu Tyr Pro Asp Ser Gly Glu Asn Ile Val Asp Ala Leu
                555                 560                 565

gca gtg ttc ctg cgc cgg ttg cat tcg att cct gtt tgt aat tgt cct 11019

Ala Val Phe Leu Arg Arg Leu His Ser Ile Pro Val Cys Asn Cys Pro
            570                 575                 580

ttt aac agc gac cgc gta ttt cgt ctc gct cag gcg caa tca cga atg 11067
Phe Asn Ser Asp Arg Val Phe Arg Leu Ala Gln Ala Gln Ser Arg Met
        585                 590                 595

aat aac ggt ttg gtt gat gcg agt gat ttt gat gac gag cgt aat ggc 11115
Asn Asn Gly Leu Val Asp Ala Ser Asp Phe Asp Asp Glu Arg Asn Gly
    600                 605                 610

tgg cct gtt gaa caa gtc tgg aaa gaa atg cat aag ctt ttg cca ttc 11163
Trp Pro Val Glu Gln Val Trp Lys Glu Met His Lys Leu Leu Pro Phe
615                 620                 625                 630

tca ccg gat tca gtc gtc act cat ggt gat ttc tca ctt gat aac ctt 11211
Ser Pro Asp Ser Val Val Thr His Gly Asp Phe Ser Leu Asp Asn Leu
                635                 640                 645

att ttt gac gag ggg aaa tta ata ggt tgt att gat gtt gga cga gtc 11259
Ile Phe Asp Glu Gly Lys Leu Ile Gly Cys Ile Asp Val Gly Arg Val
            650                 655                 660

gga atc gca gac cga tac cag gat ctt gcc atc cta tgg aac tgc ctc 11307
Gly Ile Ala Asp Arg Tyr Gln Asp Leu Ala Ile Leu Trp Asn Cys Leu
        665                 670                 675

ggt gag ttt tct cct tca tta cag aaa cgg ctt ttt caa aaa tat ggt 11355
Gly Glu Phe Ser Pro Ser Leu Gln Lys Arg Leu Phe Gln Lys Tyr Gly
    680                 685                 690

att gat aat cct gat atg aat aaa ttg cag ttt cat ttg atg ctc gat 11403
Ile Asp Asn Pro Asp Met Asn Lys Leu Gln Phe His Leu Met Leu Asp
695                 700                 705                 710

gag ttt ttc taa tcagaattgg ttaattggtt gtaacactgg cagagcatta 11455
Glu Phe Phe

cgctgacttg acgggacggc ggctttgttg aataaatcga acttttgctg agttgaagga 11515

tcagatcacg catcttcccg acaacgcaga ccgttccgtg gcaaagcaaa agttcaaaat 11575

caccaactgg tccacctaca acaaagctct catcaaccgt ggctccctca ctttctggct 11635

ggatgatggg gcgattcagg gatcacaggc agcaacgctc tgtcatcgtt acaatcaaca 11695

tgctaccctc cgcgagatca tccgtgt 11722

<210> 42
<211> 442
<212> PRT
<213> Artificial

<220>
<223> Synthetic Construct

<400> 42

Met Glu Asn Ala Lys Met Asn Ser Leu Ile Ala Gln Tyr Pro Leu Val
1               5                   10                  15

Lys Asp Leu Val Ala Leu Lys Glu Thr Thr Trp Phe Asn Pro Gly Thr
            20                  25                  30

Thr Ser Leu Ala Glu Gly Leu Pro Tyr Val Gly Leu Thr Glu Gln Asp
        35                  40                  45

Val Gln Asp Ala His Ala Arg Leu Ser Arg Phe Ala Pro Tyr Leu Ala
    50                  55                  60

Lys Ala Phe Pro Glu Thr Ala Ala Thr Gly Gly Ile Ile Glu Ser Glu
65                  70                  75                  80

Leu Val Ala Ile Pro Ala Met Gln Lys Arg Leu Glu Lys Glu Tyr Gln
                85                  90                  95

Gln Pro Ile Ser Gly Gln Leu Leu Leu Lys Lys Asp Ser His Leu Pro
            100                 105                 110

Ile Ser Gly Ser Ile Lys Ala Arg Gly Gly Ile Tyr Glu Val Leu Ala
        115                 120                 125

His Ala Glu Lys Leu Ala Leu Glu Ala Gly Leu Leu Thr Leu Asp Asp
    130                 135                 140

Asp Tyr Ser Lys Leu Leu Ser Pro Glu Phe Lys Gln Phe Phe Ser Gln
145                 150                 155                 160

Tyr Ser Ile Ala Val Gly Ser Thr Gly Asn Leu Gly Leu Ser Ile Gly
                165                 170                 175

Ile Met Ser Ala Arg Ile Gly Phe Lys Val Thr Val His Met Ser Ala
            180                 185                 190

Asp Ala Arg Ala Trp Lys Lys Ala Lys Leu Arg Ser His Gly Val Thr
        195                 200                 205

Val Val Glu Tyr Glu Gln Asp Tyr Gly Val Ala Val Glu Glu Gly Arg
    210                 215                 220

Lys Ala Ala Gln Ser Asp Pro Asn Cys Phe Phe Ile Asp Asp Glu Asn
225                 230                 235                 240

Ser Arg Thr Leu Phe Leu Gly Tyr Ser Val Ala Gly Gln Arg Leu Lys
                245                 250                 255

Ala Gln Phe Ala Gln Gln Gly Arg Ile Val Asp Ala Asp Asn Pro Leu

260                 265                 270

Phe Val Tyr Leu Pro Cys Gly Val Gly Gly Gly Pro Gly Gly Val Ala
        275                 280                 285

Phe Gly Leu Lys Leu Ala Phe Gly Asp His Val His Cys Phe Phe Ala
    290                 295                 300

Glu Pro Thr His Ser Pro Cys Met Leu Leu Gly Val His Thr Gly Leu
305                 310                 315                 320

His Asp Gln Ile Ser Val Gln Asp Ile Gly Ile Asp Asn Leu Thr Ala
                325                 330                 335

Ala Asp Gly Leu Ala Val Gly Arg Ala Ser Gly Phe Val Gly Arg Ala
            340                 345                 350

Met Glu Arg Leu Leu Asp Gly Phe Tyr Thr Leu Ser Asp Gln Thr Met
        355                 360                 365

Tyr Asp Met Leu Gly Trp Leu Ala Gln Glu Glu Gly Ile Arg Leu Glu
    370                 375                 380

Pro Ser Ala Leu Ala Gly Met Ala Gly Pro Gln Arg Val Cys Ala Ser
385                 390                 395                 400

Val Ser Tyr Gln Gln Met His Gly Phe Ser Ala Glu Gln Leu Arg Asn
                405                 410                 415

Thr Thr His Leu Val Trp Ala Thr Gly Gly Gly Met Val Pro Glu Glu
            420                 425                 430

Glu Met Asn Gln Tyr Leu Ala Lys Gly Arg
        435                 440

<210>  43
<211>  271
<212>  PRT
<213>  Artificial

<220>
<223>  Synthetic Construct

<400>  43

Met Ser His Ile Gln Arg Glu Thr Ser Cys Ser Arg Pro Arg Leu Asn
1               5                   10                  15

Ser Asn Met Asp Ala Asp Leu Tyr Gly Tyr Lys Trp Ala Arg Asp Asn

            20                  25                  30

Val Gly Gln Ser Gly Ala Thr Ile Tyr Arg Leu Tyr Gly Lys Pro Asp
        35                  40                  45

Ala Pro Glu Leu Phe Leu Lys His Gly Lys Gly Ser Val Ala Asn Asp
    50                  55                  60

Val Thr Asp Glu Met Val Arg Leu Asn Trp Leu Thr Glu Phe Met Pro
65                  70                  75                  80

Leu Pro Thr Ile Lys His Phe Ile Arg Thr Pro Asp Asp Ala Trp Leu
                85                  90                  95

Leu Thr Thr Ala Ile Pro Gly Lys Thr Ala Phe Gln Val Leu Glu Glu
            100                 105                 110

Tyr Pro Asp Ser Gly Glu Asn Ile Val Asp Ala Leu Ala Val Phe Leu
        115                 120                 125

Arg Arg Leu His Ser Ile Pro Val Cys.Asn Cys Pro Phe Asn Ser Asp
130 135 140

Arg Val Phe Arg Leu Ala Gln Ala Gln Ser Arg Met Asn Asn Gly Leu
145 150 155 160

Val Asp Ala Ser Asp Phe Asp Asp Glu Arg Asn Gly Trp Pro Val Glu
165 170 175

Gln Val Trp Lys Glu Met His Lys Leu Leu Pro Phe Ser Pro Asp Ser
180 185 190

Val Val Thr His Gly Asp Phe Ser Leu Asp Asn Leu Ile Phe Asp Glu
195 200 205

Gly Lys Leu Ile Gly Cys Ile Asp Val Gly Arg Val Gly Ile Ala Asp
210 215 220

Arg Tyr Gln Asp Leu Ala Ile Leu Trp Asn Cys Leu Gly Glu Phe Ser
225 230 235 240

Pro Ser Leu Gln Lys Arg Leu Phe Gln Lys Tyr Gly Ile Asp Asn Pro
245 250 255

Asp Met Asn Lys Leu Gln Phe His Leu Met Leu Asp Glu Phe Phe
260 265 270

<210>  44
<211>  11722
<212>  DNA
<213>  Artificial

<220>
<223>  A binary vector with maize miR166 precursor


<220>
<221>  promoter
<222>  (325)..(2312)
<223>  miaze ubiquitin promoter

<220>
<221>  CDS
<222>  (2355)..(3683)
<223>  E.coli D-serine dehydratase [dsdA] gene

<220>
<221>  terminator
<222>  (3721)..(4433)
<223>  Octapine Synthase 3 terminator

<220>
<221>  promoter
<222>  (3823)..(4433)
<223>  Sugarcane bacilliform virus [ScBV] promoter

<220>
<221>  precursor_RNA
<222>  (6016)..(6708)
<223>  Engineered maize miR166 precursor in which 21-nt miR166 sequence
       has been replaced by 21-nt of dsRed targeting dsRed mRNA

<220>
<221>  terminator
<222>  (6771)..(7023)
<223>  3'UTR of Nopaline Synthase from Agrobacterium tumefaciens T-DNA
       used as terminator

<220>
<221>  misc_feature
<222>  (6771)..(7023)
<223>  Right T-DNA Border repeat region

<220>
<221>  promoter
<222>  (7118)..(7141)
<223>  [bla] beta-lactamase promoter
<220>
<221>  rep_origin
<222>  (9500)..(9700)
<223>  ColE1 ecoli origin of replication for propigation.

<220>
<221>  CDS
<222>  (10639)..(11454)
<223>  Kanamycin Resistance selection gene

<220>

<221>  misc_feature
<222>  (10639)..(11454)
<223>  Left T-DNA Border repeat region

<400>  44
   ttacaatcaa catgctaccc tccgcgagat catccgtgtt tcaaacccgg cagcttagtt      60
   gccgttcttc cgaatagcat cggtaacatg agcaaagtct gccgccttac aacggctctc     120
   ccgctgacgc cgtcccggac tgatgggctg cctgtatcga gtggtgattt tgtgccgagc     180
   tgccggtcgg ggagctgttg gctggctggt ggcaggatat attgtggtgt aaacaaattg     240
   acgcttagac aacttaataa cacattgcgg acgtttttaa tgtactgaat tggatccgcc     300
   cgggcggtac caagcttccg cggctgcagt gcagcgtgac ccggtcgtgc ccctctctag     360
   agataatgag cattgcatgt ctaagttata aaaaattacc acatattttt tttgtcacac     420
   ttgtttgaag tgcagtttat ctatctttat acatatattt aaactttact ctacgaataa     480
   tataatctat agtactacaa taatatcagt gttttagaga atcatataaa tgaacagtta     540
   gacatggtct aaaggacaat tgagtatttt gacaacagga ctctacagtt ttatcttttt     600
   agtgtgcatg tgttctcctt tttttttgca aatagcttca cctatataat acttcatcca     660
   ttttattagt acatccattt agggtttagg gttaatggtt tttatagact aattttttta     720
   gtacatctat tttattctat tttagcctct aaattaagaa aactaaaact ctattttagt     780
   ttttttattt aatagtttag atataaaata gaataaaata aagtgactaa aaattaaaca     840
   aatacccttt aagaaattaa aaaaactaag gaaacatttt tcttgtttcg agtagataat     900
   gccagcctgt taaacgccgt cgacgagtct aacggacacc aaccagcgaa ccagcagcgt     960
   cgcgtcgggc caagcgaagc agacggcacg gcatctctgt cgctgcctct ggacccctct    1020
   cgagagttcc gctccaccgt tggacttgct ccgctgtcgg catccagaaa ttgcgtggcg    1080
   gagcggcaga cgtgagccgg cacggcaggc ggcctcctcc tcctctcacg gcaccggcag    1140
   ctacggggga ttcctttccc accgctcctt cgctttccct tcctcgcccg ccgtaataaa    1200
   tagacacccc ctccacaccc tctttcccca acctcgtgtt gttcggagcg cacacacaca    1260
   caaccagatc tcccccaaat ccacccgtcg gcacctccgc ttcaaggtac gccgctcgtc    1320
   ctcccccccc ccccccctct ctaccttctc tagatcggcg ttccggtcca tggttagggc    1380
   ccggtagttc tacttctgtt catgtttgtg ttagatccgt gtttgtgtta gatccgtgct    1440
   gctagcgttc gtacacggat gcgacctgta cgtcagacac gttctgattg ctaacttgcc    1500
   agtgtttctc tttggggaat cctgggatgg ctctagccgt tccgcagacg ggatcgattt    1560
   catgattttt tttgtttcgt tgcatagggt ttggtttgcc cttttccttt atttcaatat    1620
   atgccgtgca cttgtttgtc gggtcatctt ttcatgcttt tttttgtctt ggttgtgatg    1680
   atgtggtctg gttgggcggt cgttctagat cggagtagaa ttctgtttca aactacctgg    1740
   tggatttatt aattttggat ctgtatgtgt gtgccataca tattcatagt tacgaattga 1800
   agatgatgga tggaaatatc gatctaggat aggtatacat gttgatgcgg gttttactga 1860
   tgcatataca gagatgcttt ttgttcgctt ggttgtgatg atgtggtgtg gttgggcggt 1920
   cgttcattcg ttctagatcg gagtagaata ctgtttcaaa ctacctggtg tatttattaa 1980
   ttttggaact gtatgtgtgt gtcatacatc ttcatagtta cgagtttaag atggatggaa 2040
   atatcgatct aggataggta tacatgttga tgtgggtttt actgatgcat atacatgatg 2100
   gcatatgcag catctattca tatgctctaa ccttgagtac ctatctatta taataaacaa 2160
   gtatgtttta taattatttc gatcttgata tacttggatg atggcatatg cagcagctat 2220
   atgtggattt ttttagccct gccttcatac gctatttatt tgcttggtac tgtttctttt 2280
   gtcgatgctc accctgttgt ttggtgttac ttctgcaggg tacggatcct catctaagcg 2340
   caaagagacg tact atg gaa aac gct aaa atg aac tcg ctc atc gcc cag 2390
   Met Glu Asn Ala Lys Met Asn Ser Leu Ile Ala Gln
   1 5 10
   tat ccg ttg gta aag gat ctg gtt gct ctt aaa gaa acc acc tgg ttt 2438
   Tyr Pro Leu Val Lys Asp Leu Val Ala Leu Lys Glu Thr Thr Trp Phe
   15 20 25
   aat cct ggc acg acc tca ttg gct gaa ggt tta cct tat gtt ggc ctg 2486
   Asn Pro Gly Thr Thr Ser Leu Ala Glu Gly Leu Pro Tyr Val Gly Leu
   30 35 40
   acc gaa cag gat gtt cag gac gcc cat gcg cgc tta tcc cgt ttt gca 2534
   Thr Glu Gln Asp Val Gln Asp Ala His Ala Arg Leu Ser Arg Phe Ala
   45 50 55 60
   ccc tat ctg gca aaa gca ttt cct gaa act gct gcc act ggg ggg att 2582
   Pro Tyr Leu Ala Lys Ala Phe Pro Glu Thr Ala Ala Thr Gly Gly Ile
   65 70 75
   att gaa tca gaa ctg gtt gcc att cca gct atg caa aaa cgg ctg gaa 2630
   Ile Glu Ser Glu Leu Val Ala Ile Pro Ala Met Gln Lys Arg Leu Glu
   80 85 90
   aaa gaa tat cag caa ccg atc agc ggg caa ctg tta ctg aaa aaa gat 2678
   Lys Glu Tyr Gln Gln Pro Ile Ser Gly Gln Leu Leu Leu Lys Lys Asp
   95 100 105
   agc cat ttg ccc att tcc ggc tcc ata aaa gca cgc ggc ggg att tat 2726
   Ser His Leu Pro Ile Ser Gly Ser Ile Lys Ala Arg Gly Gly Ile Tyr
   110 115 120
   gaa gtc ctg gca cac gca gaa aaa ctg gct ctg gaa gcg ggg ttg ctg 2774
   Glu Val Leu Ala His Ala Glu Lys Leu Ala Leu Glu Ala Gly Leu Leu
   125 130 135 140
   acg ctt gat gat gac tac agc aaa ctg ctt tct ccg gag ttt aaa cag 2822
   Thr Leu Asp Asp Asp Tyr Ser Lys Leu Leu Ser Pro Glu Phe Lys Gln
   145 150 155
   ttc ttt agc caa tac agc att gct gtg ggc tca acc gga aat ctg ggg 2870
   Phe Phe Ser Gln Tyr Ser Ile Ala Val Gly Ser Thr Gly Asn Leu Gly
           160                 165                 170
   tta tca atc ggc att atg agc gcc cgc att ggc ttt aag gtg aca gtt     2918
   Leu Ser Ile Gly Ile Met Ser Ala Arg Ile Gly Phe Lys Val Thr Val
           175                 180                 185
   cat atg tct gct gat gcc cgg gca tgg aaa aaa gcg aaa ctg cgc agc     2966
   His Met Ser Ala Asp Ala Arg Ala Trp Lys Lys Ala Lys Leu Arg Ser
       190                 195                 200
   cat ggc gtt acg gtc gtg gaa tat gag caa gat tat ggt gtt gcc gtc     3014
   His Gly Val Thr Val Val Glu Tyr Glu Gln Asp Tyr Gly Val Ala Val
   205                 210                 215                 220
   gag gaa gga cgt aaa gca gcg cag tct gac ccg aac tgt ttc ttt att     3062
   Glu Glu Gly Arg Lys Ala Ala Gln Ser Asp Pro Asn Cys Phe Phe Ile
                   225                 230                 235
   gat gac gaa aat tcc cgc acg ttg ttc ctt ggg tat tcc gtc gct ggc     3110
   Asp Asp Glu Asn Ser Arg Thr Leu Phe Leu Gly Tyr Ser Val Ala Gly
               240                 245                 250
   cag cgt ctt aaa gcg caa ttt gcc cag caa ggc cgt atc gtc gat gct     3158
   Gln Arg Leu Lys Ala Gln Phe Ala Gln Gln Gly Arg Ile Val Asp Ala
           255                 260                 265
   gat aac cct ctg ttt gtc tat ctg ccg tgt ggt gtt ggc ggt ggt cct     3206
   Asp Asn Pro Leu Phe Val Tyr Leu Pro Cys Gly Val Gly Gly Gly Pro
       270                 275                 280
   ggt ggc gtc gca ttc ggg ctt aaa ctg gcg ttt ggc gat cat gtt cac     3254
   Gly Gly Val Ala Phe Gly Leu Lys Leu Ala Phe Gly Asp His Val His
   285                 290                 295                 300
   tgc ttt ttt gcc gaa cca acg cac tcc cct tgt atg ttg tta ggc gtc     3302
   Cys Phe Phe Ala Glu Pro Thr His Ser Pro Cys Met Leu Leu Gly Val
                   305                 310                 315
   cat aca gga tta cac gat cag att tct gtt cag gat att ggt atc gac     3350
   His Thr Gly Leu His Asp Gln Ile Ser Val Gln Asp Ile Gly Ile Asp
               320                 325                 330
   aac ctt acc gca gcg gat ggc ctt gca gtt ggt cgc gca tca ggc ttt     3398
   Asn Leu Thr Ala Ala Asp Gly Leu Ala Val Gly Arg Ala Ser Gly Phe
           335                 340                 345
   gtc ggg cgg gca atg gag cgt ctg ctg gat ggc ttc tat acc ctt agc     3446
   Val Gly Arg Ala Met Glu Arg Leu Leu Asp Gly Phe Tyr Thr Leu Ser
       350                 355                 360
   gat caa acc atg tat gac atg ctt ggc tgg ctg gcg cag gaa gaa ggt     3494
   Asp Gln Thr Met Tyr Asp Met Leu Gly Trp Leu Ala Gln Glu Glu Gly
   365                 370                 375                 380
   att cgt ctt gaa cct tcg gca ctg gcg ggt atg gcc gga cct cag cgc     3542
   Ile Arg Leu Glu Pro Ser Ala Leu Ala Gly Met Ala Gly Pro Gln Arg
                   385                 390                 395
   gtg tgt gca tca gta agt tac caa cag atg cac ggt ttc agc gca gaa     3590
   Val Cys Ala Ser Val Ser Tyr Gln Gln Met His Gly Phe Ser Ala Glu
               400                 405                 410
   caa ctg cgt aat acc act cat ctg gtg tgg gcg acg gga ggt gga atg 3638
   Gln Leu Arg Asn Thr Thr His Leu Val Trp Ala Thr Gly Gly Gly Met
   415 420 425
   gtg ccg gaa gaa gag atg aat caa tat ctg gca aaa ggc cgt taa 3683
   Val Pro Glu Glu Glu Met Asn Gln Tyr Leu Ala Lys Gly Arg
   430 435 440
   taacgtttca acgcagcatg gatcgtaccg agctcaatcg atcctgcttt aatgagatat 3743
   gcgagacgcc tatgatcgca tgatatttgc tttcaattct gttgtgcacg ttgtaaaaaa 3803
   cctgagcatg tgtagctcag atccttaccg ccggtttcgg ttcattctaa tgaatatatc 3863
   acccgttact atcgtatttt tatgaataat attctccgtt caatttactg attgtaccct 3923
   actacttata tgtacaatat taaaatgaaa acaatatatt gtgctgaata ggtttatagc 3983
   gacatctatg atagagcgcc acaataacaa acaattgcgt tttattatta caaatccaat 4043
   tttaaaaaaa gcggcagaac cggtcaaacc taaaagactg attacataaa tcttattcaa 4103
   atttcaaaag tgccccaggg gctagtatct acgacacacc gagcggcgaa ctaataacgc 4163
   tcactgaagg gaactccggt tccccgccgg cgcgcatggg tgagattcct tgaagttgag 4223
   tattggccgt ccgctctacc gaaagttacg ggcaccattc aacccggtcc agcacggcgg 4283
   ccgggtaacc gacttgctgc cccgagaatt atgcagcatt tttttggtgt atgtgggccc 4343
   caaatgaagt gcaggtcaaa ccttgacagt gacgacaaat cgttgggcgg gtccagggcg 4403
   aattttgcga caacatgtcg aggctcagca ggatgggccc aggtacagaa ttcgcggccg 4463
   tacaacgcgt accggttaat taaattacgc caagctatca actttgtata gaaaagttga 4523
   ggaagttgaa gacaaagaag gtcttaaatc ctggctagca acactgaact atgccagaaa 4583
   ccacatcaaa gatatgggca agcttcttgg cccattatat ccaaagacct cagagaaagg 4643
   tgagcgaagg ctcaattcag aagattggaa gctgatcaat aggatcaaga caatggtgag 4703
   aacgcttcca aatctcacta ttccaccaga agatgcatac attatcattg aaacagatgc 4763
   atgtgcaact ggatggggag cagtatgcaa gtggaagaaa aacaaggcag acccaagaaa 4823
   tacagagcaa atctgtaggt atgccagtgg aaaatttgat aagccaaaag gaacctgtga 4883
   tgcagaaatc tatggggtta tgaatggctt agaaaagatg agattgttct acttggacaa 4943
   aagagagatc acagtcagaa ctgacagtag tgcaatcgaa aggttctaca acaagagtgc 5003
   tgaacacaag ccttctgaga tcagatggat caggttcatg gactacatca ctggtgcagg 5063
   accagagata gtcattgaac acataaaagg gaagagcaat ggtttagctg acatcttgtc 5123
   caggctcaaa gccaaattag ctcagaatga accaacggaa gagatgatcc tgcttacaca 5183
   agccataagg gaagtaattc cttatccaga tcatccatac actgagcaac tcagagaatg 5243
   gggaaacaaa attctggatc cattccccac attcaagaag gacatgttcg aaagaacaga 5303
   gcaagctttt atgctaacag aggaaccagt tctactctgt gcatgcagga agcctgcaat 5363
   tcagttagtg tccagaacat ctgccaaccc aggaaggaaa ttcttcaagt gcgcaatgaa 5423
   caaatgccat tgctggtact gggcagatct cattgaagaa cacattcaag acagaattga 5483
   tgaatttctc aagaatcttg aagttctgaa gaccggtggc gtgcaaacaa tggaggagga 5543
   acttatgaag gaagtcacca agctgaagat agaagagcag gagttcgagg aataccaggc 5603
   cacaccaagg gctatgtcgc cagtagccgc agaagatgtg ctagatctcc aagacgtaag 5663
   caatgacgat tgaggaggca ttgacgtcag ggatgaccgc agcggagagt actgggccca 5723
   ttcagtggat gctccactga gttgtattat tgtgtgcttt tcggacaagt gtgctgtcca 5783
   ctttcttttg gcacctgtgc cactttattc cttgtctgcc acgatgcctt tgcttagctt 5843
   gtaagcaagg atcgcagtgc gtgtgtgaca ccacccccct tccgacgctc tgcctatata 5903
   aggcaccgtc tgtaagctct tacgatcatc ggtagttcac cacaagtttg tacaaaaaag 5963
   caggctccga attcgccctt ggaattctta attaactgca gatatcggta ccgcagcgtc 6023
   ggtggttgct tcatggctgt cgaggacggc ggcttcatct acaaacaagc cacggctgct 6083
   gctgcgctac cgccgcggct ttgtagatga agcagccgtc cacgactcac catggaaacg 6143
   gcagcgaggt gcgagcttcc agtgcgcgca gtgtttgatc agcaaacacc tgcgtggccg 6203
   aggctgagcc ttgcaagggc aggcgtccat gttctcgtca gcaagcgtct tcttcacatg 6263
   tctgaggtct gagcaattgc gggtggacat cgatggatga gcattgccgc tgcaagggaa 6323
   gatcgtccgg ctcacacttg agttgggata ccatgcacta ctcttgttgt agttagatta 6383
   gataccatgc gcttgaaccg tgcaatagca tagcgaggaa gaggaaccca gagaggactc 6443
   agaaagagat gttgtctatt tcatccagtg ctcatctagt catctagtct gcctgcgggt 6503
   gtctgcaatg aacgctgacg ctgctgccgg agacctactc tgctactggt gtggagtgga 6563
   caaacaggga gggaaggcgt ctgctagaac ctagaagacg gacacaggga ggaccaacag 6623
   gactaaagac cgtcgtacgt gttgaagcgg aaggactttc ttattacgtg tggcgcttcg 6683
   aattaaagcc agcggttaga acggcccggg ccggccaagc ttgggaaggg cgaattcgac 6743
   ccagctttct tgtacaaagt ggagctcgat cgttcaaaca tttggcaata aagtttctta 6803
   agattgaatc ctgttgccgg tcttgcgatg attatcatat aatttctgtt gaattacgtt 6863
   aagcatgtaa taattaacat gtaatgcatg acgttattta tgagatgggt ttttatgatt 6923
   agagtcccgc aattatacat ttaatacgcg atagaaaaca aaatatagcg cgcaaactag 6983
   gataaattat cgcgcgcggt gtcatctatg ttactagatc ggccggccaa ctttattata 7043
   catagttgat aattcactgg gccggccaga tcttgattgt cgtttcccgc cttcagttta 7103
   aactatcagt gtttgacagg atatattggc gggtaaacct aagagaaaag agcgtttatt 7163
   agaataatcg gatatttaaa agggcgtgaa aaggtttatc cgttcgtcca tttgtatgtg 7223
   catgccaacc acagggttcc cctcgggagt gcttggcatt ccgtgcgata atgacttctg 7283
   ttcaaccacc caaacgtcgg aaagcctgac gacggagcag cattccaaaa agatcccttg 7343
   gctcgtctgg gtcggctaga aggtcgagtg ggctgctgtg gcttgatccc tcaacgcggt 7403
   cgcggacgta gcgcagcgcc gaaaaatcct cgatcgcaaa tccgacgctg tcgaaaagcg 7463
   tgatctgctt gtcgctcttt cggccgacgt cctggccagt catcacgcgc caaagttccg 7523
   tcacaggatg atctggcgcg agttgctgga tctcgccttc aatccgggtc tgtggcggga 7583
   actccacgaa aatatccgaa cgcagcaaga tcgtcgacca attcttgaag acgaaagggc 7643
   ctcgtgatac gcctattttt ataggttaat gtcatgataa taatggtttc ttagacgtca 7703
   ggtggcactt ttcggggaaa tgtgcgcgga acccctattt gtttattttt ctaaatacat 7763
   tcaaatatgt atccgctcat gagacaataa ccctgataaa tgcttcaata atattgaaaa 7823
   aggaagagta tgagtattca acatttccgt gtcgccctta ttcccttttt tgcggcattt 7883
   tgccttcctg tttttgctca cccagaaacg ctggtgaaag taaaagatgc tgaagatcag 7943
   ttgggtgcac gagtgggtta catcgaactg gatctcaaca gcggtaagat ccttgagagt 8003
   tttcgccccg aagaacgttt tccaatgatg agcactttta aagttctgct atgtggcgcg 8063
   gtattatccc gtgttgacgc cgggcaagag caactcggtc gccgcataca ctattctcag 8123
   aatgacttgg ttgagtactc accagtcaca gaaaagcatc ttacggatgg catgacagta 8183
   agagaattat gcagtgctgc cataaccatg agtgataaca ctgcggccaa cttacttctg 8243
   acaacgatcg gaggaccgaa ggagctaacc gcttttttgc acaacatggg ggatcatgta 8303
   actcgccttg atcgttggga accggagctg aatgaagcca taccaaacga cgagcgtgac 8363
   accacgatgc cggggggggg ggggggggac atgaggttgc cccgtattca gtgtcgctga 8423
   tttgtattgt ctgaagttgt ttttacgtta agttgatgca gatcaattaa tacgatacct 8483
   gcgtcataat tgattatttg acgtggtttg atggcctcca cgcacgttgt gatatgtaga 8543
   tgataatcat tatcacttta cgggtccttt ccggtgatcc gacaggttac ggggcggcga 8603
   cctcgcgggt tttcgctatt tatgaaaatt ttccggttta aggcgtttcc gttcttcttc 8663
   gtcataactt aatgttttta tttaaaatac cctctgaaaa gaaaggaaac gacaggtgct 8723
   gaaagcgagc tttttggcct ctgtcgtttc ctttctctgt ttttgtccgt ggaatgaaca 8783
   atggaacccc cccccccccc ccctgcagca atggcaacaa cgttgcgcaa actattaact 8843
   ggcgaactac ttactctagc ttcccggcaa caattaatag actggatgga ggcggataaa 8903
   gttgcaggac cacttctgcg ctcggccctt ccggctggct ggtttattgc tgataaatct 8963
   ggagccggtg agcgtgggtc tcgcggtatc attgcagcac tggggccaga tggtaagccc 9023
   tcccgtatcg tagttatcta cacgacgggg agtcaggcaa ctatggatga acgaaataga 9083
   cagatcgctg agataggtgc ctcactgatt aagcattggt aactgtcaga ccaagtttac 9143
   tcatatatac tttagattga tttaaaactt catttttaat ttaaaaggat ctaggtgaag 9203
   atcctttttg ataatctcat gaccaaaatc ccttaacgtg agttttcgtt ccactgagcg 9263
   tcagaccccg tagaaaagat caaaggatct tcttgagatc ctttttttct gcgcgtaatc 9323
   tgctgcttgc aaacaaaaaa accaccgcta ccagcggtgg tttgtttgcc ggatcaagag 9383
   ctaccaactc tttttccgaa ggtaactggc ttcagcagag cgcagatacc aaatactgtc 9443
   cttctagtgt agccgtagtt aggccaccac ttcaagaact ctgtagcacc gcctacatac 9503
   ctcgctctgc taatcctgtt accagtggct gctgccagtg gcgataagtc gtgtcttacc 9563
   gggttggact caagacgata gttaccggat aaggcgcagc ggtcgggctg aacggggggt 9623
   tcgtgcacac agcccagctt ggagcgaacg acctacaccg aactgagata cctacagcgt 9683
   gagctatgag aaagcgccac gcttcccgaa gggagaaagg cggacaggta tccggtaagc 9743
   ggcagggtcg gaacaggaga gcgcacgagg gagcttccag ggggaaacgc ctggtatctt 9803
   tatagtcctg tcgggtttcg ccacctctga cttgagcgtc gatttttgtg atgctcgtca 9863
   ggggggcgga gcctatggaa aaacgccagc aacgcggcct ttttacggtt cctggccttt 9923
   tgctggcctt ttgctcacat gttctttcct gcgttatccc ctgattctgt ggataaccgt 9983
   attaccgcct ttgagtgagc tgataccgct cgccgcagcc gaacgaccga gcgcagcgag 10043
   tcagtgagcg aggaagcgga agagcgcctg atgcggtatt ttctccttac gcatctgtgc 10103
   ggtatttcac accgcatatg gtgcactctc agtacaatct gctctgatgc cgcatagtta 10163
   agccagtata cactccgcta tcgctacgtg actgggtcat ggctgcgccc cgacacccgc 10223
   caacacccgc tgacgcgccc tgacgggctt gtctgctccc ggcatccgct tacagacaag 10283
   ctgtgaccgt ctccgggagc tgcatgtgtc agaggttttc accgtcatca ccgaaacgcg 10343
   cgaggcagca gatcccccga tcaagtagat acactacata tatctacaat agacatcgag 10403
   ccggaaggtg atgtttactt tcctgaaatc cccagcaatt ttaggccagt ttttacccaa 10463
   gacttcgcct ctaacataaa ttatagttac caaatctggc aaaagggttg accggggggg 10523
   gggggaaagc cacgttgtgt ctcaaaatct ctgatgttac attgcacaag ataaaaatat 10583
   atcatcatga acaataaaac tgtctgctta cataaacagt aatacaaggg gtgtt atg 10641
   Met
   agc cat att caa cgg gaa acg tct tgc tcg agg ccg cga tta aat tcc 10689
   Ser His Ile Gln Arg Glu Thr Ser Cys Ser Arg Pro Arg Leu Asn Ser
   445 450 455
   aac atg gat gct gat tta tat ggg tat aaa tgg gct cgc gat aat gtc 10737
   Asn Met Asp Ala Asp Leu Tyr Gly Tyr Lys Trp Ala Arg Asp Asn Val
   460                 465                 470                 475
   ggg caa tca ggt gcg aca atc tat cga ttg tat ggg aag ccc gat gcg     10785
   Gly Gln Ser Gly Ala Thr Ile Tyr Arg Leu Tyr Gly Lys Pro Asp Ala
                   480                 485                 490
   cca gag ttg ttt ctg aaa cat ggc aaa ggt agc gtt gcc aat gat gtt     10833
   Pro Glu Leu Phe Leu Lys His Gly Lys Gly Ser Val Ala Asn Asp Val
               495                 500                 505
   aca gat gag atg gtc aga cta aac tgg ctg acg gaa ttt atg cct ctt     10881
   Thr Asp Glu Met Val Arg Leu Asn Trp Leu Thr Glu Phe Met Pro Leu
           510                 515                 520
   ccg acc atc aag cat ttt atc cgt act cct gat gat gca tgg tta ctc     10929
   Pro Thr Ile Lys His Phe Ile Arg Thr Pro Asp Asp Ala Trp Leu Leu
       525                 530                 535
   acc act gcg atc ccc ggg aaa aca gca ttc cag gta tta gaa gaa tat     10977
   Thr Thr Ala Ile Pro Gly Lys Thr Ala Phe Gln Val Leu Glu Glu Tyr
   540                 545                 550                 555
   cct gat tca ggt gaa aat att gtt gat gcg ctg gca gtg ttc ctg cgc     11025
   Pro Asp Ser Gly Glu Asn Ile Val Asp Ala Leu Ala Val Phe Leu Arg
                   560                 565                 570
   cgg ttg cat tcg att cct gtt tgt aat tgt cct ttt aac agc gac cgc     11073
   Arg Leu His Ser Ile Pro Val Cys Asn Cys Pro Phe Asn Ser Asp Arg
               575                 580                 585
   gta ttt cgt ctc gct cag gcg caa tca cga atg aat aac ggt ttg gtt     11121
   Val Phe Arg Leu Ala Gln Ala Gln Ser Arg Met Asn Asn Gly Leu Val
           590                 595                 600
   gat gcg agt gat ttt gat gac gag cgt aat ggc tgg cct gtt gaa caa     11169
   Asp Ala Ser Asp Phe Asp Asp Glu Arg Asn Gly Trp Pro Val Glu Gln
       605                 610                 615
   gtc tgg aaa gaa atg cat aag ctt ttg cca ttc tca ccg gat tca gtc     11217
   Val Trp Lys Glu Met His Lys Leu Leu Pro Phe Ser Pro Asp Ser Val
   620                 625                 630                 635
   gtc act cat ggt gat ttc tca ctt gat aac ctt att ttt gac gag ggg     11265
   Val Thr His Gly Asp Phe Ser Leu Asp Asn Leu Ile Phe Asp Glu Gly
                   640                 645                 650
   aaa tta ata ggt tgt att gat gtt gga cga gtc gga atc gca gac cga     11313
   Lys Leu Ile Gly Cys Ile Asp Val Gly Arg Val Gly Ile Ala Asp Arg
               655                 660                 665
   tac cag gat ctt gcc atc cta tgg aac tgc ctc ggt gag ttt tct cct     11361
   Tyr Gln Asp Leu Ala Ile Leu Trp Asn Cys Leu Gly Glu Phe Ser Pro
           670                 675                 680
   tca tta cag aaa cgg ctt ttt caa aaa tat ggt att gat aat cct gat     11409
   Ser Leu Gln Lys Arg Leu Phe Gln Lys Tyr Gly Ile Asp Asn Pro Asp
       685                 690                 695
   atg aat aaa ttg cag ttt cat ttg atg ctc gat gag ttt ttc taa         11454
   Met Asn Lys Leu Gln Phe His Leu Met Leu Asp Glu Phe Phe
   700                 705                 710
   tcagaattgg ttaattggtt gtaacactgg cagagcatta cgctgacttg acgggacggc 11514
   ggctttgttg aataaatcga acttttgctg agttgaagga tcagatcacg catcttcccg 11574
   acaacgcaga ccgttccgtg gcaaagcaaa agttcaaaat caccaactgg tccacctaca 11634
   acaaagctct catcaaccgt ggctccctca ctttctggct ggatgatggg gcgattcagg 11694
   gatcacaggc agcaacgctc tgtcatcg 11722

<210> 45
<211> 442
<212> PRT
<213> Artificial

<220>
<223> Synthetic Construct

<400> 45

Met Glu Asn Ala Lys Met Asn Ser Leu Ile Ala Gln Tyr Pro Leu Val
1               5                   10                  15

Lys Asp Leu Val Ala Leu Lys Glu Thr Thr Trp Phe Asn Pro Gly Thr
            20                  25                  30

Thr Ser Leu Ala Glu Gly Leu Pro Tyr Val Gly Leu Thr Glu Gln Asp
        35                  40                  45

Val Gln Asp Ala His Ala Arg Leu Ser Arg Phe Ala Pro Tyr Leu Ala
    50                  55                  60

Lys Ala Phe Pro Glu Thr Ala Ala Thr Gly Gly Ile Ile Glu Ser Glu
65                  70                  75                  80

Leu Val Ala Ile Pro Ala Met Gln Lys Arg Leu Glu Lys Glu Tyr Gln
                85                  90                  95

Gln Pro Ile Ser Gly Gln Leu Leu Leu Lys Lys Asp Ser His Leu Pro
            100                 105                 110

Ile Ser Gly Ser Ile Lys Ala Arg Gly Gly Ile Tyr Glu Val Leu Ala
        115                 120                 125

His Ala Glu Lys Leu Ala Leu Glu Ala Gly Leu Leu Thr Leu Asp Asp
    130                 135                 140

Asp Tyr Ser Lys Leu Leu Ser Pro Glu Phe Lys Gln Phe Phe Ser Gln
145                 150                 155                 160

Tyr Ser Ile Ala Val Gly Ser Thr Gly Asn Leu Gly Leu Ser Ile Gly

                165                 170                 175

Ile Met Ser Ala Arg Ile Gly Phe Lys Val Thr Val His Met Ser Ala
            180                 185                 190

Asp Ala Arg Ala Trp Lys Lys Ala Lys Leu Arg Ser His Gly Val Thr
        195                 200                 205

Val Val Glu Tyr Glu Gln Asp Tyr Gly Val Ala Val Glu Glu Gly Arg
    210                 215                 220

Lys Ala Ala Gln Ser Asp Pro Asn Cys Phe Phe Ile Asp Asp Glu Asn
225                 230                 235                 240

Ser Arg Thr Leu Phe Leu Gly Tyr Ser Val Ala Gly Gln Arg Leu Lys
                245                 250                 255

Ala Gln Phe Ala Gln Gln Gly Arg Ile Val Asp Ala Asp Asn Pro Leu
            260                 265                 270

Phe Val Tyr Leu Pro Cys Gly Val Gly Gly Gly Pro Gly Gly Val Ala
        275                 280                 285

Phe Gly Leu Lys Leu Ala Phe Gly Asp His Val His Cys Phe Phe Ala
    290                 295                 300

Glu Pro Thr His Ser Pro Cys Met Leu Leu Gly Val His Thr Gly Leu
305                 310                 315                 320

His Asp Gln Ile Ser Val Gln Asp Ile Gly Ile Asp Asn Leu Thr Ala
                325                 330                 335

Ala Asp Gly Leu Ala Val Gly Arg Ala Ser Gly Phe Val Gly Arg Ala
            340                 345                 350

Met Glu Arg Leu Leu Asp Gly Phe Tyr Thr Leu Ser Asp Gln Thr Met
        355                 360                 365

Tyr Asp Met Leu Gly Trp Leu Ala Gln Glu Glu Gly Ile Arg Leu Glu
    370                 375                 380

Pro Ser Ala Leu Ala Gly Met Ala Gly Pro Gln Arg Val Cys Ala Ser
385                 390                 395                 400

Val Ser Tyr Gln Gln Met His Gly Phe Ser Ala Glu Gln Leu Arg Asn
                405                 410                 415

Thr Thr His Leu Val Trp Ala Thr Gly Gly Gly Met Val Pro Glu Glu
            420                 425                 430

Glu Met Asn Gln Tyr Leu Ala Lys Gly Arg
        435                 440

<210> 46
<211> 271
<212> PRT
<213> Artificial

<220>
<223> Synthetic Construct

<400> 46

Met Ser His Ile Gln Arg Glu Thr Ser Cys Ser Arg Pro Arg Leu Asn
1               5                   10                  15

Ser Asn Met Asp Ala Asp Leu Tyr Gly Tyr Lys Trp Ala Arg Asp Asn
            20                  25                  30

Val Gly Gln Ser Gly Ala Thr Ile Tyr Arg Leu Tyr Gly Lys Pro Asp
        35                  40                  45

Ala Pro Glu Leu Phe Leu Lys His Gly Lys Gly Ser Val Ala Asn Asp
    50                  55                  60

Val Thr Asp Glu Met Val Arg Leu Asn Trp Leu Thr Glu Phe Met Pro
65                  70                  75                  80

Leu Pro Thr Ile Lys His Phe Ile Arg Thr Pro Asp Asp Ala Trp Leu
                85                  90                  95

Leu Thr Thr Ala Ile Pro Gly Lys Thr Ala Phe Gln Val Leu Glu Glu
            100                 105                 110

Tyr Pro Asp Ser Gly Glu Asn Ile Val Asp Ala Leu Ala Val Phe Leu
        115                 120                 125

Arg Arg Leu His Ser Ile Pro Val Cys Asn Cys Pro Phe Asn Ser Asp
    130                 135                 140

Arg Val Phe Arg Leu Ala Gln Ala Gln Ser Arg Met Asn Asn Gly Leu
145                 150                 155                 160

Val Asp Ala Ser Asp Phe Asp Asp Glu Arg Asn Gly Trp Pro Val Glu
                165                 170                 175

Gln Val Trp Lys Glu Met His Lys Leu Leu Pro Phe Ser Pro Asp Ser
        180                 185                 190

Val Val Thr His Gly Asp Phe Ser Leu Asp Asn Leu Ile Phe Asp Glu
    195                 200                 205

Gly Lys Leu Ile Gly Cys Ile Asp Val Gly Arg Val Gly Ile Ala Asp
    210                 215                 220

Arg Tyr Gln Asp Leu Ala Ile Leu Trp Asn Cys Leu Gly Glu Phe Ser
225                 230                 235                 240

Pro Ser Leu Gln Lys Arg Leu Phe Gln Lys Tyr Gly Ile Asp Asn Pro
                245                 250                 255

Asp Met Asn Lys Leu Gln Phe His Leu Met Leu Asp Glu Phe Phe
            260                 265                 270

<210>  47
<211>  693
<212>  DNA
<213>  Zea mays

<220>
<221>  precursor_RNA
<222>  (1)..(693)

<400>  47
gcagcgtcgg tggttgcttc atggctgtcg aggggaatga cgtccggtcc gaacaagcca      60

cggctgctgc tgcgctaccg ccgcggcttc ggaccaggct tcattcccca cgactcacca     120

tggaaacggc agcgaggtgc gagcttccag tgcgcgcagt gtttgatcag caaacacctg     180

cgtggccgag gctgagcctt gcaagggcag gcgtccatgt tctcgtcagc aagcgtcttc     240

ttcacatgtc tgaggtctga gcaattgcgg gtggacatcg atggatgagc attgccgctg     300

caagggaaga tcgtccggct cacacttgag ttgggatacc atgcactact cttgttgtag     360

ttagattaga taccatgcgc ttgaaccgtg caatagcata gcgaggaaga ggaacccaga     420

gaggactcag aaagagatgt tgtctatttc atccagtgct catctagtca tctagtctgc     480

ctgcgggtgt ctgcaatgaa cgctgacgct gctgccggag acctactctg ctactggtgt     540

ggagtggaca aacagggagg gaaggcgtct gctagaacct agaagacgga cacagggagg     600

accaacagga ctaaagaccg tcgtacgtgt tgaagcggaa ggactttctt attacgtgtg     660

gcgcttcgaa ttaaagccag cggttagaac ggc                                  693